(19) Europäisches Patentamt
European Patent Office
Office européen des brevets

(11) EP 1 940 811 B1

(12) **EUROPÄISCHE PATENTSCHRIFT**

(45) Veröffentlichungstag und Bekanntmachung des Hinweises auf die Patenterteilung:
**26.02.2014 Patentblatt 2014/09**

(21) Anmeldenummer: **06806107.6**

(22) Anmeldetag: **09.10.2006**

(51) Int Cl.:
***C07D 271/10*** (2006.01) ***C07D 271/06*** (2006.01)
***C07D 249/12*** (2006.01) ***C07D 249/14*** (2006.01)
***A61K 31/4196*** (2006.01) ***A61K 31/4245*** (2006.01)
***A61P 9/00*** (2006.01)

(86) Internationale Anmeldenummer:
**PCT/EP2006/009722**

(87) Internationale Veröffentlichungsnummer:
**WO 2007/045366 (26.04.2007 Gazette 2007/17)**

(54) **HETEROCYCLISCHE VERBINDUNGEN MIT CARBOXYL-ISOSTEREN GRUPPEN UND IHRE VERWENDUNG ZUR BEHANDLUNG VON HERZ-KREISLAUF-ERKRANKUNGEN**

HETEROCYCLIC COMPOUNDS WITH CARBOXYL ISOSTERE GROUPS AND THEIR USE FOR THE TREATMENT OF CARDIOVASCULAR DISEASES

COMPOSES HETEROCYCLIQUES A GROUPES CARBOXYLE-ISOSTERE ET LEUR UTILISATION POUR TRAITER DES MALADIES DU COEUR ET DE LA CIRCULATION SANGUINE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **21.10.2005 DE 102005050377**

(43) Veröffentlichungstag der Anmeldung:
**09.07.2008 Patentblatt 2008/28**

(73) Patentinhaber: **Bayer Intellectual Property GmbH**
**40789 Monheim (DE)**

(72) Erfinder:
- **BARTEL, Stephan**
**51515 Kürten (DE)**
- **HAHN, Michael**
**40764 Langenfeld (DE)**
- **MORADI, Wahed, Ahmed**
**40789 Monheim (DE)**
- **MÜNTER, Klaus**
**42489 Wülfrath (DE)**
- **RÖLLE, Thomas**
**51381 Leverkusen (DE)**
- **STASCH, Johannes-Peter**
**42651 Solingen (DE)**
- **SCHLEMMER, Karl-Heinz**
**42113 Wuppertal (DE)**
- **WUNDER, Frank**
**42117 Wuppertal (DE)**

(74) Vertreter: **BIP Patents**
**c/o Bayer Intellectual Property GmbH**
**Creative Campus Monheim**
**Alfred-Nobel-Straße 10**
**40789 Monheim (DE)**

(56) Entgegenhaltungen:
**DE-A1- 19 943 634 DE-A1- 19 943 635**
**DE-A1- 19 943 636 DE-A1- 19 943 639**

- **WERMUTH ET AL: "The Practise of Medicinal Chemistry" PRACTICE OF MEDICINAL CHEMISTRY, 1996, Seiten 203-237, XP002190259**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Anmeldung betrifft neue heterocyclische Verbindungen, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prävention kardiovaskulärer Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, am Eisen-Zentralatom des Häms anzugreifen, wobei die Stimulierung durch CO deutlich geringer ist als die durch NO.

**[0003]** Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxy-methyl-2'-furyl)-1-benzylindazol [YC-1, Wu et al., Blood 84 (1994), 4226; Mülsch et al., Br. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Br. J. Pharmacol. 114 (1995), 1587], sowie verschiedene substituierte Pyrazolderivate (WO 98/16223, WO 98/16507 und WO 98/23619).

Die vorstehend beschriebenen Stimulatoren der löslichen Guanylatcyclase stimulieren das Enzym entweder direkt über die Häm-Gruppe (Kohlenmonoxid, Stickstoffmonoxid oder Diphenyliodoniumhexafluorophosphat) durch Interaktion mit dem Eisenzentrum der Häm-Gruppe und eine sich daraus ergebende, zur Erhöhung der Enzymaktivität führende Konformationsänderung [Gerzer et al., FEBS Lett. 132 (1981), 71] oder über einen Häm-abhängigen Mechanismus, der unabhängig von NO ist, aber zu einer Potenzierung der stimulierenden Wirkung von NO oder CO führt [z.B. YC-1, Hoenicka et al., J. Mol. Med. 77 (1999) 14; oder die in WO 98/16223, WO 98/16507 und WO 98/23619 beschriebenen Pyrazolderivate].

Die in der Literatur behauptete stimulierende Wirkung von Isoliquiritigenin und von Fettsäuren, wie z.B. von Arachidonsäure, Prostaglandin-Endoperoxiden und Fettsäure-Hydroperoxiden auf die lösliche Guanylatcyclase konnte nicht bestätigt werden [vgl. z.B. Hoenicka et al., J. Mol. Med. 77 (1999), 14].

Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten bekannten Stimulatoren stimulierbar.

Es wurde eine Stimulation von Häm-freier löslicher Guanylatcyclase durch Protoporphyrin IX beschieben [Ignarro et al., Adv. Pharmacol. 26 (1994), 35]. Allerdings kann Protoporphyrin IX als Mimik für das NO-Häm-Addukt angesehen werden, weshalb die Zugabe von Protoporphyrin IX zur löslichen Guanylatcyclase zur Bildung einer der durch NO stimulierten Häm-haltigen löslichen Guanylatcyclase entsprechenden Struktur des Enzyms führen dürfte. Dies wird auch durch die Tatsache belegt, dass die stimulierende Wirkung von Protoporphyrin IX durch den vorstehend beschriebenen NO-unabhängigen, aber Häm-abhängigen Stimulator YC-1 erhöht wird [Mülsch et al., Naunyn Schmiedebergs Arch. Pharmacol. 355, R47].

Im Gegensatz zu den vorstehend beschriebenen Stimulatoren der löslichen Guanylatcyclase sind die Verbindungen der vorliegenden Erfindung in der Lage, sowohl die Häm-haltige als auch die Häm-freie Form der löslichen Guanylatcyclase zu aktivieren. Die Stimulierung des Enzyms verläuft bei diesen neuen Aktivatoren also über einen Häm-unabhängigen Weg, was auch dadurch belegt wird, dass die neuen Aktivatoren am Häm-haltigen Enzym einerseits keine synergistische Wirkung mit NO zeigen und andererseits sich die Wirkung dieser neuartigen Aktivatoren nicht durch den Häm-abhängigen Inhibitor der löslichen Guanylatcyclase, 1*H*-1,2,4-Oxadiazol-(4,3-a)-chinoxalin-1-on (ODQ), blockieren lässt.

In EP 0 341 551-A1 werden Alkensäure-Derivate als Leukotrien-Antagonisten für die Behandlung von Erkrankungen des Kreislauf- und Atmungssystems offenbar. In WO 01/19355, WO 01/19776, WO 01/19778, WO 01/19780, WO 02/070462 und WO 02/070510 werden Dicarbonsäure- bzw. Aminodicarbonsäure-Derivate als Stimulatoren der löslichen Guanylatcyclase zur Behandlung von Herz-Kreislauf-Erkrankungen beschrieben. Allerdings zeigte es sich, dass diese Verbindungen hinsichtlich ihrer pharmakokinetischen Eigenschaften Nachteile aufweisen, wie insbesondere eine geringe Bioverfügbarkeit und/oder eine nur kurze Wirkdauer nach oraler Gabe.

Aufgabe der vorliegenden Erfindung war daher die Bereitstellung neuer Verbindungen, welche als Aktivatoren der löslichen Guanylatcyclase wirken, jedoch nicht die vorstehend aufgeführten Nachteile der Verbindungen aus dem Stand der Technik aufweisen.

Erfindungsgemäße Verbindungen sind die Verbindungen der Formel (IA) und deren Salze, solvate und Solvate der Salze, die von Formel (IA) umfassten Verbindungen der nachfolgend genannten Formeln und deren Salze, Solvate und Solvate der Salze sowie die von Formel (IA) umfassten, nachfolgend als Ausführungsbeispiele genannten Verbindungen, und deren Salze, Solvate und Solvate der Salze, soweit es sich bei den von Formel (IA) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholln, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:

$(C_1-C_6)$-Alkyl und $(C_1-C_4)$-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien gebannt: Methyl, Ethyl, n-Propyl, Isopropyl, n-Butyl, *iso*-Butyl, *sec.*-Butyl, *tert.*-Butyl, 1-Ethyl-propyl, n-Pentyl und n-Hexyl.

$(C_1-C_7)$-Alkandiyl steht im Rahmen der Erfindung für einen geradkettigen oder verzweigten divalenten Alkylrest mit

1 bis 7 Kohlenstoffatomen. Bevorzugt ist ein geradkettiger Alkandiylrest mit 1 bis 6 Kohlenstoffatomen. Beispielhaft und vorzugsweise seien genannt: Methylen, 1,2-Ethylen, Ethan-1,1-diyl, 1,3-Propylen, Propan-1,1-diyl, Propan-1,2-diyl, Propan-2,2-diyl, 1,4-Butylen, Butan-1,2-diyl, Butan-1,3-diyl, Butan-2,3-diyl, Pentan-1,5-diyl, Penten-2,4-diyl, 3-Methyl-pentan-2,4-diyl und Hexan-1,6-diyl.

($C_2$-$C_7$)-Alkendiyl steht im Rahmen der Erfindung für einen geradkettigen oder verzweigten divalenten Alkenylrest mit 2 bis 7 Kohlenstoffatomen und bis zu 3 Doppelbindungen. Bevorzugt ist ein geradkettiger Alkendiylrest mit 2 bis 6 Kohlenstoffatomen und bis zu 2 Doppelbindungen. Beispielhaft und vorzugsweise seien genannt: Ethen-1,1-diyl, Ethen-1,2-diyl, Propen-1,1-diyl, Propen-1,2-diyl, Propen-1,3-diyl, But-1-en-1,4-diyl, But-1-en-1,3-diyl, But-2-en-1,4-diyl, Buta-1,3-dien-1,4-diyl, Pent-2-en-1,5-diyl, Hex-3-en-1,6-diyl und Hexa-2,4-dien-1,6-diyl.

($C_2$-$C_7$)-Alkindiyl steht im Rahmen der Erfindung für einen geradkettigen oder verzweigten divalenten Alkinylrest mit 2 bis 7 Kohlenstoffatomen und bis 3 Dreifachbindungen. Bevorzugt ist ein geradkettiger Alkindiylrest mit 2 bis 6 Kohlenstoffatomen und bis zu 2 Dreifachbindungen. Beispielhaft und vorzugsweise seien genannt: Ethin-1,2-diyl, Propin-1,3-diyl, But-1-in-1,4-diyl, But-1-in-1,3-diyl, But-2-in-1,4-diyl, Pent-2-in-1,5-diyl, Pent-2-in-1,4-diyl und Hex-3-in-1,6-diyl.

($C_1$-$C_6$)-Alkoxy und ($C_1$-$C_4$)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy, n-Butoxy, *tert.*-Butoxy, n-Pentoxy und n-Hexoxy.

Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Gegenstand der vorliegenden Erfindung sind Verbindungen der Formel (I-A)

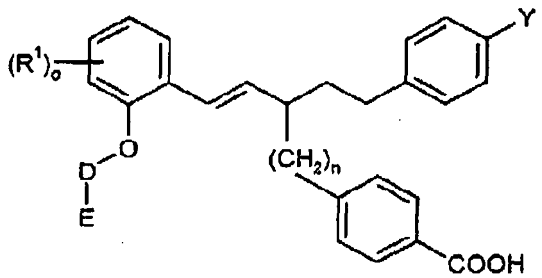

(I-A),

in welcher

D für ($C_1$-$C_7$)-Alkandiyl, welches ein- oder mehrfach mit Fluor substituiert sein kann, steht,

E für Wasserstoff, Trifluormethyl oder für eine Gruppe der Formel

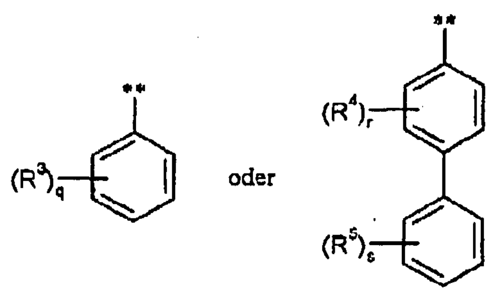

steht, worin ** die Verknüpfungsstelle mit der Gruppe D bedeutet,

Y für eine Gruppe der Formel

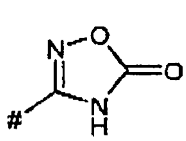, 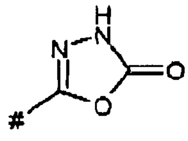 oder 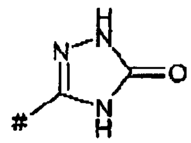 ,

worin # die Verknüpfungsstelle mit dem Phenylring bedeutct, steht,

n für die Zahl 1 oder 2 steht,

$R^1$, $R^3$, $R^4$ und $R^5$ unabhängig voneinander für einen Substituenten ausgewählt aus der Reihe Fluor, Chlor, Brom, Methyl, *tert.*-Butyl, Trifluormethyl, Methoxy und Trifluormethoxy stehen,

und

o, q, r und s unabhängig voneinander jeweils für die Zahl 0, 1 oder 2 stehen, wobei für den Fall, dass $R^1$, $R^3$, $R^4$ oder $R^5$ mehrfach auftreten, ihre Bedeutungen jeweils gleich oder verschieden sein können,

sowie ihre Salze, Solvate und Solvate der Salze.

Besonders bevorzugt im Rahmen der vorliegenden Erfindung sind auch Verbindungen der Formel (I-B)

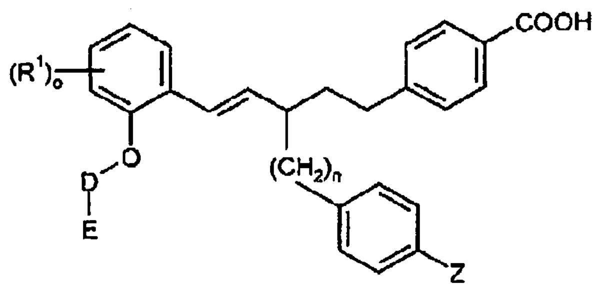

(I-B),

in welcher

D für ($C_1$-$C_7$)-Alkandiyl, welches ein- oder mehrfach mit Fluor substituiert sein kann, steht,

E für Wasserstoff, Trifluormethyl oder für eine Gruppe der Formel

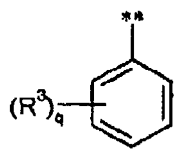 oder 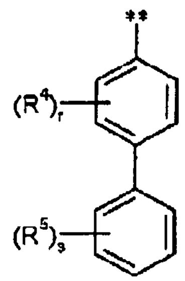

steht, worin ** die Verknüpfungsstelle mit der Gruppe D bedeutet,

Z für eine Gruppe der Formel

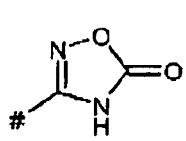, 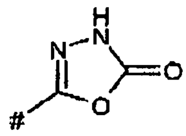 oder 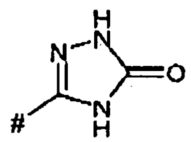 ,

worin # die Verknüpfungsstelle mit dem Phenylring bedeutet, steht,

n für die Zahl 1 oder 2 steht,

$R^1$, $R^3$, $R^4$ und $R^5$ unabhängig voneinander für einen Substituenten ausgewählt aus der Reihe Fluor, Chlor, Brom, Methyl, *tert.*-Butyl, Trifluormethyl, Methoxy und Trifluormethoxy stehen,

und

o, q, r und s unabhängig voneinander jeweils für die Zahl 0, 1 oder 2 stehen,
wobei für den Fall, dass $R^1$, $R^3$, $R^4$ oder $R^5$ mehrfach auftreten, ihre Bedeutungen jeweils gleich oder verschieden sein können,
sowie ihre Salze, Solvate und Solvate der Salze.

Insbesondere bevorzugt im Rahmen der vorliegenden Erfindung sind Verbindungen der Formeln (I-A) und (I-3), in welchen

D und E zusammen für n-Butyl, n-Pentyl, n-Hexyl, 5,5,5-Trifluorpentan-1-yl, 6,6,6-Trifluorhexan-1-yl oder für eine Gruppe der Formel

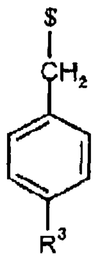

oder

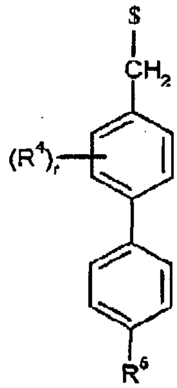

stehen, worin S die Verknüpfungsstelle mit dem O-Atom und
$R^3$ und $R^5$ jeweils *tert.*-Butyl, Trifluormethyl, Methoxy oder Trifluomethoxy bedeuten,

n für die Zahl 1 steht,
$R^1$ für Fluor steht,
$R^4$ für Fluor oder Chlor steht,

und
*o* und r unabhängig voneinander jeweils für die Zahl 0 oder 1 stehen,
sowie ihre Salze, Solvate und Solvate der Salze.
Insbesondere bevorzugt im Rahmen der vorliegenden Erfindung sind auch Verbindungen der Formel (I-C), in welcher

D und E zusammen für n-Butyl, n-Pentyl, n-Hexyl, 5,5,5-Trifluorpentan-1-yl, 6,6,6-Trifluorhexan-1-yl oder für eine Gruppe der Formel

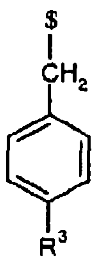

oder

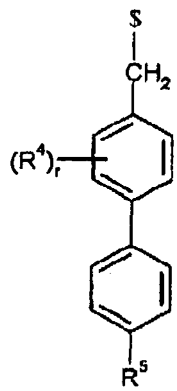

stehen, worin $ die Verkrüpfungsstelle mit dem O-Atom und
$R^3$ und $R^5$ jeweils *tert.*-Butyl, Trifluormethyl, Methoxy oder Trifluormethoxy bedeuten,

n für die Zahl 1 oder 2 steht,
$R^1$ für Fluor steht,
$R^4$ für Fluor oder Chlor steht,

und
o und r unabhängig voneinander jeweils für die Zahl 0 oder 1 stehen,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung sind Verfahren zur Herstellung der erfindungsgemäßen Verbindungen der Formel (I-A) und (I-B), dadurch gekennzeichnet, dass man entweder

[A-1] Verbindungen der Formel (II-1)

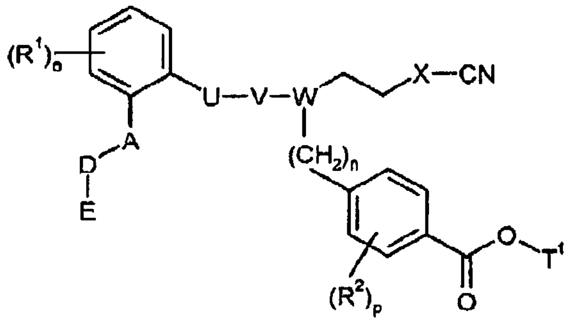

(II-1),

in welcher $R^1$, $R^2$, A, D, E, U, V, W, X, n, o und p jeweils die oben angegebenen Bedeutungen haben und
$T^1$ für $(C_1\text{-}C_4)$-Alkyl steht,
zunächst in einem inerten Lösungsmittel mit Hydroxylamin in Verbindungen der Formel (III-1)

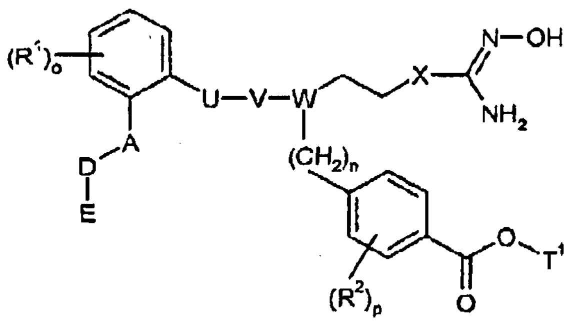

(III-1),

in welcher $R^1$, $R^2$, A, D, E, U, V, W, X, n, o, p und $T^1$ jeweils die oben angegebenen Bedeutungen haben,
überführt und dann in einem inerten Lösungsmittel in Gegenwart einer Base mit einem Chlorameisensäureester der Formel (IV)

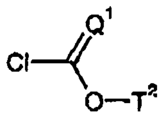

(IV),

in welcher

$Q^1$ für O oder S

und

$T^2$ für $(C_1\text{-}C_{10})$-Alkyl steht,

sowie gegebenenfalls durch nachfolgendes Erhitzen in einem inerten Lösungsmittel zu Verbindungen der Formel (V-1)

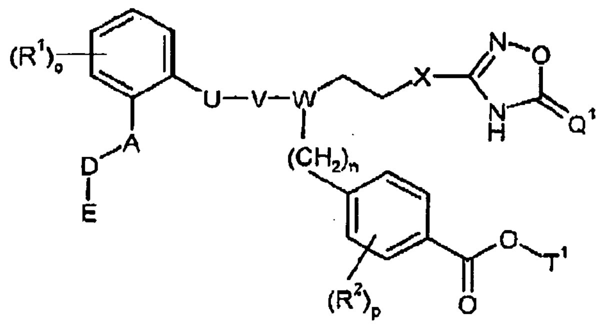

(V-1),

in welcher $R^1$, $R^2$, A, D, E, U, V, W, X, n, o, p, $Q^1$ und $T^1$ jeweils die oben angegebenen Bedeutungen haben, umsetzt
oder

[A-2] Verbindungen der Formel (II-2)

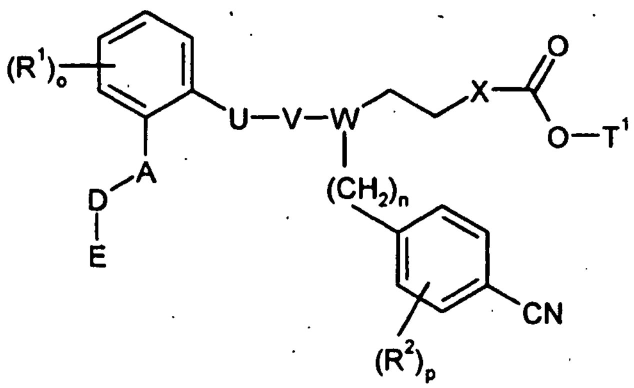

(II-2),

in welcher $R^1$, $R^2$, A, D, E, U, V, W, X, n, o, p und $T^1$ jeweils die oben angegebenen Bedeutungen haben, auf zum Verfahren [A-1] analoge Weise in Verbindungen der Formel (V-2)

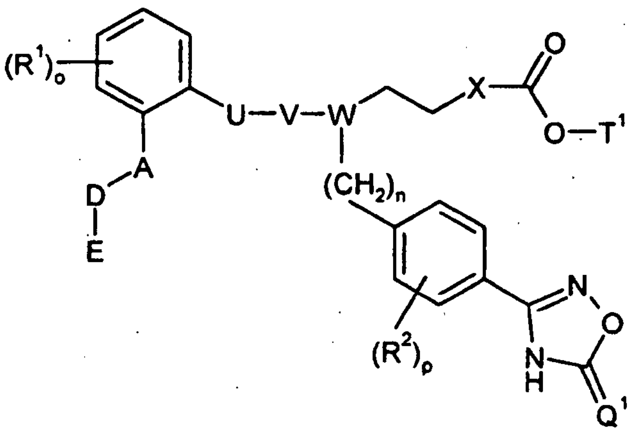

(V-2),

in welcher $R^1$, $R^2$, A, D, E, U, V, W, X, n, o, p, $Q^1$ und $T^1$ jeweils die oben angegebenen Bedeutungen haben, überführt
oder

[B-1] Verbindungen der Formel (VI-1)

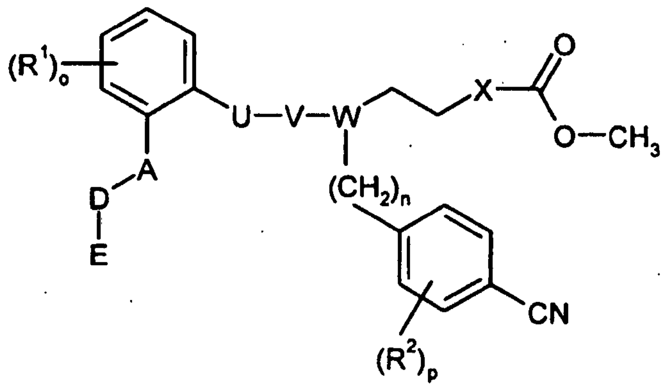

(VI-1),

in welcher $R^1$, $R^2$, A, D, E, U, V, W, X, n, o und p jeweils die oben angegebenen Bedeutungen haben, zunächst in einem inerten Lösungsmittel mit Hydrazin in Verbindungen der Formel (VII-1)

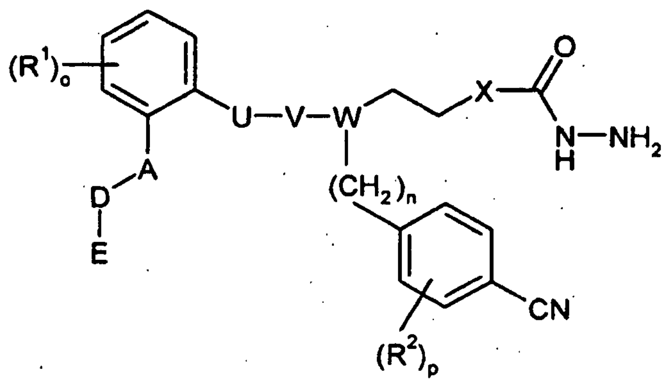

(VII-1),

in welcher $R^1$, $R^2$, A, D, E, U, V, W, X, n, o und p jeweils die oben angegebenen Bedeutungen haben, überführt und dann in einem inerten Lösungsmittel mit Phosgen oder einem Phosgen-Derivat wie beispielsweise Di- oder Triphosgen zu Verbindungen der Formel (VIII-1)

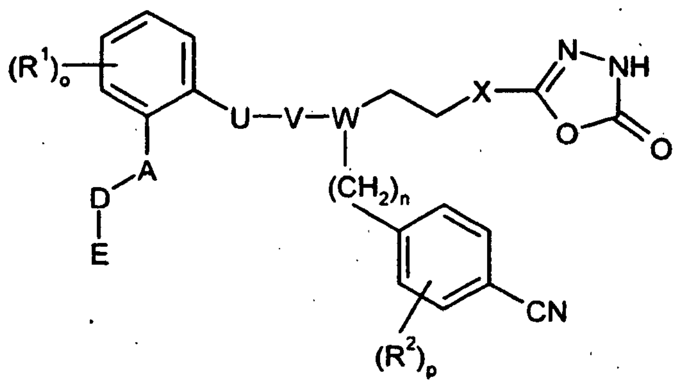

(VIII-1),

in welcher $R^1$, $R^2$, A, D, E, U, V, W, X, n, o und p jeweils die oben angegebenen Bedeutungen haben, umsetzt
oder

[B-2] Verbindungen der Formel (VI-2)

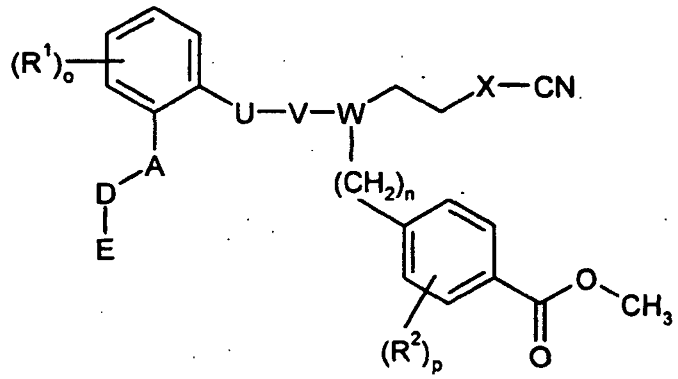

(VI-2),

in welcher $R^1$, $R^2$, A, D, E, U, V, W, X, n, o und p jeweils die oben angegebenen Bedeutungen haben, auf zum Verfahren [B-1] analoge Weise in Verbindungen der Formel (VIII-2)

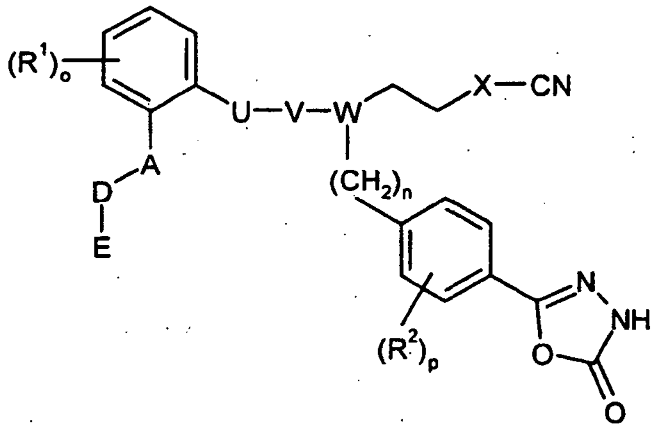

(VIII-2),

in welcher $R^1$, $R^2$, A, D, E, U, V, W, X, n, o und p jeweils die oben angegebenen Bedeutungen haben, überführt
oder

[C-1] Verbindungen der Formel (IX-1)

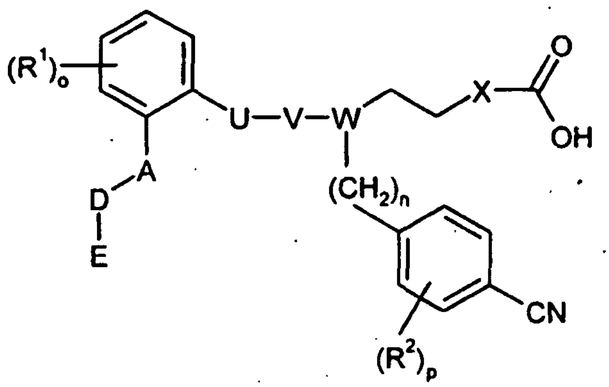

(IX-1),

in welcher $R^1$, $R^2$, A, D, E, U, V, W, X, n, o und p jeweils die oben angegebenen Bedeutungen haben, zunächst in einem inerten Lösungsmittel mit Oxalylchlorid, Thionylchlorid oder Phosphorylchlorid in die entsprechenden Carbonsäurechloride der Formel (X-1)

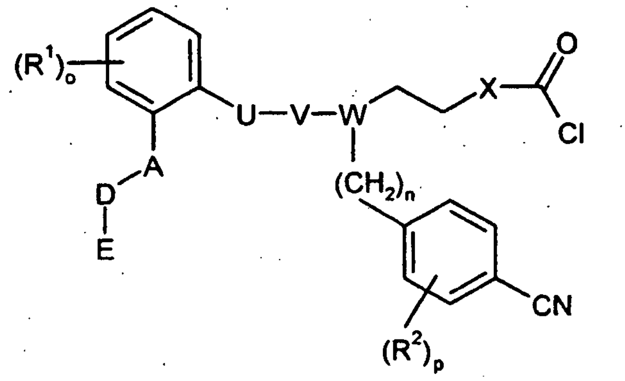

(X-1),

in welcher $R^1$, $R^2$, A, D, E, U, V, W, X, n, o und p jeweils die oben angegebenen Bedeutungen haben, überführt, diese dann in einem inerten Lösungsmittel mit einem Semicarbazid der Formel (XI)

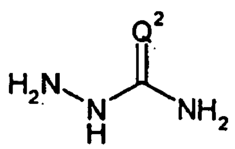

(XI),

in welcher
$Q^2$ für O, S oder NH steht,
zu Verbindungen der Formel (XII-1)

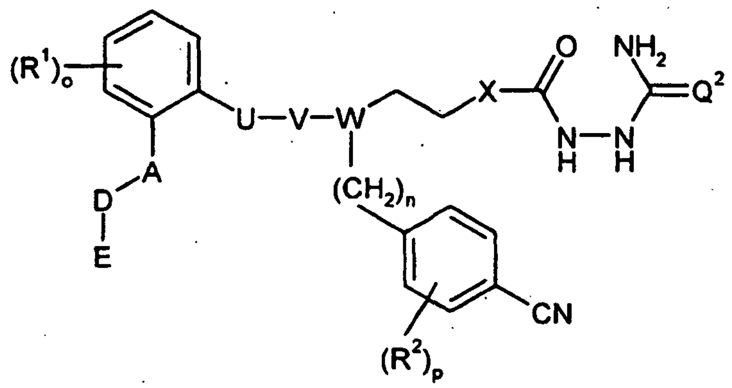

(XII-1),

in welcher $R^1$, $R^2$, A, D, E, U, V, W, X, n, o, p und $Q^2$ jeweils die oben angegebenen Bedeutungen haben, umsetzt und anschließend in Gegenwart einer Base unter gleichzeitiger Hydrolyse der Nitril-Gruppe zu Verbindungen der Formel (XIII-1)

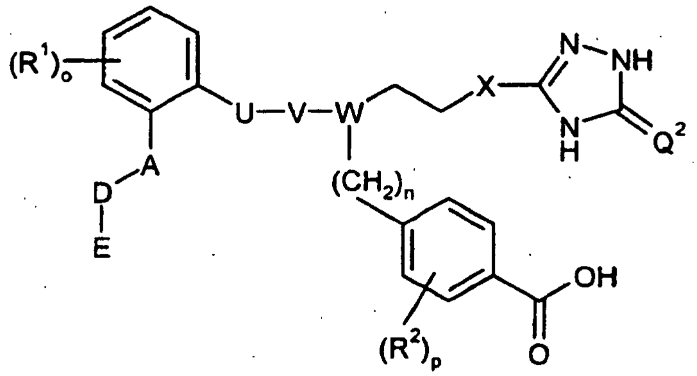

(XIII-1);

in welcher $R^1$, $R^2$, A, D, E, U, V, W, X, n, o, p und $Q^2$ jeweils die oben angegebenen Bedeutungen haben, cyclisiert
oder

[C-2] Verbindungen der Formel (IX-2)

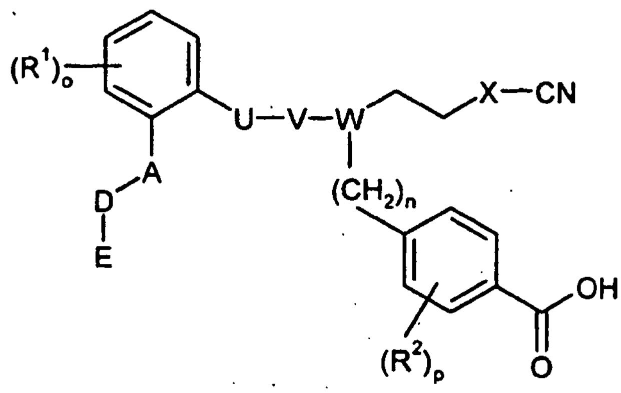

(IX-2),

in welcher $R^1$, $R^2$, A, D, E, U, V, W, X, n, o und p jeweils die oben angegebenen Bedeutungen haben, auf zum Verfahren [C-1] analoge Weise in Verbindungen der Formel (XIII-2)

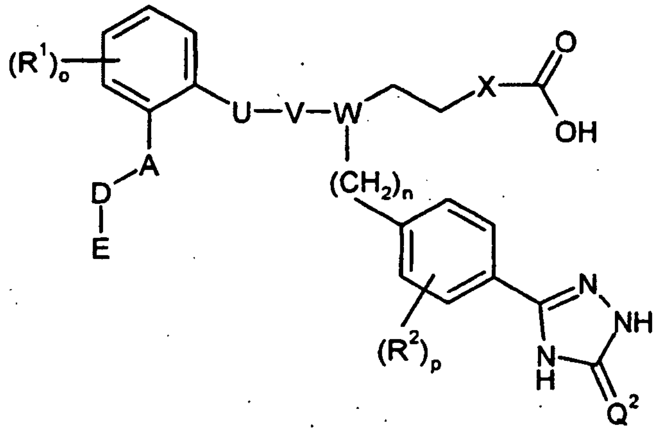

(XIII-2),

in welcher $R^1$, $R^2$, A, D, E, U, V, W, X, n, o, p und $Q^2$ jeweils die oben angegebenen Bedeutungen haben, überführt
und die jeweils resultierenden Verbindungen der Formel (V-1), (V-2), (VIII-1) bzw. (VIII-2) durch Hydrolyse der Ester-Gruppierung-C(O)O$T^1$ bzw. Hydrolyse der Nitril-Gruppe in die entsprechenden Carbonsäuren der Formel (I) überführt

und die Verbindungen der Formel (I), einschließlich der Verbindungen der Formeln (XIII-1) und (XIII-2), gegebenenfalls nach dem Fachmann bekannten Methoden in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

Als inerte Lösungsmittel für den Verfahrensschritt (II-1) → (III-1) eignen sich insbesondere Dimethylsulfoxid oder Dimethylformamid. Die Reaktion wird im Allgemeinen in einem Temperaturbereich von +50°C bis +100°C durchgeführt.

Der Verfahrensschritt (III-1) + (IV) → (V-1) wird in seiner ersten Stufe (Acylierungsschritt) bevorzugt in Dimethylformamid als Lösungsmittel in Gegenwart einer organischen Amin-Base wie beispielsweise Triethylamin, *N,N*-Diisopropylethylamin, *N*-Methylmorpholin, *N*-Methylpiperidin oder Pyridin durchgeführt. Besonders bevorzugt wird Pyridin als Base verwendet.

Als Reagenz (IV) [worin $Q^1$ = O] wird bevorzugt Chlorameisensäure-2-ethylhexylester eingesetzt. Die Umsetzung erfolgt hierbei im Allgemeinen in einem Temperaturbereich von -20°C bis +40°C, bevorzugt bei 0°C bis +20°C.

Ein beim Verfahrensschritt (III-1) + (IV) → (V-1) gegebenenfalls erforderliches Erhitzen zur Vervollständigung des Ringschlusses zum 5-Oxo-[1,2,4]oxadiazol wird bevorzugt als separate Reaktionsstufe in einem inerten Lösungsmittel wie Toluol oder Xylol in einem Temperaturbereich von +100°C bis +150°C durchgeführt.

Inerte Lösungsmittel für den Verfahrensschritt (VI-1) → (VII-1) sind beispielsweise Alkohole wie Methanol, Ethanol, n-Propanol, Isopropanol, n-Butanol oder *tert*.-Butanol, Ether wie Diethylether, Dioxan, Tetrahydrofuran, Glykoldimethylether oder Diethylenglykoldimethylether, oder andere Lösungsmittel wie Dimethylformamid, Dimethylsulfoxid, *N,N'*-Dimethylpropylenharnstoff (DMPU) oder *N*-Methylpyrrolidon (NMP). Ebenso ist es möglich, Gemische der genannten Lösungsmittel einzusetzen. Bevorzugt sind Methanol, Ethanol, Tetrahydrofuran oder Gemische hieraus.

Die Umsetzung (VI-1) → (VII-1) wird im Allgemeinen in einem Temperaturbereich von +20°C bis +80°C durchgeführt.

Inerte Lösungsmittel für den Verfahrensschritt (VII-1) → (VIII-1) sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, Glykoldimethylether oder Diethylenglykoldimethylether, oder Kohlenwasserstoffe wie Benzol, Xylol, Toluol, Hexan, Cyclohexan oder Erdölfraktionen. Ebenso ist es möglich, Gemische der genannten Lösungsmittel einzusetzen. Bevorzugt wird Dioxan verwendet.

Die Umsetzung (VII-1) → (VIII-1) wird im Allgemeinen in einem Temperaturbereich von +50°C bis +150°C, bevorzugt bei +80°C bis +120°C durchgeführt.

Der Verfahrensschritt (IX-1) → (X-1) wird mit üblichen Chlorierungsmitteln wie beispielsweise Oxalylchlorid, Thionylchlorid oder Phosphorylchlorid durchgeführt. Bevorzugt wird Oxalylchlorid, in Gegenwart geringer Mengen von Dimethylformamid, eingesetzt. Die Umsetzung erfolgt im Allgemeinen in inerten Lösungsmitteln wie Tetrahydrofuran oder Toluol in einem Temperaturbereich von 0°C bis +120°C.

Inerte Lösungsmittel für den Verfahrensschritt (X-1) + (XI) → (XII-1) sind beispielsweise Halogenkohlenwasserstoffe wie Dichlormethan, Trichlormethan, Tetrachlormethan, Trichlorethan, Tetrachlorethan oder 1,2-Dichlorethan, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid, Dimethylsulfoxid, *N,N'*-Dimethylpropylenharnstoff (DMPU), *N*-Methytpyrrotidon (NMP), Acetonitril oder auch Wasser. Ebenso ist es möglich, Gemische der genannten Lösungsmittel einzusetzen. Bevorzugt sind Tetrahydrofuran oder Tetrahydrofuran/Wasser-Gemische.

Die Umsetzung (X-1) + (XI) → (XII-1) wird im Allgemeinen in einem Temperaturbereich von -20°C bis +30°C durchgeführt.

Die Cyclisierung im Verfahrensschritt (XII-1) → (XIII-1) wird vorteilhafterweise unter gleichzeitiger Hydrolyse der Nitril-Gruppe durchgeführt. Hierfür eignen sich insbesondere Basen wie Natrium- oder Kaliumhydroxid. Die Umsetzung erfolgt bevorzugt in Wasser oder alkoholischen Lösungsmitteln wie Methanol, Ethanol, n-Propanol, Isopropanol, n-Butanol oder *tert*.-Butanol oder in Gemischen dieser Alkohole mit Wasser. Besonders bevorzugt sind Wasser oder n-Propanol. Die Reaktion wird im Allgemeinen in einem Temperaturbereich von +50°C bis +150°C, bevorzugt bei +90°C bis +110°C durchgeführt.

Die Hydrolyse der Carbonsäureester im Verfahrensschritt (V-1) / (V-2) → (I) bzw. der Nitrile im Verfahrensschritt (VIII-1) / (VIII-2) → (I) erfolgt nach üblichen Methoden, indem man die Ester bzw. Nitrile in inerten Lösungsmitteln mit Säuren oder Basen behandelt, wobei bei letzterem die zunächst entstehenden Salze durch Behandeln mit Säure in die freien Carbonsäuren überführt werden. Im Falle der tert.-Butylester erfolgt die Esterspaltung bevorzugt mit Säuren.

Als inerte Lösungsmittel eignen sich hierfür Wasser oder die für eine Esterspaltung üblichen organischen Lösungsmittel. Hierzu gehören bevorzugt Alkohole wie Methanol, Ethanol, n-Propanol, Isopropanol, n-Butanol oder *tert*.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit

Dioxan, Tetrahydrofuran, Methanol und/oder Ethanol, bei der Nitril-Hydrolyse bevorzugt Wasser oder n-Propanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium-, Kalium- oder Lithiumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der tert.-Butylester und Salzsäure im Falle der Methylester.

Die Esterspaltung erfolgt im Allgemeinen in einem Temperaturbereich von 0°C bis +100°C, bevorzugt bei +20°C bis +60°C. Die Nitril-Hydrolyse wird im Allgemeinen in einem Temperaturbereich von +50°C bis +150°C, bevorzugt bei +90°C bis +110°C durchgeführt.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Die Verbindungen der Formeln (II-1), (II-2), (VI-1), (VI-2), (IX-1) und (IX-2) können nach den in EP 0 341 551-A1, WO 01/19355, WO 01/19776, WO 01/19778, WO 01/19780, WO 02/070462 sowie WO 02/070510 beschriebenen Verfahren hergestellt werden (vgl. auch die nachfolgenden Reaktionsschemata 1-18); der diesbezügliche Inhalt dieser Druckschriften wird hiermit ausdrücklich als Bestandteil der Offenbarung einbezogen.

Die Verbindungen der Formeln (IV) und (XI) sind kommerziell erhältlich, literaturbekannt oder können auf einfache Weise nach literaturbekannten Verfahren hergestellt werden.

Eine Trennung der erfindungsgemäßen Verbindungen in die entsprechenden Enantiomere und/oder Diastereomere kann gegebenenfalls, je nach Zweckmäßigkeit, auch bereits auf der Stufe der Verbindungen (II-1), (II-2), (V-1), (V-2), (VI-1), (VI-2), (VIII-1), (VIII-2), (IX-1), (IX-2) oder der in den Schemata 11-18 dargestellten phenolischen Vorstufen hierzu erfolgen, welche dann in separierter Form entsprechend den beschriebenen Verfahrenssequenzen weiter umgesetzt werden. Eine solche Auftrennung der Stereoisomeren läßt sich nach üblichen, dem Fachmann bekannten Methoden durchführen; vorzugsweise werden chromatographische Verfahren oder eine Trennung über diastereomere Salze verwendet.

Die Herstellung der erfindungsgemäßen Verbindungen kann durch die folgenden Syntheseschemata veranschaulicht werden:

Schema 1

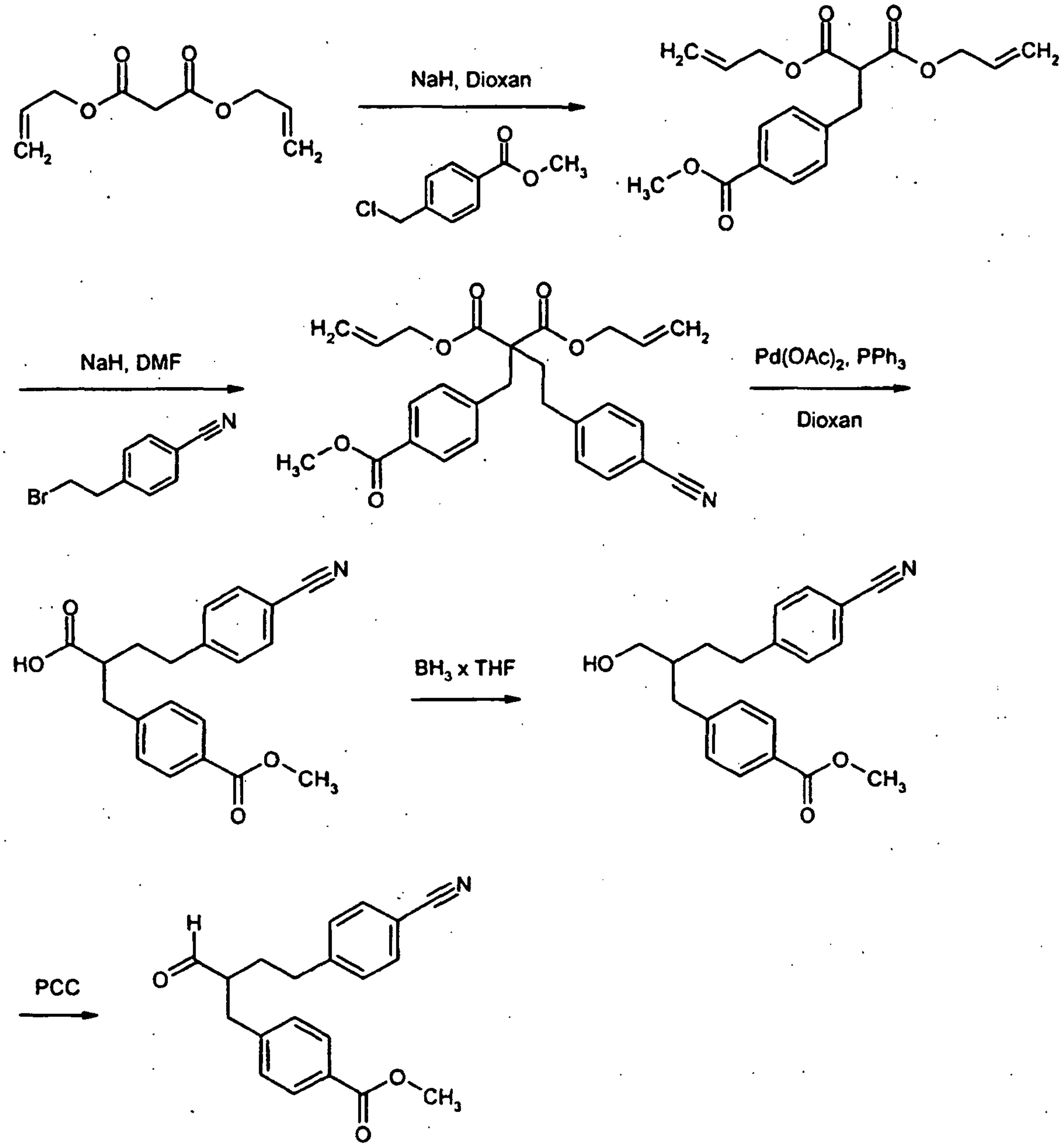

Schema 2

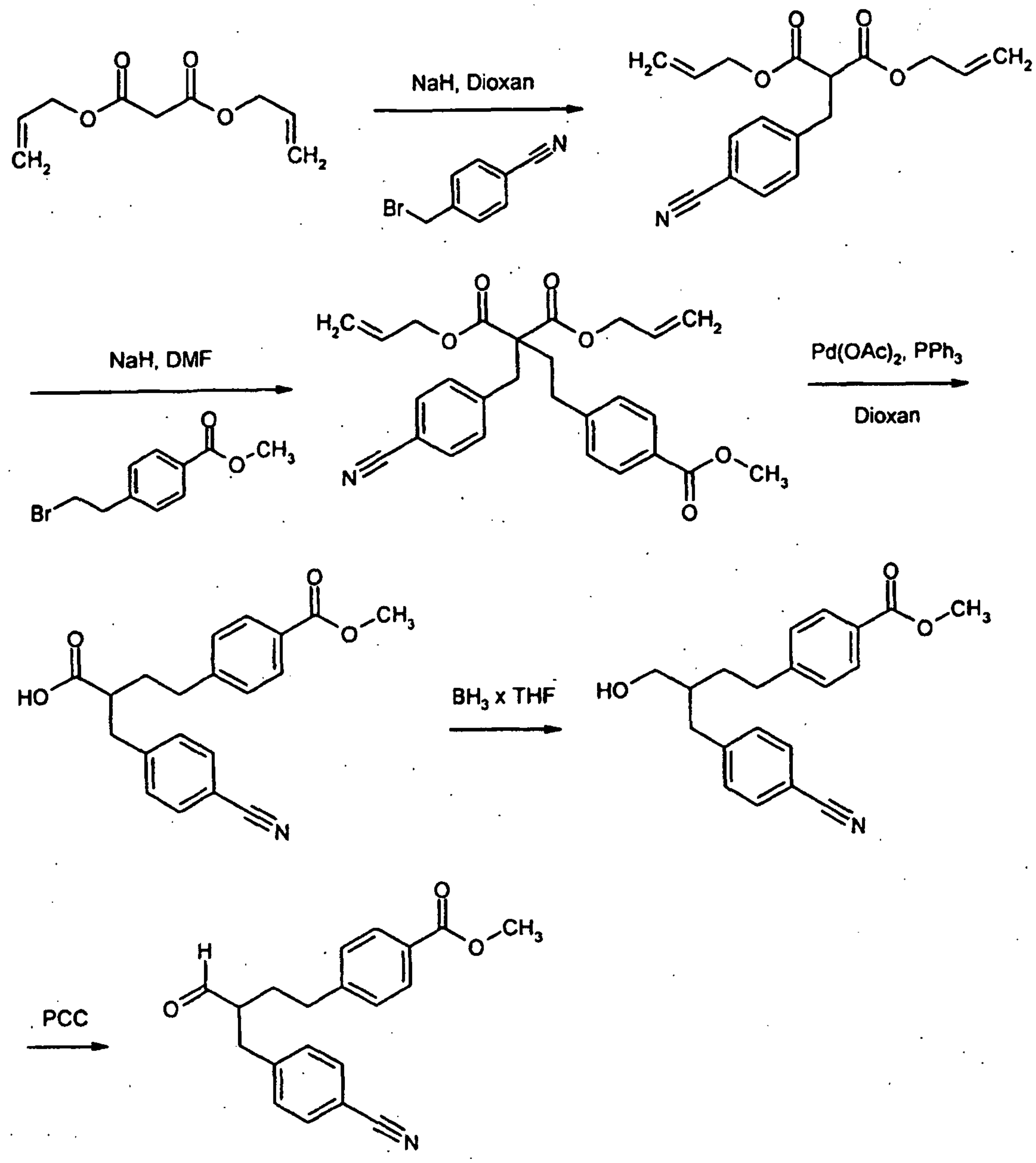

Schema 3

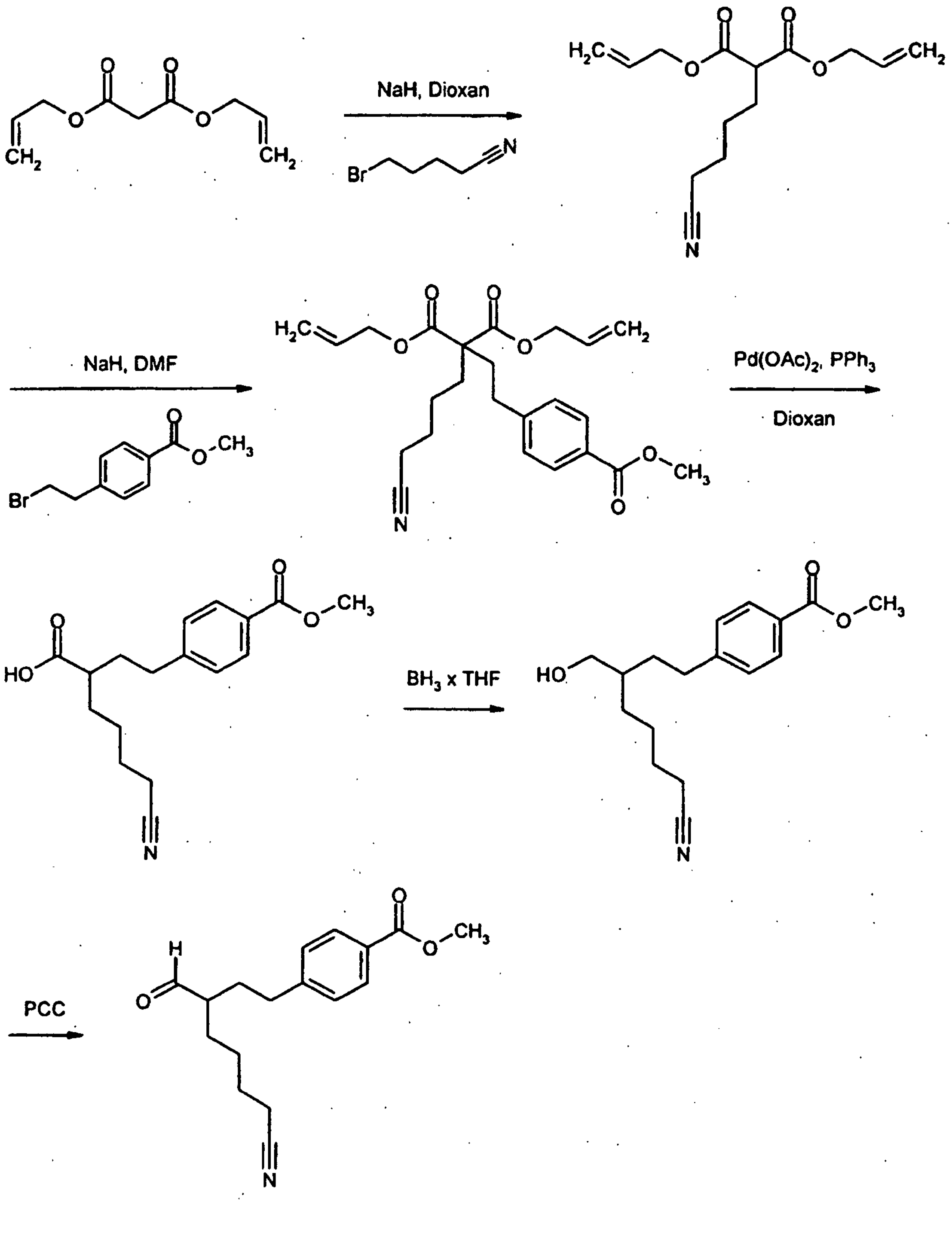

Schema 4

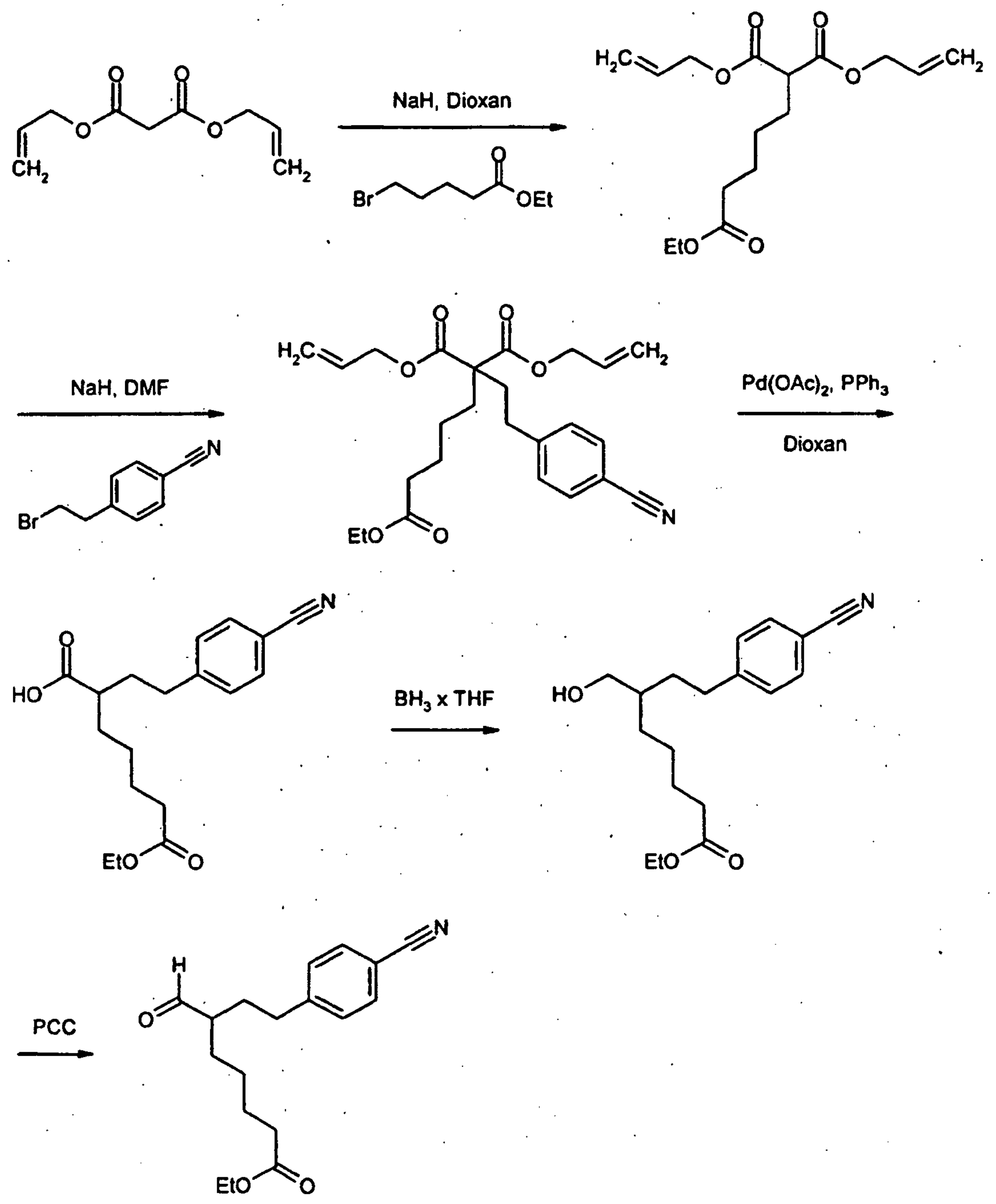

Schema 5

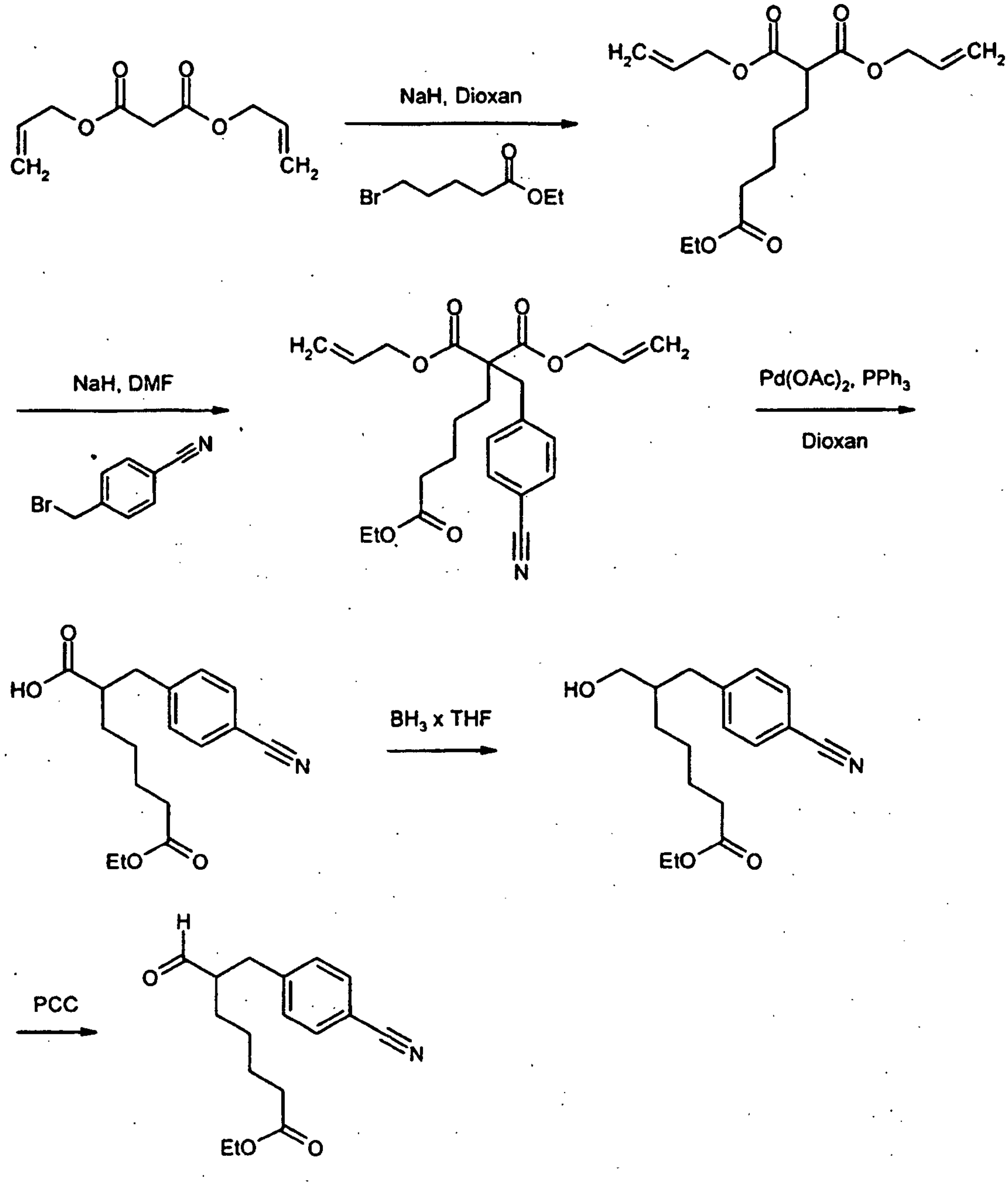

Schema 6

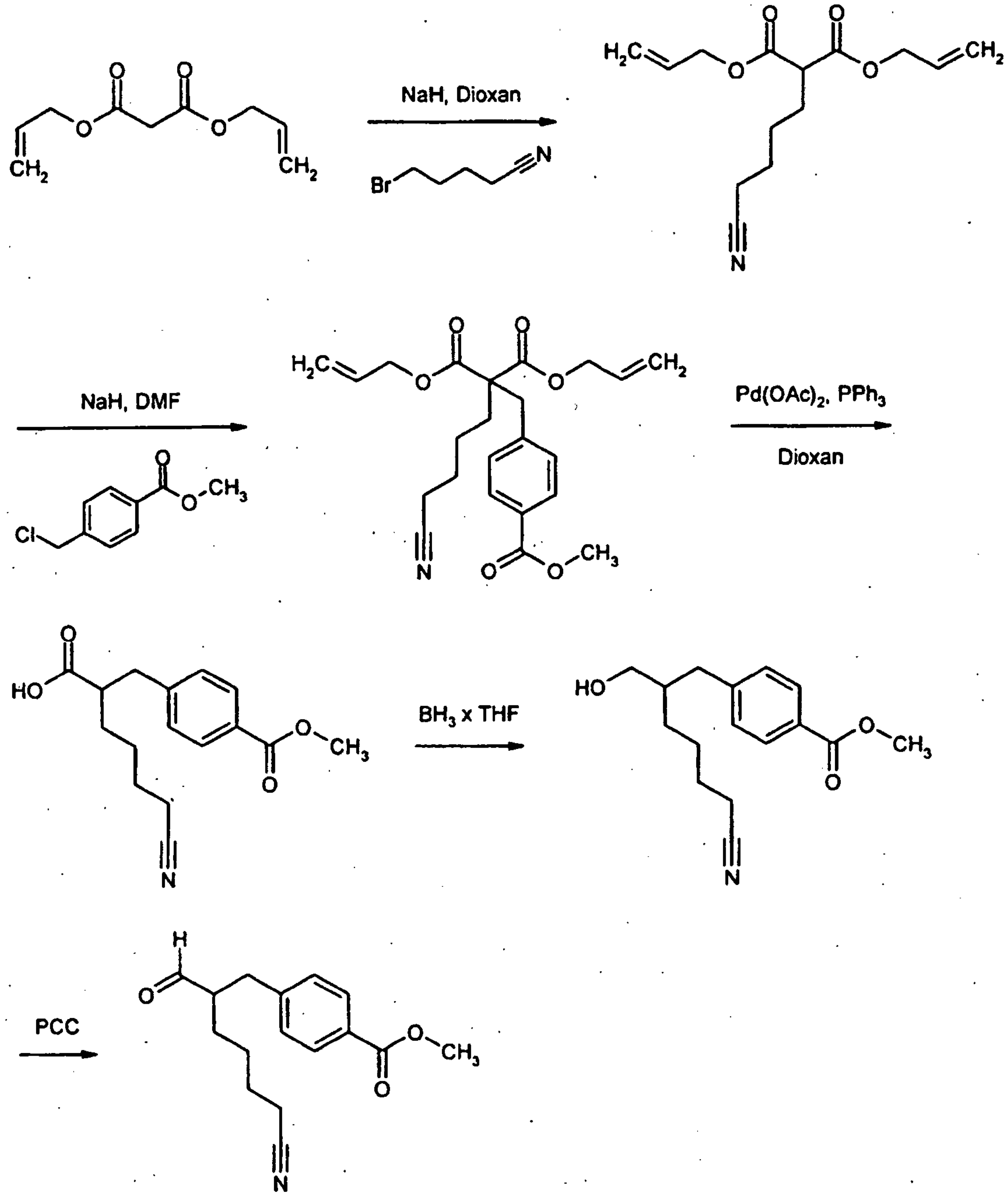


Schema 7

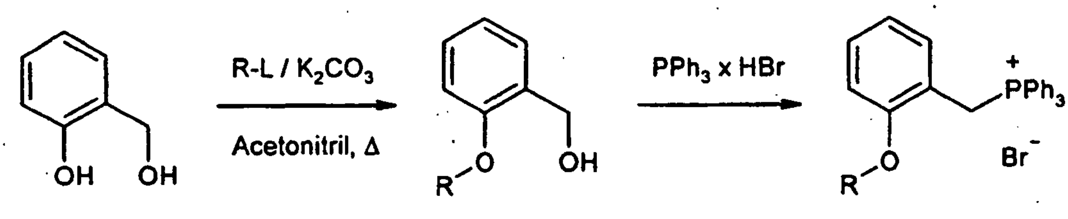

Schema 8

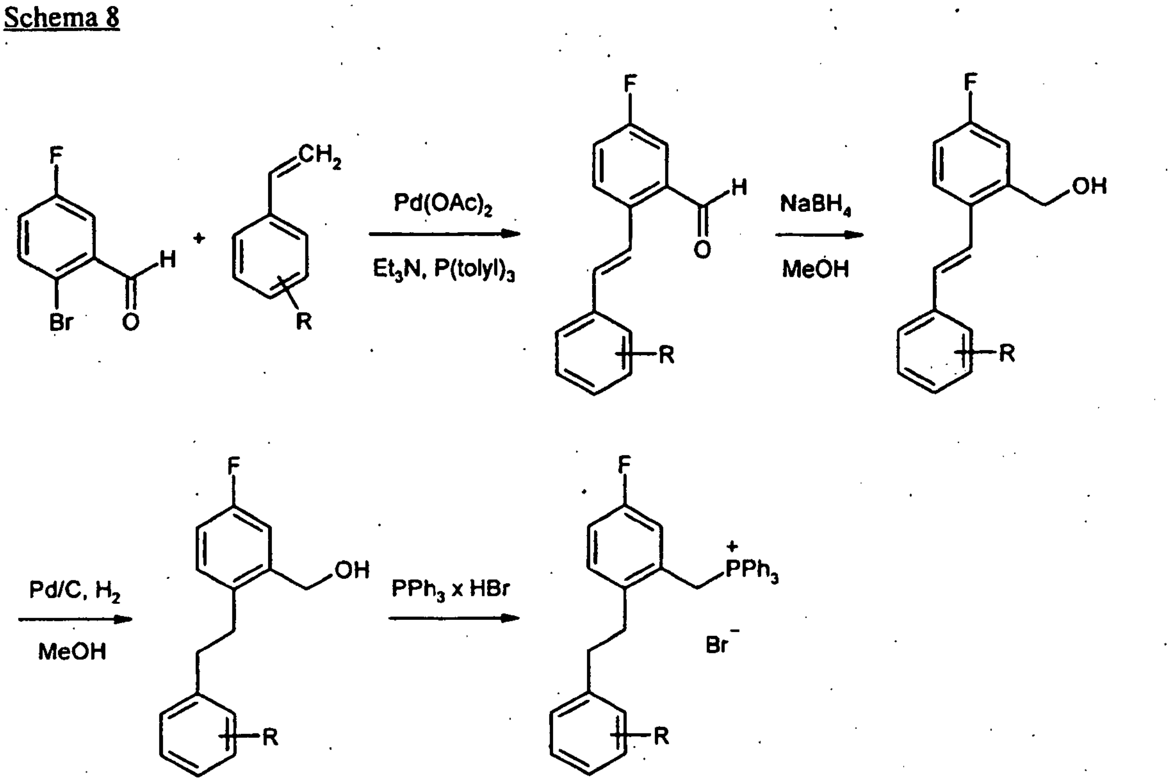

Schema 9

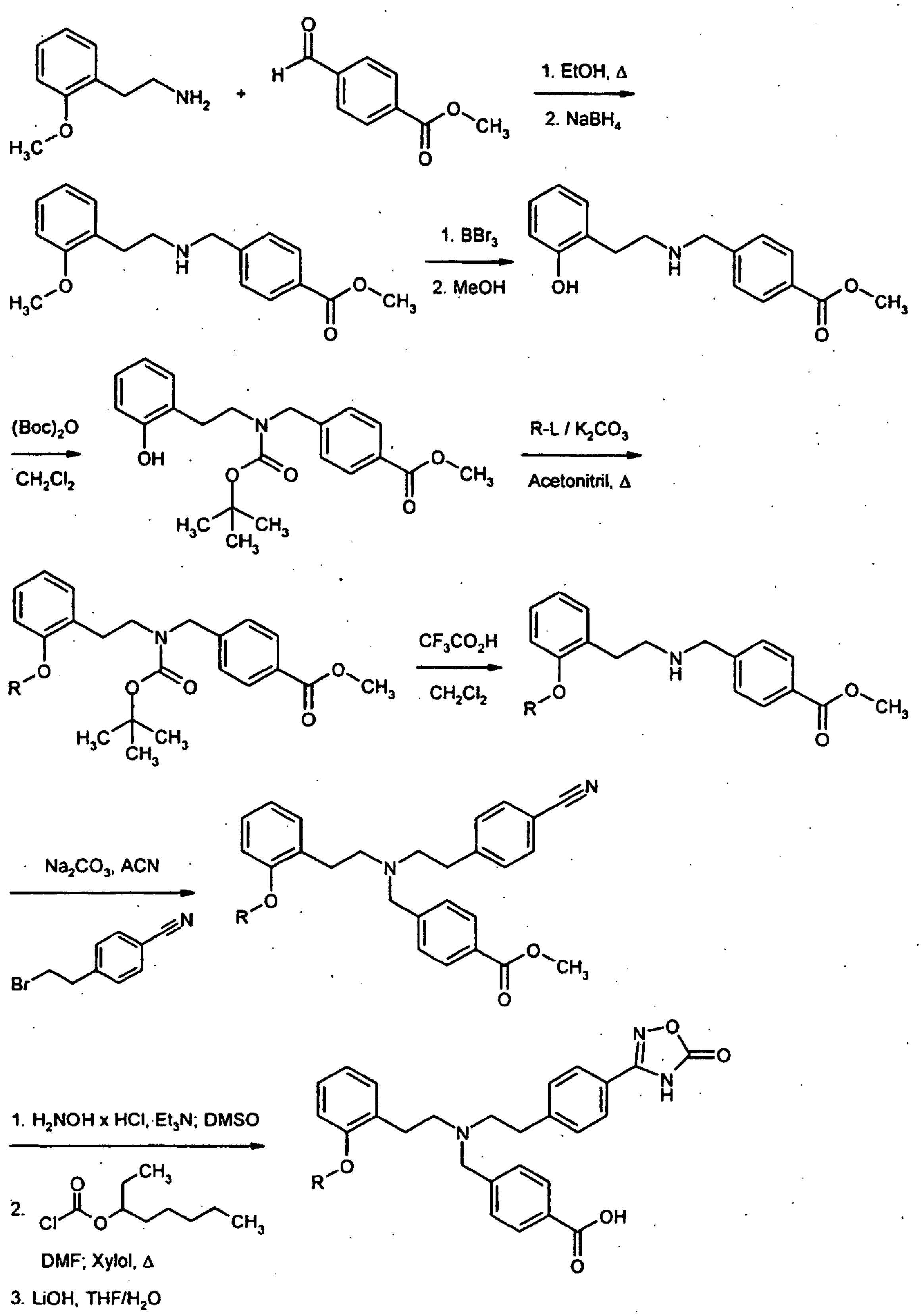
1. EtOH, Δ
2. NaBH4
1. BBr3
2. MeOH
(Boc)2O
CH2Cl2
R-L / K2CO3
Acetonitril, Δ
CF3CO2H
CH2Cl2
Na2CO3, ACN
1. H2NOH x HCl, Et3N; DMSO
2.
DMF; Xylol, Δ
3. LiOH, THF/H2O

Schema 10

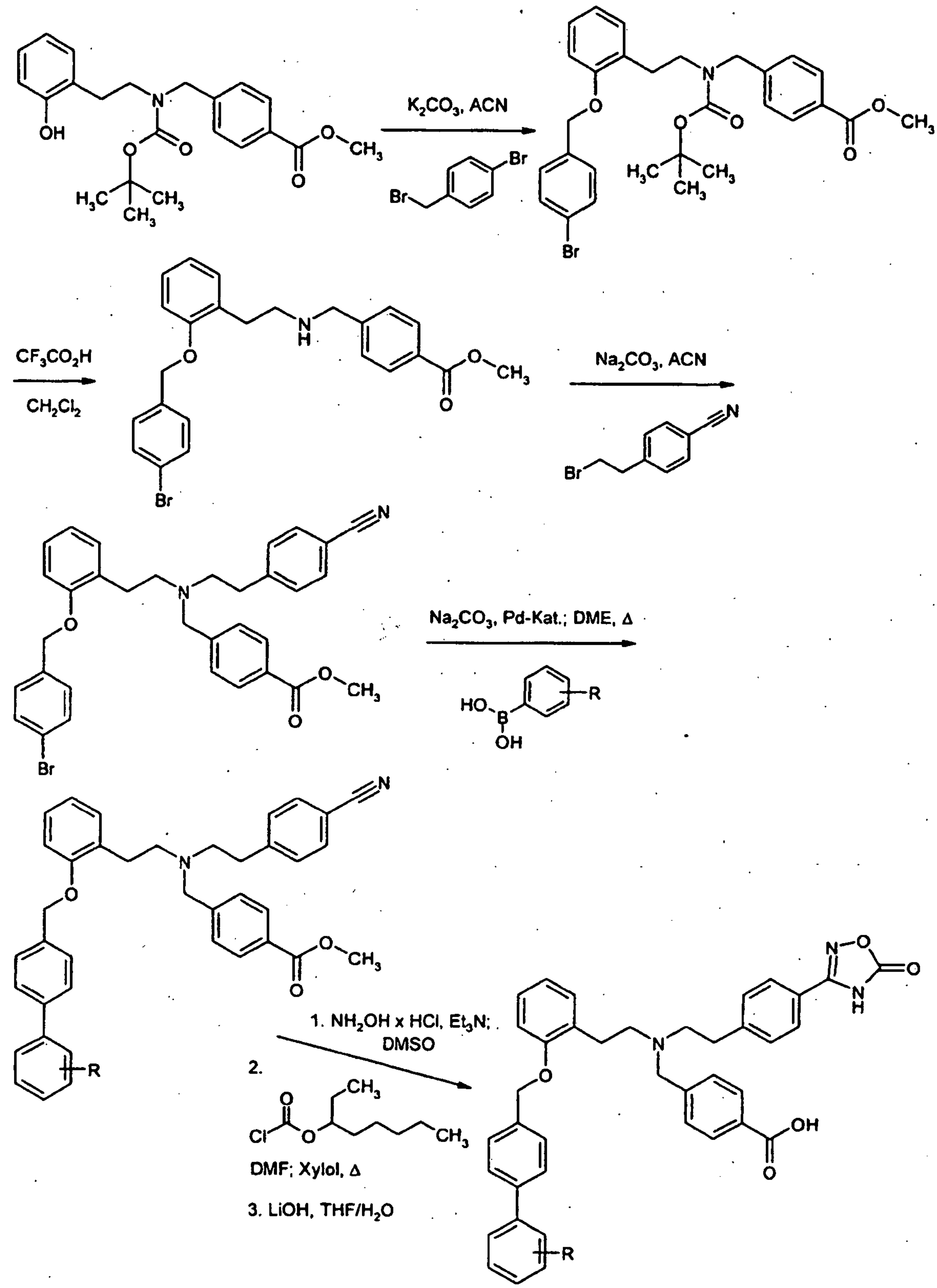

$K_2CO_3$, ACN
$CF_3CO_2H$
$CH_2Cl_2$
$Na_2CO_3$, ACN
$Na_2CO_3$, Pd-Kat.; DME, Δ
1. $NH_2OH$ x HCl, $Et_3N$; DMSO
2.
DMF; Xylol, Δ
3. LiOH, THF/$H_2O$

Schema 11

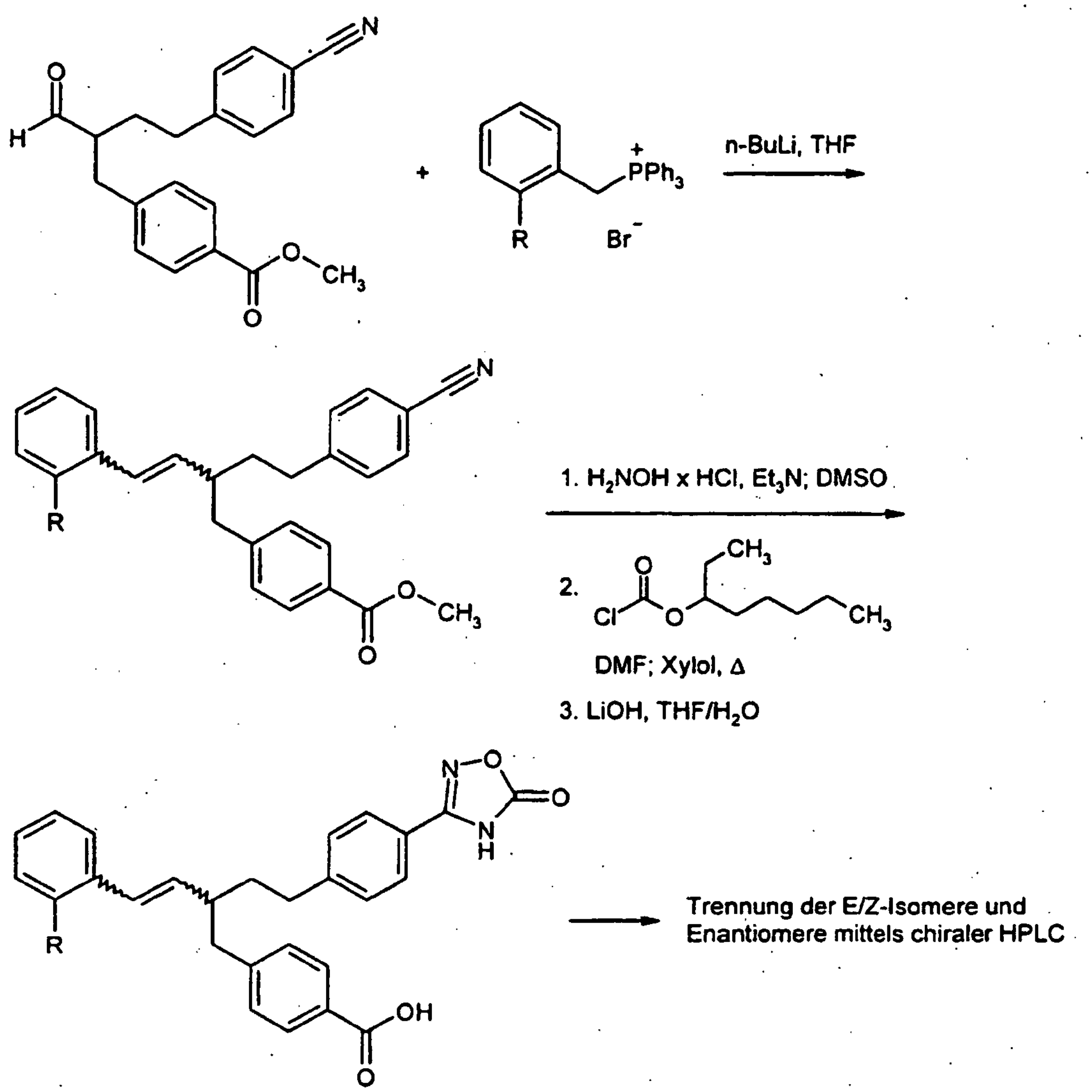
n-BuLi, THF
1. $H_2NOH$ x HCl, $Et_3N$; DMSO
2.
DMF; Xylol, Δ
3. LiOH, THF/$H_2O$
Trennung der E/Z-Isomere und Enantiomere mittels chiraler HPLC

Schema 12

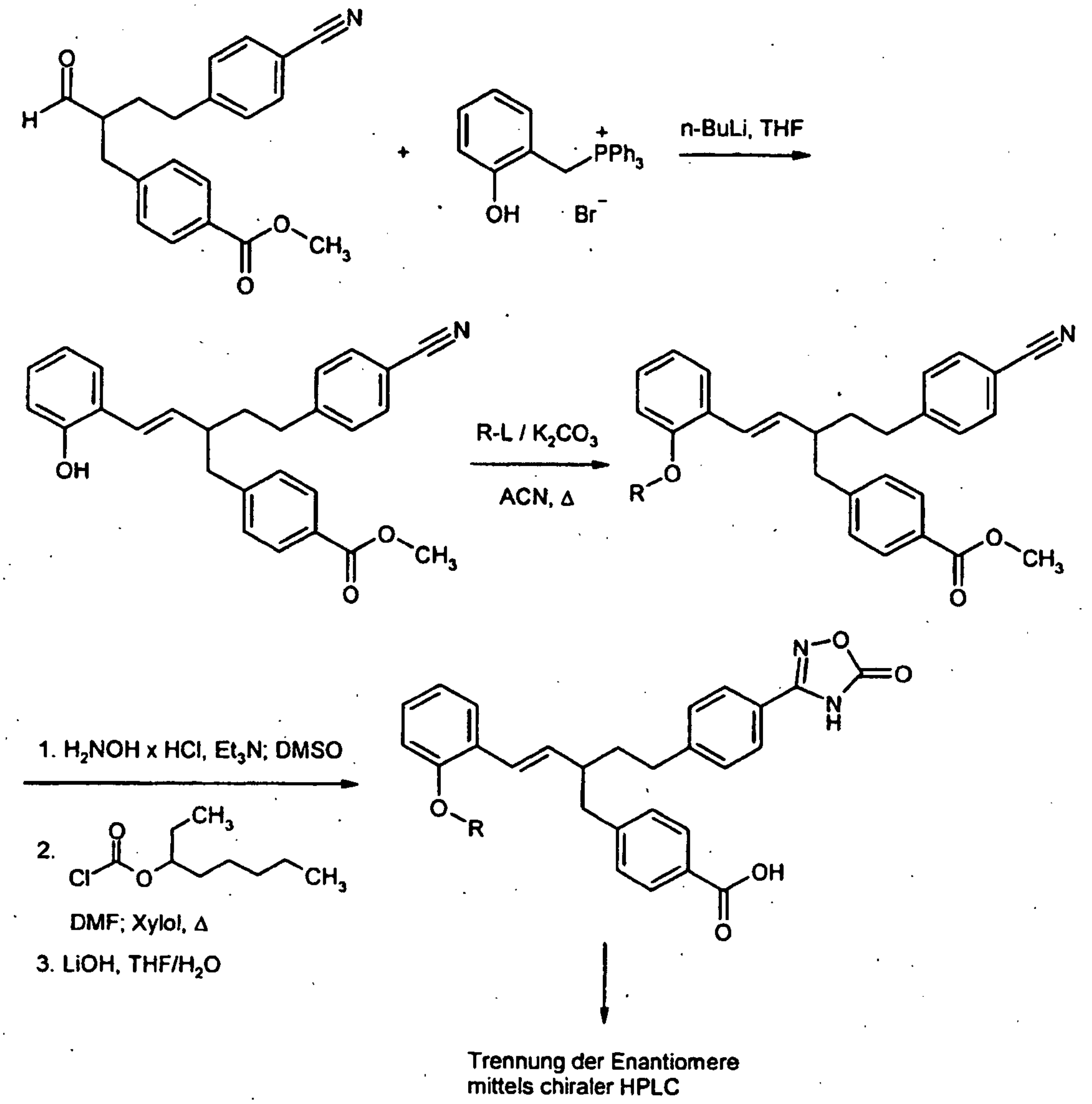
n-BuLi, THF
R-L / $K_2CO_3$
ACN, Δ
1. $H_2NOH$ x HCl, $Et_3N$; DMSO
2.
DMF; Xylol, Δ
3. LiOH, THF/$H_2O$
Trennung der Enantiomere mittels chiraler HPLC

Schema 13

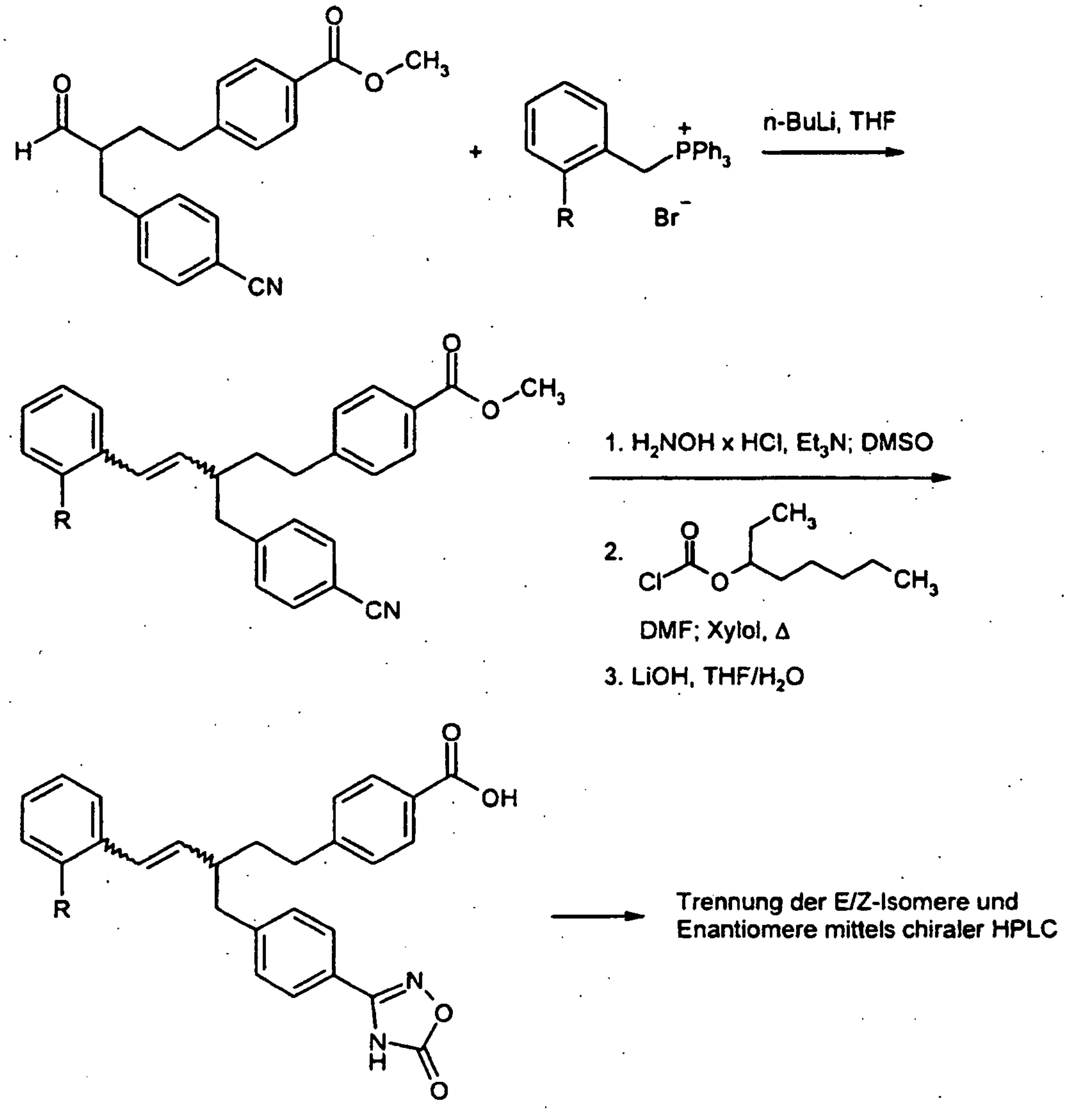
n-BuLi, THF
1. $H_2NOH$ x HCl, $Et_3N$; DMSO
2.
DMF; Xylol, Δ
3. LiOH, THF/$H_2O$
Trennung der E/Z-Isomere und Enantiomere mittels chiraler HPLC

Schema 14

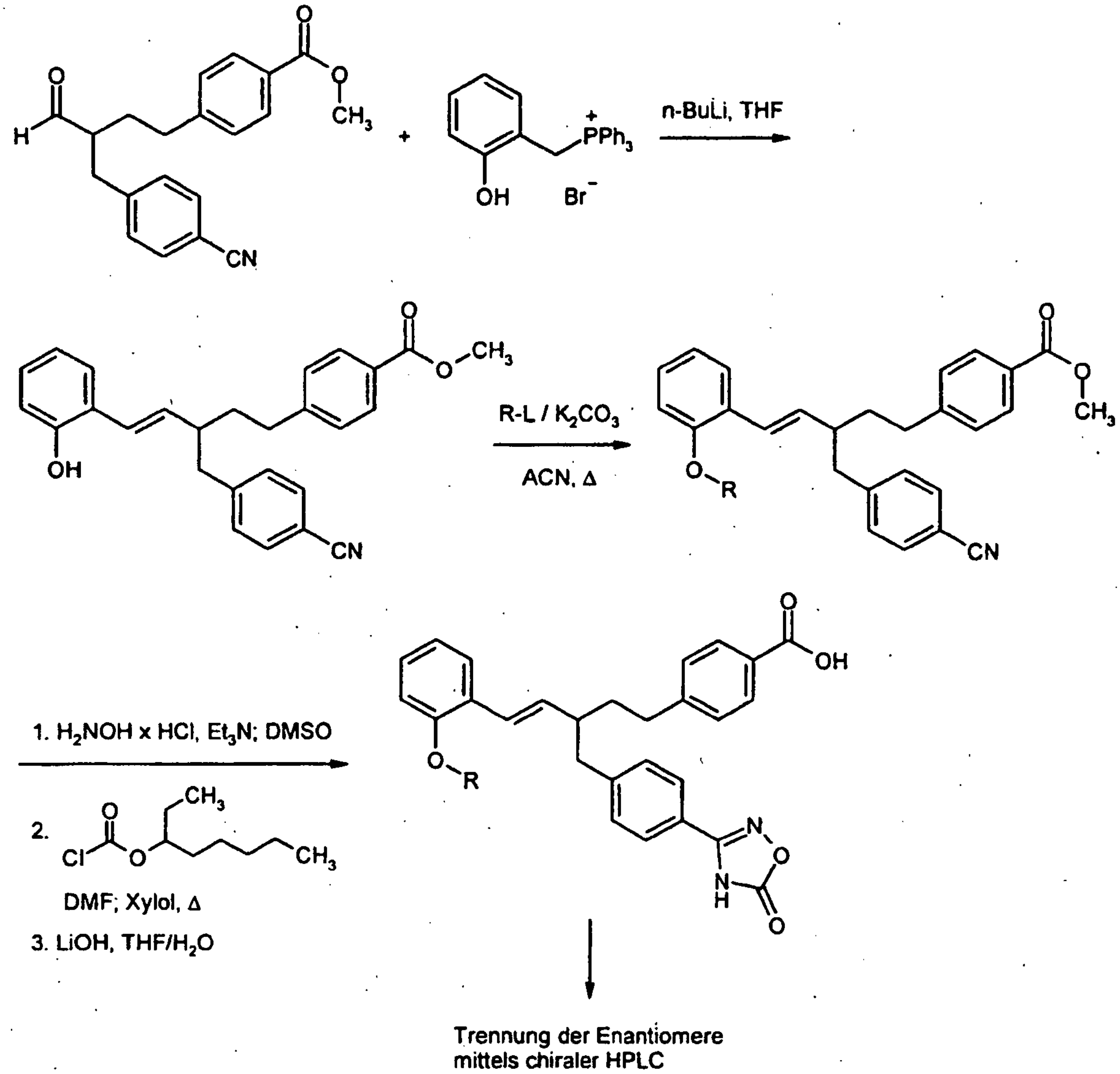
n-BuLi, THF
R-L / $K_2CO_3$
ACN, Δ
1. $H_2NOH$ x HCl, $Et_3N$; DMSO
2.
DMF; Xylol, Δ
3. LiOH, THF/$H_2O$
Trennung der Enantiomere
mittels chiraler HPLC

Schema 15

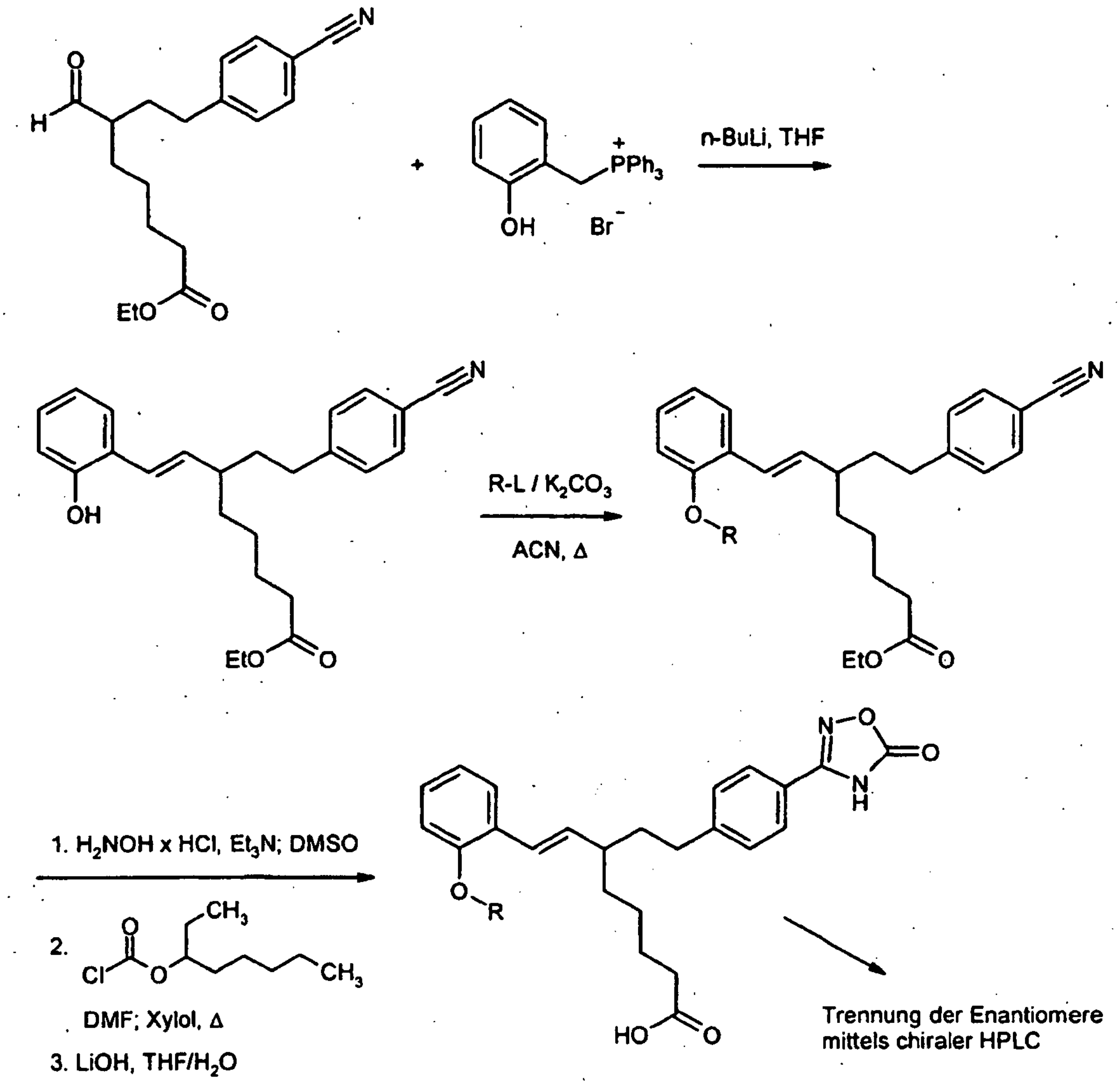
n-BuLi, THF
R-L / $K_2CO_3$
ACN, Δ
1. $H_2NOH$ x HCl, $Et_3N$; DMSO
2.
DMF; Xylol, Δ
3. LiOH, THF/$H_2O$
Trennung der Enantiomere mittels chiraler HPLC

Schema 16

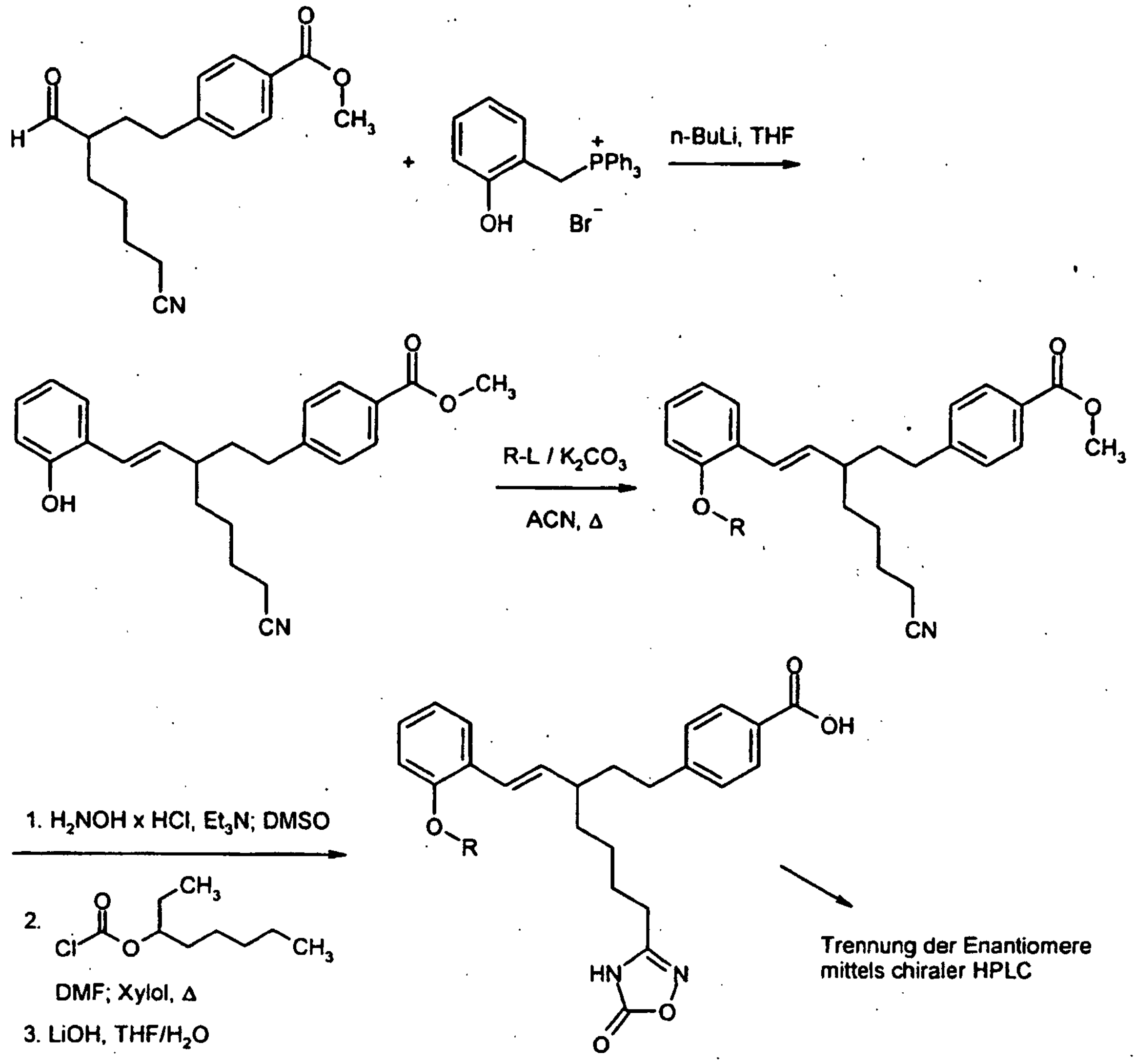
n-BuLi, THF
R-L / $K_2CO_3$
ACN, Δ
1. $H_2NOH$ x HCl, $Et_3N$; DMSO
2.
DMF; Xylol, Δ
3. LiOH, THF/$H_2O$
Trennung der Enantiomere mittels chiraler HPLC

Schema 17

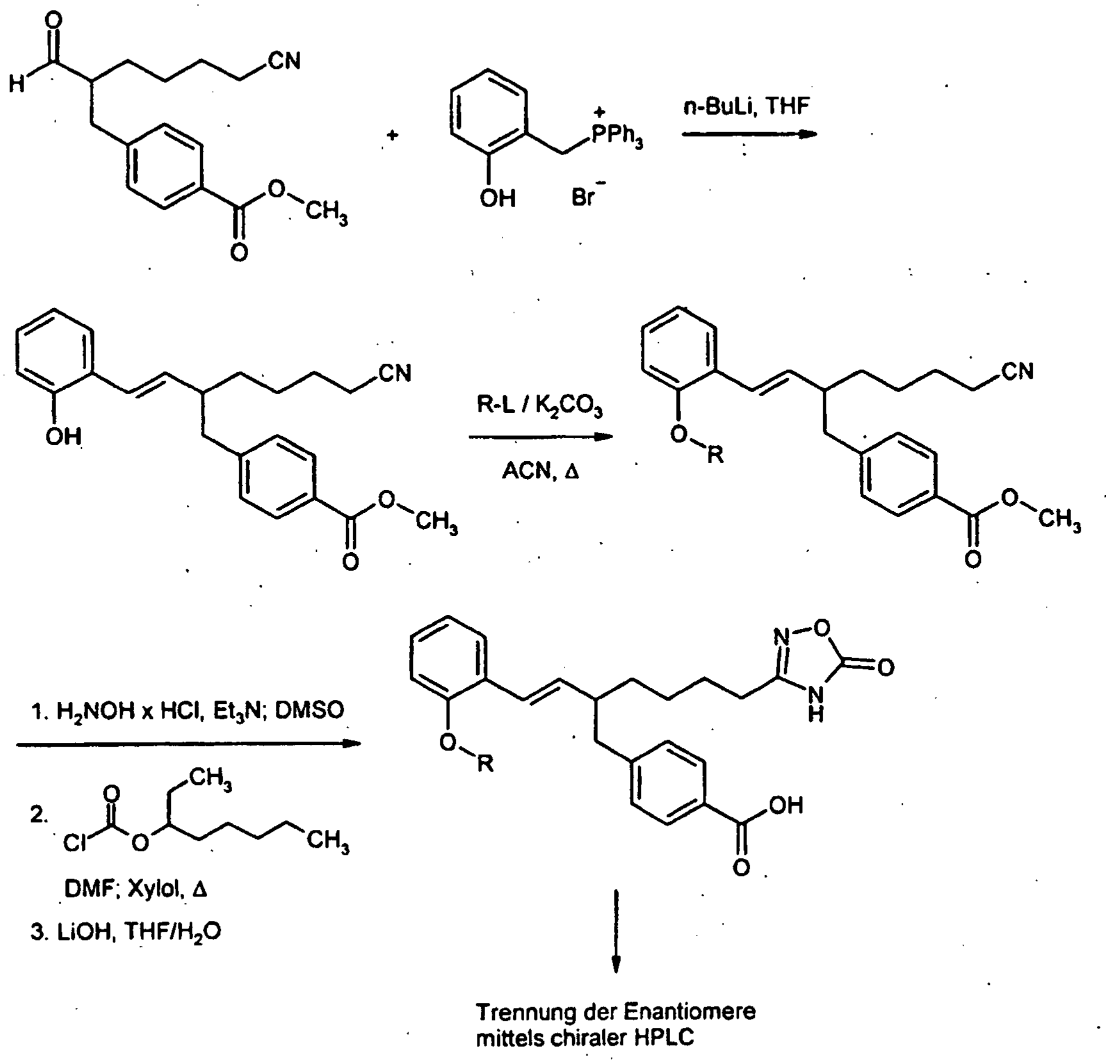
n-BuLi, THF
R-L / $K_2CO_3$
ACN, Δ
1. $H_2NOH$ x HCl, $Et_3N$; DMSO
2.
DMF; Xylol, Δ
3. LiOH, THF/$H_2O$
Trennung der Enantiomere
mittels chiraler HPLC

Schema 18

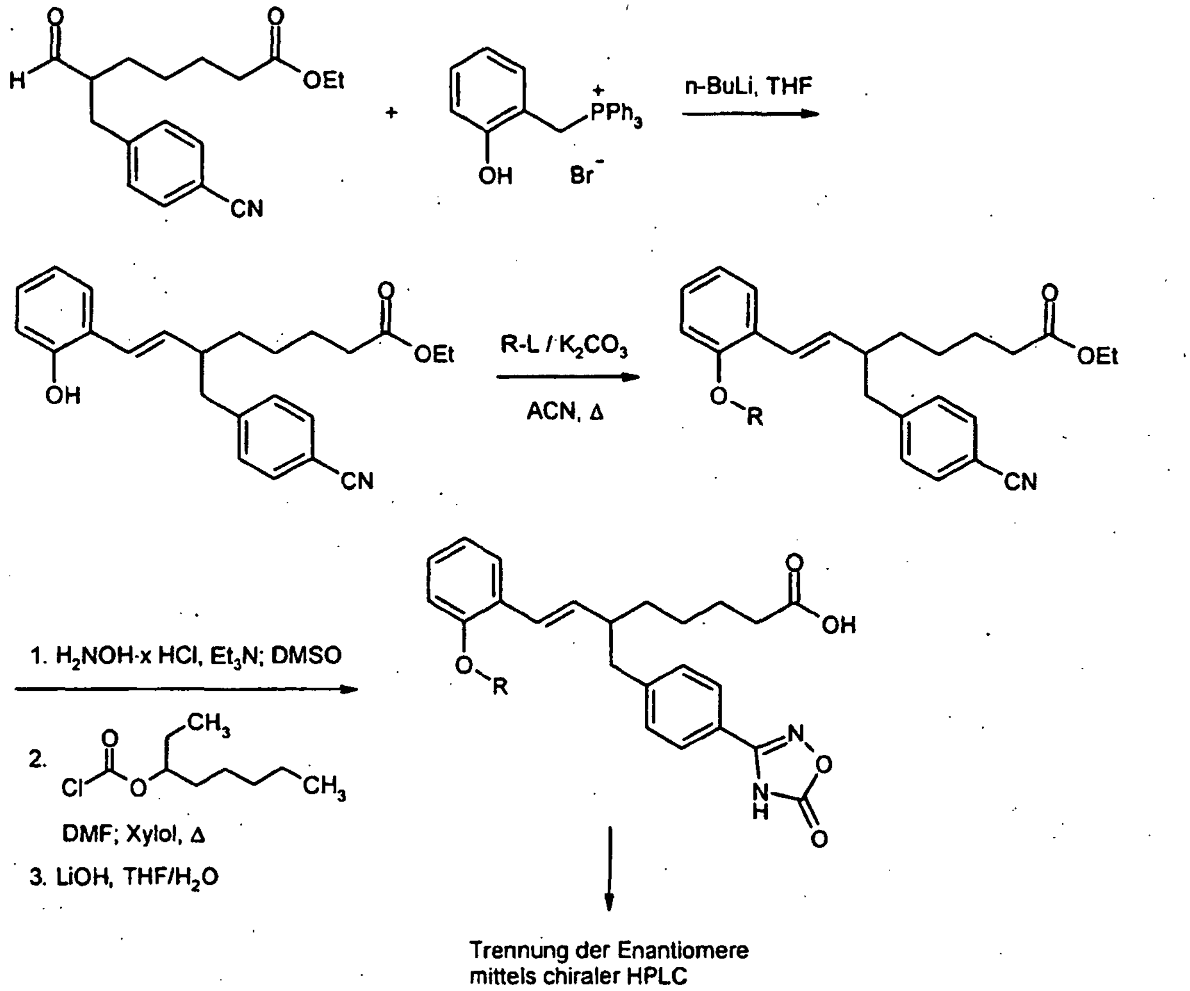


[Abkürzungen: Ac = Acetyl; ACN = Acetonitril; $(Boc)_2O$ = Di-*tert*.-butylpyrocarbonat; Bu = Butyl; DME = 1,2-Dimethoxyethan; DMF = Dimethylformamid; DMSO = Dimethylsulfoxid; Et = Ethyl; Kat. = Katalysator; L = Abgangsgruppe, z.B. Halogen; Me = Methyl; PCC = Pyridiniumchlorochromat; Ph = Phenyl; THF = Tetrahydrofuran].

Die erfindungsgemäßen Verbindungen besitzen wertvolle pharmakologische Eigenschaften und können zur Vorbeugung und Behandlung von Erkrankungen bei Menschen und Tieren verwendet werden.

Als besonderes und überraschendes Merkmal weisen die Verbindungen der vorliegenden Erfindung vorteilhafte pharmakokinetische Eigenschaften wie beispielsweise eine erhöhte Bioverfügbarkeit und/oder eine verlängerte Wirkdauer nach oraler Gabe auf.

Die erfindungsgemäßen Verbindungen führen zu einer Gefäßrelaxation, zu einer Thrombozytenaggregationshemmung und zu einer Blutdrucksenkung sowie zu einer Steigerung des koronaren Blutflusses. Diese Wirkungen sind über eine direkte Aktivierung der löslichen Guanylatcyclase und einen intrazellulären cGMP-Anstieg vermittelt.

Die erfindungsgemäßen Verbindungen können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, pulmonaler Hypertonie, peripheren und kardialen Gefäßerkrankungen, Arrhythmien, zur Behandlung von thromboembolischen Erkrankungen und Ischämien wie Myokardinfarkt, Hirnschlag, transistorischen und ischämischen Attacken, peripheren Durchblutungsstörungen, zur Verhinderung von Restenosen wie nach Thrombolysetherapien, percutan-transluminalen Angioplastien (PTA), percutan-transluminalen Koronarangioplastien (PTCA) und Bypass sowie zur Behandlung von Arteriosklerose, asthmatischen Erkrankungen, Krankheiten des Urogenitalsystems wie beispielsweise Prostatahypertrophie, erektile Dysfunktion, weibliche sexuelle Dysfunktion und Inkontinenz, von Osteoporose, Glaukom und Gastroparese eingesetzt werden.

Außerdem können die erfindungsgemäßen Verbindungen zur Behandlung von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, CREST-Syndrom,

Erythematose, Onychomykose sowie von rheumatischen Erkrankungen verwendet werden.

Ferner eignen sich die erfindungsgemäßen Verbindungen zur Behandlung von Respiratory Distress-Syndromen und chronisch-obstruktiven Atemwegserkrankungen (COPD), von akuter und chronischer Niereninsuffizienz sowie zur Förderung der Wundheilung.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfälten auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerrzuständen eingesetzt werden.

Zudem besitzen die erfindungsgemäßen Verbindungen antiinflammatorische Wirkung und können daher als entzündungshemmende Mittel eingesetzt werden.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:

- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;

- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;

- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;

- antithrombotisch wirkende Mittel, beispielhaft und vorzugsweise aus der Gruppe der Thrombozytenaggregationshemmer, der Antikoagulantien oder der profibrinolytischen Substanzen;

- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antigonisten sowie der Diuretika; und/oder

- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise BAY 59-7939, DU-176b, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol,.Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gerncabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten

oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beilspielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

## A. Beispiele

### Abkürzungen:

| | |
|---|---|
| abs. | absolut |
| aq. | wässrig |
| Bsp. | Beispiel |
| CI | chemische Ionisation (bei MS) |
| DC | Dünnschichtchromatographie |
| DCI | direkte chemische Ionisation (bei MS) |
| DMF | Dimethylformamid |
| DMSO | Dimethylsulfoxid |
| d. Th. | der Theorie (bei Ausbeute) |
| ee | Enantiomerenüberschuss |
| EI | Elektronenstoß-Ionisation (bei MS) |
| eq. | Äquivalent(e) |
| ESI | Elektrospray-Ionisation (bei MS) |
| GC | Gaschromatographie |
| h | Stunde(n) |
| HPLC | Hochdruck-, Hochleistungsflüssigchromatographie |
| LC/MS | Flüssigchromatographie-gekoppelte Massenspektroskopie |
| min | Minute(n) |
| MS | Massenspektroskopie |
| NMR | Kernresonanzspektroskopie |
| quant. | quantitativ |
| $R_f$ | Retentionsindex (bei DC) |
| RT | Raumtemperatur |
| $R_t$ | Retentionszeit (bei HPLC) |
| THF | Tetrahydrofuran |

UV Ultraviolett-Spektroskopie
v/v Volumen zu Volumen-Verhältnis (einer Lösung)

**LC/MS-Methoden:**

Methode 1 (LC-MS)

Gerätetyp MS: Micromass ZQ; Gerätetyp HPLC: HP 1100 Series; UV DAD; Säule: Phenomenex Synergi 2μ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

Methode 2 (LC-MS)

Gerätetyp MS: Micromass ZQ; Gerätetyp HPLC: Waters Alliance 2795; Säule: Phenomenex Synergi 2μ Hydro-RP Mercury 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

Methode 3 (LC-MS)

Instrument: Micromass Platform LCZ mit HPLC Agilent' Serie 1100; Säule: Phenomenex Synergi 2μ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

Methode 4 (LC-MS)

Instrument: Micromass Quattro LCZ mit HPLC Agilent Serie 1100; Säule: Phenomenex Synergi 2μ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

Methode 5 (LC-MS)

Instrument: Micromass Platform LCZ mit HPLC Agilent Serie 1100; Säule: Thermo Hypersil GOLD 3μ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

Methode 6 (LC-MS)

Gerätetyp MS: Micromass ZQ; Gerätetyp HPLC: HP 1100 Series; UV DAD; Säule: Phenomenex Gemini 3μ 30 mm x 3.00 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

**GC/MS-Methoden:**

Methode 1 (GC-MS)

Instrument: Micromass GCT, GC6890; Säule: Restek RTX-35MS, 30 m x 250 μm x 0.25 μm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

Methode 2 (GC-MS)

Instrument: Micromass GCT, GC6890; Säule: Restek RTX-35MS, 30 m x 250 μm x 0.25 μm; konstanter Fluss

mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (8.7 min halten).

HPLC-Methoden:

Methode 1 (HPLC)

Instrument: HP 1100 mit DAD-Detektion; Säule: Kromasil 100 RP-18, 60 mm x 2.1 mm, 3.5 μm; Eluent A: 5 ml $HClO_4$ (70%-ig) / l Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Säulentemperatur: 30°C; UV-Detektion: 210 nm.

Methode 2 (HPLC)

Instrument: HP 1100 mit DAD-Detektion; Säule: Kromasil 100 RP-18, 60 mm x 2.1 mm, 3.5 μm; Eluent A: 5 ml $HClO_4$ (70%-ig)/l Wasser, Eluent B: Acetonitril; Gradient: 0 min 2% B → 0.5 min 2% B → 4.5 min 90% B → 15 min 90% B → 15.2 min 2% B → 16 min 2% B; Fluss: 0.75 ml/min; Säulentemperatur: 30°C; UV-Detektion: 210 nm.

**Ausgangsverbindungen** und **Intermediate:**

**Beispiel 1A**

4-({[2-(2-Methoxyphenyl)ethyl]amino}methyl)benzoesäuremethylester

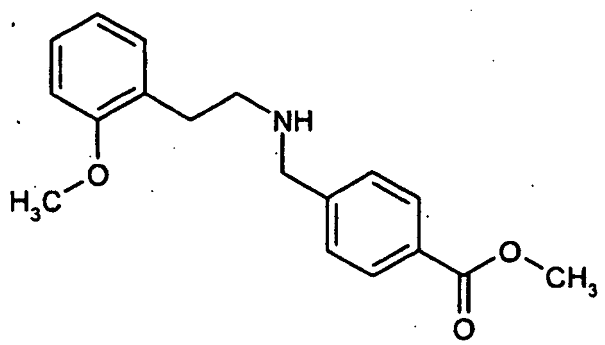

Eine Lösung von 92.08 g (0.597 mol) 2-Methoxyphenethylamin und 98 g (0.597 mol) 4-Formylbenzoesäuremethylester in 2 1 Ethanol wird 2 Stunden zum Rückfluss erhitzt. Anschließend wird das Lösungsmittel im Vakuum abgezogen und der erhaltene Rückstand in 1 1 Methanol gelöst. Portionsweise werden insgesamt 46.14 g (1.220 mol) festes Natriumborhydrid zugesetzt. Nach zwei Stunden Rühren bei Raumtemperatur wird der Ansatz in Wasser gegossen und mit Essigsäureethylester extrahiert. Der organische Extrakt wird mit gesättigter Kochsalz-Lösung gewaschen und über Natriumsulfat getrocknet. Nach Filtration wird das Lösungsmittel im Vakuum entfernt. Es werden 167.7 g (0.559 mol, 77% Ausbeute) eines farblosen Öls erhalten, das ohne weitere Reinigung in der nächsten Stufe eingesetzt wird.

$^1$H-NMR (200 MHz, DMSO-$d_6$, δ/ppm): 7,90 (2H, d), 7,45 (2H, d), 7,17 (1H, t), 7,12 (1H, d), 6,92 (1H, d), 6,83 (1H, t), 3,83 (3H, s), 3,78 (2H, s), 3,73 (3H, s), 2,75-2,63 (4H, m).

MS (DCI, $NH_3$): 300 ($M+H^+$).

**Beispiel 2A**

4-({[2-(2-Hydroxyphenyl)ethyl]amino}methyl)benzoesäuremethylester-Hydrobrömid

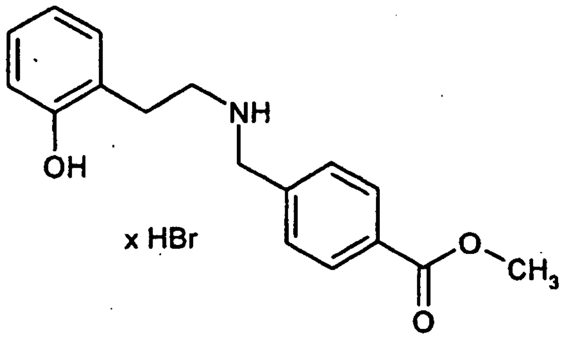

Eine Lösung von 60 g (0.2 mol) 4-({[2-(2-Methoxyphenyl)ethyl]amino}methyl)benzoesäuremethylester aus Beispiel 1A in 200 ml Dichlormethan wird bei 0°C mit 661.4 ml (0.66 mol) einer 1 M Lösung von Bortribromid in Dichlormethan versetzt. Man lässt eine Stunde bei 0°C weiterrühren. Dann werden 300 ml Methanol zugefügt und der Ansatz 18 Stunden zum Rückfluss erhitzt. Beim Abkühlen fällt das Produkt aus und wird abfiltriert. Weiteres Produkt wird nach Aufkonzentrieren der Mutterlauge erhalten. Die gesammelten Produktfraktionen werden mit Diethylether gewaschen. Es werden 45.04 g (0.16 mol, 56% Ausbeute) eines weißen kristallinen Feststoffs erhalten.

$R_f$ (Dichlormethan/Methanol 10: 1): 0.54.

$^1$H-NMR (300 MHz, DMSO-$d_6$, $\delta$/ppm): 9,58 (1H, breit), 9,02 (2H, breit), 8,03 (2H, d), 7,68 (2H, d), 7,09 (1H, d), 7,07 (1H, t), 6,82 (1H, d), 6,77 (1H, t), 4,29 (2H, s), 3,89 (3H, s), 3,18-3,10 (2H, m), 2,94-2,88 (2H, m).

MS (ESI): 286 ($M+H^+$).

__Beispiel 3A__

4-({*tert*.-Butoxycarbonyl-[2-(2-hydroxyphenyl)ethyl]amino}methyl)benzoesäuremethylester

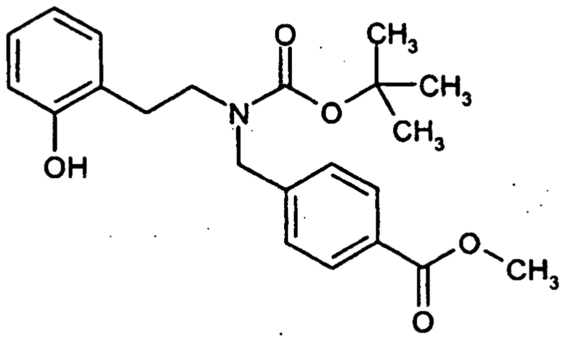

Eine Lösung von 11.15 g (39.08 mmol) 4-({[2-(2-Hydroxyphenyl)ethyl]amino}methyl)benzoesäuremethylester-Hydrobromid aus Beispiel 2A und 10.89 ml (78.15 mmol) Triethylamin in 300 ml THF wird bei 0°C mit 8.95 g (41.03 mmol) Di-*tert*.-butyldicarbonat, gelöst in 200 ml THF, versetzt. Nach 12 Stunden Rühren bei Raumtemperatur wird das Lösungsmittel im Vakuum abgezogen, der erhaltene Rückstand in Dichlormethan aufgenommen und mit halbgesättigter Ammoniumchlorid-Lösung gewaschen. Nach Trocknen über Natriumsulfat wird filtriert und eingeengt. Es werden 12.21 g (31.7 mmol, 82% Ausbeute) eines farblosen Öls erhalten, das ohne weitere Reinigung in der nächsten Stufe eingesetzt wird.

$^1$H-NMR (300 MHz, DMSO-$d_6$, $\delta$/ppm): 9,28 (1H, s), 7,95 (2H, d), 7,34 (2H, d), 7,01 (2H, t), 6,78 (2H, d), 6,69 (2H, t), 4,41 (2H, s), 3,86 (3H, s), 3,33 (2H, s, breit), 2,71 (2H, t), 1,48 (9H, s).

MS (ESI): 386 ($M+H^+$).

__Beispiel 4A__

4-[({2-[2-(4-Brombenzyloxy)phenyl]ethyl}-*tert*.-butoxycarbonylamino)methyl]benzoesäuremethylester

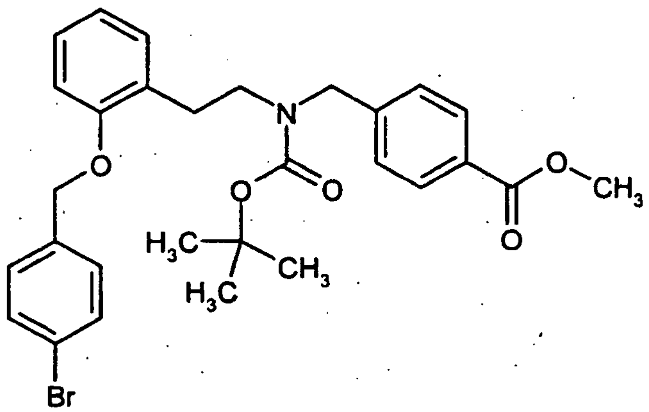

Eine Lösung von 5 g (12.87 mmol) 4-({*tert*.-Butoxycarbonyl-[2-(2-hydroxyphenyl)ethyl]amino}-methyl)benzoesäuremethylester aus Beispiel 3A in 100 ml trockenem Acetonitril wird mit 3.89 g (15.57 mmol) 4-Brombenzylbromid und 2.69 g (19.46 mmol) wasserfreiem Kaliumcarbonat versetzt und 12 Stunden zum Rückfluss erhitzt. Anschließend wird der Ansatz zur Trockene eingeengt. Der Rückstand wird in Essigsäureethylester aufgenommen, mit Wasser und gesättigter Kochsalz-Lösung gewaschen und über Natriumsulfat getrocknet. Die erhaltene organischen Phase wird eingeengt. Das Rohprodukt wird durch Flash-Chromatographie an Kieselgel (Laufmittel: Cyclohexan/Essigsäureethylester 20:1 $\rightarrow$ 10:1) gereinigt. Es werden 6.88 g (12.4 mmol, 89% Ausbeute) eines Feststoffs erhalten.

$^1$H-NMR (300 MHz, DMSO-$d_6$, $\delta$/ppm): 7,90 (2H, d), 7,57 (2H, d), 7,38 (2H, d), 7,27-7,06 (4H, m), 7,00 (1H, d), 6,88 (1H, t), 5,06 (2H, s), 4,38 (2H, m), 3,84 (3H, s), 3,36 (2H, m), 2,79 (2H, t), 1,30 (9H, s).

MS (ESI): 576 (M+Na$^+$), 578 (M+Na$^+$), 553 (M$^+$), 555 (M$^+$).

__Beispiel 5A__

4-({2-[2-(4-Brombenzyloxy)phenyl]ethylamino}methyl)benzoesäuremethylester

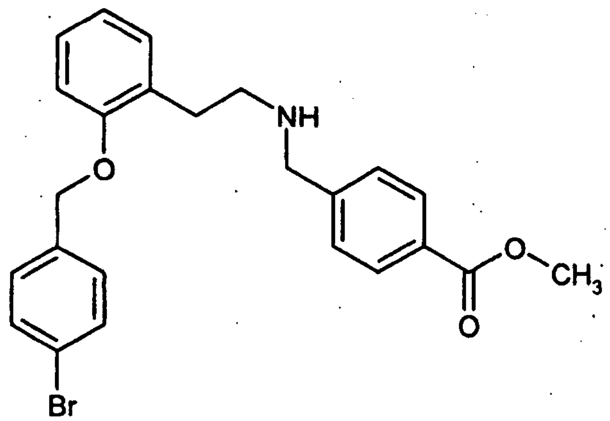

Eine Lösung von 4.2 g (7.57 mmol) 4-[({2-[2-(4-Brombenzyloxy)phenyl]ethyl}-*tert*.-butoxycarbonylamino)methyl]benzoesäuremethylester aus Beispiel 4A in 15 ml Dichlormethan wird mit 15 ml Trifluoressigsäure 4 Stunden bei Raumtemperatur gerührt. Anschließend wird die Reaktionslösung mit wässriger Natriumhydrogencarbonat-Lösung neutralisiert, die organische Phase abgetrennt und über Natriumsulfat getrocknet. Nach Filtration wird das Lösemittel im Vakuum entfernt. Es werden 3.2 g (7.04 mmol, 92% Ausbeute) eines farblosen Öls erhalten, das ohne weitere Reinigung in der nächsten Stufe eingesetzt wird.

$^1$H-NMR (300 MHz, DMSO-$d_6$, $\delta$/ppm): 7,86 (2H, d), 7,57 (2H, d), 7,42 (2H, d), 7,37 (2H, d), 7,14 (2H, t), 6,97 (1 H, d), 6,86 (1H, t), 5,13 (2H, s), 3,85 (3H, s), 3,79 (2H, s), 2,81-2,67 (4H, m).

MS (CI): 454 (M$^+$).

**Beispiel 6A**

[4-(2-Bromethyl)phenyl]methanol

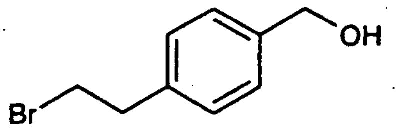

Eine Lösung von 2 g (8.73 mmol) 4-(2-Bromethyl)benzoesäure in 50 ml trockenem THF wird bei -10°C langsam und tropfenweise mit 13.10 ml (13.10 mmol) Boran-THF-Komplex versetzt. Nach Erwärmung auf Raumtemperatur wird der Ansatz eine Stunde nachgerührt. Nach vollständiger Umsetzung wird der Ansatz mit gesättigter Ammoniumchlorid-Lösung versetzt, in Essigsäureethylester aufgenommen und die organische Phase abgetrennt und über Natriumsulfat getrocknet. Nach Filtration wird das Lösemittel im Vakuum entfernt. Es werden 1.67 g (7.76 mmol, 79% Ausbeute) eines farblosen Öls erhalten, das ohne weitere Reinigung in der nächsten Stufe eingesetzt wird.

$^1$H-NMR (300 MHz, DMSO-$d_6$, $\delta$/ppm): 7,33-7,28 (4H, m), 5,14 (1H, t), 4,48 (2H, d), 3,77 (2H, t), 3,11 (2H, t).

MS (DCI, $NH_3$): 232 ($M+NH_4^+$).

**Beispiel 7A**

4-(2-Bromethyl)benzaldehyd

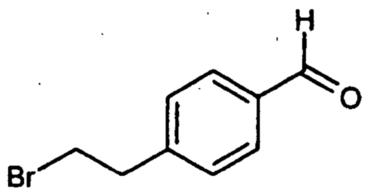

Verfahren 1:

Eine Lösung von 200 mg (0.93 mmol) [4-(2-Bromethyl)phenyl]methanol aus Beispiel 6A in 20 ml Dichlormethan wird mit 240.5 mg (1.12 mmol) Pyridiniumchlorochromat (PCC) versetzt und 3 Stunden bei Raumtemperatur gerührt. Anschließend wird die Reaktionslösung mit ca. 2 g Kieselgel versetzt und bis zur Trockene eingeengt. Der erhaltene Rückstand wird durch Flash-Chromatographie an Kieselgel (Laufmittel: Cyclohexan/Essigsäureethylester 4:1) gereinigt. Es werden 183 mg (0.85 mmol, 82% Ausbeute) eines farblosen Feststoffes erhalten.

Verfahren 2:

Eine Lösung von 44.4 g (0.38 mol) Dichlormethylmethylether in 230 ml Dichlormethan wird unter Kühlung (4-5°C) innerhalb von 10 min mit 42.26 ml (0.385 mol) Titantetrachlorid versetzt und 1 Stunde nachgerührt. Anschließend werden 64.89 g (0.34 mol) 2-Bromethylbenzol, gelöst in 24 ml Dichlormethan, bei 5-7°C innerhalb von 50 min zu der Reaktionslösung zudosiert. Dann erwärmt man die Reaktionslösung langsam auf Raumtemperatur und lässt den Ansatz über Nacht nachrühren. Nach vollständiger Umsetzung werden sehr vorsichtig innerhalb von 1 Stunde 140 ml Wasser zugetropft (Vorsicht: zunächst endotherme Reaktion durch Gasentwicklung, dann exotherme Reaktion bis 30°C, Kühlung erforderlich). Die Reaktionslösung wird dreimal mit Dichlormethan extrahiert, die vereinigten organischen Phasen mit 170 ml Wasser gewaschen sowie mit 115 ml Natriumhydrogencarbonat-Lösung neutralisiert und über Natriumsulfat getrocknet. Nach Filtration wird das Lösemittel im Vakuum entfernt. Der erhaltene Rückstand wird durch Flash-Chromatographie an Kieselgel (Laufmittel: Dichlormethan/Petrolether 1:2 $\rightarrow$ 1:1) gereinigt. Es werden 29.3 g (0.14 mol, 37% Ausbeute) eines farblosen Feststoffes erhalten.

$^1$H-NMR (300 MHz, DMSO-$d_6$, $\delta$/ppm): 9,99 (1H, s), 7,88 (2H, d), 7,52 (2H, d), 3,80 (2H, t), 3,24 (2H, t).

MS (EI): 212 ($M^+$).

**Beispiel 8A**

4-(2-Bromethyl)benzonitril

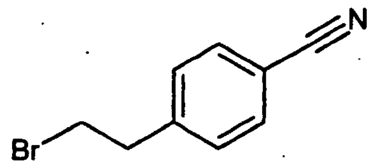

Eine Lösung von 29.3 g (0.14 mol) 4-(2-Bromethyl)benzaldehyd aus Beispiel 7A in 112.4 ml Ameisensäure wird mit 12.42 g (0.18 mol) Hydroxylaminhydrochlorid versetzt und 2 Stunden zum Rückfluss erhitzt. Nach langsamem Abkühlen auf Raumtemperatur werden 670 ml Wasser zugesetzt und das Reaktionsgemisch langsam unter Kühlung mit 6 N Natronlauge neutralisiert. Anschließend wird der Ansatz dreimal mit Methyl-*tert.*-butylether extrahiert. Die vereinigten organischen Phasen werden über Magnesiumsulfat getrocknet und zur Trockene eingeengt. Der erhaltene Rückstand wird durch Flash-Chromatographie an Kieselgel (Laufmittel: Dichlormethan) gereinigt. Es werden 21.3 g (0.10 mol, 74% Ausbeute) eines gelblichen Feststoffes erhalten.
$^1$H-NMR (400 MHz, DMSO-$d_6$, $\delta$/ppm): 7,80 (2H, d), 7,51 (2H, d), 3,77 (2H, t), 3,22 (2H, t).
MS (DCI, $NH_3$): 227 ($M+NH_4^+$).

**Beispiel 9A**

4-({{2-[2-(4-Brombenzyloxy)phenyl]ethyl}-[2-(4-cyanophenyl)ethyl]amino}methyl)benzoesäuremethylester

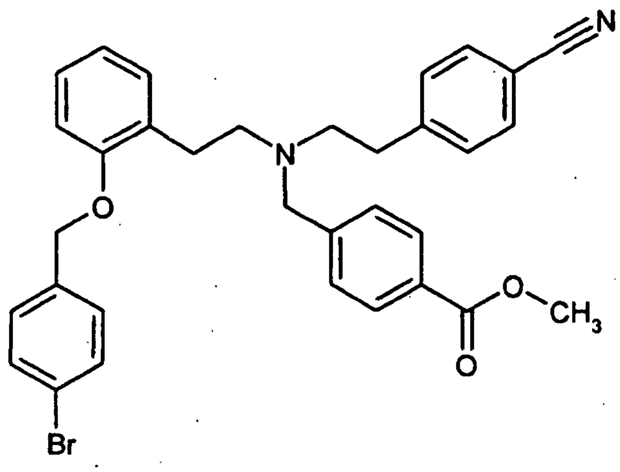

200 mg (0.44 mmol) 4-({2-[2-(4-Brombenzyloxy)phenyl]ethylamino}methyl)benzoesäuremethylester aus Beispiel 5A, 101 mg (0.48 mmol) 4-(2-Bromethyl)benzonitril und 51.4 mg (0.48 mmol) Natriumcarbonat werden in 5 ml Acetonitril 5 Stunden zum Rückfluss erhitzt. Anschließend werden erneut 101 mg (0.48 mmol) 4-(2-Bromethyl)benzonitril hinzugefügt und die Reaktionslösung über Nacht weiter unter Rückfluss gerührt. Nach Abkühlen der Reaktionslösung wird der Ansatz zur Trockene eingeengt, der Rückstand mit Essigsäureethylester aufgenommen und mit Wasser und gesättigter Kochsalz-Lösung gewaschen. Nach Trocknen über Natriumsulfat wird filtriert und eingeengt. Das Rohprodukt wird durch präparative HPLC gereinigt. Es werden 183 mg (0.31 mmol, 69% Ausbeute) eines blassgelben Öls erhalten,
**[0162]** $^1$H-NMR (300 MHz, DMSO-$d_6$, $\delta$/ppm): 7,79 (2H, d), 7,63 (2H, d), 7,49 (2H, d), 7,30 (2H, d), 7,24 (4H, d), 7,18 (1H, t), 7,09 (1H, d), 6,99 (1H, d), 6,86 (1H, t), 5,00 (2H, s), 3,85 (3H, s), 3,70 (2H, s), 2,79-2,59 (8H, m).
MS (ESI): 583 ($M+H^+$).

**Beispiel 10A**

4-[([2-(4-Cyanophenyl)ethyl]-{2-[2-(4'-trifluormethyl-biphenyl-4-ylmethoxy)phenyl]ethyl}amino)-methyl]benzoesäure-methylester

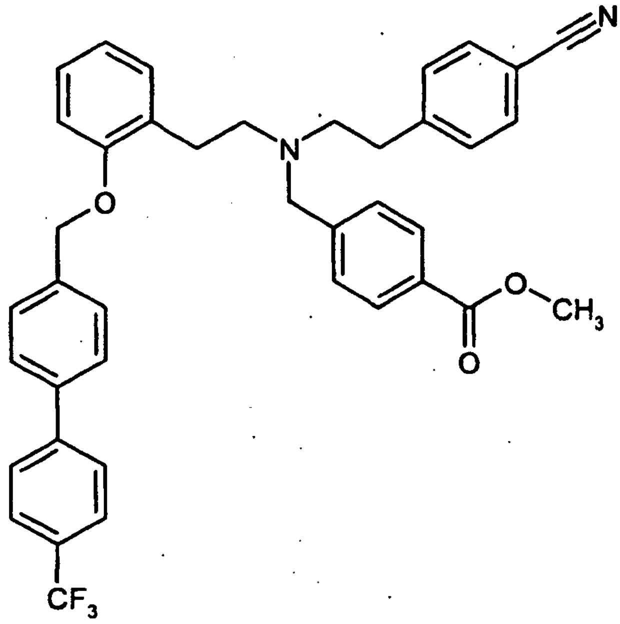

200 mg (0.34 mmol) 4-({{2-[2-(4-Brombenzyloxy)phenyl]ethyl}-[2-(4-cyanophenyl)ethyl]amino}-methyl)benzoesäuremethylester aus Beispiel 9A werden in 2 ml 1,2-Dimethoxyethan gelöst und unter Argon mit 78 mg (0.41 mmol) 4-Trifluormethylphenylboronsäure, 2.5 mg Bis(triphenylphosphin)palladium(II)chloirid und 0.38 ml einer 2 M wässrigen Natriumcarbonat-Lösung versetzt. Das Reaktionsgemisch wird anschließend 12 Stunden unter Rückfluss gerührt. Nachfolgend wird der Ansatz abgekühlt, über 1 g Extrelut filtriert, mit Dichlormethan gewaschen und eingeengt. Das erhaltene Rohprodukt wird durch präparative HPLC gereinigt. Es werden 158 mg (0.24 mmol, 71% Ausbeute) eines farblosen Öls erhalten.

$^1$H-NMR (300 MHz, $CDCl_3$, $\delta$/ppm): 7,85 (2H, d), 7,69 (2H, d), 7,62 (2H, d), 7,54 (2H, d), 7,43 (4H, dd), 7,19 (3H, t), 7,10-7,01 (3H, m), 6,92 (2H, d), 5,06 (2H, s), 3,89 (3H, s), 3,69 (2H, s), 2,90-2,76 (4H, m), 2,70 (4H, s).

MS (ESI): 649 ($M+H^+$).

**Beispiel 11A**

4-[({2-[4-(5-Oxo-4,5-dihydro-[1,2,4]oxadiazol-3-yl)phenyl]ethyl}-{2-[2-(4'-trifluormethyl-biphenyl-4-ylmethoxy)phenyl] ethyl} amino)methyl]benzoesäuremethylester

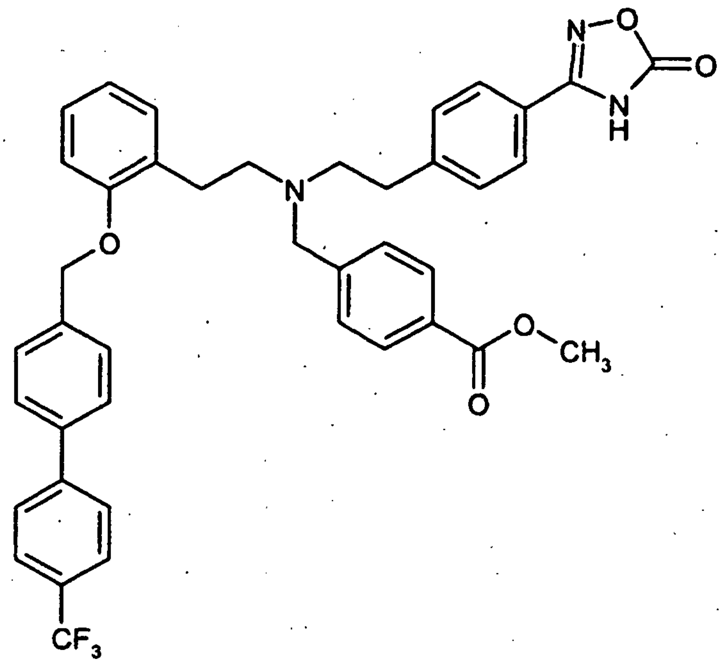

Eine Lösung von 80.34 mg (1.16 mmol) Hydroxylaminhydrochlorid in 5 ml DMSO wird mit 0.16 ml (1.16 mmol) Triethylamin versetzt und 10 min bei Raumtemperatur gerührt. Der anfallende Niederschlag wird abfiltriert. In das Filtrat werden anschließend 150 mg (0.23 mmol) 4-[([2-(4-Cyanophenyl)ethyl]-{2-[2-(4'-trifluoromethyl-biphenyl-4-ylmethoxy) phenyl]ethyl}amino)methyl]-benzoesäuremethylester aus Beispiel 10A zudosiert. Die Reaktionslösung wird 12 Stunden bei 75°C gerührt. Nach vollständigem Umsatz und Abkühlung werden zu der Reaktionslösung 10 ml Wasser gegeben, mit Essigsäureethylester dreimal extrahiert und die vereinigten organischen Phasen mit gesättigter Kochsalz-Lösung gewaschen. Anschließend wird die organische Phase über Natriumsulfat getrocknet und bis zur Trockene eingeengt. Es werden 162 mg eines farblosen Öls erhalten, das ohne weitere Reinigung direkt weiter umgesetzt wird.

Das oben erhaltene Öl wird in 10 ml DMF gelöst und mit 0.02 ml (0.26 mmol) Pyridin versetzt. Anschließend wird die Lösung auf 0°C abgekühlt und langsam mit 46 mg (0.24 mmol) Chlorameisensäure-2-ethylhexylester versetzt. Der Ansatz wird ca. 30 min bei 0°C nachgerührt, dann in Wasser aufgenommen und mit Essigsäureethylester extrahiert. Die organische Phase wird über Natriumsulfat getrocknet und bis zur Trockene eingeengt. Der erhaltene Rückstand wird in 20 ml Xylol aufgenommen und 2 Stunden zum Rückfluss erhitzt. Nach vollständiger Umsetzung wird die Reaktionslösung abgekühlt und bis zur Trockene eingeengt. Das Rohprodukt wird mittels präparativer HPLC gereinigt. Man erhält 32.7 mg (0.05 mmol, 19% Ausbeute) eines farblosen Öls.

LC-MS (Methode 2): R, 2.49 min; m/z 708 ($M+H^+$).

**Beispiel 12A**

2-(4-Methoxycarbonyl-benzyl)-malonsäurediallylester

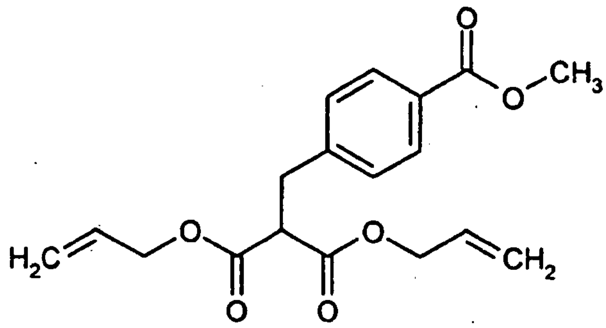

Eine Lösung von 56.7 g (0.3 mol) Malonsäurediallylester in 375 ml Dioxan und 75 ml THF wird bei 0°C portionsweise mit 14.42 g (0.36 mol) Natriumhydrid versetzt. Nach Erwärmen auf Raumtemperatur wird der Ansatz 1 Stunde bei 40°C gerührt. Anschließend werden 111.88 g (0.6 mol) 4-Chlormethyl-benzoesäuremethylester, gelöst in 375 ml Dioxan, langsam bei 40°C zugetropft und die Reaktionslösung über Nacht bei 110°C gerührt. Nach Abkühlen auf Raumtemperatur wird das Reaktionsgemisch auf 1200 ml Wasser gegeben. Dabei ist darauf zu achten, dass der pH-Wert < 7 ist (gegebenenfalls werden wenige ml 1 M Salzsäure bis ca. pH 2 zudosiert). Der Ansatz wird dann dreimal mit Essigsäureethylester extrahiert, die vereinigten organischen Phasen mit gesättigter Natriumchlorid-Lösung gewaschen und über Natriumsulfat getrocknet. Nach der Filtration wird das Lösungsmittel im Vakuum bis zur Trockene eingeengt. Das erhaltene Rohprodukt wird durch Flash-Chromatographie (Laufmittel: Petrolether/Essigsäureethylester 10:1; 3 kg Kieselgel) gereinigt. Es werden 85.4 g (0.26 mol, 85% Ausbeute) eines farblosen Feststoffes erhalten.

$^1$H-NMR (300 MHz, $CDCl_3$, $\delta$/ppm): 7,96 (2H, d), 7,29 (2H, d), 5,91-5,74 (2H, m), 5,32-5,17 (4H, m), 4,59 (4H, d), 3,93 (3H, s), 3,74 (1H, t), 3,31 (2H, d).

MS (DCI): 349 ($M+NH_4^+$).

**Beispiel 13A**

2-[2-(4-Cyanophenyl)ethyl]-2-(4-methoxycarbonyl-benzyl)-malonsäurediallylester

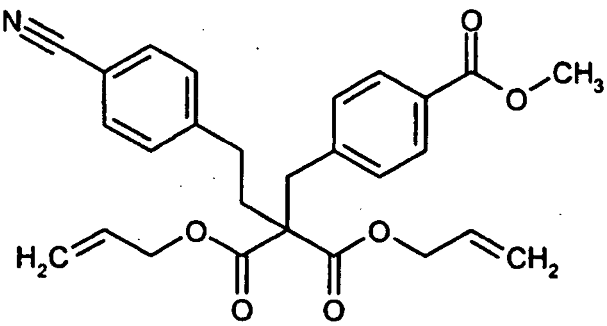

Eine Lösung von 55.71 g (0.17 mol) 2-(4-Methoxycarbonyl-benzyl)-malonsäurediallylester aus Beispiel 12A in 34 ml DMF wird bei 0°C portionsweise mit 6.70 g (0.17 mol) Natriumhydrid versetzt. Anschließend lässt man die Reaktionslösung auf Raumtemperatur kommen und rührt 1 Stunde nach. Nachfolgend wird die Reaktionslösung auf 0°C abgekühlt, mit 42.98 g (0.20 mol) 4-(2-Bromethyl)benzonitril aus Beispiel 8A in 21 ml DMF versetzt und 30 min bei dieser Temperatur nachgerührt. Der Ansatz wird danach bei Raumtemperatur über Nacht gerührt. Das Reaktionsgemisch wird tropfenweise mit Wasser versetzt, dreimal mit Essigsäureethylester extrahiert, die vereinigten organischen Phasen mit gesättigter Kochsalz-Lösung gewaschen und über Natriumsulfat getrocknet. Nach der Filtration wird das Lösungsmittel'im Vakuum bis zur Trockene eingeengt. Das erhaltene Rohprodukt wird durch Flash-Chromatographie (Laufmittel: Petrolether/ Essigsäureethylester 3:1; 3 kg Kieselgel) gereinigt: Es werden 36 g (0.078 mol, 46% Ausbeute) eines farblosen Feststoffes erhalten.

$^1$H-NMR (300 MHz, $CDCl_3$, $\delta$/ppm): 7,95 (2H, d), 7,55 (2H, d), 7,21 (4H, t), 5,97-5,69 (2H, m), 5,40-5,23 (4H, m), 4,62 (4H, d), 3,92 (3H, s), 3,40 (2H, s), 2,72-2,61 (2H, m), 2,13-2,01 (2H, m).

MS (DCI): 479 ($M+NH_4^+$).

**Beispiel 14A**

4-[2-Carboxy-4-(4-cyanophenyl)butyl]benzoesäuremethylester

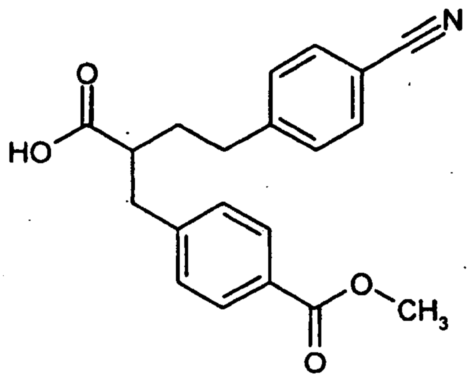

Eine Lösung von 43.5 g (0.09 mol) 2-[2-(4-Cyanophenyl)ethyl]-2-(4-methoxycarbonyl-benzyl)-malonsäurediallylester aus Beispiel 13A, 1.67 g (0.01 mol) Triphenylphosphin und 410 mg Palladiumacetat in 505 ml Dioxan wird bei Raumtemperatur mit einer Lösung von 41.8 ml (0.3 mol) Triethylamin und 8.6 ml (0.23 mol) Ameisensäure in 500 ml Dioxan versetzt. Das Reaktionsgemisch wird anschließend bei 100°C 2 Stunden lang gerührt. Nach vollständigem Umsatz wird die Reaktionslösung abgekühlt und das Lösungsmittel im Vakuum entfernt. Der erhaltene Rückstand wird durch Flash-Chromatographie an Kieselgel (Laufmittel: Dichlormethan/Methanol 50:1) gereinigt. Es werden 25 g (0.074 mol, 82% Ausbeute) eines farblosen Feststoffes erhalten.

$^1$H-NMR (400 MHz, DMSO-$d_6$, $\delta$/ppm): 12,55-12,24 (1H, breit), 7,86 (2H, d), 7,72 (2H, d), 7,38 (2H, d), 7,32 (2H, d), 3,84 (3H, s), 2,99-2,81 (2H, m), 2,78-2,55 (3H, m), 1,90-1,67 (2H, m).

MS (ESI): 338 (M+H$^+$).

**Beispiel 15A**

4-[4-(4-Cyanophenyl)-2-hydroxymethyl-butyl]benzoesäuremethylester

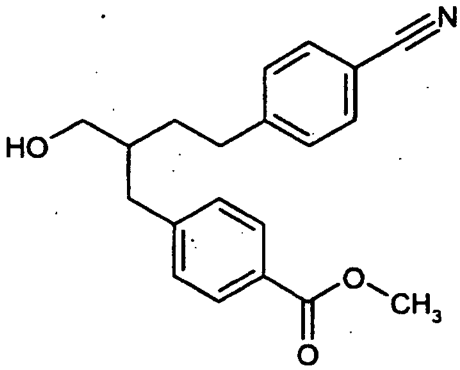

Zu einer Lösung von 5.32 g (77% Reinheit, 12.14 mmol) 4-[2-Carboxy-4-(4-cyanophenyl)-butyl]-benzoesäuremethylester aus Beispiel 14A in 40 ml THF werden 24.28 ml einer 1 M Boran-THF-Komplex-Lösung (24.28 mmol) bei -15°C getropft und die Lösung 4 h bei dieser Temperatur nachgerührt. Nach vollständiger Umsetzung wird das Reaktionsgemisch mit gesättigter Natriumhydrogencarbonat-Lösung versetzt und das Lösungsmittel bis zur Trockene eingeengt. Der Rückstand wird in Dichlormethan aufgenommen, über Natriumsulfat getrocknet und nach Filtration wiederum vom Lösungsmittel befreit. Das erhaltene Rohprodukt wird durch Flash-Chromatographie an Kieselgel (Laufmittel: Cyclohexan/Essigsäureethylester 4:1) gereinigt. Es werden 2.25 g (55% Ausbeute) eines farblosen Feststoffes erhalten.

$^1$H-NMR (300 MHz, DMSO-$d_6$, $\delta$/ppm): 7,88 (2H, d), 7,71 (2H, d), 7,46 (4H, t), 4,54 (1H, t), 3,83 (3H, s), 3,41 (2H, t), 2,80-2,55 (4H, m), 1,79-1,39 (3H, m).

MS (ESI): 324 (M+H$^+$).

**Beispiel 16A**

4-[4-(4-Cyanophenyl)-2-formyl-butyl]benzoesäuremethylester

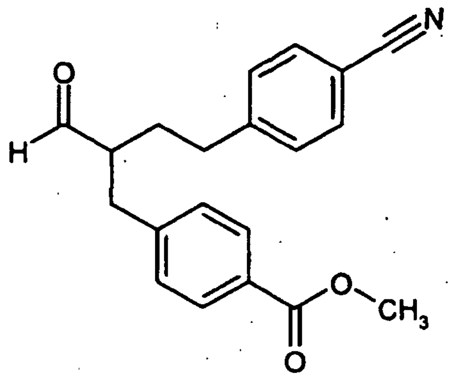

Eine Lösung von 5.7 g (17.63 mmol) 4-[4-(4-Cyanophenyl)-2-hydroxymethyl-butyl]-benzoesäure-methylester aus Beispiel 15A in 250 ml Dichlormethan wird mit 4.56 g (21.15 mmol) Pyridiniumchlorochromat (PCC) versetzt und 5 Stunden bei Raumtemperatur gerührt. Nach vollständigem Umsatz werden ca. 10 g Kieselgel zugegeben und das Lösemittel im Vakuum bis zur Trockene entfernt. Der Rückstand wird durch Flash-Chromatographie an Kieselgel (Laufmittel: Cyclohexan/ Essigsäureethylester 10:1 → 4:1) gereinigt. Es werden 4.16 g (12.94 mmol, 73% Ausbeute) eines farblosen Feststoffes erhalten.

$^1$H-NMR (300 MHz, DMSO-$d_6$, δ/ppm): 9,68 (1H, s), 7,88 (2H, d), 7,73 (2H, d), 7,47 (4H, dd), 3,86 (3H, s), 3,14-3,02 (1H, m), 2,92-2,80 (1H, m), 2,78-2,54 (3H, m), 1,98-1,81 (1H, m), 1,76-1,60 (1H, m).

MS (DCI): 339 (M+$NH_4^+$).

**Beispiel 17A**

*E*-4-[2-[2-(4-Cyanophenyl)ethyl]-4-(2-hydroxyphenyl)-but-3-enyl]benzoesäuremethylester

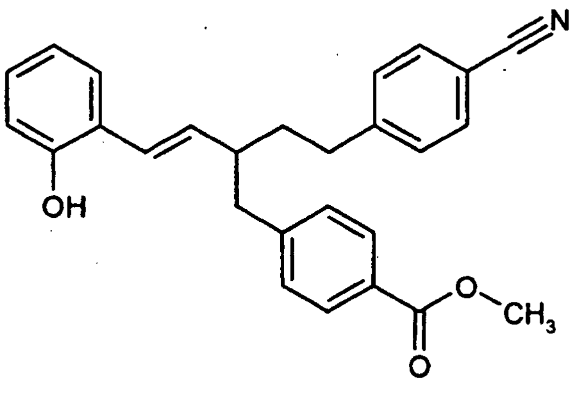

Zu einer Lösung von 2.459 g (5.36 mmol) (2-Hydroxybenzyl)-triphenylphosphoniumbromid in 65 ml wasserfreiem THF werden bei 0°C 9.39 ml (15.02 mmol) einer 1.6 M Lösung von n-Butyl-lithium in Hexan langsam hinzugefügt. Anschließend werden bei dieser Temperatur 1.744 g (5.36 mmol) 4-[4-(4-Cyanophenyl)-2-formyl-butyl]benzoesäuremethylester aus Beispiel 16A, gelöst in 65 ml THF, langsam zudosiert. Nach Erwärmung auf Raumtemperatur wird die Reaktionslösung 12 Stunden nachgerührt und dann mit etwas Wasser versetzt und bis zur Trockene eingeengt. Der erhaltene Rückstand wird in Essigsäureethylester aufgenommen, mit Wasser und gesättigter Kochsalz-Lösung gewaschen und über Natriumsulfat getrocknet. Nach Filtration wird das Lösungsmittel bis zur Trockene eingeengt. Das Rohprodukt wird durch Flash-Chromatographie an Kieselgel (Laufmittel: Cyclohexan/Essigsäureethylester 4: 1) gereinigt. Es werden 1.44 g (3.50 mmol, 65% Ausbeute) eines farblosen Feststoffes erhalten.

$^1$H-NMR (300 MHz, DMSO-$d_6$, δ/ppm): 9,39 (1H, s), 7,82 (2H, d), 7,60 (2H, d), 7,41-7,27 (5H, m), 7,01 (1H, t), 6,81-6,68 (2H, m), 6,45 (1H, d), 6,13-5,99 (1H, m), 3,81 (3H, s), 2,92-2,58 (5H, m), 1,86-1,56 (2H, m).

MS (DCI): 429 ($M+NH_4^+$).

**Beispiel 18A**

*E*-4-{4-[2-(4-Brombenzyloxy)phenyl]-2-[2-(4-cyanophenyl)ethyl]-but-3-enyl}benzoesäuremethylester

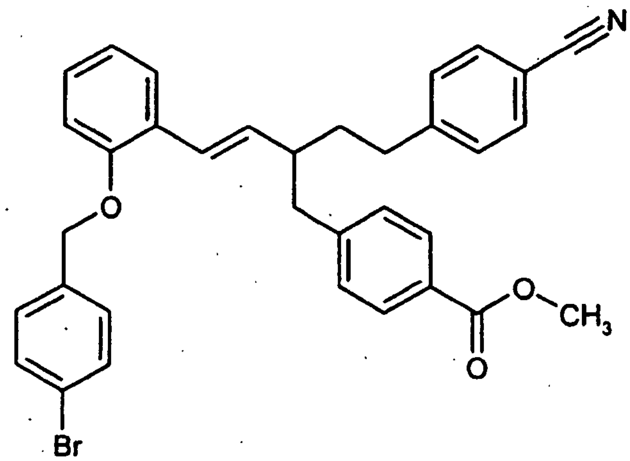

Eine Lösung von 230 mg (0.56 mmol) *E*-4-[2-[2-(4-Cyanophenyl)ethyl]-4-(2-hydroxyphenyl)-but-3-enyl]-benzoesäuremethylester aus Beispiel 17A in 5 ml trockenem Acetonitril wird mit 209 mg (0.84 mmol) 4-Brombenzylbromid und 116 mg (0.84 mmol) wasserfreiem Kaliumcarbonat versetzt und 12 Stunden zum Rückfluss erhitzt. Anschließend wird der Ansatz zur Trockene eingeengt. Der Rückstand wird in Essigsäureethylester aufgenommen, mit Wasser und gesättigter Kochsalz-Lösung gewaschen und über Natriumsulfat getrocknet. Die erhaltene organische Phase wird eingeengt. Das Rohprodukt wird durch Flash-Chromatographie an Kieselgel (Laufmittel: Cyclohexan) gereinigt. Es werden 286 mg (0.49 mmol, 88% Ausbeute) eines Feststoffs erhalten.

$^1$H-NMR (400 MHz, DMSO-$d_6$, δ/ppm): 7,82 (2H, d), 7,68 (2H, d), 7,54 (2H, d), 7,40 (1H, d), 7,38-7,25 (6H, m), 7,18 (1H, t), 7,00 (1H, d), 6,90 (1H, t), 6,44 (1H, d), 6,09 (1H, dd), 5,06 (2H, s), 3,81 (3H, s), 2,91-2,82 (1H, m), 2,79-2,69 (2H, m), 2,68-2,56 (1H, m), 2,54-2,41 (1H, m), 1,85-1,72 (1H, m), 1,71-1,59 (1H, m).

MS (ESI): 602 ($M+Na^+$), 597 ($M+NH_4^+$).

**Beispiel 19A**

*E*-4-{2-[2-(4-Cyanophenyl)ethyl]-4-[2-(4'-trifluormethyl-biphenyl-4-ylmethoxy)phenyl]-but-3-enyl}benzoesäuremethylester

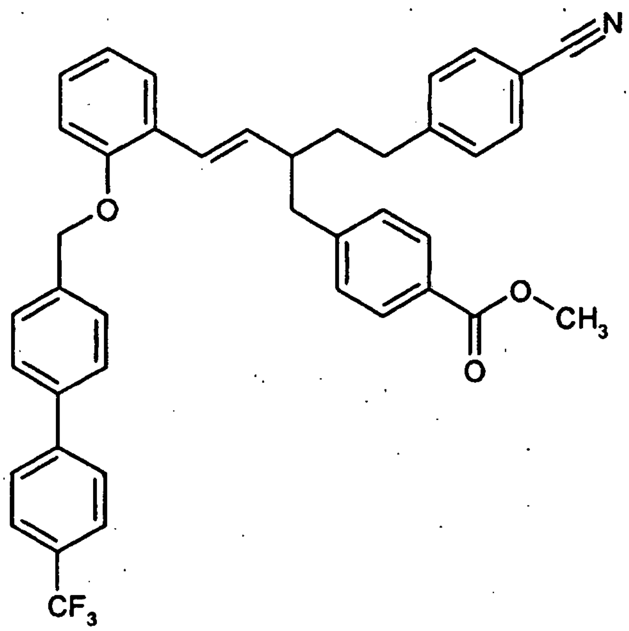

270 mg (0.47 mmol) *E*-4-{4-[2-(4-Brombenzyloxy)phenyl]-2-[2-(4-cyanophenyl)ethyl]-but-3-enyl}benzoesäuremethylester aus Beispiel 18A werden in 3 ml 1,2-Dimethoxyethan gelöst und unter Argon mit 106 mg (0.56 mmol) 4-Trifluormethylphenylboronsäure, 3.3 mg Bis(triphenylphosphin)palladium(II)chlorid und 0.51 ml einer 2 M Lösung von Natriumcarbonat in Wasser versetzt. Das Reaktionsgemisch wird anschließend 12 Stunden unter Rückfluss gerührt. Nachfolgend wird der Ansatz abgekühlt, über 1 g Extrelut filtriert, mit Dichlormethan gewaschen und eingeengt. Das erhaltene Rohprodukt wird durch präparative HPLC gereinigt. Es werden 175 mg (0.27 mmol, 58% Ausbeute) eines farblosen Feststoffes erhalten.

$^1$H-NMR (400 MHz, DMSO-$d_6$, $\delta$/ppm): 7,91 (2H, d), 7,71 (4H, s), 7,59 (2H, d), 7,50-7,41 (4H, m), 7,39 (1H, d), 7,22-7,12 (5H, m), 6,99-6,89 (2H, m), 6,66 (1H, d), 6,00 (1H, dd), 5,12 (2H, s), 3,87 (3H, s), 2,79 (2H, d), 2,78-2,70 (1H, m), 2,67-2,54 (1H, m), 2,53-2,42 (1H, m), 1,89-1,74 (1H, m), 1,73-1,58 (1H, m).

MS (ESI): 668 ($M+Na^+$), 663 ($M+NH_4^+$).

## Beispiel 20A

*E*-4-{2-{2-[4-(5-Oxo-4,5-dihydro-[1,2,4]oxadiazol-3-yl)phenyl]ethyl}-4-[2-(4'-trifluormethyl-biphenyl-4-ylmethoxy)phenyl]-but-3-enyl}benzoesäuremethylester

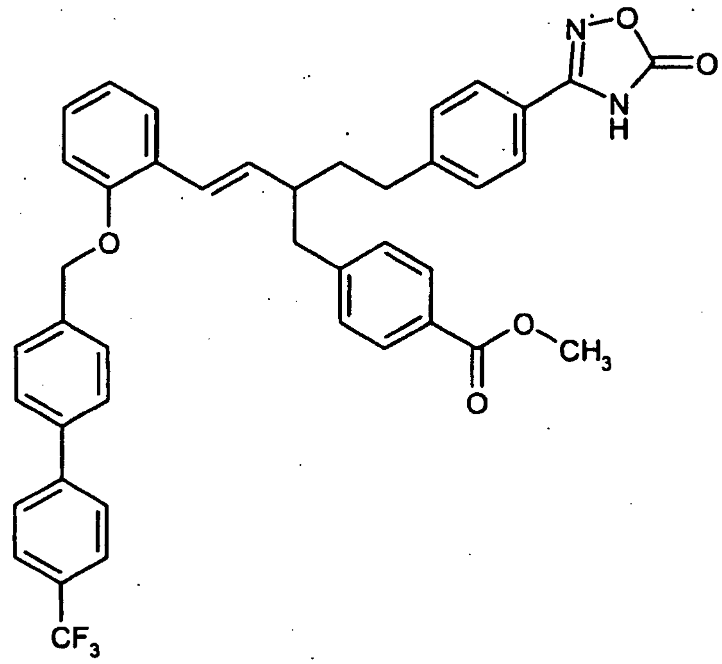

Eine Lösung von 96.8 mg (1.39 mmol) Hydroxylaminhydrochlorid in 18 ml DMSO wird mit 0.19 ml (1.39 mmol) Triethylamin versetzt und 10 min bei Raumtemperatur gerührt. Der anfallende Niederschlag wird abfiltriert. In das Filtrat werden anschließend 180 mg (0.28 mmol) *E*-4-{2-[2-(4-Cyanophenyl)ethyl]-4-[2-(4'-trifluormethyl-biphenyl-4-ylmethoxy) phenyl]-but-3-enyl}-benzoesäuremethylester aus Beispiel 19A zudosiert. Die Reaktionslösung wird dann 12 Stunden bei 75°C gerührt. Nach vollständigem Umsatz und Abkühlung werden zu der Reaktionslösung 10 ml Wasser gegeben, dreimal mit Essigsäureethylester extrahiert und die vereinigten organischen Phasen mit gesättigter Kochsalz-Lösung gewaschen. Anschließend wird die organische Phase über Natriumsulfat getrocknet und bis zur Trockene eingeengt. Es werden 213 mg eines farblosen Öls erhalten, das ohne weitere Reinigung direkt weiter umgesetzt wird.

Das oben erhaltene Öl wird in 8 ml DMF gelöst und mit 0.02 ml (0.29 mmol) Pyridin versetzt. Anschließend wird die Lösung auf 0°C gekühlt und langsam mit 47.1 mg (0.26 mmol) Chlorameisensäure-2-ethylhexylester versetzt. Der Ansatz wird ca. 30 min bei 0°C nachgerührt, in Wasser aufgenommen und mit Essigsäureethylester extrahiert. Die organische Phase wird über Natriumsulfat getrocknet und bis zur Trockene eingeengt. Der erhaltene Rückstand wird in 8 ml Xylol aufgenommen und 4 Stunden zum Rückfluss erhitzt. Nach vollständiger Umsetzung wird die Reaktionslösung abgekühlt und bis zur Trockene eingeengt. Das Rohprodukt wird mittels präparativer HPLC gereinigt. Man erhält 110 mg (0.15 mmol, 57% Ausbeute) eines farblosen Öls.

$^1$H-NMR (300 MHz, DMSO-$d_6$, δ/ppm): 12,86 (1H, s, breit), 7,90-7,75 (6H, m), 7,74-7,62 (4H, m), 7,50 (2H, d), 7,43 (1H, d), 7,33 (4H, t), 7,19 (1H, t), 7,07 (1H, d), 6,91 (1H, t), 6,50 (1H, d), 6,12 (1H, dd), 5,16 (2H, s), 3,76 (3H, s), 2,94-2,82 (1H, m), 2,81-2,56 (3H, m), 2,55-2,43 (1H, m), 1,90-1,59 (2H, m).

MS (ESI): 727 (M+Na$^+$).

__Beispiel 21A__

4'-Trifluormethyl-biphenyl-4-carbonsäureethylester

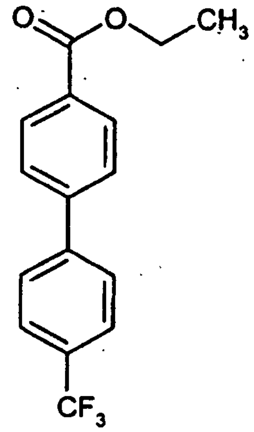

7 g (30.56 mmol) 4-Brombenzoesäureethylester werden in 60 ml 1,2-Dimethoxyethan gelöst und unter Argon mit 6.96 g (36.67 mmol) 4-Trifluormethylphenylboronsäure, 271 mg Bis(triphenylphosphin)palladium(II)chlorid und 40.7 ml einer 2 M Lösung von Natriumcarbonat in Wasser versetzt. Das Reaktionsgemisch wird anschließend 12 Stunden unter Rückfluss erhitzt. Nachfolgend wird der Ansatz abgekühlt, über 1 g Extrelut filtriert, mit Dichlormethan gewaschen und eingeengt. Das Rohprodukt wird durch Flash-Chromatographie an Kieselgel (Laufmittel: Cyclohexan/Dichlormethan 2:1) gereinigt. Es werden 6.31 g (21.4 mmol, 70% Ausbeute) eines farblosen Feststoffes erhalten.

$^1$H-NMR (300 MHz, $CDCl_3$, δ/ppm): 8,17 (2H, d), 7,72 (4H, s), 7,67 (2H, d), 4,41 (2H, q), 1,43 (3H, t).

MS (EI): 294 ($M^+$).

**Beispiel 22A**

(4'-Trifluormethyl-biphenyl-4-yl)-methanol

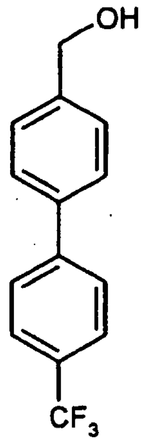

Eine Lösung von 6.24 g (21.21 mmol) 4'-Trifluormethyl-biphenyl-4-carbonsäureethylester aus Beispiel 21A in 60 ml trockenem THF wird bei 0°C tropfenweise mit 12.73 ml (12.73 mmol) einer 1 M Lösung von Lithiumaluminiumhydrid in THF versetzt. Nach vollständiger Umsetzung wird der Ansatz mit gesättigter Ammoniumchlorid-Lösung versetzt, in Essigsäureethylester aufgenommen, die organische Phase abgetrennt und über Natriumsulfat getrocknet. Nach Filtration wird das Lösemittel im Vakuum entfernt. Das erhaltene Rohprodukt wird durch Flash-Chromatographie an Kieselgel (Laufmittel: Cyclohexan/Essigsäureethylester 5:1) gereinigt. Es werden 5.1 g (20.21 mmol, 95% Ausbeute) eines farblosen Feststoffes erhalten.

$^1$H-NMR (300 MHz, DMSO-$d_6$, δ/ppm): 7,88 (2H, d), 7,82 (2H, d), 7,71 (2H, d), 7,46 (2H, d), 5,23 (1H, t), 4,58 (2H, d).

MS (EI): 252 ($M^+$).

**Beispiel 23A**

4-Chlormethyl-4'-trifluormethyl-biphenyl

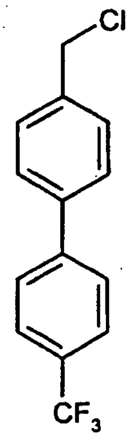

Eine Lösung von 5.0 g (19.82 mmol) (4'-Trifluormethyl-biphenyl-4-yl)-methanol aus Beispiel 22A in 40 ml Chloroform wird mit 2.89 ml (39.65 mmol) Thionylchlorid, gelöst in 10 ml Chloroform, versetzt und 12 Stunden lang bei Raumtemperatur gerührt. Nach vollständiger Umsetzung wird das Reaktionsgemisch bis zur Trockene eingeengt, der Rückstand in Essigsäureethylester aufgenommen und mit gesättigter Natriumcarbonat-Lösung gewaschen. Die organische Phase wird anschließend abgetrennt, über Natriumsulfat getrocknet und nach Filtration eingeengt. Das Rohprodukt wird durch Flash-Chromatographie an Kieselgel (Laufmittel: Cyclohexan/Essigsäureethylester 9:1) gereinigt. Es werden 5.26 g (19.43 mmol, 98% Ausbeute) eines farblosen Feststoffes erhalten.
$^1$H-NMR (300 MHz, DMSO-$d_6$, $\delta$/ppm): 7,91 (2H, d), 7,82 (2H, d), 7,78 (2H, d), 7,58 (2H, d), 4,83 (2H, s).
MS (EI): 270 ($M^+$).

**Beispiel 24A**

[2-(4'-Trifluormethyl-biphenyl-4-ylmethoxy)phenyl]methanol

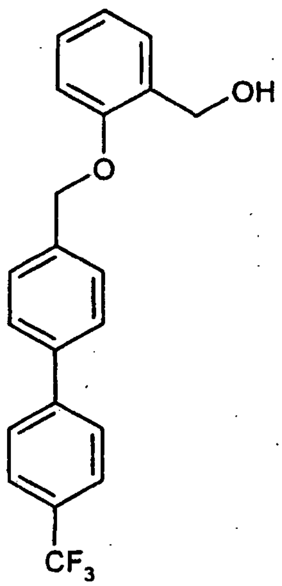

Eine Lösung von 4.59 g (36.94 mmol) 2-Hydroxybenzylalkohol in 200 ml trockenem Acetonitril wird mit 10 g

(36.94 mmol) 4-Chlormethyl-4'-trifluormethyl-biphenyl aus Beispiel 23A und 6.13 g (44.33 mmol) wasserfreiem Kaliumcarbonat versetzt und 12 Stunden zum Rückfluss erhitzt. Anschließend wird der Ansatz zur Trockene eingeengt. Der Rückstand wird in Essigsäureethylester aufgenommen, mit Wasser und gesättigter Kochsalz-Lösung gewaschen und über Natriumsulfat getrocknet. Die erhaltene organische Phase wird eingeengt. Das Rohprodukt wird durch Flash-Chromatographie an Kieselgel (Laufmittel: Cyclohexan/Essigsäureethylester 5:1) gereinigt. Es werden 11.8 g (32.92 mmol, 89% Ausbeute) eines Feststoffs erhalten.

$^1$H-NMR (300 MHz, DMSO-$d_6$, δ/ppm): 7,92 (2H, d), 7,87-7,72 (4H, m), 7,60 (2H, d), 7,41 (1H, d), 7,21 (1H, t), 7,03 (1H, d), 6,96 (1H, t), 5,20 (2H, s), 5,03 (1H, t), 4,58 (2H, d).

MS (DCI): 376 ($M+NH_4^+$).

**Beispiel 25A**

Triphenyl-[2-(4'-trifluormethyl-biphenyl-4-ylmethoxy)benzyl]-phosphoniumbromid

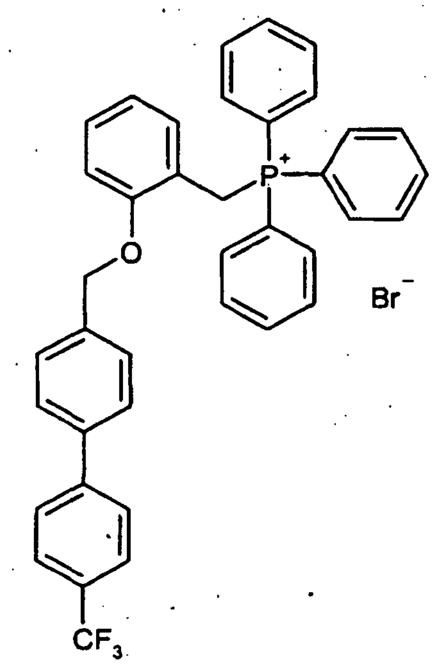

Eine Lösung von 11.7 g (32.65 mmol) [2-(4'-Trifluormethyl-biphenyl-4-ylmethoxy)phenyl]-methanol aus Beispiel 24A in 100 ml Acetonitril wird mit 10.64 g (31.02 mmol) Triphenylphosphoniumhydrobromid versetzt und 3 Stunden zum Rückfluss erhitzt. Anschließend wird die Reaktionslösung bis zur Trockene eingeengt und das erhaltene Öl in Diethylether aufgenommen und verrieben. Dabei kristallisiert das Produkt als weißer Feststoff aus. Nach Filtration wird der Feststoff bei 50°C im Trockenofen über Nacht getrocknet. Es werden 20.5 g (30 mmol, 92% Ausbeute) kristallines Produkt erhalten.

$^1$H-NMR (300 MHz, DMSO-$d_6$, δ/ppm): 7,99-7,79 (8H, m), 7,78-7,50 (12H, m), 7,32 (4H, d), 7,08 (1H, d), 6,97 (1H, d), 6,86 (1H, t), 5,03 (2H, d), 4,70 (2H, s).

**Beispiel 26A**

(5-Brompentyl)-benzol

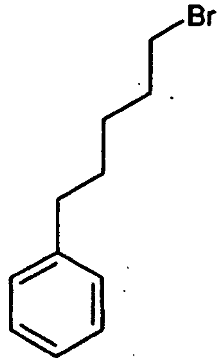

Eine Lösung von 416.7 ml (1.83 mol) 48%-ige Bromwasserstoffsäure wird mit 50 g (0.304 mol) 5-Phenylpentan-1-ol bei 0°C versetzt und 30 min bei 0°C nachgerührt. Anschließend wird die Reaktionslösung 12 Stunden bei 100°C gerührt. Nach vollständiger Umsetzung wird der Ansatz auf Raumtemperatur abgekühlt und mit 200 ml Essigsäureethylester versetzt. Nach Extraktion wird die organische Phase abgetrennt, mit gesättigter Natriumhydrogencarbonat-Lösung gewaschen und über Natriumsulfat getrocknet. Nach Filtration wird das Filtrat bis zur Trockene eingeengt. Das erhaltene Rohprodukt wird durch Flash-Chromatographie an Kieselgel (Laufmittel: Cyclohexan) gereinigt. Es werden 59.4 g (0.26 mol, 86% Ausbeute) einer farblosen Flüssigkeit erhalten.
$^1$H-NMR (300 MHz, $CDCl_3$, δ/ppm): 7,32-7,22 (2H, m), 7,21-7,11 (3H, m), 3,40 (2H, t), 2,61 (2H, t), 1,97-1,81 (2H, m), 1,72-1,58 (2H, m), 1,56-1,39 (2H, m).
MS (CI): 226 ($M^+$).

**Beispiel 27A**

[2-(5-Phenylpentyloxy)phenyl]methanol

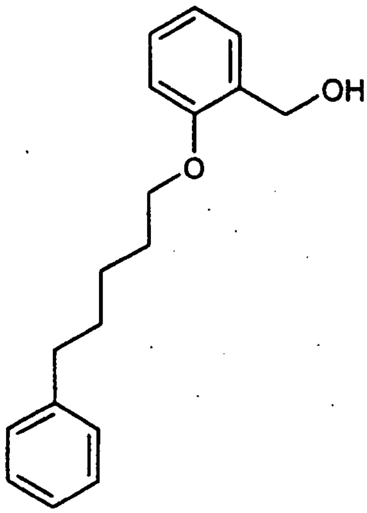

Eine Lösung von 10 g (80.56 mmol) 2-Hydroxybenzylalkohol in 200 ml trockenem Acetonitril wird mit 27.45 g (120.83 mmol) (5-Brompentyl)-benzol aus Beispiel 26A und 12.25 g (88.61 mmol) wasserfreiem Kaliumcarbonat versetzt und 12 Stunden unter Rückfluss erhitzt. Anschließend wird der Ansatz zur Trockene eingeengt. Der Rückstand wird in Essigsäureethylester aufgenommen, mit Wasser und gesättigter Kochsalz-Lösung gewaschen und über Natriumsulfat getrocknet. Die erhaltene organische Phase wird eingeengt. Das Rohprodukt wird durch Flash-Chromatographie an Kieselgel (Laufmittel: Cyclohexan/Essigsäureethylester 10:1) gereinigt. Es werden 18.7 g (81% Ausbeute) eines farblosen Feststoffes erhalten.
$^1$H-NMR (300 MHz, DMSO-$d_6$, δ/ppm): 7,38 (1H, d), 7,31-7,10 (6H, m), 6,91 (2H, t), 4,92 (1H, t), 4,50 (2H, d), 3,95 (2H, t), 2,59 (2H, t), 1,81-1,68 (2H, m), 1,67-1,55 (2H, m), 1,52-1,36 (2H, m).
MS (CI): 288 ($M+NH_4^+$), 270 ($M^+$).

**Beispiel 28A**

Triphenyl-[2-(5-phenylpentyloxy)benzyl]-phosphoniumbromid

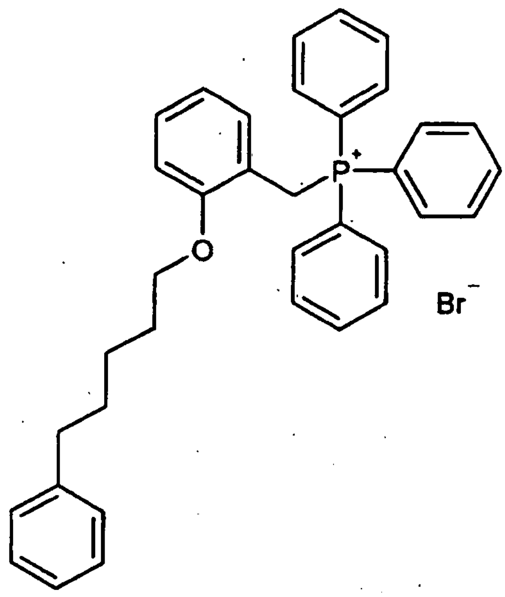

Eine Lösung von 18.7 g (69.16 mmol) [2-(5-Phenylpentyloxy)phenyl]methanol aus Beispiel 27A in 120 ml Acetonitril wird mit 22.55 g (65.71 mmol) Triphenylphosphoniumhydrobromid versetzt und drei Stunden lang zum Rückfluss erhitzt. Anschließend wird die Reaktionslösung bis zur Trockene eingeengt. Es werden 36.6 g (61.45 mmol, 83% Ausbeute) kristallines Produkt erhalten, welches ohne weitere Reinigung umgesetzt wird.

$^1$H-NMR (400 MHz, DMSO-$d_6$, δ/ppm): 7,89 (3H, t), 7,78-7,66 (6H, m), 7,64-7,52 (6H, m), 7,32-7,24 (3H, m), 7,21-7,12 (3H, m), 7,01 (1H, d), 6,89-6,77 (2H, m), 4,90 (2H, d), 3,44 (2H, t), 2,56 (2H, t), 1,59-1,46 (2H, m), 1,38-1,25 (2H, m), 1,23-1,12 (2H, m).

MS (ESI): 515 ($M^+$-Br).

**Beispiel 29A**

*E*-5-Fluor-2-[2-(4-methoxyphenyl)vinyl]benzaldehyd

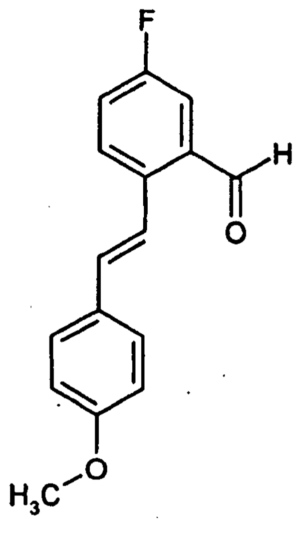

Eine Lösung von 10 g (49.26 mmol) 2-Brom-5-fluorbenzaldehyd in 200 ml trockenem DMF wird mit 7.27 g (54.18 mmol) 4-Methoxystyrol, 1.5 g (4.93 mmol) Tri-2-tolylphosphin, 330 mg (1.48 mmol) Palladium(II)acetat und 10.3 ml (73.89 mmol) Triethylamin unter Argon versetzt und 12 Stunden lang bei 100°C gerührt. Nach vollständiger Umsetzung wird die Reaktionslösung auf Raumtemperatur abgekühlt und bis zur Trockene eingeengt. Der erhaltene Rückstand wird in 100 ml Wasser aufgenommen und dreimal mit jeweils 50 ml Diethylether extrahiert. Die vereinigten organischen Phasen werden über Natriumsulfat getrocknet. Nach Filtration wird die Lösung bis zur Trockene eingeengt und der Rückstand durch Flash-Chromatographie an Kieselgel (Laufmittel: Cyclohexan/Essigsäureethylester 10:1) gereinigt. Es werden 7.79 g (55% Ausbeute) eines farblosen Feststoffes erhalten.

$^1$H-NMR (400 MHz, DMSO-$d_6$, $\delta$/ppm): 10,40 (1H, s), 8,01-7,89 (2H, m), 7,68-7,49 (4H, m), 7,22 (1H, d), 6,99 (2H, d), 3,80 (3H, s).

MS (DCI): 274 ($M+NH_4^+$).

**Beispiel 30A**

*E*-{5-Fluor-2-[2-(4-methoxyphenyl)vinyl]phenyl}methanol

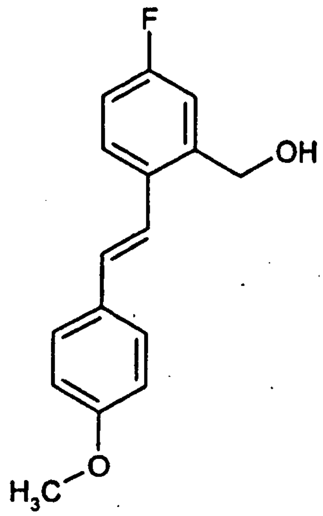

Eine Lösung von 7.79 g (30.40 mmol) *E*-5-Fluor-2-[2-(4-methoxyphenyl)vinyl)benzaldehyd aus Beispiel 29A in 500 ml Methanol wird bei 0°C portionsweise mit 1.73 g (45.60 mmol) Natriumborhydrid versetzt und anschließend 2 Stunden bei Raumtemperatur gerührt. Nach vollständiger Umsetzung wird der Ansatz bis zur Trockene eingeengt und dann in Wasser und Dichlormethan aufgenommen. Anschließend wird die wässrige Phase noch zweimal mit Dichlormethan extrahiert. Die vereinigten organischen Phasen werden über Natriumsulfat getrocknet, filtriert und bis zur Trockene eingeengt. Der erhaltene Rückstand wird durch Flash-Chromatographie an Kieselgel (Laufmittel: Cyclohexan/Essigsäureethylester 4:1 $\rightarrow$ 1:1) gereinigt. Es werden 6.8 g (85% Ausbeute) eines farblosen Feststoffes erhalten.

$^1$H-NMR (400 MHz, DMSO-$d_6$, $\delta$/ppm): 7,71 (1H, m), 7,54 (2H, d), 7,27-7,12 (2H, m), 7,11-6,99 (2H, m), 6,97 (2H, d), 5,47 (1H, t), 4,67 (2H, d), 3,79 (3H, s).

LC-MS (Methode 1): $R_t$ 2.49 min; m/z 259 ($M+H^+$).

**Beispiel 31A**

{5-Fluor-2-[2-(4-methoxyphenyl)ethyl]phenyl}methanol

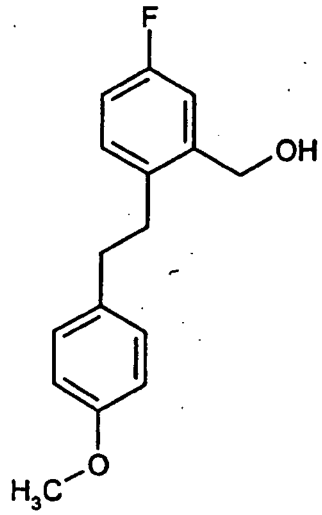

Eine Mischung von 6.8 g (26.33 mmol) *E*-{5-Fluor-2-[2-(4-methoxyphenyl)vinyl]phenyl}methanol aus Beispiel 30A und 0.5 g Palladium auf Kohle (10%-ig) in 50 ml Methanol und 250 ml Ethanol wird bei Raumtemperatur 1 Stunde lang unter Normaldruck hydriert. Nach Beendigung der Umsetzung wird der Ansatz über Kieselgur filtriert und das Filtrat anschließend bis zur Trockene eingeengt. Der Rückstand wird durch Flash-Chromatographie an Kieselgel (Laufmittel: Cyclo-hexan/Essigsäureethylester 10:1 → 4:1 → 1:1) gereinigt. Es werden 5.95 g (87% Ausbeute) eines farblosen Feststoffes erhalten.

$^1$H-NMR (400 MHz, DMSO-$d_6$, δ/ppm): 7,21-7,09 (4H, m), 6,97 (1H, t), 6,83 (2H, d), 5,24 (1H, t), 4,51 (2H, d), 3,72 (3H, s), 2,81-2,68 (4H, m).

LC-MS (Methode 1): $R_t$ 2.49 min; m/z 278 ($M+NH_4^+$).

**Beispiel 32A**

{5-Fluor-2-[2-(4-methoxyphenyl)ethyl]benzyl}-triphenylphosphoniumbromid

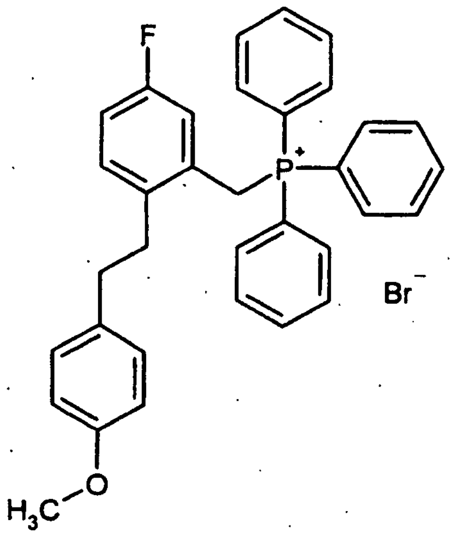

Eine Lösung von 5.95 g (22.86 mmol) {5-Fluor-2-[2-(4-methoxyphenyl)ethyl]phenyl}methanol aus Beispiel 31A in 130 ml Acetonitril wird mit 7.45 g (21.71 mmol) Triphenylphosphoniumhydrobromid versetzt und 3 Stunden lang zum Rückfluss erhitzt. Anschließend wird die Reaktionslösung bis zur Trockene eingeengt und das erhaltene Öl in Diethylether aufgenommen und verrieben. Dabei kristallisiert das Produkt als weißer Feststoff aus. Nach Filtration wird der Feststoff bei 50°C im Trockenofen über Nacht getrocknet. Es werden 11.5 g (77% Ausbeute) kristallines Produkt erhalten.

$^1H$-NMR (300 MHz, DMSO-$d_6$, δ/ppm): 7,92 (3H, t), 7,81-7,69 (6H, m), 7,68-7,57 (6H, m), 7,30-7,21 (1H, m), 7,19-7,08 (1H, m), 6,94 (2H, d), 6,81 (2H, d), 6,70-6,58 (1H, m), 4,94 (2H, d), 3,71 (3H, s), 2,57-2,46 (2H, t), 2,18 (2H, t).

**Beispiel 33A**

1-Brom-4-[brom(difluor)methyl]benzol

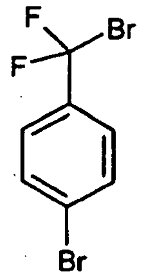

Unter Sauerstoffausschluss wird eine Lösung von 14.0 g (67.63 mmol) 1-Brom-4-(difluormethyl)-benzol [CAS 51776-71-7] und 25.3 g (142 mmol) *N*-Bromsuccinimid (NBS) in 190 ml Tetrachlorkohlenstoff mit einer Tageslichtlampe bestrahlt. Dabei erreicht das Lösemittel seine Siedetemperatur. Es wird 24 Stunden lang unter Rückfluss bestrahlt. Anschließend lässt man auf Raumtemperatur abkühlen und filtriert ausgefallenes Succinimid ab. Das Filtrat wird erneut mit 25 g NBS versetzt und nochmals für 24 Stunden unter Sauerstoffausschluss und Rückfluss bestrahlt. Nach dem Abkühlen wird wiederum filtriert und das Filtrat zur Trockene eingeengt. Es werden als Rohprodukt 18 g eines orangefarbenen Öls erhalten, das durch Vakuumdestillation bei 13 mm Hg weiter gereinigt wird. Es werden 12.7 g (44.4 mmol, 66% Ausbeute) eines farblosen Öls erhalten.

Siedepunkt (13 mm Hg): 90-92°C

$^1H$-NMR (300 MHz, DMSO-$d_6$, δ/ppm): 7,79 (2H, d), 7,63 (2H, d).

MS (ESI): 205/207 ($M-Br^+$).

**Beispiel 34A**

4-Fluor-2-(hydroxymethyl)phenol

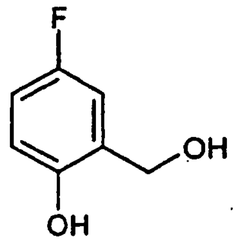

Unter Sauerstoffausschluss werden 27.1 g (159.28 mmol) Methyl-5-fluor-2-hydroxybenzoat in 500 ml trockenem THF vorgelegt und auf 0°C abgekühlt. Anschließend tropft man langsam unter Kühlung 238 ml (238 mmol) einer 1 M Lösung von Lithiumaluminiumhydrid in THF hinzu und rührt 1 Stunde bei 0°C und dann über Nacht bei RT nach. Nach vollständiger Umsetzung wird der Ansatz mit gesättigter Ammoniumchlorid-Lösung versetzt, in Methylenchlorid aufgenommen, die organische Phase abgetrennt und über Natriumsulfat getrocknet. Nach Filtration wird das Lösungsmittel im Vakuum entfernt. Das Rohprodukt wird über Kieselgel chromatographisch (Laufmittel: Cyclohexan/Essigsäureethylester 20:1) gereinigt. Es werden 18.0 g (126.6 mmol, 79% Ausbeute) eines farblosen Feststoffes isoliert.

$^1H$-NMR (400 MHz, DMSO-$d_6$, δ/ppm): 9,32 (1H, s), 7,06-7,03 (1H, m), 6,86-6,81 (1H, m), 6,74-6,71 (1H, m), 5,09 (1H, t), 4,45 (2H, d).

**Beispiel 35A**

4-Brom-2-fluorbenzoesäuremethylester

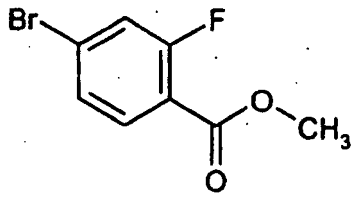

Eine Lösung von 8 g (36.53 mmol) 4-Brom-2-fluorbenzoesäure in 44 ml Methanol wird mit 0.46 ml (3.65 mmol) Chlortrimethylsilan versetzt und 12 h lang zum Rückfluss erhitzt. Anschließend wird der Ansatz eingeengt und der Rückstand mit Cyclohexan aufgenommen und über Kieselgel filtriert. Es werden 4.49 g (19.25 mmol, 52% Ausbeute) eines weißen Feststoff erhalten.

$R_f$(Cyclohexan/Essigsäureethylester 2:1): 0.5.

$^1$H-NMR (200 MHz, DMSO-$d_6$, $\delta$/ppm): 7,84 (1H, t), 7;75 (1H, dd), 7,58 (1H, dd), 3,88 (3H, s).

MS (EI): 232 ($M^+$).

**Beispiel 36A**

3-Fluor-4'-methoxy-1,1'-biphenyl-4-carbonsäuremethylester

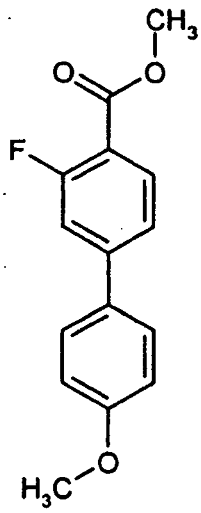

1.45 g (6.22 mmol) 4-Brom-2-fluorbenzoesäuremethylester aus Beispiel 35A werden in 15 ml 1,2-Dimethoxyethan gelöst und unter Argon mit 1.13 g (7.47 mmol) 4-Methoxybenzolboronsäure, 80 mg (0.11 mmol) Bis(triphenylphosphin)palladium(II)chlorid und 7 ml einer 2 M Lösung von Natriumcarbonat in Wasser versetzt. Das Reaktionsgemisch wird anschließend 12 h unter Rückfluss gerührt. Nachfolgend wird der Ansatz abgekühlt, über 10 g Extrelut filtriert, mit Dichlormethan gewaschen und eingeengt. Das erhaltene Rohprodukt wird durch Flash-Chromatographie (Kieselgel; Laufmittel: Cyclohexan/Essigsäureethylester 2:1) gereinigt. Es werden 1.36 g (5.23 mmol, 84% Ausbeute) eines weißen Feststoffs erhalten.

$R_f$(Cyclohexan/Essigsäureethylester 2:1): 0.44.

$^1$H-NMR (200 MHz, DMSO-$d_6$, $\delta$/ppm): 7,96 (1H, t), 7,80-7,62 (2H, m), 7,49-7,28 (3H, m), 7,02 (1H, dd), 3,89 (3H, s), 3,82 (3H, s).

MS (EI): 260 ($M^+$).

**Beispiel 37A**

(3-Fluor-4'-methoxy-1,1'-biphenyl-4-yl)methanol

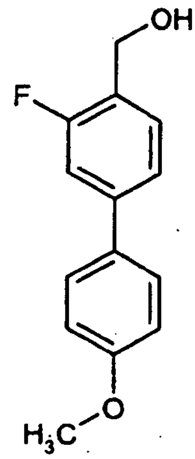

3.14 ml (3.14 mmol) einer 1 M Lösung von Lithiumaluminiumhydrid in wasserfreiem THF werden bei 0°C tropfenweise mit einer Lösung von 1.36 g (5.23 mmol) 3-Fluor-4'-methoxy-1,1'-biphenyl-4-Carbonsäuremethylester aus Beispiel 36A in 10 ml wasserfreiem THF versetzt. Es wird 2 h bei 0°C gerührt. Anschließend wird vorsichtig mit 10 ml gesättigter Ammoniumchlorid-Lösung versetzt, mit Essigsäureethylester verdünnt und die organische Phase abgetrennt. Die organische Phase wird nacheinander mit Wasser und gesättigter Kochsalz-Lösung gewaschen, über Natriumsulfat getrocknet und nach Filtration vom Lösungsmittel befreit. Das erhaltene Rohprodukt wird durch Flash-Chromatographie (Kieselgel; Laufmittel: Cyclohexan/Essigsäureethylester 5:1) gereinigt. Es werden 914 mg (3.94 mmol, 73% Ausbeute) der Titelverbindung erhalten.

$R_f$(Cyclohexan/Essigsäureethylester 2:1): 0.29.

$^1$H-NMR (300 MHz, DMSO-$d_6$, $\delta$/ppm): 7,58-7,45 (3H, m), 7,39 (1H, t), 7,29-7,20 (2H, m), 6,95 (1H, dd), 5,30 (1H, t), 4,58 (2H, d), 3,83 (3H, s).

MS (EI): 232 ($M^+$).

**Beispiel 38A**

4-(Chlormethyl)-3-fluor-4'-methoxy-1,1'-biphenyl

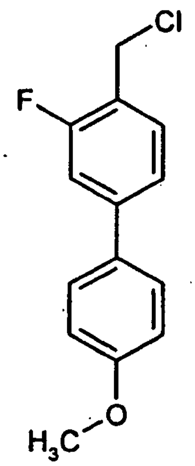

Eine Lösung von 864 mg (3.72 mmol) (3-Fluor-4'-methoxy-1,1'-biphenyl-4-yl)methanol aus Beispiel 37A in 3

ml Chloroform wird langsam mit 0.54 ml (7.45 mmol) Thionylchlorid, gelöst in 2 ml Chloroform, versetzt und 12 h bei Raumtemperatur gerührt. Anschließend wird der Ansatz eingeengt, mit Essigsäureethylester aufgenommen und mit Wasser und zweimal mit gesättigter Natriumhydrogencarbonat-Lösung gewaschen. Nach Trocknen über Magnesiumsulfat, Filtration und Einengen wird das Produkt durch Flash-Chromatographie (Kieselgel; Laufmittel: Cyclohexan/ Essigsäureethylester 100:1) gereinigt. Es werden 511 mg (2.04 mmol, 55% Ausbeute) eines farblosen Öls erhalten.

$R_f$(Cyclohexan/Essigsäureethylester 2:1): 0.52.

$^1$H-NMR (200 MHz, DMSO-$d_6$, δ/ppm): 7,68-7,52 (3H, m), 7,46-7,21 (3H, m), 6,99 (1H, dd), 4,86 (2H, s), 3,86 (3H, s).

MS (EI): 250 ($M^+$).

**Beispiel 39A**

{2-[(4-Bromphenyl)(difluor)methoxy]-5-fluorphenyl}methanol

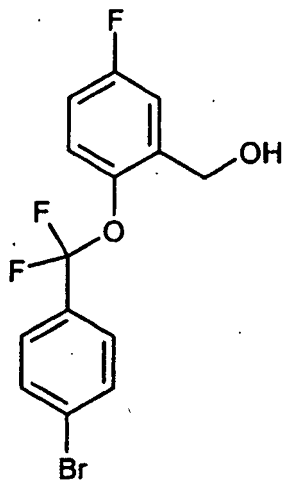

Eine Suspension von 1.17 g (8.23 mmol) 4-Fluor-2-(hydroxymethyl)phenol aus Beispiel 34A, 2.59 g (12.63 mmol) 1-Brom-4-[brom(difluor)methyl]benzol aus Beispiel 33A und 1.25 g (9.06 mmol) Kaliumcarbonat in 10 ml 2-Propanol wird 12 Stunden zum Rückfluss erhitzt. Anschließend wird der Ansatz zur Trockene eingeengt. Der Rückstand wird in Methylenchlorid suspendiert, auf Kieselgel aufgenommen, im Vakuum getrocknet und chromatographisch gereinigt (Laufmittel: Cyclohexan/Essigsäureethylester 10:1, anschließend 100% Essigsäureethylester). Man erhält 1.02 g (2.95 mmol, 35% Ausbeute) der Titelverbindung.

$^1$H-NMR (400 MHz, $CDCl_3$, δ/ppm): 7,65-7,58 (4H, m), 7,32-7,24 (2H, m), 7,03-6,96 (1H, m), 4,74 (2H, s), 1,73 (1H, s).

MS (DCI): 364/366 ($M+NH_4^+$).

**Beispiel 40A**

(2-{Difluor-[4'-(trifluormethyl)biphenyl-4-yl]methoxy}-5-fluorphenyl)methanol

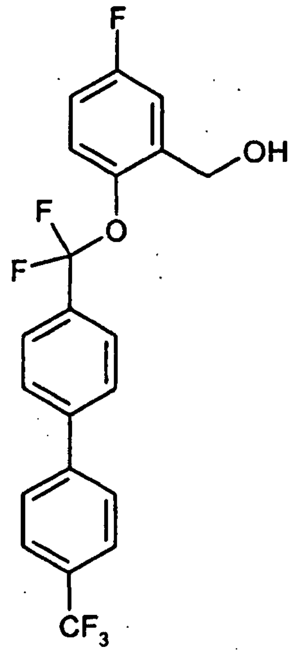

473 mg (1.36 mmol) {2-[(4-Bromphenyl)(difluor)methoxy]-5-fluorphenyl)methanol aus Beispiel 39A werden in 8 ml 1,2-Dimethoxyethan gelöst und unter Argon mit 362.6 mg (1.91 mmol) 4-Trifluormethylphenylboronsäure, 67 mg Bis(triphenylphosphin)palladium(II)chlorid und 1.5 ml einer 2 M Lösung von Natriumcarbonat in Wasser versetzt. Das Reaktionsgemisch wird anschließend 12 Stunden unter Rückfluss gerührt. Nachfolgend wird der Ansatz abgekühlt, über 5 g Celit filtriert, mit Dichlormethan gewaschen und eingeengt. Das erhaltene Rohprodukt wird mittels Kieselgelchromalographie (Laufmittel: Cyclohexan/Essigsäureethylester 10:1) gereinigt. Es werden 532 mg (1.29 mmol, 90% Reinheit, 85% Ausbeute) eines farblosen Öls erhalten.

$^1$H-NMR (300 MHz, $CDCl_3$, δ/ppm): 7,85-7,82 (2H, m), 7,76-7,69 (6H, m), 7,37-7,27 (2H, m), 7,04-6,98 (1H, s), 4,79 (2H, s), 1,76 (1H, s).

MS (DCI): 430 ($M+NH_4^+$).

**Beispiel 41A**

2-(Brommethyl)-4-fluorphenyl-difluor[4'-(trifluormethyl)biphenyl-4-yl]methylether

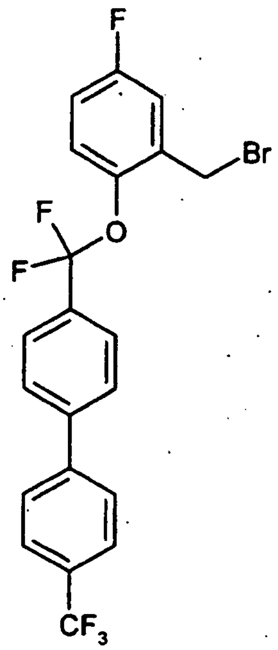

220 mg (0.53 mmol) (2-{Difluor-(4'-(trifluormethyl)biphenyl-4-yl]methoxy}-5-fluorphenyl)-methanol aus Beispiel 40A, 154 mg (0.59 mmol) Triphenylphosphin und 194.6 mg (0.59 mmol) Tetrabrommethan werden in 5 ml Dichlormethan 12 Stunden bei RT gerührt. Zu der Reaktionsmischung werden 2 g Kieselgel gegeben und die Mischung anschließend im Vakuum getrocknet und mittels Kieselgelchromatographie gereinigt (Laufmittel: Cyclohexan/Essigsäureethylester 10:1). Es werden 217 mg (0.46 mmol, 95% Reinheit, 81% Ausbeute) eines Feststoffes isoliert.

MS (ESI): 474 ($M^+$).

**Beispiel 42A**

{2-[Difluor-(4'-trifluormethyl-biphenyl-4-yl)methoxy]-5-fluorbenzyl}-triphenylphosphonium-bromid

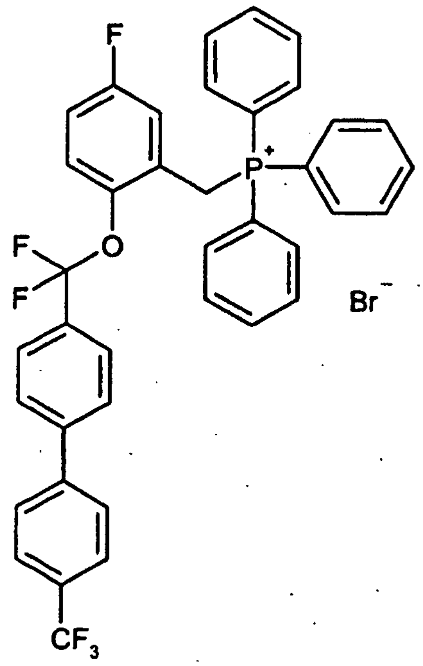

217 mg (0.46 mmol) 2-(Brommethyl)-4-fluorphenyl-difluor[4'-(trifluormethyl)biphenyl-4-yl]-methylether aus Beispiel 41A und 148 mg (0.57 mmol) Triphenylphosphin werden in 5 ml THF bei RT 12 Stunden lang gerührt. Das Reaktionsgemisch wird eingeengt und der Rückstand mit Diethylether und n-Hexan gewaschen. Das Rohprodukt wird ohne weitere Aufreinigung in der Folgestufe eingesetzt.
MS (ESI): 657 $(M+H)^+$.

__Beispiel 43A__

2-(2-Chlorbenzyloxy)-5-fluorbenzoesäuremethylester

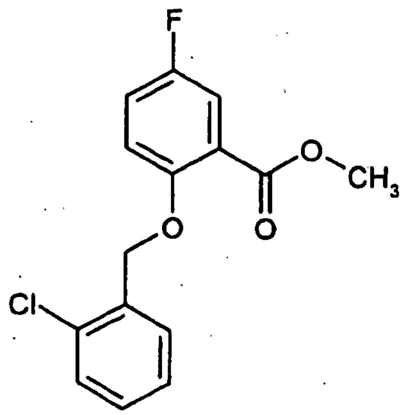

10.0 g (58 mmol) 5-Fluor-2-hydroxybenzoesäuremethylester [CAS 391-92-4], 13.3 g (64.5 mmol) 2-Chlorbenzylbromid [CAS 611-17-6], 40.6 g (293 mmol) Kaliumcarbonat und 14.6 g (88 mmol) Kaliumiodid werden in 50 ml Aceton suspendiert und die Mischung für 12 h unter Rückfluss gerührt. Das Lösungsmittel wird im Vakuum abdestilliert, der Rückstand in Ethylacetat aufgenommen und je zweimal mit 10%-iger Natronlauge, mit 1 N Salzsäure und mit Wasser gewaschen. Man trocknet über Magnesiumsulfat, filtriert, wäscht mit Ethylacetat nach und engt ein. Den Rückstand rührt man mit Petrolether aus, saugt den Feststoff ab und trocknet auf einem Tonteller. Es werden 10.0 g (33.9 mmol, 59% d. Th.) der Titelverbindung isoliert.
$^1$H-NMR (400 MHz, DMSO-$d_6$, $\delta$/ppm): 7.72 (1H, $m_c$), 7.51 (2H, $m_c$), 7.46-7.36 (3H, m), 7.31 (1H, dd), 5.22 (2H, s), 3.81 (3H, s).
LC-MS (Methode 2): $R_t$ 2.6 min; m/z 295 $(M+H)^+$.

__Beispiel 44A__

2-(2-Chlorbenzyloxy)-5-fluorbenzoesäure

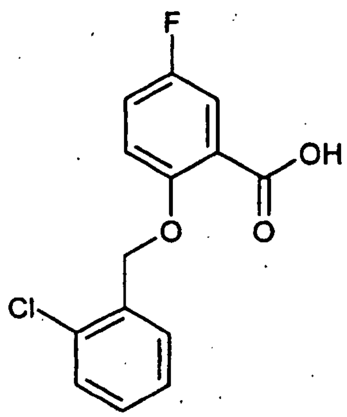

10.0 g (34 mmol) 2-(2-Chlorbenzyloxy)-5-fluorbenzoesäuremethylester werden in 80 ml Methanol und 40 ml 40%-iger Natronlauge gelöst und für 12 h bei RT gerührt. Das Lösungsmittel wird im Vakuum abdestilliert, der Rückstand mit so viel Wasser versetzt, bis alles gelöst ist, und dann mit Ethylacetat extrahiert. Die wässrige Phase stellt man mit Salzsäure sauer und extrahiert dreimal mit Ethylacetat. Man trocknet die vereinigten organischen Phasen über Magnesiumsulfat, filtriert, wäscht mit Ethylacetat nach und engt ein. Den Rückstand versetzt man mit Petrolether, saugt die ausgefallenen Kristalle ab und trocknet auf einem Tonteller. Es werden 6.00 g (21.3 mmol, 63% d. Th.) der Titelverbindung isoliert.

$^1$H-NMR (400 MHz, DMSO-$d_6$, $\delta$/ppm): 7.61 (1H, $m_c$), 7.51 (1H, $m_c$), 7.42-7.37 (2H, m), 7.17 (1H, dd), 7.08-6.97 (2H, m), 5.19 (1H, t), 5.16 (2H, s), 4.55 (2H, d).

HPLC (Methode 1): $R_t$ 4.7 min.

MS (DCI): 284 ($M+NH_4^+$).

**Beispiel 45A**

[2-(2-Chlorbenzyloxy)-5-fluorphenyl]methanol

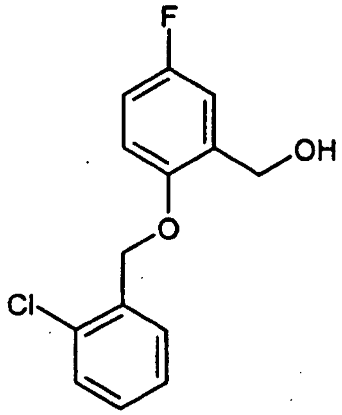

5.50 g (20 mmol) 2-(2-Chlorbenzyloxy)-5-fluorbenzoesäure werden in 25 ml THF gelöst, zum Rückfluss erhitzt und dann tropfenweise mit 20 ml 1 M Boran-Dimethylsulfid-Komplex versetzt. Anschließend wird die Reaktionslösung auf Raumtemperatur abgekühlt und 1 h bei dieser Temperatur nachgerührt. Das Lösungsmittel wird im Vakuum abdestilliert und der Rückstand mit Wasser versetzt. Anschließend wird vorsichtig (starke Gasentwicklung) mit 1 N Salzsäure sauer gestellt. Durch Zugabe von 1 N Natronlauge wird dann leicht basisch gestellt und die wässrige Phase dreimal mit Dichlormethan extrahiert. Die organischen Phasen trocknet man über Magnesiumsulfat, filtriert, wäscht mit Dichlormethan nach und engt das Filtrat ein. Den Rückstand versetzt man mit Petrolether, saugt das ausgefallene Kristallisat ab und trocknet auf einem Tonteller. Es werden 3.65 g (13.6 mmol, 68% d. Th.) der Titelverbindung isoliert.

$^1$H-NMR (400 MHz, DMSO-$d_6$, $\delta$/ppm): 7.61 (1H, $m_c$), 7.51 (1H, $m_c$), 7.42-7.37 (2H, m), 7.17 (1H, dd), 7.08-6.97 (2H, m), 5.19 (1H, t), 5.16 (2H, s), 4.55 (2H, d).

HPLC (Methode 1): $R_t$ 4.7 min.

MS (DCI): 284 ($M+NH_4^+$).

**Beispiel 46A**

[2-(2-Chlorbenzyloxy)-5-fluorbenzyl]-triphenylphosphoniumbromid

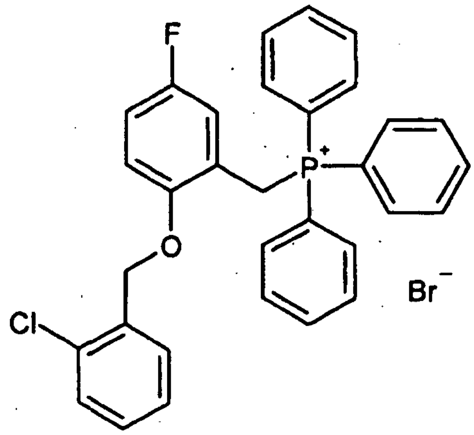

4.60 g (17 mmol) [2-(2-Chlorbenzyloxy)-5-fluorphenyl]methanol werden in 10 ml Acetonitril gelöst, 5.62 g (16 mmol) Triphenylphosphin-Hydrobromid werden zugegeben und die Suspension wird 12 h unter Rückfluss gerührt. Weitere 2.80 g (8 mmol) Triphenylphosphin-Hydrobromid werden hinzugefügt und die Mischung weitere 3 h unter Rückfluss gerührt. Das Lösungsmittel wird im Vakuum größtenteils abdestilliert, und die ausgefallenen Kristalle mit Petrolether verrührt, abgesaugt und auf einem Tonteller getrocknet. Es werden 9.82 g (15.7 mmol, 92% d. Th.) der Titelverbindung erhalten.

$^1$H-NMR (300 MHz, DMSO-$d_6$, δ/ppm): 7.86 (3H, $m_c$), 7.70-7.46 (13H, m), 7.42-7.30 (3H, m), 7.15 (1H, $m_c$), 6.96 (1H, dd), 6.90 (1 H, dt), 4.99 (2H, d), 4.66 (2H, d).

HPLC (Methode 1): $R_t$ 5.0 min.

MS (ESI): 511 (M-$Br^+$).

**Beispiel 47A**

Methyl-5-fluor-2-{[2-(trifluormethyl)benzyl]oxy}benzoat

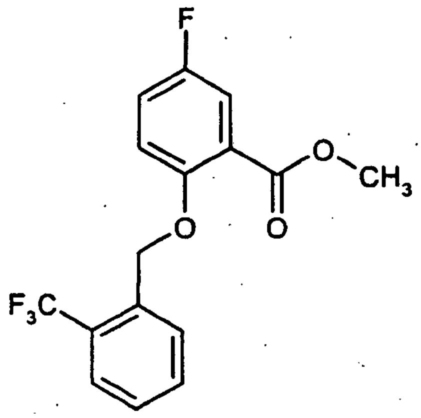

Eine Lösung von 7.50 g (44.08 mmol) Methyl-5-fluor-2-hydroxybenzoat in 40 ml trockenem Acetonitril wird mit 9.44 g (48.49 mmol) 2-Trifluormethylbenzylchlorid und 18.28 g (132.25 mmol) wasserfreiem Kaliumcarbonat versetzt und 12 Stunden unter Rückfluss erhitzt. Anschließend wird der Ansatz abgekühlt, filtriert, mit Dichlormethan nachgewaschen, eingeengt und der Rückstand chromatographisch (Kieselgel; Laufmittel: Cyclohexan/Essigsäureethylester 5:1) gereinigt. Es werden 9.18 g (28 mmol, 89% Reinheit, 56% Ausbeute) der Titelverbindung erhalten.

$^1$H-NMR (400 MHz, DMSO-$d_6$, δ/ppm): 7,93-7,91 (1H, m), 7,80-7,74 (2H, m), 7,60-7,56 (1H, m), 7,53-7,50 (1H, s), 7,46-7,41 (1H, m), 7,29-7,26 (1H, m), 5,32 (2H, s), 3,80 (3H, s).

MS (DCI): 346 (M+$NH_4^+$), 329 (M+$H^+$).

**Beispiel 48A**

(5-Fluor-2-{[2-(trifluormethyl)benzyl]oxy}phenyl)methanol

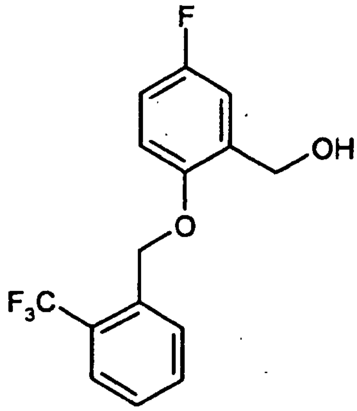

Die Titelverbindung wird in 72% Ausbeute aus Methyl-5-fluor-2-{[2-(trifluormethyl)benzyl]oxy}-benzoat (Beispiel 47A) analoge zu [2-(2-Chlorbenzyloxy)-5-fluorphenyl]methanol (Beispiel 45A) hergestellt.
$^1$H-NMR (400 MHz, DMSO-$d_6$, δ/ppm): 7,81-7,72 (3H, m), 7,61-7,57 (1H, m), 7,20-7,18 (1H, m), 7,06-6,99 (2H, s), 5,23 (2H, s), 5,20 (1H, d), 4,52 (2H, d).
MS (EI): 300 ($M^+$).

**Beispiel 49A**

2-(Brommethyl)-4-fluor-1-{[2-(trifluormethyl)benzyl]oxy}benzol

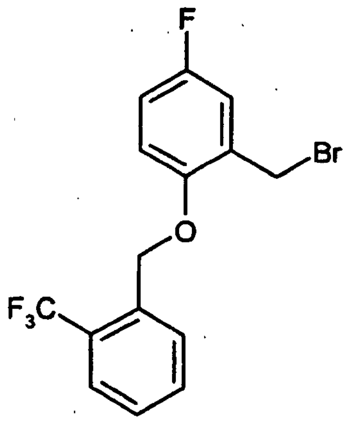

Die Titelverbindung wird in 80% Ausbeute aus (5-Fluor-2-{[2-(trifluormethyl)benzyl]oxy}-phenyl)methanol (Beispiel 48A) analog zu 2-(Brommethyl)-4-fluorphenyl-difluor-[4'-(trifluor-methyl)biphenyl-4-yl]methylether (Beispiel 41A) hergestellt.
$^1$H-NMR (400 MHz, DMSO-$d_6$, δ/ppm): 7,89-7,74 (3H, m), 7,62-7,58 (1H, m), 7,38-7,35 (1H, m), 7,21-7,16 (1H, m), 5,32 (2H, s), 4,66 (2H, s).
MS (DCI): 380 ($M+NH_4^+$).

**Beispiel 50A**

[5-Fluor-2-(2-trifluormethyl-benzyloxy)benzyl]-triphenylphosphoniumbromid

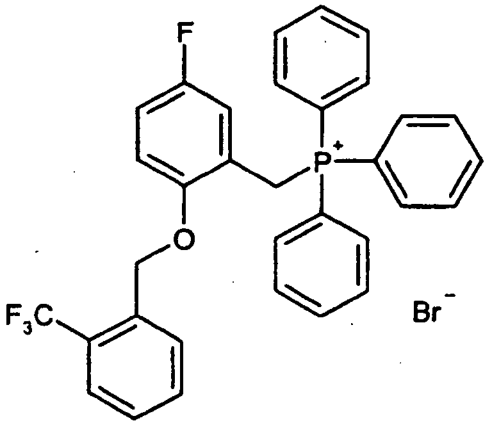

Die Titelverbindung wird aus 2-(Brommethyl)-4-fluor-1-{[2-(trifluormethyl)benzyl]oxy}benzol (Beispiel 49A) analog zu Beispiel 42A hergestellt.

$^1$H-NMR (400 MHz, DMSO-$d_6$, δ/ppm): 7,87-7,83 (3H, m), 7,79-7,77 (1H, m), 7,69-7,55 (14H, m), 7,47-7,44 (1H, m), 7,20-7,14 (1H, m), 6,94-6,90 (2H, m), 5,02 (2H, d), 4,75 (2H, s).

MS (DCI): 545 $(M+H)^+$.

Auf analoge Weise werden die in der folgenden Tabelle aufgeführten Verbindungen erhalten:

| Beispiel | Struktur | Analytische Daten |
|---|---|---|
| **51A**<br><br>(ausgehend von Bsp. 34A und Bsp. 38A) | 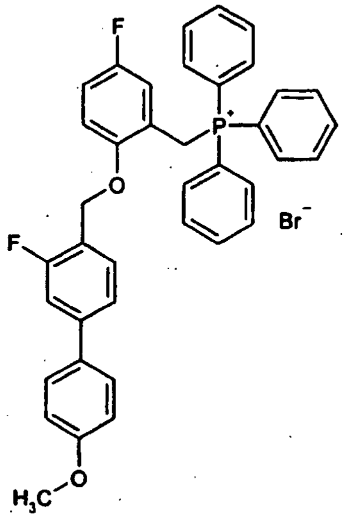 | $^1$H-NMR (300 MHz, DMSO-$d_6$, δ/ppm): 7,94-6,88 (25H, m), 5,00-4,92 (2H, m), 4,72 (2H, s), 3,82 (3H, s).<br>MS (ESI): 601 $(M^+)$. |

(fortgesetzt)

| Beispiel | Struktur | Analytische Daten |
|---|---|---|
| **52A** (ausgehend von Bsp. 34A und 4-Chlormethyl-4'-trifluormethylbiphenyl [CAS 454464-38-1] | 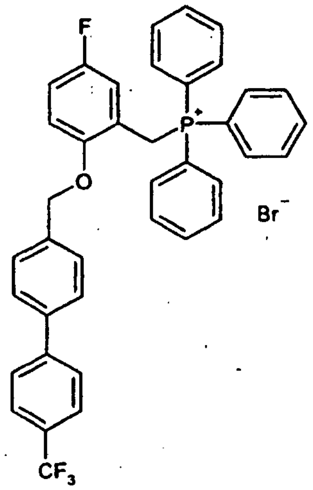 | $^1$H-NMR (300 MHz, DMSO-$d_6$, δ/ppm): 7,95-7,82 (7H, m), 7,75-7,57 (14H, m), 7,35-7,30 (2H, m), 7,20-7,12 (1H, m), 7,00-6,96 (1H, m), 6,92-6,86 (1H, m), 5,05-4,98 (2H, d), 4,72 (2H, s). MS (ESI): 621 ($M^+$-Br). |
| **53A** (ausgehend von Bsp. 34A und 1-Chlormethyl-2-fluorbenzol) | 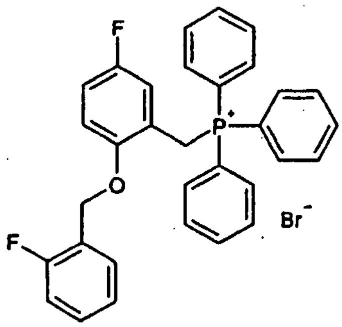 | MS (DCI): 495 ($M^+$-Br). |
| **54A** (ausgehend von Bsp. 34A und 1-Chlormethyl-3,5-bis(trifluor-methyl)-benzol) | 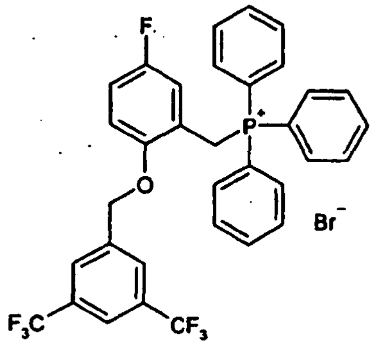 | MS (DCI): 613 ($M^+$-Br). |
| **55A** (ausgehend von Bsp. 34A und Chlormethylbenzol) | 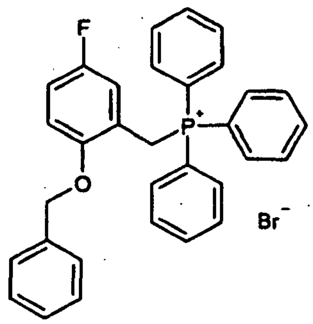 | $^1$H-NMR (400 MHz, DMSO-$d_6$, δ/ppm): 7,88-7,84 (3H, m), 7,68-7,55 (12H, m), 7,39-7,33 (3H, m), 7,21-7,12 (3H, m), 6,96-6,93 (1H, m), 6,89-6,85 (1H, m), 4,98 (2H, d), 4,64 (2H, m). MS (ESI): 557 ($M^+$). |

**Beispiel 56A**

2-(4-*tert*.-Butylbenzyloxy)-5-fluorbenzoesäuremethylester

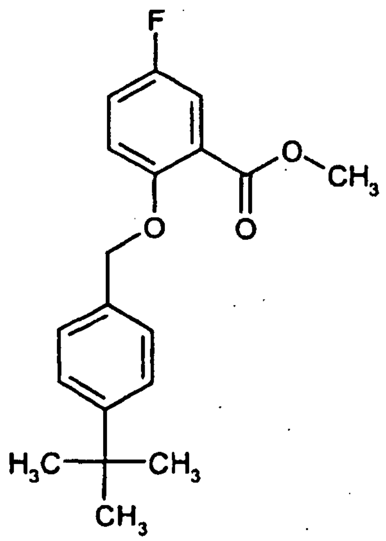

4.44 g (26 mmol) 5-Fluor-2-hydroxybenzoesäuremethylester [CAS 391-92-4], 5.25 g (28.7 mmol) 4-*tert*.-Butylbenzylchlorid [CAS 19692-45-6], 18.0 g (130 mmol) Kaliumcarbonat und 6.5 g (39.2 mmol) Kaliumiodid werden in 25 ml Aceton gelöst und für 12 h unter Rückfluss gerührt. Das Lösungsmittel wird im Vakuum abdestilliert, der Rückstand in Ethylacetat aufgenommen und einmal mit 10%-iger Natronlauge und dreimal mit 1 N Salzsäure gewaschen. Man trocknet über Natriumsulfat, filtriert, wäscht mit Ethylacetat nach und engt ein. Es werden 8.36 g (26 mmol, 100% d. Th.) der Titelverbindung isoliert.

$^1$H-NMR (400 MHz, DMSO-$d_6$, $\delta$/ppm): 7.49-7.35 (6H, m), 7.25 (1H, dd), 5.12 (2H, s), 3.81 (3H, s), 1.28 (9H, s).

LC-MS (Methode 4): $R_t$ 3.1 min; m/z 334 $(M+NH_4)^+$.

**Beispiel 57A**

2-(4-*tert*.-Butylbenzyloxy)-5-fluorbenzoesäure

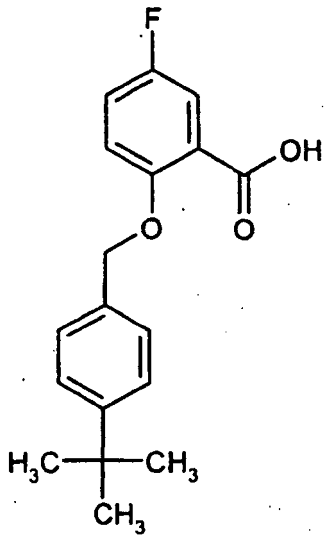

8.0 g (25 mmol) 2-(4-*tert*.-Butylbenzyloxy)-5-fluorbenzoesäuremethylester werden in 64 ml Methanol und 32 ml 40%-iger Natronlauge gelöst und für 12 h unter Rückfluss gerührt. Die ausgefallenen Kristalle werden abgesaugt, mit Wasser, 1 N Salzsäure und Petrolether gewaschen und auf einem Tonteller getrocknet. Es werden 7.10 g (23.5 mmol, 94% d. Th.) der Titelverbindung isoliert.
$^{1}$H-NMR (300 MHz, DMSO-$d_6$, δ/ppm): 7.42-7.33 (4H, m), 6.89-6.70 (3H, m), 4.97 (2H, s), 1.28 (9H, s).
LC-MS (Methode 4): $R_t$ 2.7 min; m/z 302 $(M+H)^+$.

**Beispiel 58A**

[2-(4-*tert*.-Butylbenzyloxy)-5-fluorphenyl]methanol

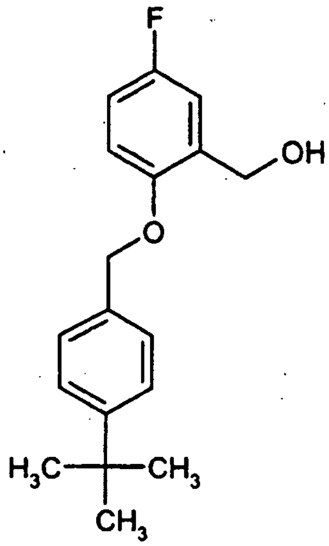

6.65 g (22 mmol) 2-(4-*tert*.-Butylbenryloxy)-5-fluorbenzoesäure werden in 25 ml THF suspendiert und auf 0°C gekühlt. 2.4 ml (22 mmol) *N*-Methylmorpholin und 2.1 ml (22 mmol) Chlorameisensäureethylester werden zugegeben, und die Lösung wird für 15 min bei Raumtemperatur nachgerührt. 2.50 g (66 mmol) Natriumborhydrid werden hinzugefügt, und nach 3 h wird solange Methanol zugesetzt, bis die Gasentwicklung abgeklungen ist. Es wird mit 1 N Salzsäure sauer gestellt, im Vakuum eingeengt und der Rückstand in Ethylacetat aufgenommen. Die organische Phase wird mit gesättigter Kochsalz-Lösung gewaschen und anschließend über Natriumsulfat getrocknet. Das Lösungsmittel wird im Vakuum abdestilliert und der Rückstand an Kieselgel chromatographiert (Laufmittel: Cyclohexan/Ethylacetat 10:1). Es werden 3.52 g (12.2 mmol, 55% d. Th.) der Titelverbindung isoliert.
$^{1}$H-NMR (400 MHz, DMSO-$d_6$, δ/ppm): 7.41-7.35 (4H, m), 7.17-7.15 (1H, m), 7.02-6.99 (2H, m), 5.19 (1H, t), 5.05 (2H, s), 4.52 (2H, d), 1.28 (9H, s).
LC-MS (Methode 4): $R_t$ 2.8 min; m/z 288 $(M+H)^+$.

**Beispiel 59A**

[2-(4-*tert*.-Butylbenzyloxy)-5-fluorbenzyl]-triphenylphosphoniumbromid

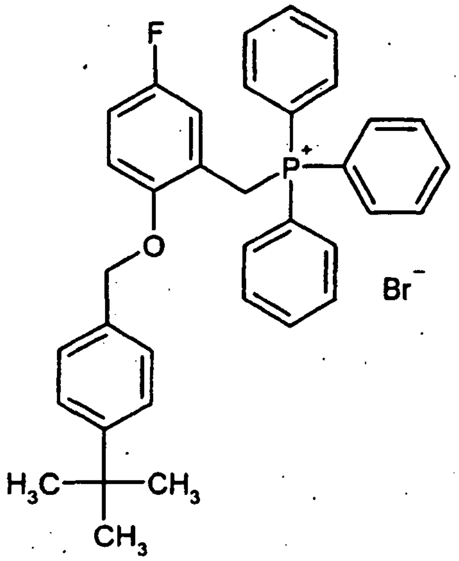

5.80 g (20 mmol) [2-(4-*tert*.-Butylbenzyloxy)-5-fluorphenyl]methanol werden in 70 ml Acetonitril gelöst, 6.56 g (19.5 mmol) Triphenylphosphin-Hydrobromid werden zugegeben und die Suspension wird für 24 h unter Rückfluss gerührt. Das Lösungsmitteln wird im Vakuum größtenteils abdestilliert und die ausgefallenen Kristalle mit Diethylether verrührt, abgesaugt, mit Petrolether gewaschen und auf einem Tonteller getrocknet. Das erhaltene Rohprodukt wird per präparative HPLC gereinigt. Es werden 2.64 g (4.3 mmol, 2 1 % d. Th.) der Titelverbindung erhalten.

$^1$H-NMR (300 MHz, DMSO-$d_6$, δ/ppm): 7.86 (3H, $m_c$), 7.69-7.52 (12H, m), 7.37 (2H, $d_{AB}$), 7.14 (2+1 H, $d_{AB}$+m), 6.95 (1H, dd), 6.88 (1H, dd), 4.94 (2H, d), 4.40 (2H, s), 1.28 (9H, s).

LC-MS (Methode 2): $R_t$ 2.4 min; m/z 533 (M+H-Br)$^+$.

__Beispiel 60A__

2-(4-Trifluormethylphenyl)ethanol

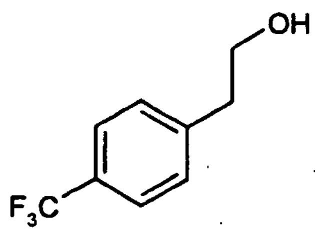

3.0 g (14.7 mmol) 4-Trifluormethylphenylessigsäure werden bei 0°C in 30 ml abs. THF vorgelegt. Eine 1 M Lösung von 557.8 mg (14.7 mmol) Lithiumaluminiumhydrid in 14.7 ml THF wird zugetropft und die Lösung bei Raumtemperatur bis zur vollständigen Umsetzung gerührt. Der Ansatz wird auf Eis gegeben, mit Salzsäure angesäuert und mit Essigsäureethylester extrahiert. Die organische Phase wird eingeengt und das Rohprodukt an Kieselgel chromatographisch (Laufmittel: Cyclohexan/Essigsäureethylester 6:1) gereinigt. Es werden 2 g (92% d. Th.) der Titelverbindung erhalten.

$^1$H-NMR (400 MHz, $CDCl_3$, δ/ppm): 2.95 (t, 2H), 3.9 (t, 2H), 7.35 (d, 2H), 7.6 (t, 2H).

GC-MS (Methode 1): $R_t$ 4.61 min; m/z 190 (M$^+$).

__Beispiel 61A__

5-Fluor-2-[2-(4-trifluormethyl-phenyl)ethoxy]benzoesäuremethylester

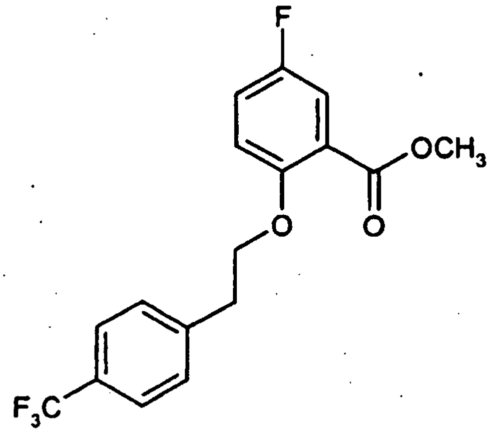

1.79 g (10.5 mmol) 5-Fluorsalicylsäuremethylester, 20 g (10.5 mmol) 2-(4-Trifluormethyl-phenyl)-ethanol und 2.76 g (10.5 mmol) Triphenylphosphin werden in 50 ml THF vorgelegt, und bei 0°C eine Lösung von 1.83 g (10.5 mmol) Diethylazodicarboxylat in 10 ml THF zugetropft. Es wird über Nacht bei RT nachgerührt und dann die flüchtige Komponenten im Vakuum entfernt. Das erhaltene Rohprodukt wird durch Chromatographie an Kieselgel (Laufmittel: Cyclohexan/Essigsäureethylester 7:1) gereinigt. Es werden 2.09 g (58% Ausbeute) der Titelverbindung erhalten.

$^1$H-NMR (300 MHz, $CDCl_3$, δ/ppm): 3.2 (t, 2H), 3.85 (s, 3H), 4.25 (t, 2H), 6.85 (dd, 1H), 7.15 (m, 1H), 7.5 (m, 3H), 7.6 (d, 2H).

GC-MS (Methode 2): $R_t$ 10.54 min; m/z 342 ($M^+$).

__Beispiel 62A__

{5-Fluor-2-[2-(4-trifluormethyl-phenyl)ethoxy]phenyl}methanol

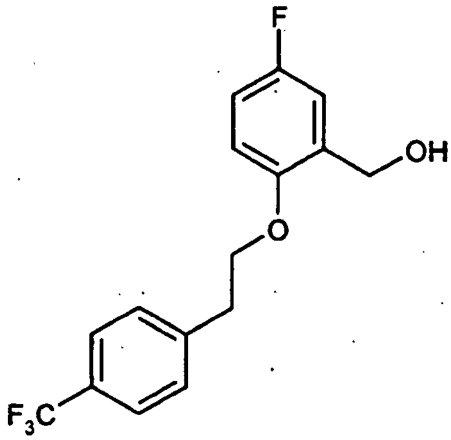

2.0 g (5.84 mmol) 5-Fluor-2-[2-(4-trifluormethyl-phenyl)ethoxy]benzoesäuremethylester werden in 25 ml THF vorgelegt. Bei 0°C werden 4.38 ml (4.38 mmol) einer 1 M Lithiumaluminiumhydrid-Lösung in THF zugetropft und die Mischung bei RT bis zur vollständigen Umsetzung nachgerührt. Der Ansatz wird auf Eiswasser gegeben, mit Salzsäure angesäuert und mit Essigsäureethylester extrahiert. Alle flüchtigen Komponenten werden im Vakuum entfernt und das Rohprodukt chromatographisch an Kieselgel (Laufmittel: Cyclohexan/Essigsäureethylester 5:1) gereinigt. Es werden 1.7 g (82% Ausbeute) der Titelverbindung erhalten.

$^1$H-NMR (400 MHz, $CDCl_3$, δ/ppm): 3,2 (t, 2H); 4,25 (t, 2H); 4,6 (s, 2H); 6,75 (m, 1H); 6,9 (m, 1H); 7,05 (dd, 1H); 7,4 (d, 2H); 7,6 (d, 2H).

GC-MS (Methode 1): $R_t$ 10.71 min; m/z 314 ($M^+$).

**Bespiel 63A**

{5-Fluor-2-[2-(4-trifluormethyl-phenyl)ethoxy]benzyl}-triphenylphosphoniumbromid

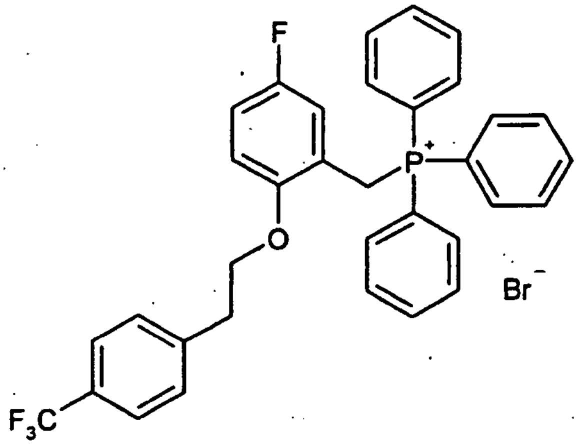

1.7 g (5.41 mmol) {5-Fluor-2-[2-(4-trifluormethyl-phenyl)ethoxy]phenyl}methanol und 1.76 g (5.14 mmol) Triphenylphosphoniumhydrobromid werden in 20 ml Acetonitril drei Stunden unter Rückfluss erhitzt. Eine erste Produktfraktion wird bei -20°C auskristallisiert. Die Mutterlauge wird eingeengt, der Rückstand in Dichlormethan gelöst und mit Diethylether eine zweite Fraktion kristallisiert. Es werden insgesamt 2.71 g (76% Ausbeute) der Titelverbindung erhalten.
$^1$H-NMR (400 MHz, DMSO-$d_6$, $\delta$/ppm): 2,75 (t, 2H); 3,75 (t, 2H); 4,85 (d, 2H); 6,8 (m, 1H); 6,9 (m, 1H); 7,15 (m, 1H); 7,45 (d, 2H); 7,6 (m, 8H); 7,7 (m, 6H); 7,9 (t, 3H).
LC-MS (Methode 2): $R_t$ 2.15 min; m/z 559 $[M+H]^+$.

**Beispiel 64A**

5-Fluor-2-[3-(4-trifluormethyl-phenyl)-prop-2-ynyloxy]-benzoesäuremethylester

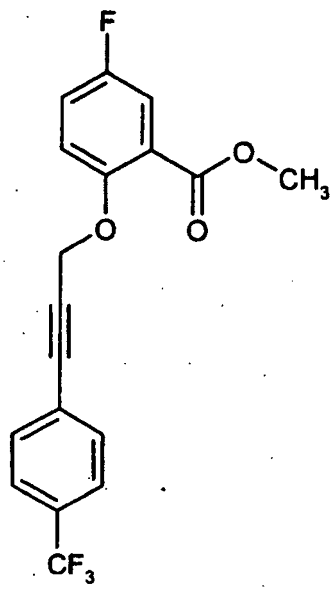

4.12 g (24.2 mmol) 3-Fluorsalicylsäuremethylester [CAS 391-92-4] werden in 100 ml Aceton gelöst, 10.0 g (72.6 mmol) Pottasche, 6.02 g (36.3 mmol) Kaliumiodid und 7.00 g (26.6 mmol) 1-(3-Bromprop-1-ynyl)-4-trifluormethyl-benzol [Beilstein Record-Nr. 7919066] werden zugegeben und die Mischung für 2.5 h unter Rückfluss gerührt. Das Lösungsmittel wird im Vakuum abdestilliert und der Rückstand in Ethylacetat aufgenommen. Die organische Phase wird mit Wasser gewaschen und über Natriumsulfat getrocknet. Das Lösungsmittel wird im Vakuum abdestilliert und der Rückstand an Kieselgel chromatographiert (Laufmittel: Cyclohexan/Ethylacetat 20:1 → 5:1). Es werden 4.63 g (13.1 mmol, 49% d. Th.) der Titelverbindung erhalten.

$^1$H-NMR (400 MHz, DMSO-$d_6$, δ/ppm): 7.75 (2H, $d_{AB}$), 7.65 (2H, $d_{AB}$), 7.52-7.45 (2H, m), 7.37 (1H, dd), 5.17 (2H, s), 3.82 (3H, s).

LC-MS (Methode 4): $R_t$ 2.9 min; m/z 352 $(M+H)^+$.

**<u>Beispiel</u>** <u>65A</u>

5-Fluor-2-[3-(4-trifluormethyl-phenyl)-prop-2-ynyloxy]-benzoesäure

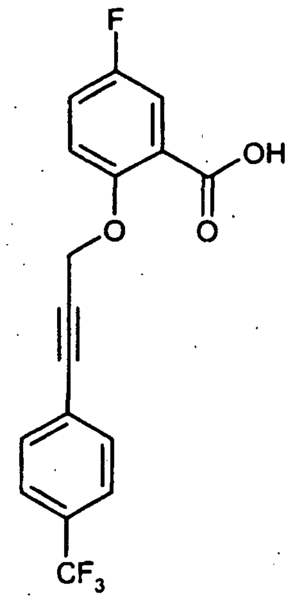

100 mg (0.28 mmol) 5-Fluor-2-[3-(4-trifluormethyl-phenyl)-prop-2-ynyloxy]-benzoesäuremethyl-ester werden in 2 ml Methanol und 2 ml Wasser gelöst und mit 20.3 mg (0.85 mmol) Lithium-hydroxid versetzt. Nach 12 h Rühren bei Raumtemperatur werden die ausgefallenen Kristalle abgesaugt, mit Wasser, 1 N Salzsäure und Petrolether gewaschen und auf einem Tonteller getrocknet. Es werden 74.9 mg (0.20 mmol, 71% d. Th.) der Titelverbindung isoliert.

$^1$H-NMR (300 MHz, DMSO-$d_6$, δ/ppm): 13.0 (1H, breites s), 7.76 (2H, $d_{AB}$), 7.65 (2H, $d_{AB}$), 7.49-7.39 (2H, m), 7.33 (1H, dd), 5.15 (2H, s).

LC-MS (Methode 2): $R_t$ 2.4 min; m/z 338 $(M+H)^+$.

**<u>Beispiel 66A</u>**

{5-Fluor-2-[3-(4-trifluormethyl-phenyl)-prop-2-ynyloxy]phenyl}methanol

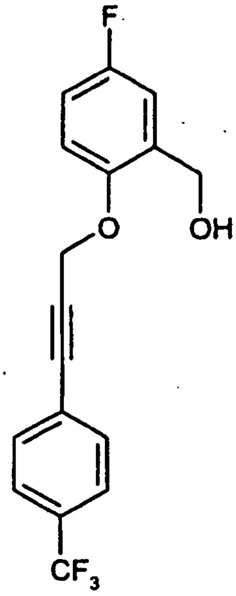

3.50 g (10.3 mmol) 5-Fluor-2-[3-(4-trifluormethyl-phenyl)-prop-2-ynyloxy]-benzoesäure werden in 10 ml THF suspendiert, 1.04 g (10.3 mmol) *N*-Methylmorpholin zugegeben, die Mischung auf 0°C abgekühlt und 1.12 g (10.3 mmol) Chlorameisensäureethylester zugegeben. Es wird auf Raum-temperatur erwärmt und 30 min nachgerührt. Zu dieser Lösung werden 1.17 g (31.0 mmol) Natriumborhydrid gegeben und der Ansatz für 6 h bei Raumtemperatur gerührt. Dann wird solange Methanol zugegeben, bis die Gasentwicklung abgeklungen ist, und anschließend mit 1 N Salzsäure sauer gestellt. Das Lösungsmittel wird im Vakuum abdestilliert und der Rückstand in Ethylacetat aufgenommen. Die organische Phase wird mit gesättigter Kochsalz-Lösung gewaschen, über Natriumsulfat getrocknet und das Lösungsmittel im Vakuum abdestilliert. Es werden 4.62 g (8.69 mmol, 85% d. Th.) der Titelverbindung isoliert.

LC-MS (Methode 4): $R_t$ 2.7 min; m/z 348 $(M+Na)^+$.

**Beispiel 67A**

{5-Fluor-2-[3-(4-trifluormethyl-phenyl)-prop-2-ynyloxy]benzyl}-triphenylphosphoniumbromid

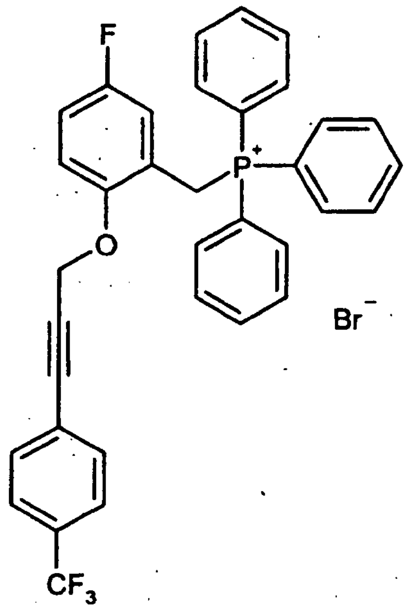

*Stufe a):*

834 mg (3.18 mmol) Triphenylphosphin und 1.05 g (3.18 mmol) Tetrabromkohlenstoff werden in 2 ml THF gelöst und 10 min bei Raumtemperatur gerührt. Dann werden 860 mg (2.63 mmol) {5-Fluor-2-[3-(4-trifluormethyl-phenyl)-prop-2-ynyloxy]phenyl}methanol, in 2 ml THF gelöst, zugetropft und die Mischung über Nacht bei Raumtemperatur gerührt. Das Lösungsmittel wird im Vakuum abdestilliert und der Rückstand an Kieselgel chromatographiert (Laufmittel: Cyclohexan/ Essigsäureethylester 10:1). Es werden 240 mg (0.62 mmol, 24% d. Th.) 5-Fluor-2-[3-(4-trifluormethyl-phenyl)-prop-2-ynyloxy]benzylbromid erhalten.

*Stufe b):*

63.7 mg (0.24 mmol) Triphenylphosphin werden in 2 ml Dichlormethan gelöst, 94 mg (0.24 mmol) 5-Fluor-2-[3-(4-trifluormethyl-phenyl)-prop-2-ynyloxy]benzylbromid, in 2 ml Dichlormethan gelöst, zugetropft und die Mischung über Nacht bei Raumtemperatur gerührt. Das Lösungsmittel wird im Vakuum abdestilliert und der kristalline Rückstand mit Petrolether verrührt. Die erhaltenen farblosen Kristalle werden abgesaugt und auf einem Tonteller getrocknet. Es werden 109 mg (0.17 mmol, 69% d. Th.) der Titelverbindung erhalten.

$^1$H-NMR (300 MHz, DMSO-$d_6$, δ/ppm): 7.89-7.58 (19H, m), 7.21 (1H, tt), 7.06 (1H, dd), 6.86 (1H, dt), 6.88 (1H, dd), 4.98 (2H, d), 4.61 (2H, s).

LC-MS (methode 1): $R_t$ 2.4 min; m/z 569 $(M+H-Br)^+$.

**Beispiel 68A**

*E*/*Z*-4-(2-[2-(4-Cyanophenyl)ethyl]-4-{5-fluor-2-[2-(4-methoxyphenyl)ethyl]phenyl}-but-3-enyl)-benzoesäuremethylester

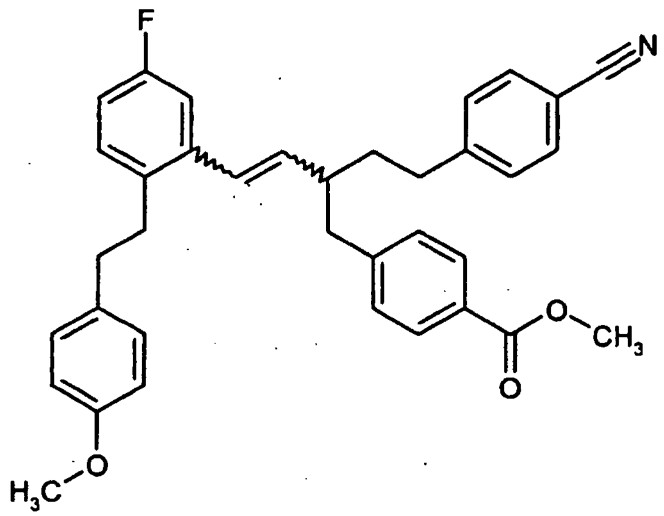

Zu einer Lösung von 1093 mg (1.87 mmol) ({5-Fluor-2-[2-(4-methoxyphenyl)ethyl]benzyl}-tri-phenylphosphoniumbromid (Beispiel 32A) in 20 ml THF werden bei 0°C 1.46 ml (2.33 mmol) einer 1.6 M Lösung von n-Butyllithium in Hexan langsam hinzugefügt. Anschließend werden bei dieser Temperatur 500 mg (1.56 mmol) 4-[4-(4-Cyanophenyl)-2-formylbutyl]-benzoesäuremethylester aus Beispiel 16A langsam zudosiert und 4 Stunden bei 0°C nachgerührt. Nach Erwärmung auf Raumtemperatur wird die Reaktionslösung 12 Stunden nachgerührt und nachfolgend mit etwas Wasser versetzt und bis zur Trockene eingeengt. Der Rückstand wird in Essigsäureethylester aufgenommen, mit Wasser und gesättigter Kochsalz-Lösung gewaschen und über Natriumsulfat getrocknet. Nach Filtration wird das Lösungsmittel bis zur Trockene eingeengt. Das erhaltene Rohprodukt wird durch Flash-Chromatographie an Kieselgel (Laufmittel: Cyclohexan/Essigsäureethylester 50:1 → 20:1) gereinigt. Es werden 719 mg (1.31 mmol, 84% Ausbeute) eines farblosen Feststoffes erhalten.

$^1$H-NMR (300 MHz, DMSO-$d_6$, δ/ppm): 2:1-*E*/*Z*-Mischung: 7,79 (3H, d), 7,71 (2H, d), 7,64 (1H, d), 7,44-6,72 (9H, m), 6,43 (0.33H, d), 6,32 (0.66H, d), 6,14-5,99 (1H, m), 5,62 (0,33H, t), 3,79 (0.66H, s), 3,74 (2H, s), 3,71 (3H, s), 2,99-2,37 (9H, m), 1,92-1,52 (2H, m).

MS (DCI): 565 ($M+NH_4^+$).

**Beispiel 69A**

*E*/*Z*-4-(4-{5-Fluor-2-[2-(4-methoxyphenyl)ethyl]phenyl}-2-{2-[4-(5-oxo-4,5-dihydro-[1,2,4]oxa-diazol-3-yl)phenyl] ethyl}-but-3-enyl)-benzoesäuremethylester

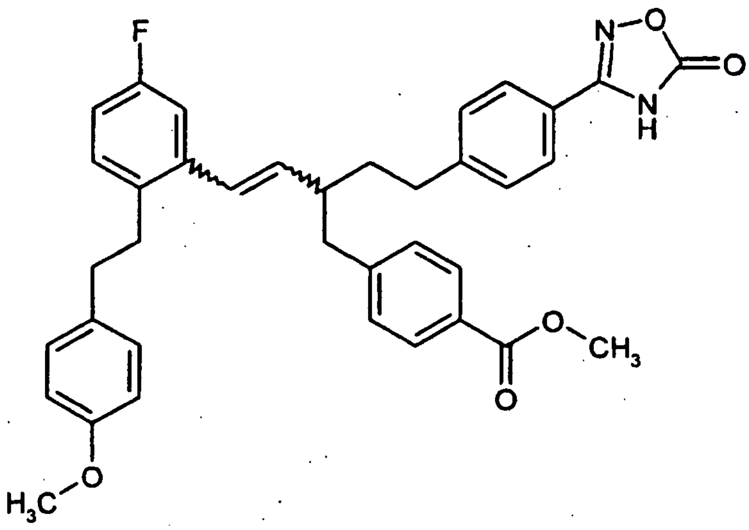

Eine Lösung von 444.1 mg (6.39 mmol) Hydroxylaminhydrochlorid in 10 ml DMSO wird mit 0.89 ml (6.39 mmol) Triethylamin versetzt und 10 min bei Raumtemperatur gerührt. Der anfallende Niederschlag wird abfiltriert. In das Filtrat werden anschließend 180 mg (0.28 mmol) *E*/*Z*-4-(2-[2-(4-Cyanophenyl)ethyl]-4-{5-fluor-2-[2-(4-methoxyphenyl)ethyl] phenyl}-but-3-enyl)-benzoesäure-methylester aus Beispiel 68A zudosiert. Die Reaktionslösung wird 12 Stunden bei 75°C gerührt. Nach vollständigem Umsatz und Abkühlung werden zu der Reaktionslösung 10 ml Wasser gegeben, mit Essigsäureethylester dreimal extrahiert und die vereinigten organischen Phasen mit gesättigter Kochsalz-Lösung gewaschen. Anschließend wird die organische Phase über Natriumsulfat getrocknet und bis zur Trockene eingeengt. Es werden 632 mg eines farblosen Öls erhalten, das ohne weitere Reinigung direkt weiter umgesetzt wird.

Das oben erhaltene Öl wird in 10 ml DMF gelöst und mit 0.1 ml (1.19 mmol) Pyridin versetzt. Anschließend wird die Lösung auf 0°C gekühlt und langsam mit 193.83 mg (1.08 mmol) Chlorameisensäure-2-ethylhexylester versetzt. Der Ansatz wird ca. 30 min bei 0°C nachgerührt, dann in Wasser aufgenommen und mit Essigsäureethylester extrahiert. Die organische Phase wird über Natriumsulfat getrocknet und bis zur Trockene eingeengt. Der erhaltene Rückstand wird in 10 ml Xylol aufgenommen und 4 Stunden zum Rückfluss erhitzt. Nach vollständiger Umsetzung wird die Reaktionslösung abgekühlt und bis zur Trockene eingeengt. Das Rohprodukt wird mittels Flash-chromatographie an Kieselgel gereinigt (Laufmittel: Cyclohexan/Essigsäureethylester 4:1). Man erhält 447 mg (0.59 mmol, 56% Ausbeute; Reinheit 82%) eines farblosen Öls.

LC-MS (Methode 1): R, 3.05 min; m/z 607 ($M+H^+$).

**Beispiel 70A**

2-(4-Cyanobenzyl)-malonsäurediallylester

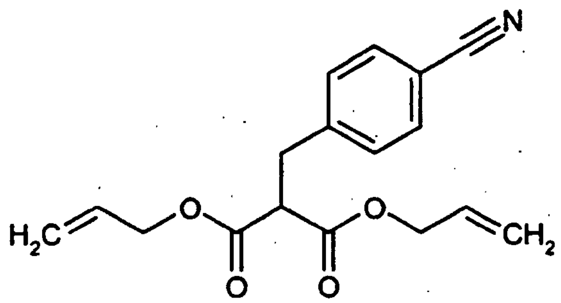

Eine Lösung von 121.5 g (0.659 mol) Malonsäurediallylester in 1.5 Liter Dioxan wird bei 0°C portionsweise mit 19.79 g (0.494 mol) Natriumhydrid versetzt (Vorsicht Wasserstoffentwicklung). Nach Erwärmen auf Raumtemperatur wird der Ansatz 1 Stunde bei 40°C gerührt. Anschließend werden 50 g (0.329 mol) 4-Chlormethylbenzonitril, gelöst in 500 ml Dioxan, langsam bei 40°C zugetropft und die Reaktionslösung über Nacht bei 110°C gerührt. Nach Abkühlen auf Raumtemperatur wird das Reaktionsgemisch auf 1200 ml Wasser gegeben. Dabei ist darauf zu achten, dass der pH-Wert < 7 bleibt (gegebenenfalls werden wenige ml 1 M Salzsäure bis ca. pH 2 zudosiert).. Der Ansatz wird dann dreimal mit Essigsäureethylester extrahiert, die vereinigten organischen Phasen mit gesättigter Natriumchlorid-Lösung gewaschen und über Natriumsulfat getrocknet. Nach der Filtration wird das Lösungsmittel im Vakuum bis zur Trockene eingeengt. Anschließend wird durch Hochvakuum-Destillation überschüssiger Malonsäurediallylester entfernt (Siedepunkt: 57°C; 0.074 mbar). Der Destillationsrückstand wird durch Flash-Chromatographie an Kieselgel (Laufmittel: Petrolether/Essigsäureethylester 20:1) gereinigt. Es werden 67 g (0.22 mol, 67% Ausbeute) eines farblosen Feststoffes erhalten.

$^1$H-NMR (300 MHz, DMSO-$d_6$, δ/ppm): 7,77 (2H, d), 7,48 (2H, d), 5,90-5,73 (2H, m), 5,29-5,13 (4H, m), 4,64-4,50 (4H, m), 4,09 (1H, t), 3,21 (2H, d).

MS (DCI): 317 ($M+NH_4^+$).

**Beispiel 71A**

2-(4-Cyanobenzyl)-2-[2-(4-methoxycarbonylphenyl)ethyl]-malonsäurediallylester

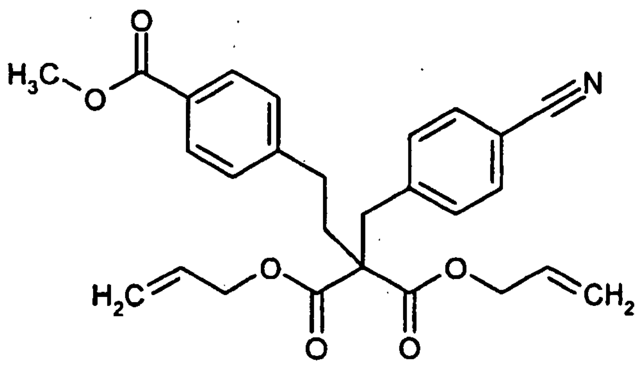

Eine Lösung von 48.53 g (162.14 mmol) 2-(4-Cyanobenzyl)-malonsäurediallylester aus Beispiel 70A in 180 ml DMF wird bei 0°C portionsweise mit 7.13 g (178.36 mmol) Natriumhydrid versetzt. Anschließend lässt man die Reaktionslösung auf Raumtemperatur kommen und 30 min lang nachrühren. Nachfolgend wird die Reaktionslösung wieder auf 0°C abgekühlt, mit 55 g (194.57 mmol) 4-(2-Bromethyl)-benzoesäuremethylester [CAS 136333-97-6] in 195 ml DMF versetzt und 30 min bei dieser Temperatur nachgerührt. Der Ansatz wird danach bei Raumtemperatur über Nacht gerührt. Das Reaktionsgemisch wird tropfenweise mit Wasser versetzt, mit Essigsäureethylester dreimal extrahiert, die vereinigten organischen Phasen mit gesättigter Kochsalz-Lösung gewaschen und über Natriumsulfat getrocknet. Nach der Filtration wird das Lösungsmittel im Vakuum bis zur Trockene eingeengt. Das erhaltene Rohprodukt wird durch Flash-Chromatographie an Kieselgel (Laufmittel: Petrolether/Essigsäureethylester 10:1) gereinigt. Es werden 33.4 g (72.37 mol, 44% Ausbeute) eines farblosen Feststoffes erhalten.

$^1$H-NMR (300 MHz, $CDCl_3$, δ/ppm): 7,89 (2H, d), 7,79 (2H, d), 7,38 (2H, d), 7,32 (2H, d), 5,97-5,81 (2H, m), 5,38-5,20 (4H, m), 4,61 (4H, d), 3,82 (3H, s), 3,39 (2H, s), 2,77-2,61 (2H, m), 1,99-1,84 (2H, m).

MS (DCI): 479 ($M+NH_4^+$).

**Beispiel 72A**

4-[3-Carboxy-4-(4-cyanophenyl)butyl]-benzoesäuremethylester

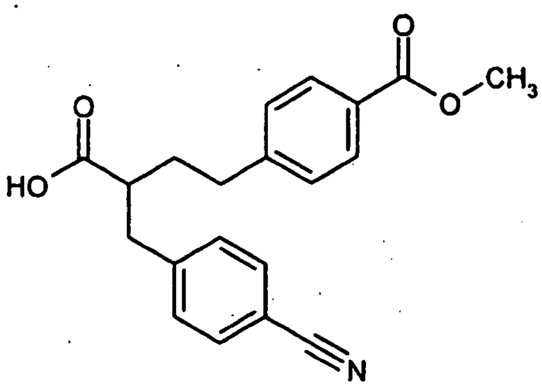

Eine Lösung von 7.5 g (16.25 mmol) 2-(4-Cyanobenzyl)-2-[2-(4-methoxycarbonylphenyl)ethyl]-malonsäurediallylester aus Beispiel 71A, 0.3 g (1.14 mmol) Triphenylphosphin und 70 mg Palladiumacetat in 170 ml Dioxan wird bei Raumtemperatur mit einer Lösung von 7.47 ml (53.63 mmol) Triethylamin und 1.53 ml (40.63 mmol) Ameisensäure in 170 ml Dioxan versetzt. Das Reaktionsgemisch wird anschließend bei 100°C 2 Stunden gerührt. Nach vollständigem Umsatz wird die Reaktionslösung abgekühlt und das Lösungsmittel im Vakuum entfernt. Anschließend wird der Rückstand in Essigsäureethylester und Wasser aufgenommen, mit 1 N Salzsäure angesäuert und die organische Phase abgetrennt. Die wässrige Phase wird noch dreimal mit Essigsäureethylester extrahiert, die organischen Phasen werden anschließend vereinigt, mit gesättigter Koch-salz-Lösung gewaschen und über Natriumsulfat getrocknet. Nach Filtration wird die Lösung im Vakuum eingeengt. Es werden 5.48 g (89% Ausbeute, 90% Reinheit) eines farblosen Feststoffes erhalten.

$^1$H-NMR (400 MHz, DMSO-$d_6$, $\delta$/ppm): 12,46-12,29 (1H, breit), 7,88 (2H, d), 7,74 (2H, d), 7,39 (2H, d), 7,31 (2H, d), 3,83 (3H, s), 2,99-2,83 (2H, m), 2,79-2,56 (3H, m), 1,93-1,67 (2H, m).

MS (DCI): 355 ($M+NH_4^+$).

**Beispiel 73A**

4-[3-(4-Cyanobenzyl)-4-hydroxybutyl]-benzoesäuremethylester

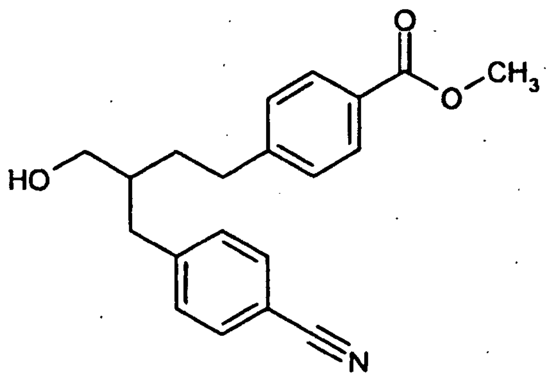

Zu einer Lösung von 8 g (23.71 mmol) 4-[3-Carboxy-4-(4-cyanophenyl)butyl]-benzoesäuremethyl-ester aus Beispiel 72A in 200 ml THF werden 47.43 ml einer 1 M Boran-THF-Komplex-Lösung (47.73 mmol) bei -10°C zugetropft. Nach Erwärmen auf -5°C wird noch 4 Stunden bei dieser Temperatur nachgerührt. Nach vollständiger Umsetzung wird

das Reaktionsgemisch mit gesättigter Natriumhydrogencarbonat-Lösung versetzt und das Lösungsmittel bis zur Trockene eingeengt. Der Rückstand wird in Dichlormethan aufgenommen, über Natriumsulfat getrocknet und wiederum vom Lösungsmittel befreit. Das erhaltene Rohprodukt wird durch Flash-Chromatographie an Kieselgel (Laufmittel: Essigsäureethylester/Cyclohexan 1:10) gereinigt. Es werden 5.8 g (98% Reinheit, 74% Ausbeute) eines farblosen Feststoffes erhalten.

$^1$H-NMR (300 MHz, DMSO-$d_6$, δ/ppm): 7,86 (2H, d), 7,73 (2H, d), 7,38 (2H, d), 7,30 (2H, d), 4,60 (1H, t), 3,83 (3H, s), 3,32 (2H, t), 2,81-2,57 (4H, m), 1,79-1,56 (2H, m), 1,54-1,39 (1H, m).

MS (DCI): 341 ($M+NH_4^+$).

**Beispiel 74A**

4-[3-(4-Cyanobenzyl)-4-oxobutyl]-benzoesäuremethylester

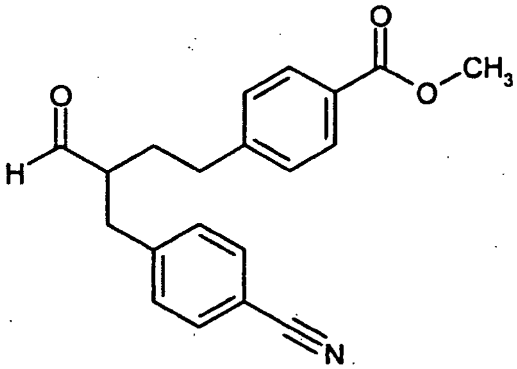

Eine Lösung von 400 mg (1.24 mmol) 4-[3-(4-Cyanobenzyl)-4-hydroxybutyl]-benzoesäuremethyl-ester aus Beispiel 73A in 7 ml Dichlormethan wird mit 320 mg (1.48 mmol) Pyridiniumchlorochromat (PCC) versetzt und 5 Stunden bei Raumtemperatur gerührt. Nach vollständigem Umsatz wird ca. 1 g Kieselgel zugegeben und das Lösungsmittel, im Vakuum bis zur Trockene entfernt. Der Rückstand wird durch Flash-Chromatographie an Kieselgel (Laufmittel: Cyclohexan/Essigsäureethylester 10:1 → 4:1) gereinigt. Es werden 302 mg (90% Reinheit, 69% Ausbeute) eines farblosen Feststoffes erhalten.

$^1$H-NMR (300 MHz, DMSO-$d_6$, δ/ppm): 9,68 (1H, s), 7,87 (2H, d), 7,77 (2H, d), 7,43 (2H, d), 7,31 (2H, d), 3,86 (3H, s), 3,16-3,03 (1H, m), 2,94-2,81 (1H, m), 2,80-2,55 (3H, m), 1,99-1,81 (1H, m), 1,78-1,61 (1H, m).

MS (DCI): 339 ($M+NH_4^+$).

**Beispiel 75A**

*E*-4-[3-(4-Cyanobenzyl)-5-(2-hydroxyphenyl)-pent-4-enyl]-benzoesäuremethylester

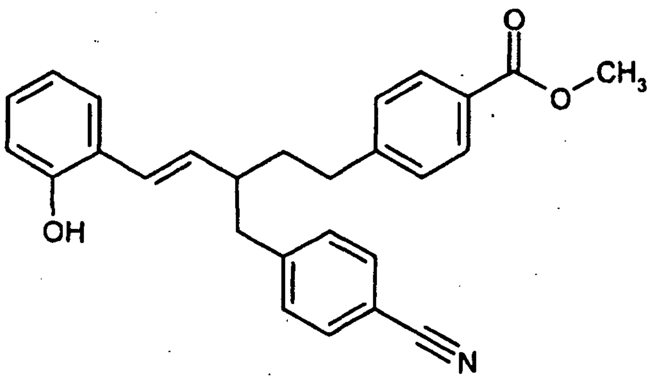

Zu einer Lösung von 1820 mg (4.05 mmol) (2-Hydroxybenzyl)-triphenylphosphoniumbromid in 10 ml wasserfreiem THF werden bei 0°C 5.91 ml (9.45 mmol) einer 1.6 M Lösung von n-Butyllithium in Hexan langsam hinzugefügt. Anschließend werden bei dieser Temperatur 1085 mg (3.38 mmol) 4-[3-(4-Cyanobenzyl)-4-oxobutyl]-benzoesäuremethylester aus Beispiel 74A, gelöst in 10 ml THF, langsam zudosiert. Nach Erwärmung auf Raumtemperatur wird die Reaktionslösung 12 Stunden nachgerührt und anschließend mit etwas Wasser versetzt und bis zur Trockene eingeengt. Der Rückstand wird in Essigsäureethylester aufgenommen, mit Wasser und gesättigter Kochsalz-Lösung gewaschen und über Natriumsulfat getrocknet. Nach Filtration wird das Lösungsmittel bis zur Trockene eingeengt. Das erhaltene Rohprodukt wird durch Flash-Chromatographie an Kiesel-gel (Laufmittel: Cyclohexan/Essigsäureethylester 4:1 → 2:1) gereinigt. Es werden 1150 mg (2.79 mmol, 83% Ausbeute) eines farblosen Feststoffes erhalten.

$^1$H-NMR (300 MHz, DMSO-$d_6$, δ/ppm): 9,47 (1H, s), 7,88 (2H, d), 7,71 (2H, d), 7,42-7,27 (5H, m), 7,01 (1H, t), 6,81-6,68 (2H, m), 6,45 (1H, d), 6,12-6,00 (1H, m), 3,84 (3H, s), 3,42 (2H, m), 2,95-2,56 (3H, m), 1,88-1,56 (2H, m).

MS (DCI): 429 ($M+NH_4^+$).

**Beispiel 76A**

*E*-4-{3-(4-Cyanobenzyl)-5-[2-(4'-trifluormethyl-biphenyl-4-ylmethoxy)phenyl]-pent-4-enyl}-benzoesäuremethylester

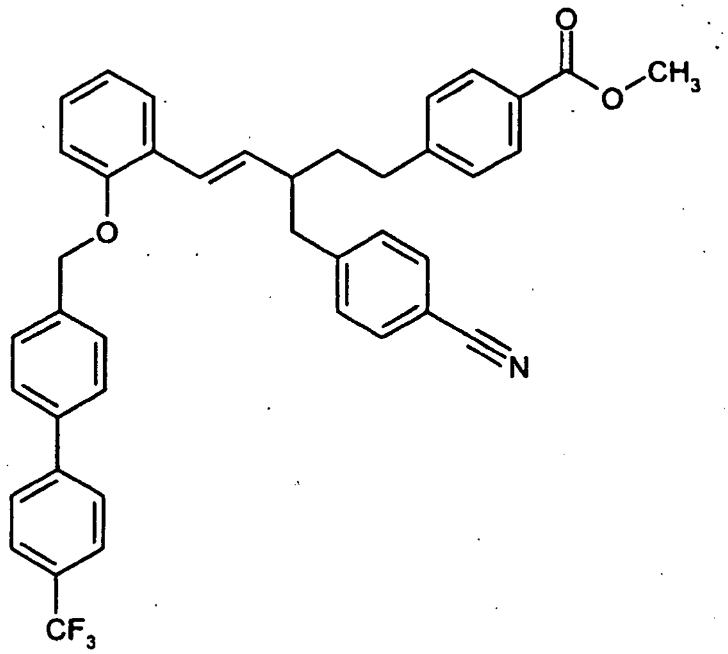

Eine Lösung von 1150 mg (2.79 mmol) *E*-4-[3-(4-Cyanobenzyl)-5-(2-hydroxyphenyl)-pent-4-enyl]-benzoesäuremethylester aus Beispiel 75A in 50 ml trockenem Acetonitril wird mit 908 mg (3.35 mmol) 4-Chlormethyl-4'-trifluormethylbiphenyl aus Beispiel 23A und 579 mg (4.19 mmol) wasserfreiem Kaliumcarbonat versetzt und 12 Stunden zum Rückfluss erhitzt. Anschließend wird der Ansatz zur Trockene eingeengt. Der Rückstand wird in Essigsäureethylester aufgenommen, mit Wasser und gesättigter Kochsalz-Lösung gewaschen und über Natriumsulfat getrocknet. Die organische Phase wird eingeengt. Das erhaltene Rohprodukt wird durch Flash-Chromatographie an Kieselgel (Laufmittel: Cyclohexan/Essigsäureethylester 7:3) gereinigt. Es werden 1620 mg (2.51 mmol, 90% Ausbeute) eines Feststoffs erhalten.

$^1$H-NMR (300 MHz, DMSO-$d_6$, δ/ppm): 7,87 (2H, d), 7,80 (4H, d), 7,77-7,64 (4H, m), 7,51 (2H, d), 7,42 (1H, d), 7,37 (2H, d), 7,28 (2H, d), 7,20 (1H, t), 7,07 (1H, d), 6,92 (1H, t), 6,48 (1H, d), 6,18-6,04 (1H, m), 5,18 (2H, s), 3,78 (3H, s), 2,95-2,82 (1H, m), 2,81-2,41 (4H, m), 1,88-1,55 (2H, m).

LC-MS (Methode 4): $R_t$ 3.57 min; m/z 646 ($M+H^+$).

**Beispiel 77A**

*E*-4-{3-[4-(5-Oxo-4,5-dihydro-[1,2,4]oxadiazol-3-yl)benzyl]-5-[2-(4'-trifluormethyl-biphenyl-4-ylmethoxy)phenyl]-pent-4-enyl}-benzoesäuremethylester

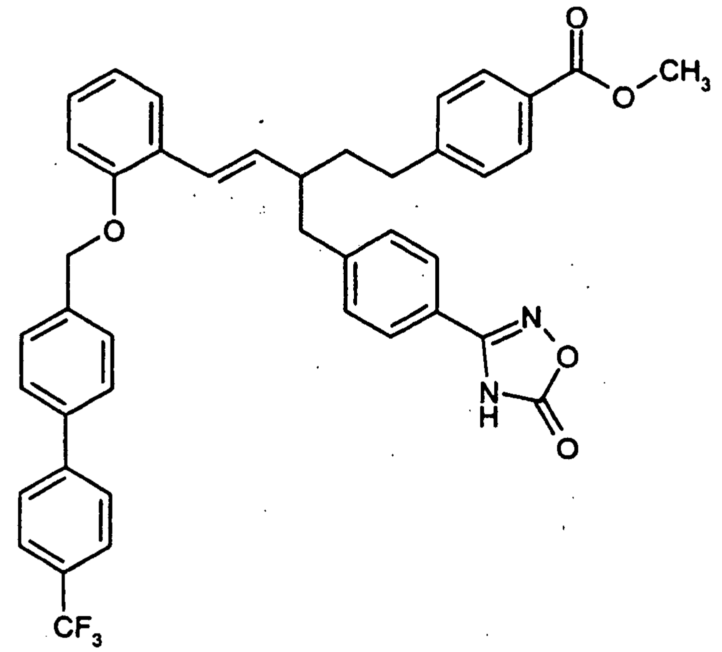

Eine Lösung von 872 mg (12.54 mmol) Hydroxylaminhydrochlorid in 30 ml DMSO wird mit 1.75 ml (12.54 mmol) Triethylamin versetzt und 10 min bei Raumtemperatur gerührt. Der anfallende Niederschlag wird abfiltriert. In das Filtrat werden anschließend 1620 mg (2.51 mmol) *E*-4-{3-(4-Cyanobenzyl)-5-[2-(4'-trifluormethyl-biphenyl-4-ylmethoxy)phenyl]-pent-4-enyl}-benzoesäure-methylester aus Beispiel 76A zudosiert. Die Reaktionslösung wird 12 Stunden bei 75°C gerührt. Nach vollständigem Umsatz und Abkühlung werden zu der Reaktionslösung 20 ml Wasser gegeben, mit Essigsäureethylester dreimal extrahiert und die vereinigten organischen Phasen mit gesättigter Kochsalz-Lösung gewaschen. Anschließend wird die organische Phase über Natriumsulfat getrocknet und bis zur Trockene eingeengt. Es werden 1265 mg eines farblosen Öls erhalten, das ohne weitere Reinigung direkt weiter umgesetzt wird.

Das oben erhaltene Öl wird in 50 ml DMF gelöst und mit 0.18 ml (2.24 mmol) Pyridin versetzt. Anschließend wird die Lösung auf 0°C abgekühlt und langsam mit 333 mg (1.86 mmol) Chlorameisensäure-2-ethylhexylester versetzt. Der Ansatz wird ca. 30 min bei 0°C nachgerührt, dann in Wasser aufgenommen und mit Essigsäureethylester extrahiert. Die organische Phase wird über Natriumsulfat getrocknet und bis zur Trockene eingeengt. Der erhaltene Rückstand wird in 50 ml Xylol aufgenommen und 4 Stunden zum Rückfluss erhitzt. Nach vollständiger Umsetzung wird die Reaktionslösung abgekühlt und bis zur Trockene eingeengt. Das Rohprodukt wird mittels präparativer HPLC gereinigt. Man erhält 1000 mg (71% Ausbeute) eines farblosen Öls.

$^1$H-NMR (400 MHz, DMSO-$d_6$, δ/ppm): 7,90-7,77 (6H, m), 7,74-7,64 (4H, t), 7,49 (2H, d), 7,42 (1H, d), 7,34 (2H, d), 7,29 (2H, d), 7,20 (1H, t), 7,08 (1H, d), 6,92 (1 H, t), 6,51 (1H, d), 6,19-6,08 (1H, m), 5,18 (2H, s), 3,79 (3H, s), 2,92-2,81 (1H, m), 2,79-2,44 (4H, m), 1,89-1,76 (1H, m), 1,75-1,62 (1H, m).

MS (EI): 703 (M-H$^-$).

**Beispiel 78A**

[2-(2-Chlorbenzyloxy)phenyl]methanol

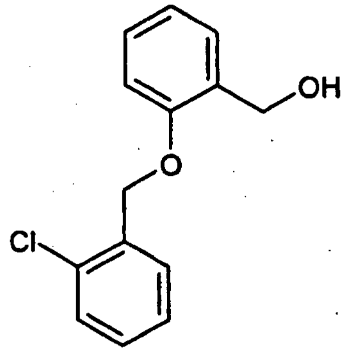

Eine Lösung von 2.42 g (19.47 mmol) 2-Hydroxybenzylalkohol in 100 ml trockenem Acetonitril wird mit 4.12 g (19.47 mmol) 2-Chlorbenzylbromid und 2.96 g (21.42 mmol) wasserfreiem Kaliumcarbonat versetzt und 12 Stunden zum Rückfluss erhitzt. Anschließend wird der Ansatz zur Trockene eingeengt. Der Rückstand wird in Essigsäureethylester aufgenommen, mit Wasser und gesättigter Kochsalz-Lösung gewaschen und über Natriumsulfat getrocknet. Die organische Phase wird eingeengt. Das erhaltene Rohprodukt wird durch Flash-Chromatographie an Kieselgel (Lauf-mittel: Cyclohexan/Essigsäureethylester 7:3) gereinigt. Es werden 3.856 g (15.5 mmol, 79% Ausbeute) eines Feststoffs erhalten.

$^1$H-NMR (300 MHz, DMSO-$d_6$, δ/ppm): 7,66-7,56 (1H, m), 7,55-7,48 (1H, m), 7,45-7,33 (3H, m), 7,21 (1H, t), 7,08-6,91 (2H, m), 5,18 (2H, s), 5,02 (1H, t), 4,56 (2H, d).

LC-MS (Methode 2): $R_t$ 2.22 min; m/z 231 [M+H-$H_2O^+$].

**Beispiel 79A**

[2-(2-Chlorbenzyloxy)benzyl]-triphenylphosphoniumbromid

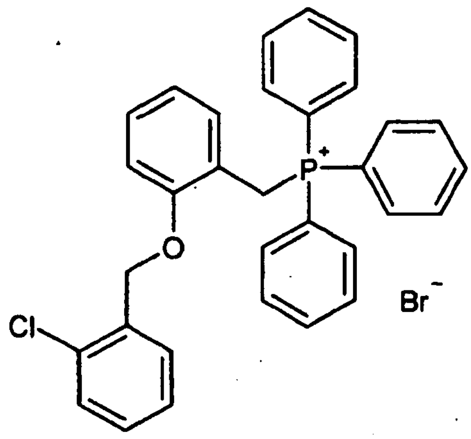

Eine Lösung von 3.775 g (15.18 mmol) [2-(2-Chlorbenzyloxy)phenyl]methanol aus Beispiel 78A in 60 ml Acetonitril wird mit 4.949 g (14.42 mmol) Triphenylphosphoniumhydrobromid versetzt und 3 Stunden zum Rückfluss erhitzt. Anschließend wird die Reaktionslösung bis zur Trockene eingeengt und das erhaltene Öl in Diethylether aufgenommen und verrieben. Dabei kristallisiert das Produkt als weißer Feststoff aus. Nach Filtration wird der Feststoff bei 50°C im Trockenofen über Nacht getrocknet. Es werden 8.247 g (91% Ausbeute) kristallines Produkt erhalten.

$^1$H-NMR (300 MHz, DMSO-$d_6$, δ/ppm): 7,87 (3H, t), 7,71-7,46 (13H, m), 7,43-7,28 (4H, m), 7,10 (1H, d), 6,96 (1H, d), 6,89 (1H, t), 5,02 (2H, d), 4,64 (2H, s).

**Beispiel 80A**

[2-(2-Trifluormethyl-benzyloxy)phenyl]methanol

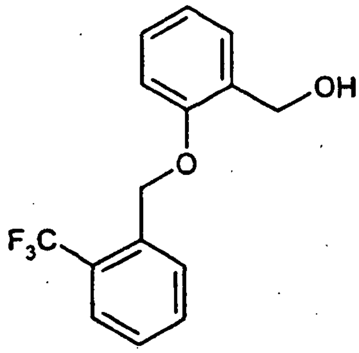

Eine Lösung von 2.42 g (19.47 mmol) 2-Hydroxybenzylalkohol in 100 ml trockenem Acetonitril wird mit 3.91 g (19.47 mmol) 2-Trifluormethylbenzylbromid und 2.96 g (21.42 mmol) wasserfreiem Kaliumcarbonat versetzt und 12 Stunden zum Rückfluss erhitzt. Anschließend wird der Ansatz zur Trockene eingeengt. Der Rückstand wird in Essigsäureethylester aufgenommen, mit Wasser und gesättigter Kochsalz-Lösung gewaschen und über Natriumsulfat getrocknet. Die organische Phase wird eingeengt. Das erhaltene Rohprodukt wird durch Flash-Chromatographie an Kieselgel (Laufmittel: Cyclohexan/Essigsäureethylester 7:3) gereinigt. Es werden 5.08 g (87% Ausbeute) eines Feststoffs erhalten.

$^1$H-NMR (300 MHz, DMSO-$d_6$, $\delta$/ppm): 7,85-7,67 (3H, m), 7,59 (1H, t), 7,41 (1H, d), 7,21 (1H, t), 7,04-6,89 (2H, m), 5,24 (2H, s), 5,02 (1H, t), 4,53 (2H, d).

MS (DCI): 300 ($M+NH_4^+$).

**Beispiel 81A**

Triphenyl-[2-(2-trifluormethyl-benzyloxy)benzyl]-phosphoniumbromid

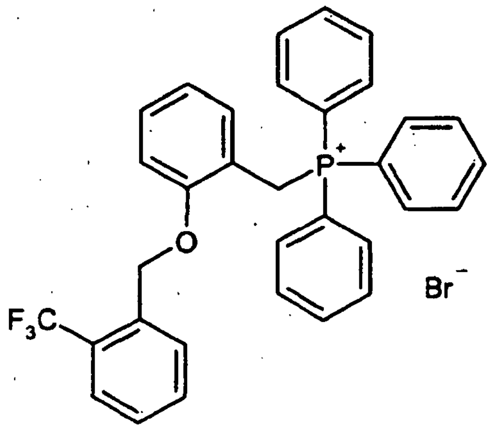

Eine Lösung von 4.913 g (17.41 mmol) [2-(2-Trifluormethyl-benzyloxy)phenyl]methanol aus Beispiel 80A in 70 ml Acetonitril wird mit 5.675 g (16.54 mmol) Triphenylphosphoniumhydrobromid versetzt und 3 Stunden zum Rückfluss erhitzt. Anschließend wird die Reaktionslösung bis zur Trockene eingeengt und das erhaltene Öl in Diethylether aufgenommen und verrieben. Dabei kristallisiert das Produkt als weißer Feststoff aus. Nach Filtration wird der Feststoff bei 50°C im Trockenofen über Nacht getrocknet. Es werden 9.621 g (88% Ausbeute) kristallines Produkt erhalten.

$^1$H-NMR (300 MHz, DMSO-$d_6$, $\delta$/ppm): 7,84 (3H, t), 7,79 (1H, d), 7,70-7,49 (15H, m), 7,46 (1H, d), 7,32 (1H, t), 7,10 (1H, d), 6,89 (1H, t), 5,02 (2H, d), 4,72 (2H, s).

**Beispiel 82A**

2-Allyloxycarbonyl-heptandisäure-1-allylester-7-ethylester

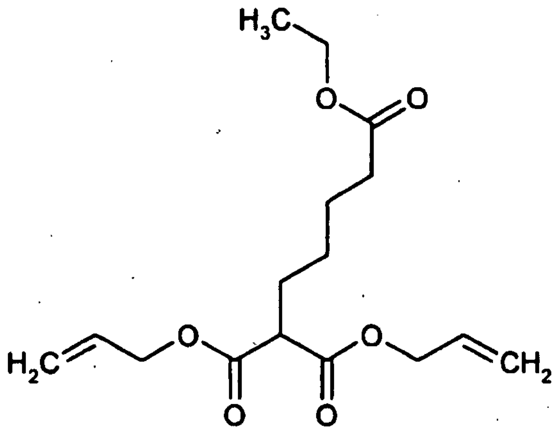

Zu einer Lösung von 100 g (542.92 mmol) Malonsäurediallylester in 900 ml trockenem Dioxan werden bei 5°C 16.29 g (407.19 mmol) Natriumhydrid portionweise zugegeben. Nach Beendigung der Gasentwicklung wird das Reaktionsgemisch auf 40°C erwärmt und 30 min nachgerührt. Anschließend werden 56.76 g (271.46 mmol) 5-Bromvaleriansäureethylester in 100 ml trockenem Dioxan zugetropft und die Mischung 12 Stunden bei 110°C gerührt. Nach Beendigung der Reaktion wird der Ansatz auf Raumtemperatur abgekühlt und auf ca. 400 ml Eiswasser gegeben. Nach Neutralisation des Reaktionsgemisches mit 1 N Salzsäure wird die organische Phase abgetrennt und die wässrige Phase dreimal mit jeweils 250 ml Essigsäureethylester extrahiert. Nach Vereinigung der organischen Phasen wird mit gesättigter Kochsalz-Lösung gewaschen und über Natriumsulfat getrocknet. Nach Filtration wird die Reaktionslösung im Vakuum eingeengt. Darauf folgend wird durch Hochvakuum-Destillation überschüssiger Malonsäurediallylester entfernt (Siedepunkt: 57°C, 0.074 mbar). Der Destillationsrückstand wird durch Flash-Chromatographie an Kieselgel (Laufmittel: Cyclohexan/Essigsäureethylester 2:1) gereinigt. Es werden 73.92 g (236.65 mmol, 44% d. Th.) einer farblosen Flüssigkeit erhalten.

$^1$H-NMR (400 MHz, DMSO-$d_6$, $\delta$/ppm): 5,99-5,81 (2H, m), 5,38-5,16 (4H, m), 4,68-4,51 (4H, m), 4,04 (2H, q), 3,59 (1H, t), 2,28 (2H, t), 1,86-1,71 (2H, m), 1,61-1,45 (2H, m), 1,35-1,20 (2H, m), 1,17 (3H, t).

MS (DCI): 330 ($M+NH_4^+$).

__Beispiel 83A__

2-Allyloxycarbonyl-2-[2-(4-cyanophenyl)ethyl]-heptandisäure-1-allylester-7-ethylester

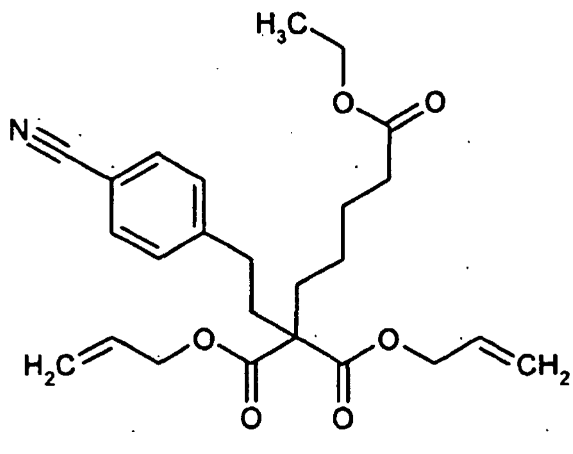

Eine Lösung von 45.23 g (144.79 mmol) 2-Allyloxycarbonyl-heptandisäure-1-allylester-7-ethyl-ester. aus Beispiel 82A in 250 ml DMF wird bei 0°C portionsweise mit 6.37 g (159.27 mmol; Gehalt 60%) Natriumhydrid versetzt. Anschließend lässt man die Reaktionslösung auf Raumtemperatur kommen und 30 min lang nachrühren. Die Reaktionslösung wird dann wieder auf 0°C abgekühlt, mit 36.50 g (173.75 mmol) 4-(2-Bromethyl)benzonitril aus Beispiel 8A in 250 ml DMF versetzt und 30 min bei dieser Temperatur nachgerührt. Der Ansatz wird danach bei Raumtemperatur über Nacht gerührt. Das Reaktionsgemisch wird tropfenweise mit Wasser versetzt, mit Essigsäureethylester dreimal

extrahiert, die vereinigten organischen Phasen mit gesättigter Kochsalz-Lösung gewaschen und über Natriumsulfat getrocknet. Nach Filtration wird das Lösungsmittel im Vakuum bis zur Trockene eingeengt. Das erhaltene Rohprodukt wird durch Flash-Chromatographie an Kieselgel (Laufmittel: Cyclohexan/Essigsäureethylester 10:1 → 4:1 → 1:1) gereinigt. Es werden 17.85 g (40.43 mol, 28% Ausbeute) eines farblosen Feststoffes erhalten.

$^1$H-NMR (400 MHz, DMSO-$d_6$, δ/ppm): 7,77 (2H, d), 7,42 (2H, d), 5,97-5,82 (2H, m), 5,37-5,18 (4H, m), 4,60 (4H, d), 4,04 (2H, q), 2,59-2,45 (2H, m), 2,30 (2H, t), 2,14-2,02 (2H, m), 1,96-1,83 (2H, m), 1,60-1,47 (2H, m), 1,24-1,07 (5H, m).

MS (DCI): 459 ($M+NH_4^+$).

**Beispiel 84A**

2-[2-(4-Cyanophenyl)ethyl]-heptandisäure-7-ethylester

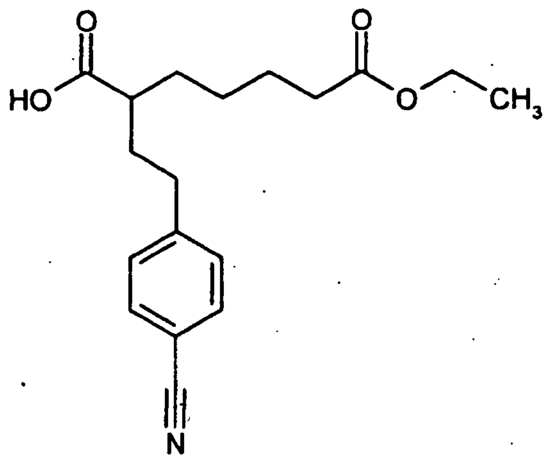

Eine Lösung von 21 g (40.43 mmol) 2-Allyloxycarbonyl-2-[2-(4-cyanophenyl)ethyl]-heptandisäure-1-allylester-7-ethylester aus Beispiel 83A, 742 mg (2.83 mmol) Triphenylphosphin und 181 mg (0.81 mmol) Palladiumacetat in 175 ml Dioxan wird bei Raumtemperatur mit einer Lösung von 18.6 ml (133.4 mmol) Triethylamin und 3.81 ml (101.1 mmol) Ameisensäure in 175 ml Dioxan versetzt. Das Reaktionsgemisch wird anschließend bei 100°C 12 Stunden lang gerührt. Nach vollständigem Umsatz wird die Reaktionslösung abgekühlt und das Lösungsmittel im Vakuum entfernt. Anschließend wird der Rückstand in Essigsäureethylester und Wasser aufgenommen, mit 1 N Salzsäure angesäuert und die organische Phase abgetrennt. Die wässrige Phase wird dreimal mit Essigsäureethylester extrahiert, die organischen Phasen vereinigt, mit gesättigter Kochsalz-Lösung gewaschen und über Natriumsulfat getrocknet. Nach Filtration wird die Reaktionslösung im Vakuum eingeengt. Das erhaltene Rohprodukt wird durch Flash-Chromatographie an Kieselgel (Laufmittel: Cyclohexan/Essigsäureethylester 4:1 → 1:1) gereinigt. Es werden 8.6 g (64% Ausbeute, 95% Reinheit) eines farblosen Feststoffes erhalten.

$^1$H-NMR (400 MHz, DMSO-$d_6$, δ/ppm): 12,25-12,09 (1H, breit), 7,76 (2H, d), 7,40 (2H, d), 4,04 (2H, q), 2,71-2,57 (2H, m), 2,30-2,14 (4H, m), 1,87-1,74 (1H, m), 1,73 (1H, m), 1,58-1,38 (3H, m), 1,31-1,19 (2H, m), 1,18 (3H, t).

MS (EI): 316 ($M-H^-$).

**Beispiel 85A**

8-(4-Cyanophenyl)-6-hydroxymethyl-octansäureethylester

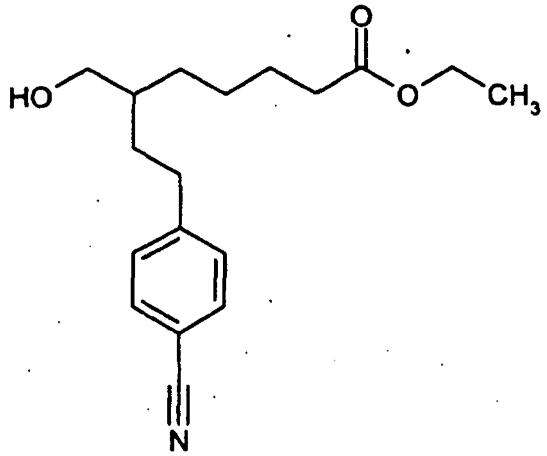

Zu einer Lösung von 8.6 g (27.1 mmol) 2-[2-(4-Cyanophenyl)ethyl]-heptandisäure-7-ethylester aus Beispiel 84A in 200 ml THF werden 54.19 ml einer 1 M Boran-THF-Komplex-Lösung (54.19 mmol) bei -10°C zugetropft. Nach Erwärmen auf 0°C wird noch 2 Stunden bei dieser Temperatur nachgerührt. Nach vollständiger Umsetzung wird das Reaktionsgemisch mit gesättigter Natriumhydrogencarbonat-Lösung versetzt und das Lösungsmittel bis zur Trockene eingeengt. Der Rückstand wird in Dichlormethan aufgenommen, über Natriumsulfat getrocknet und wiederum vom Lösungsmittel befreit. Das erhaltene Rohprodukt wird durch Flash-Chromatographie an Kieselgel (Laufmittel: Essigsäureethylester/Cyclohexan 1:10 → 1:4) gereinigt. Es werden 5.1 g (97% Reinheit, 60% Ausbeute) eines farblosen Feststoffes erhalten.

$^1$H-NMR (300 MHz, DMSO-$d_6$, ö/ppm): 7,74 (2H, d), 7,41 (2H, d), 4,41 (1H, t), 4,03 (2H, q), 3,41-3,29 (2H, m), 2,67 (2H, t), 2,28 (2H, t), 1,69-1,11 (9H, m), 1,18 (3H, t).

MS (DCI): 321 ($M+NH_4^+$).

**Beispiel 86A**

8-(4-Cyanophenyl)-6-formyl-octansäureethylester

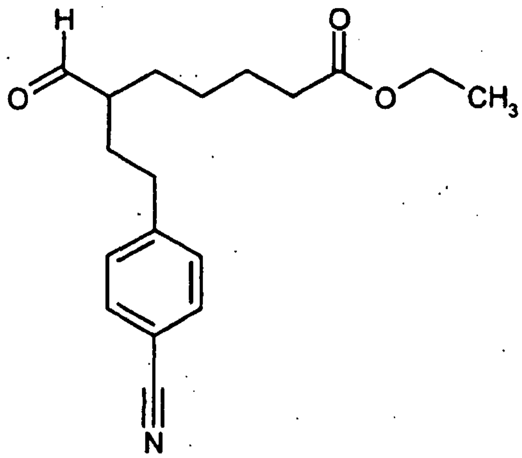

Eine Lösung von 4 g (13.18 mmol) 8-(4-Cyanophenyl)-6-hydroxymethyl-octansäureethylester aus Beispiel 85A in 100 ml Dichlormethan wird mit 3.41 g (15.82 mmol) Pyridiniumchlorochromat (PCC) versetzt und 12 Stunden bei Raumtemperatur gerührt. Nach vollständigem Umsatz wird das Lösungsmittel im Vakuum bis zur Trockene eingeengt. Der erhaltene Rückstand wird durch Flash-Chromatographie an Kieselgel (Laufmittel: Cyclohexan/Essigsäureethylester 10:1 → 4:1) gereinigt. Es werden 2.74 g (9.09 mmol, 69% Ausbeute) eines farblosen Feststoffes erhalten.

LC-MS (Methode 2): $R_t$ 2.38 min; m/z 302 ($M+H^+$).

**Beispiel 87A**

*E*-6-[2-(4-Cyanophenyl)ethyl]-8-(2-hydroxyphenyl)-oct-7-ensäureethylester

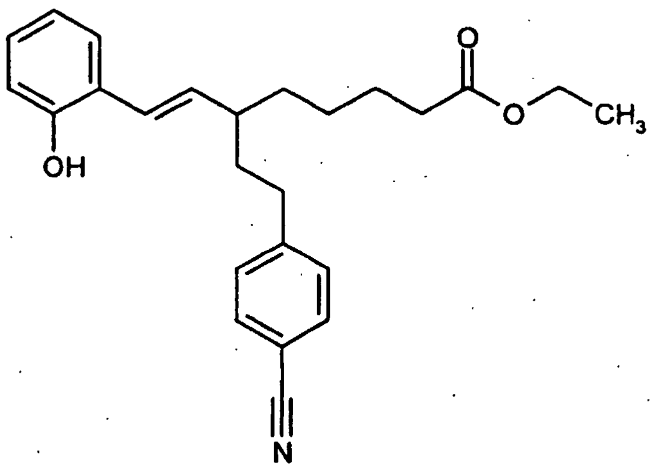

Zu einer Lösung von 5.066 g (10.91 mmol) (2-Hydroxybenzyl)-triphenylphosphoniumbromid in 38 ml wasserfreiem THF werden bei 0°C 15.91 ml (25.45 mmol) einer 1.6 M Lösung von n-Butyl-lithium in Hexan langsam hinzugefügt. Anschließend werden bei dieser Temperatur 2.740 g (9.09 mmol) 8-(4-Cyanophenyl)-6-formyl-octansäureethylester aus Beispiel 86A, gelöst in 38 ml THF, langsam zudosiert. Nach Erwärmung auf Raumtemperatur wird die Reaktionslösung 12 Stunden nachgerührt, dann mit etwas Wasser versetzt und bis zur Trockene eingeengt. Der Rückstand wird in Essigsäureethylester aufgenommen, mit Wasser und gesättigter Kochsalz-Lösung gewaschen und über Natriumsulfat getrocknet. Nach Filtration wird das Lösungsmittel bis zur Trockene eingeengt. Das erhaltene Rohprodukt wird durch Flash-Chromatographie an Kieselgel (Laufmittel: Cyclohexan/Essigsäureethylester 10:1 → 4:1) gereinigt. Es werden 2.30 g (5.88 mmol, 63% Ausbeute) eines farblosen Feststoffes erhalten.

$^1$H-NMR (400 MHz, DMSO-$d_6$, δ/ppm): 9,48 (1H, s), 7,73 (2H, d), 7,42 (2H, d), 7,38 (1H, d), 7,03 (1H, t), 6,80 (1H, d), 6,74 (1H, t), 6,57 (1 H, d), 6,06-5,93 (1H, m), 4,03 (2H, q), 2,76-2,57 (2H, m), 2,26 (2H, t), 2,17-2,02 (1H, m), 1,80-1,68 (1H, m), 1,67-1,56 (1H, m), 1,56-1,39 (3H, m), 1,38-1,19 (3H, m), 1,13 (3H, t).

MS (DCI): 409 ($M+NH_4^+$).

**Beispiel 88A**

*E*-8-[2-(4-*tert.*-Butylbenzyloxy)phenyl]-6-[2-(4-cyanophenyl)ethyl]-oct-7-ensäureethylester

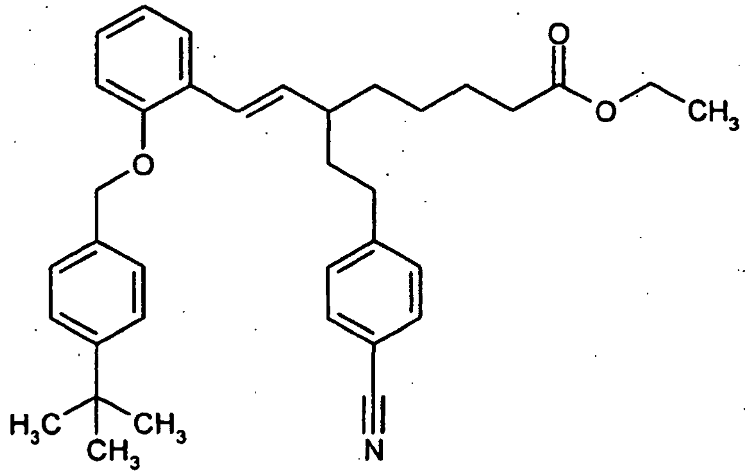

Eine Lösung von 2300 mg (5.87 mmol) E-6-[2-(4-Cyanophenyl)ethyl]-8-(2-hydroxyphenyl)-oct-7-ensäureethylester aus Beispiel 87A in 160 ml trockenem Acetonitril wird mit 1600 mg (7.05 mmol) 4-(*tert*.-Bütyl)benzylbromid und 1220 mg (8.81 mmol) wasserfreiem Kaliumcarbonat versetzt und 12 Stunden zum Rückfluss erhitzt. Anschließend wird der Ansatz zur Trockene eingeengt. Der Rückstand wird in Essigsäureethylester aufgenommen, mit Wasser und gesättigter Kochsalz-Lösung gewaschen und über Natriumsulfat getrocknet. Die organische Phase wird eingeengt. Das erhaltene Rohprodukt wird durch Flash-Chromatographie an Kieselgel (Laufmittel: Cyclo-hexan/Essigsäureethylester 10: 1) gereinigt. Es werden 2800 mg (5.21 mmol, 88% Ausbeute) eines Feststoffs erhalten.

$^1$H-NMR (300 MHz, DMSO-$d_6$, δ/ppm): 7,68 (2H, d), 7,45 (1H, d), 7,41-7,32 (6H, m), 7,20 (1H, t), 7,09 (1H, d), 6,91 (1H, t), 6,61 (1H, d), 6,08-5,95 (1H, m), 5,10 (2H, s), 4,00 (2H, q), 2,77-2,45 (3H, m), 2,23 (2H, t), 2,12-1,98 (1H, m), 1,78-1,37 (5H, m), 1,32-1,19 (2H, m), 1,28 (9H, s), 1,13 (3H, t).

MS (DCI): 555 ($M+NH_4^+$).

**Beispiel 89A**

*E*-8-[2-(4-*tert*.-Butylbenzyloxy)phenyl]-6-{2-[4-(5-oxo-4,5-dihydro-[1,2,4]oxadiazol-3-yl)phenyl]-ethyl}-oct-7-ensäureethylester

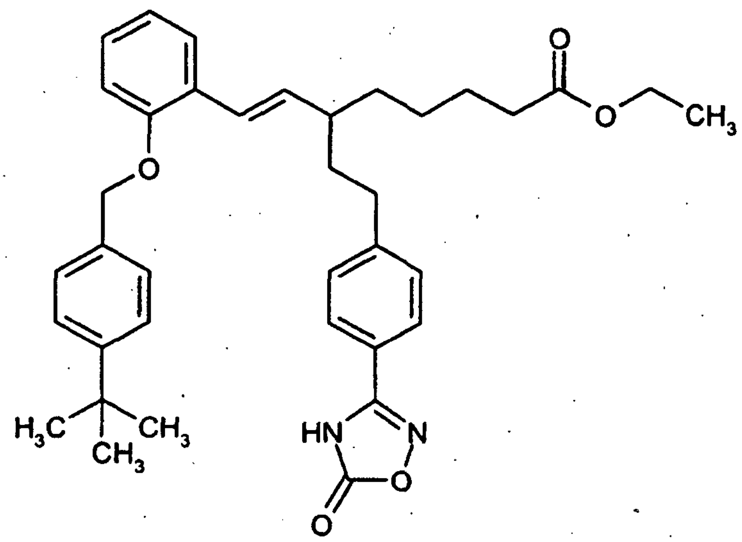

Eine Lösung von 1.16 g (16.74 mmol) Hydroxylaminhydrochlorid in 20 ml DMSO wird mit 2.33 ml (16.74 mmol) Triethylamin versetzt und 10 min bei Raumtemperatur gerührt. Der anfallende Niederschlag wird abfiltriert. In das Filtrat werden anschließend 1.8 g (3.35 mmol) *E*-8-[2-(4-*tert*.-Butyl-benzyloxy)phenyl]-6-[2-(4-cyanophenyl)ethyl]-oct-7-ensäureethylester aus Beispiel 88A zudosiert. Die Reaktionslösung wird 12 Stunden bei 75°C gerührt. Nach vollständigem Umsatz und Abkühlung werden zu der Reaktionslösung 20 ml Wasser gegeben, mit Essigsäureethylester dreimal extrahiert und die vereinigten organischen Phasen mit gesättigter Kochsalz-Lösung gewaschen. Anschließend wird die organische Phase über Natriumsulfat getrocknet und bis zur Trockene eingeengt. Es werden 1.6 g eines farblosen Öls erhalten, das ohne weitere Reinigung direkt weiter umgesetzt wird.

Das oben erhaltene Öl wird in 20 ml DMF gelöst und mit 0.27 ml (3.36 mmol) Pyridin versetzt. Anschließend wird die Lösung auf 0°C abgekühlt und langsam mit 500 mg (2.80 mmol) Chlorameisensäure-2-ethylhexylester versetzt. Der Ansatz wird ca. 30 min bei 0°C nachgerührt; dann in Wasser aufgenommen und mit Essigsäureethylester extrahiert. Die organische Phase wird über Natriumsulfat getrocknet und bis zur Trockene eingeengt. Der erhaltene Rückstand wird in 20 ml Xylol aufgenommen und 2 Stunden zum Rückfluss erhitzt. Nach vollständiger Umsetzung wird die Reaktionslösung abgekühlt und bis zur Trockene eingeengt. Das erhaltene Rohprodukt wird durch Flash-Chromatographie an Kieselgel (Laufmittel: Cyclohexan/Essigsäureethylester 4:1 → 1:1) gereinigt. Es werden 1.19 g (70% Ausbeute) eines Feststoffs erhalten.

$^1$H-NMR (400 MHz, DMSO-$d_6$, δ/ppm): 13,01-12,78 (1H, breit), 7,70 (2H, d), 7,48 (1H, d), 7,42-7,31 (6H, m), 7,20 (1H, t), 7,09 (1H, d), 6,91 (1H, t), 6,63 (1H, d), 6,11-5,98 (1H, m), 5,11 (2H, s), 3,99 (2H, q), 2,76-2,46 (2H, m), 2,24 (2H, t), 2,17-2,02 (1H, m), 1,82-1,69 (1H, m), 1,68-1,55 (1H, m), 1,54-1,38 (3H, m), 1,37-1,17 (3H, m), 1,28 (9H, s), 1,13 (3H, t).

MS (DCI): 614 ($M+NH_4^+$).

**Beispiel 90A**

2-(4-Cyanobutyl)-malonsäurediallylester

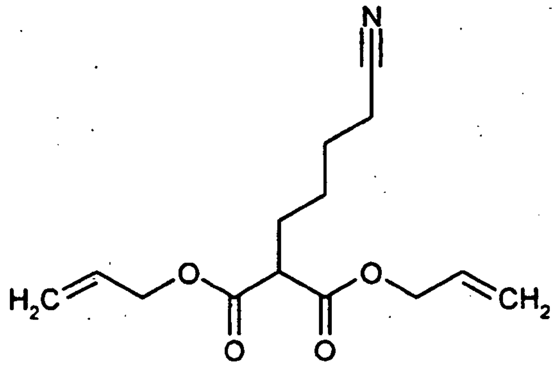

Zu einer Lösung von 100 g (542.9 mmol) Malonsäurediallylester in 700 ml trockenem Dioxan werden bei 0°C 21.71 g (542.9 mmol; 60%-ig) Natriumhydrid portionsweise zugegeben. Nach Beendigung der Gasentwicklung wird das Reaktionsgemisch auf 40°C erwärmt und 1 Stunde nachgerührt. Anschließend werden 43.98 g (271.5 mmol) 5-Bromvaleriansäurenitril in 350 ml trockenem Dioxan zugetropft und die Mischung 12 Stunden bei 110°C gerührt. Nach Beendigung der Reaktion wird der Ansatz auf Raumtemperatur abgekühlt, mit 400 ml gesättigter Ammonium-chlorid-Lösung versetzt und mit Essigsäureethylester extrahiert. Nach Phasentrennung wird die wässrige Phase dreimal mit jeweils 250 ml Essigsäureethylester nachextrahiert. Nach Vereinigung der organischen Phasen wird mit gesättigter Kochsalz-Lösung gewaschen, über Natriumsulfat getrocknet und anschließend das Lösungsmittel im Vakuum abgezogen. Darauf folgend wird durch Hochvakuum-Destillation überschüssiger Malonsäurediallylester entfernt (Siedepunkt: 57°C; 0.074 mbar). Der Destillationsrückstand wird durch Flash-Chromatographie an Kieselgel (Lauf-mittel: Cyclohexan/Essigsäureethylester 2:1) gereinigt. Es werden 105 g (233 mmol, ca. 60% Reinheit, 43% Ausbeute) einer Flüssigkeit erhalten. Dieses Produkt wird ohne weitere Aufreinigung in der Folgestufe umgesetzt. Eine kleine Menge wird mittels präparativer HPLC für die analytische Charakterisierung aufgereinigt.

$^1H$-NMR (300 MHz, DMSO-$d_6$, $\delta$/ppm): 5,96-5,83 (2H, m), 5,35-5,19 (4H, m), 4,64-4,59 (4H, m), 3,66-3,61 (1H, t), 2,54-2,47 (2H, t), 1,86-1,75 (2H, m), 1,62-1,50 (2H, m), 1,42-1,29 (2H, m).

LC-MS (Methode 1): $R_t$ 2.44 min, m/z 266 $(M+H)^+$.

**Beispiel 91A**

2-(4-Cyanobutyl)-2-[2-(4-methoxycarbonyl-phenyl)ethyl]-malonsäurediallylester

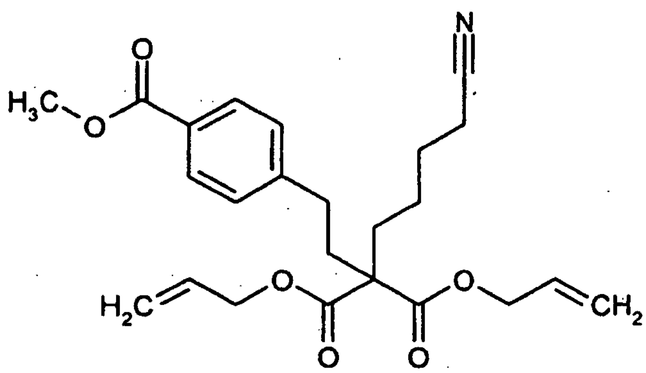

Eine Lösung von 48.64 g (127.73 mmol) 2-(4-Cyanobutyl)-malonsäurediallylester aus Beispiel 90A in 160 ml

trockenem DMF wird bei 0°C portionsweise mit 5.62 g (140 mmol; 60% Gehalt) Natriumhydrid versetzt. Anschließend lässt man die Reaktionslösung auf Raumtemperatur kommen und rührt 90 min nach. Die Reaktionslösung wird dann wieder auf 0°C abgekühlt, mit 45.72 g (153.3 mmol) 4-(2-Bromethyl)benzoesäuremethylester in 80 ml trockenem DMF versetzt und 45 min bei dieser Temperatur nachgerührt. Der Ansatz wird danach bei Raumtemperatur über Nacht gerührt. Das Reaktionsgemisch wird mit Wasser versetzt und mit Essigsäureethylester extrahiert. Nach Phasentrennung wird die wässrige Phase dreimal mit jeweils 200 ml Essigsäureethylester extrahiert. Die vereinigten organischen Phasen wird mit gesättigter Kochsalz-Lösung gewaschen, über Natriumsulfat getrocknet und anschließend das Lösungsmittel im Vakuum abgezogen. Das erhaltene Rohprodukt wird mittels Kieselgel-Chromatographie (Laufmittel: Cyclohexan/ Essigsäureethylester 10:1 $\rightarrow$ 100% Essigsäureethylester) aufgereinigt. Es werden 18.53 g (43.3 mmol, 34% Ausbeute) einer farblosen Flüssigkeit erhalten.

$^1$H-NMR (300 MHz, DMSO-$d_6$, $\delta$/ppm): 7,90-7,88 (2H, d), 7,36-7,34 (2H, d), 5,97-5,83 (2H, m), 5,35-5,21 (4H, m), 4,64-4,59 (4H, m), 3,84 (3H, s), 2,57-2,48 (4H, t), 2,15-2,09 (2H, m), 1,98-1,89 (2H, m), 1,63-1,52 (2H, m), 1,34-1,21 (2H, m).

LC-MS (Methode 2): $R_t$ 2.59 min; m/z 428 $(M+H)^+$.

**Beispiel 92A**

4-(3-Carboxy-7-cyanoheptyl)-benzoesäuremethylester

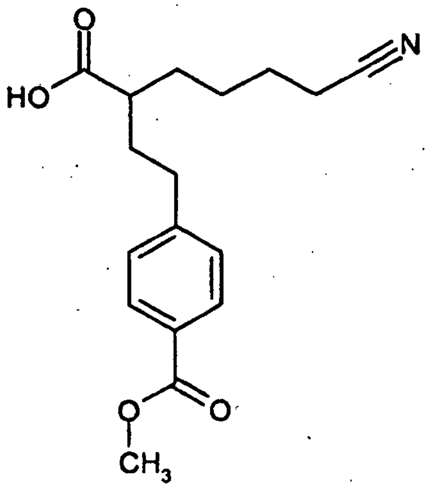

Eine Lösung von 6.92 g (16.19 mmol) 2-(4-Cyanobutyl)-2-[2-(4-methoxycarbonyl-phenyl)ethyl]-malonsäurediallylester aus Beispiel 91A, 594 mg (2.26 mmol) Triphenylphosphin und 145 mg (0.64 mmol) Palladiumacetat in 67 ml Dioxan wird bei Raumtemperatur mit einer Lösung von 7.45 ml (53.42 mmol) Triethylamin und 1.53 ml (40.47 mmol) Ameisensäure in 67 ml Dioxan versetzt. Das Reaktionsgemisch wird anschließend bei 100°C 12 Stunden lang gerührt. Nach vollständigem Umsatz wird die Reaktionslösung abgekühlt und das Lösungsmittel im Vakuum entfernt. Der Rückstand wird in Essigsäureethylester und Wasser aufgenommen, mit 1 N Salzsäure angesäuert und die organische Phase abgetrennt. Die wässrige Phase wird dreimal mit Essigsäureethylester extrahiert, die organischen Phasen anschließend vereinigt, mit gesättigter Kochsalz-Lösung gewaschen und über Natriumsulfat getrocknet. Nach Filtration wird die organische Phase im Vakuum eingeengt. Das erhaltene Rohprodukt wird durch Flash-Chromatographie an Kieselgel (Laufmittel: Cyclohexan/Essigsäureethylester 2:1 $\rightarrow$ 1:3, mit 1% Ameisensäure) gereinigt. Es werden 2.1 g (43% Ausbeute, 100% Reinheit) eines farblosen Feststoffes erhalten.

LC-MS (Methode 2): R, 1.88 min; m/z 303 $(M^+)$.

**Beispiel 93A**

4-(7-Cyano-3-hydroxymethyl-heptyl)-benzoesäuremethylester

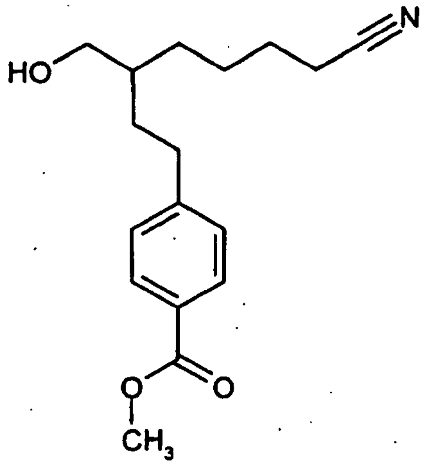

Zu einer Lösung von 5 g (16.48 mmol) 4-(3-Carboxy-7-cyanoheptyl)-benzoesäuremethylester aus Beispiel 92A in 62 ml THF werden 33 ml einer 1 M Boran-THF-Komplex-Lösung (33 mmol) bei -15°C zugetropft und die Lösung 2 Stunden bei dieser Temperatur gerührt. Anschließend werden nochmals 16 ml 1 M Boran-THF-Komplex-Lösung zugetropft und weitere 45 Minuten nachgerührt. Nachfolgend wird die Reaktionsmischung auf 0°C erwärmt und bei dieser Temperatur noch 1 Stunde nachgerührt. Nach vollständiger Umsetzung wird das Reaktionsgemisch mit 100 ml gesättigter Natriumhydrogencarbonat-Lösung versetzt und das Lösungsmittel bis zur Trockene eingeengt. Der Rückstand wird in Essigsäureethylester und Wasser aufgenommen und die wässrige Phase mit Essigsäureethylester extrahiert. Die vereinigten organischen Phasen wird über Natriumsulfat getrocknet und wiederum vom Lösungsmittel befreit. Das erhaltene Rohprodukt wird durch Flash-Chromatographie an Kieselgel (Laufmittel: Cyclohexan/Essigsäureethylester 2:1 → 1:3 → 100% Essigsäureethylester) gereinigt. Es werden 2.4 g (93% Reinheit, 47% Ausbeute) eines farblosen Feststoffes erhalten.

$^1$H-NMR (400 MHz, DMSO-$d_6$, δ/ppm): 7,88-7,86 (2H, d), 7,36-7,34 (2H, d), 4,43-4,40 (1H, t), 3,83 (3H, s), 3,37-3,34 (2H, t), 2,68-2,64 (2H, t), 2,49-2,46 (2H, t), 1,67-1,57 (1H, m), 1,56-1,45 (3H, m), 1,42-1,25 (5H, m).

LC-MS (Methode 2): $R_t$ 1.93 min; m/z 290 $(M+H)^+$.

__Beispiel 94A__

4-(7-Cyano-3-formyl-heptyl)-benzoesäuremethylester

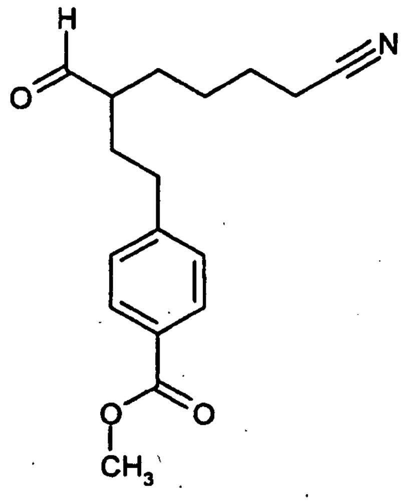

Eine Lösung von 2.23 g (7.71 mmol) 4-(7-Cyano-3-hydroxymethyl-heptyl)-benzoesäuremethylester aus Beispiel 93A in 100 ml Dichlormethan wird mit 1.99 g (9.26 mmol) Pyridiniumchlorochromat (PCC) versetzt und 6 Stunden bei Raumtemperatur gerührt. Nach vollständigem Umsatz wird das Lösungsmittel im Vakuum bis zur Trockene eingeengt. Der erhaltene Rückstand wird durch Flash-Chromatographie an Kieselgel (Laufmittel: Cyclohexan/Essigsäureethylester

10:1 → 4:1) gereinigt. Es werden 1.50 g (9.09 mmol, 68% Ausbeute) eines Öls erhalten.

$^1$H-NMR (400 MHz, $CDCl_3$, δ/ppm): 9,62 (1H, d), 7,98-7,96 (2H, d), 7,25-7,23 (2H, d), 3,91 (3H, s), 2,77-2,62 (2H, m), 2,35 (2H, t), 2,38-2,29 (1H, m), 2,07-1,98 (1H, m), 1,82-1,63 (4H, m), 1,55-1,47 (3H, m).

MS (ESI): 310 $(M+Na)^+$.

__Beispiel 95A__

*E*-4-{7-Cyano-3-[2-(2-hydroxyphenyl)vinyl]heptyl}-benzoesäuremethylester

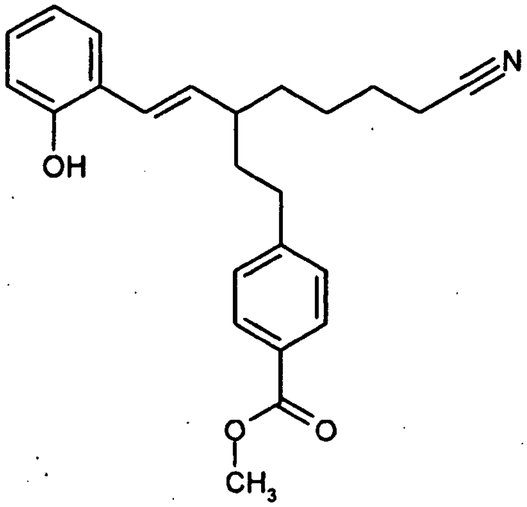

Zu einer Suspension von 3.26 g (7.26 mmol) 2-Hydroxyphenyl-triphenylphosphoniumbromid in 40 ml trockenem THF werden bei 0°C 9.07 ml (14.52 mmol) einer 1.6 M Lösung von n-Butyllithium in Hexan langsam zugetropft und die Mischung 5 Minuten nachgerührt. Anschließend werden bei dieser Temperatur 1.49 g (5.19 mmol) 4-(7-Cyano-3-formylheptyl)-benzoesäuremethylester aus Beispiel 94A in 10 ml trockenem THF langsam zugetropft. Die Reaktionsmischung wird 10 Minuten bei 0°C gerührt. Anschließend wird die Kühlung entfernt, die Reaktionslösung 10 Minuten bei Raumtemperatur nachgerührt, dann mit Kieselgel versetzt und bis zur Trockene eingeengt. Der erhaltene Rückstand wird direkt durch Flash-Chromatographie an Kieselgel (Laufmittel: Cyclohexan/Essigsäureethylester 1:2 → 1:5) gereinigt. Es werden 1.71 g (75% Reinheit, 3.40 mmol, 65% Ausbeute) eines Öls erhalten.

$^1$H-NMR (300 MHz, DMSO-$d_6$, δ/ppm): 9,50 (1H, s), 7,88-7,86 (2H, d), 7,40-7,34 (3H, m), 7,05-7,00 (1H, m), 6,84-6,68 (2H, m), 6,62-6,56 (1H, m), 6,05-5,97 (1H, dd), 3,83 (3H, s), 2,75-2,56 (2H, m), 2,52-2,44 (3H, m), 2,18-2,06 (1H, m), 1,81-1,28 (7H, m).

LC-MS (Methode 2): $R_t$ 2.60 min; m/z 377 $(M^+)$.

__Beispiel 96A__

*E*-4-(3-{2-[2-(4-*tert*.-Butylbenzyloxy)phenyl]vinyl}-7-cyanoheptyl)-benzoesäuremethylester

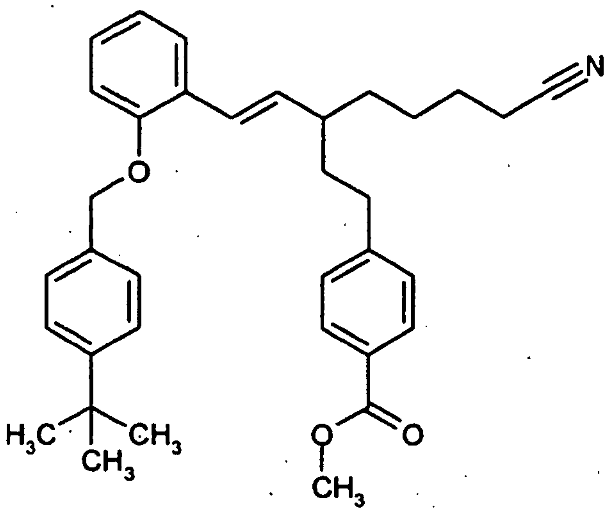

Eine Lösung von 1.70 g (3.38 mmol, 75%-ig) 4-{7-Cyano-3-[2-(2-hydroxyphenyl)vinyl]heptyl}-benzoesäuremethylester aus Beispiel 95A in 20 ml trockenem Acetonitril wird mit 1.53 g (7.76 mmol) 4-(*tert.*-Butyl)benzylbromid und 1.4 g (10.13 mmol) wasserfreiem Kaliumcarbonat versetzt und 12 Stunden zum Rückfluss erhitzt. Anschließend wird der Ansatz filtriert und das Filtrat zur Trockene eingeengt. Das erhaltene Rohprodukt wird auf Kieselgel aufgenommen und durch Flash-Chromatographie (Laufmittel: Cyclohexan/Essigsäureethylester 10:1) gereinigt. Es werden 1700 mg (3.12 mmol, 96% Reinheit, 92% Ausbeute) eines Öls erhalten.

$^1$H-NMR (400 MHz, $CDCl_3$, δ/ppm): 7,93-7,71 (2H, d), 7,46-7,35 (5H, m), 7,24-7,18 (3H, m), 6,97-6,93 (2H, m), 6,77-6,73 (1H, d), 5,98-5,92 (1H, dd), 5,11-5,05 (2H, m), 3,90 (3H, s), 2,79-2,70 (1H, m), 2,66-2,57 (1H, m), 3,36 (2H, t), 2,20-2,11 (1H, m), 1,82-1,73 (1H, m), 1,70-1,56 (3H, m), 1,56-1,35 (9H, s).

LC-MS (Methode 4): $R_t$ 3.36 min; m/z 523 ($M^+$).

## Beispiel 97A

4-[3-((*E*)-2-{2-[(4-*tert.*-Butylbenzyl)oxy]phenyl}vinyl)-7-cyanoheptyl]-benzohydrazid

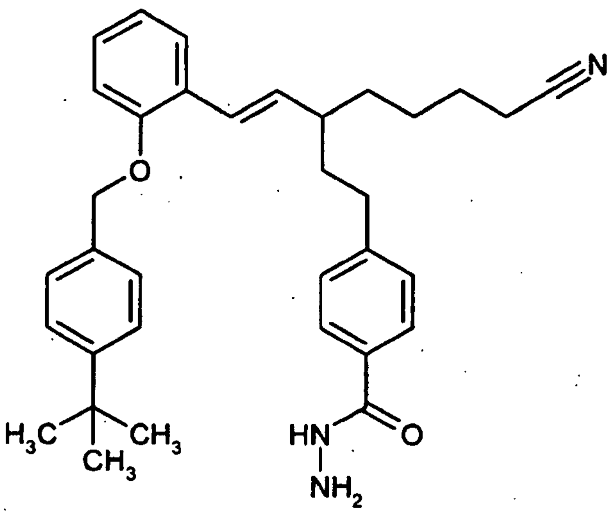

Eine Lösung von 1 g (1.83 mmol, 96%-ig) 4-(3-{2-[2-(4-*tert.*-Butyl-benzyloxy)phenyl]vinyl}-7-cyanoheptyl)-benzoesäuremethylester aus Beispiel 96A in 1.90 ml Methanol und 3.80 ml THF wird mit 3.82 g (76.38 mmol) Hydrazinmonohydrat versetzt und 12 Stunden bei 60°C gerührt. Das Reaktionsgemisch wird zur Trockene eingeengt und der Rückstand mit Dichlormethan nochmals co-evaporiert. Das erhaltene Rohprodukt (1.1 g) wird direkt in der Folgestufe umgesetzt.

LC-MS (Methode 2): $R_t$ 2.88 min; m/z 524 $(M+H)^+$.

**Beispiel 98A**

*E*-8-[2-(4-*tert*.-Butylbenzyloxy)phenyl]-6-{2-[4-(5-oxo-4,5-dihydro-[1,3,4]oxadiazol-2-yl)phenyl]-ethyl}-okt-7-ennitril

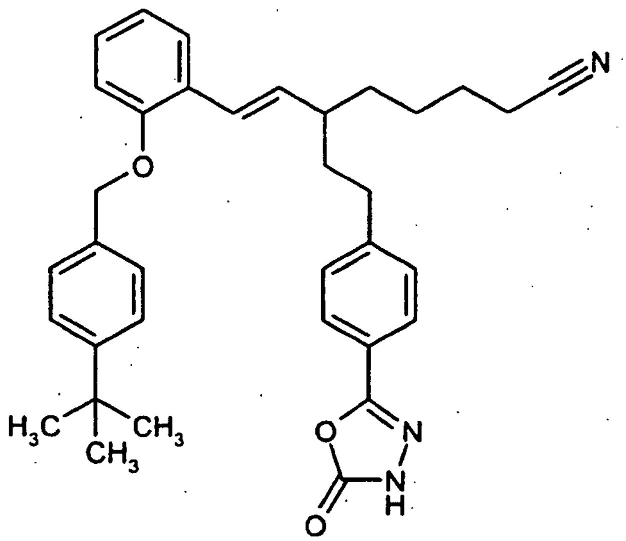

Eine Lösung von 566.6 mg (2.86 mmol) Chlorameisensäuretrichlormethylester in 2.5 ml trockenem Dioxan wird mit 1.0 g 4-[3-((*E*)-2-{2-[(4-*tert*.-Butylbenzyl)oxy]phenyl}vinyl)-7-cyanoheptyl]-benzohydrazid aus Beispiel 97A (Rohprodukt) in 7.5 ml Dioxan versetzt und 3 Stunden zum Rückfluss erhitzt. Anschließend wird der Ansatz direkt auf Kieselgel aufgenommen, bis zur Trockene eingeengt und durch Flash-Chromatographie (Laufmittel: Dichlormethan/Methanol 5: 1) gereinigt. Es werden 236 mg (0.429 mmol, 23% Ausbeute bezogen auf beide Stufen) eines Feststoffes erhalten.

$^1$H-NMR (300 MHz, DMSO-$d_6$, δ/ppm): 12,52 (1H, s), 7,68-7,62 (2H, d), 7,50-7,45 (1H, m), 7,40-7,32 (6H, m), 7,24-7,11 (1H, m), 7,11-7,06 (1H, m), 6,95-6,88 (1H, t), 6,68-6,61 (1H, d), 6,01-6,02 (1H, dd), 5,12-5,08 (2H, m), 2,75-2,55 (3H, m), 2,17-2,05 (1H, m), 1,81-1,28 (9H, m), 1,25 (9H, s).

LC-MS (Methode 4): $R_t$ 3.15 min; m/z 549 $(M)^+$.

**Beispiel 99A**

4-[(4*E*)-5-{2-[(4-*tert*.-Butylbenzyl)oxy]phenyl}-3-(4-cyanobenzyl)pent-4-en-1-yl]benzoesäuremethylester

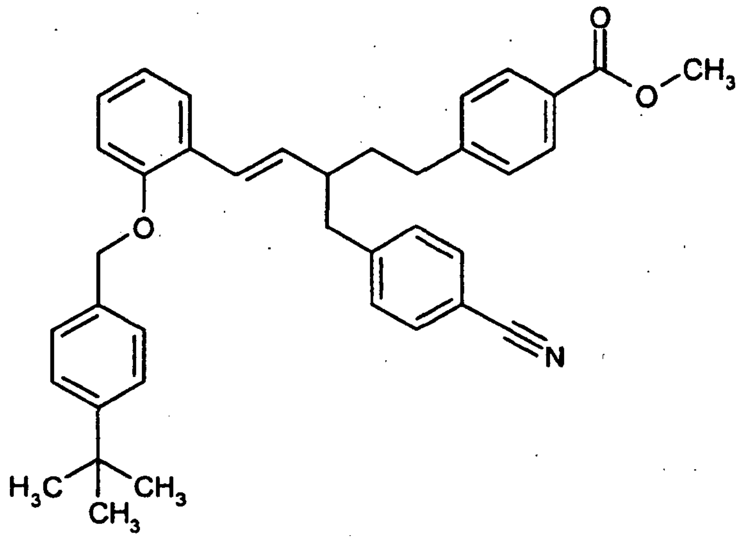

Eine Lösung von 1.4 g (3.13 mmol, 92%-ig) *E*-4-[3-(4-Cyanobenzyl)-5-(2-hydroxyphenyl)-pent-4-enyl]-benzoesäuremethylester (Beispiel 75A) in 7 ml trockenem Acetonitril wird mit 1.42 g (6.26 mmol) 4-(*tert.*-Butyl)benzylbromid und 1.30 g (9.39 mmol) wasserfreiem Kaliumcarbonat versetzt und 12 Stunden zum Rückfluss erhitzt. Anschließend wird der Ansatz filtriert und das Filtrat zur Trockene eingeengt. Der Rückstand wird direkt chromatographisch an Kieselgel (Laufmittel: Cyclohexan/Essigsäureethylester 10:1, dann 1:5) aufgereinigt. Es werden 1.85 g (3.32 mmol, 93% Reinheit, 98% Ausbeute) eines Öls isoliert.

$^{1}H$-NMR (300 MHz, $CDCl_3$, $\delta$/ppm): 7,94-7,88 (2H, m), 7,52-7,14 (12H, m), 6,97-6,89 (2H, m), 6,67-6,57 (1H, m), 6,02-5,92 (1H, m), 5,05-5,02 (2H, m), 3,90 (3H, s), 2,85-2,67 (3H, m), 2,67-2,55 (1H, m), 2,54-2,42 (1H, m), 1,88-1,61 (2H, m), 1,33 (9H, s).

LC-MS (Methode 4): $R_t$ 3.53 min; m/z 557 ($M^+$).

__**Beispiel 100A**__

4-[(4*E*)-5-{2-[(4-*tert.*-Butylbenzyl)oxy]phenyl}-3-(4-cyanobenzyl)pent-4-en-1-yl]benzoesäure

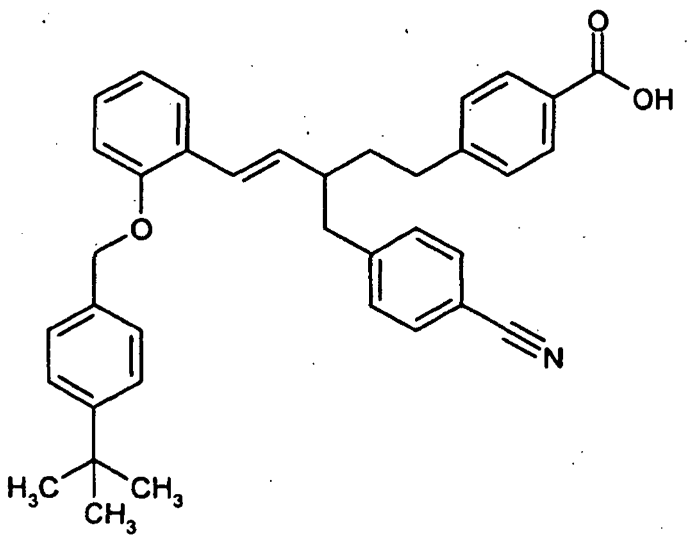

Eine Lösung von 350 mg (0.63 mmol) 4-[(4*E*)-5-{2-[(4-*tert.*-Butylbenzyl)oxy]phenyl}-3-(4-cyano-benzyl)-pent-4-en-1-yl]-benzoesäuremethylester aus Beispiel 99A in 2 ml THF und 1 ml Wasser wird mit 60 mg (2.51 mmol) Lithiumhydroxid versetzt und 12 h bei 60°C gerührt. Die Reaktionsmischung wird mit 1 M Salzsäure auf pH 3-4 eingestellt und

eingeengt. Der Rückstand wird direkt auf Kieselgel aufgenommen und chromatographisch (Laufmittel: Cyclohexan/ Essigsäureethylester 1:2, dann 100% Essigsäureethylester) aufgereinigt. Es werden 350 mg (0.61 mmol, 95% Reinheit, 97% Ausbeute) eines Feststoffs isoliert.

$^1$H-NMR (400 MHz, DMSO-$d_6$, δ/ppm): 12,92-12,66 (1H, breit), 7,85-7,79 (2H, d), 7,69-7,64 (2H, d), 7,43-7,23 (9H, m), 7,21-7,14 (1H, m), 7,06-7,02 (1H, d), 6,93-6,86 (1H, t), 6,60-6,42 (1H, d), 6,13-6,03 (1H, dd), 5,09-5,00 (2H, m), 2,93-2,84 (1H, m), 2,79-2,67 (2H, m), 2,67-2,55 (1H, m), 1,85-1,59 (2H, m), 1,26 (9H, s).

LC-MS (Methode 2): $R_t$ 3.39 min; m/z 543 $(M)^+$.

**Beispiel 101A**

2-{4-[(4*E*)-5-{2-[(4-*tert.*-Butylbenzyl)oxy]phenyl}-3-(4-cyanobenzyl)pent-4-en-1-yl]benzoyl}-hydrazincarbothioamid

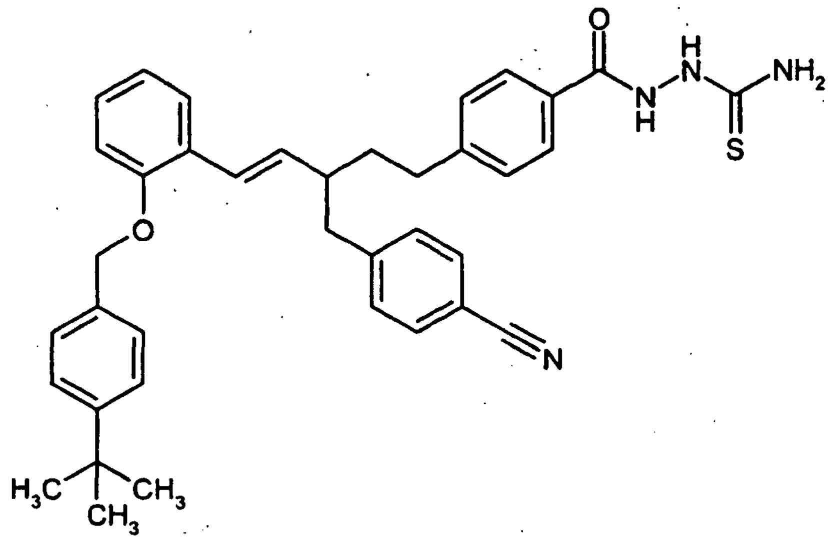

Zu einer Lösung von 12.7 mg (0.02 mmol) 4-[(4*E*)-5-{2-[(4-*tert.*-Butylbenzyl)oxy]phenyl}-3-(4-cyanobenzyl)pent-4-en-1-yl]-benzoesäure aus Beispiel 100A in 0.2 ml trockenem THF mit 1 Tropfen DMF werden 5.9 mg (0.05 mmol) Oxalylchlorid unter Eiskühlung langsam zugetropft. Die Reaktionsmischung wird 30 Minuten bei RT gerührt, anschließend im Vakuum eingeengt, der Rückstand zweimal mit Dichlormethan co-evaporiert und dann in 0.2 ml THF aufgenommen. Diese Lösung wird zu einer Lösung von 4.2 mg (0.05 mmol) Thiosemicarbazid in 0.25 ml THF bei 0°C zugetropft. Die Mischung wird 1 Stunde bei RT gerührt, dann eingeengt und der Rückstand ohne weitere Aufreinigung in der Folgestufe umgesetzt.

LC-MS (Methode 4): R, 3.09 min; m/z 616 $(M)^+$.

**Beispiel 102A**

Methyl-4-{(3*E*)-4-{2-[(4-*tert.*-butylbenzyl)oxy]phenyl}-2-[2-(4-cyanophenyl)ethyl]but-3-en-1-yl}-benzoat

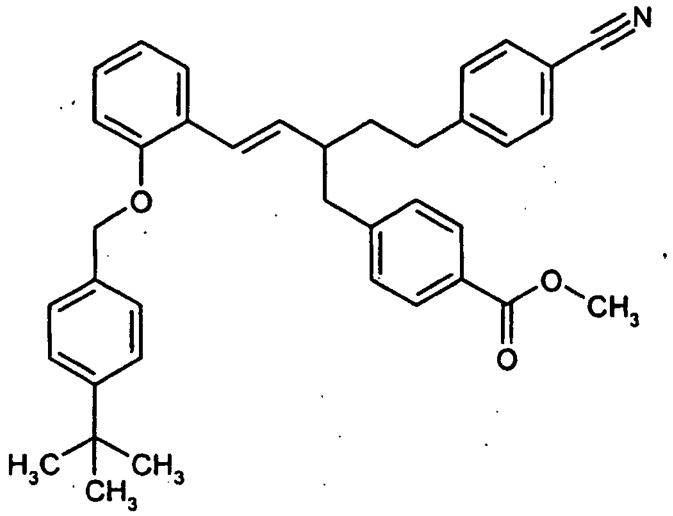

Eine Lösung von 2 g (3.6 mmol) *E*-4-[2-[2-(4-Cyanophenyl)ethyl]-4-(2-hydroxyphenyl)-but-3-enyl]-benzoesäuremethylester (Beispiel 17A) in 8 ml trockenem Acetonitril wird mit 2.20 g (9.71 mmol) 4-(*tert.*-Butyl)benzylbromid und 2.02 g (14.59 mmol) wasserfreiem Kaliumcarbonat versetzt und 12 Stunden zum Rückfluss erhitzt. Anschließend wird der Ansatz filtriert und das Filtrat zur Trockene eingeengt. Der Rückstand wird chromatographisch an Kieselgel (Laufmittel: Cyclohexan/Essigsäureethylester 10:1) aufgereinigt. Es werden 1.9 g (3.30 mmol, 97% Reinheit, 92% Ausbeute) eines Öls isoliert.

$^1$H-NMR (400 MHz, $CDCl_3$, $\delta$/ppm): 7,94-7,89 (2H, m), 7,49-7,30 (7H, m), 7,21-7,12 (5H, m), 6,97-6,90 (2H, m), 6,68-7,62 (1H, m), 5,06-5,02 (2H, m), 3,89 (3H, s), 2,83-2,69 (3H, m), 2,65-2,39 (2H, m), 1,86-1,59 (2H, m), 1,33 (9H, s).

LC-MS (Methode 2): $R_t$ 3.37 min; m/z 575 ($M+NH_4^+$), 557 ($M^+$).

## Beispiel 103A

4-{(3*E*)-4-{2-[(4-*tert.*-Butylbenzyl)oxy]phenyl}-2-[2-(4-cyanophenyl)ethyl]but-3-en-1-yl}benzo-hydrazid

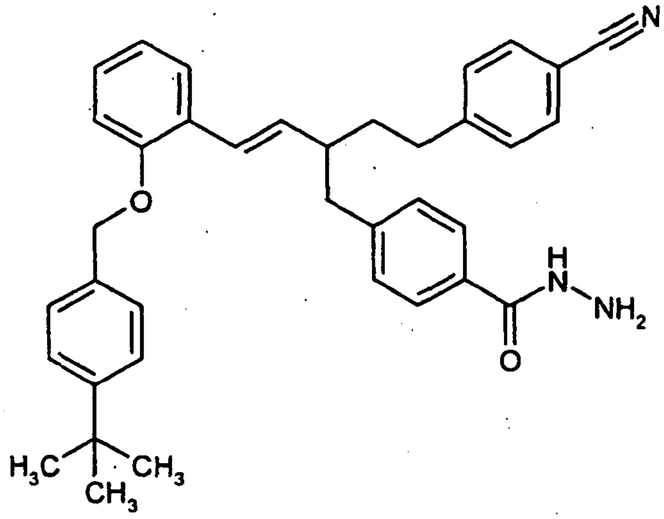

800 mg (1.43 mmol) 4-{(3*E*)-4-{2-[(4-*tert.*-Butylbenzyl)oxy]phenyl}-2-[2-(4-cyanophenyl)ethyl]-but-3-en-1-yl} benzoesäuremethylester (Beispiel 102A) werden in 3 ml Methanol und 1.5 ml THF vorgelegt und tropfenweise mit 2.88 g (57.4 mmol) Hydrazinmonohydrat versetzt. Das Reaktionsgemisch wird 3 Stunden bei 65°C und weitere 12 Stunden bei RT gerührt. Nach Entfernung des Lösungsmittels im Vakuum wird der Rückstand durch Flash-Chromatographie an Kieselgel (Laufmittel: Dichlormethan/Methanol/aq. Ammoniak 20:1:0.1) aufgereinigt. Man erhält 185 mg (89% Reinheit)

der Titelverbindung.

LC-MS (Methode 4): $R_t$ 3.25 min; m/z 557 ($M^+$).

**Beispiel 104A**

4-{(4*E*)-5-{2-[(4-*tert*.-Butylbenzyl)oxy]phenyl}-3-[4-(5-oxo-4,5-dihydro-1,3,4-oxadiazol-2-yl)-benzyl]pent-4-en-1-yl}benzonitril

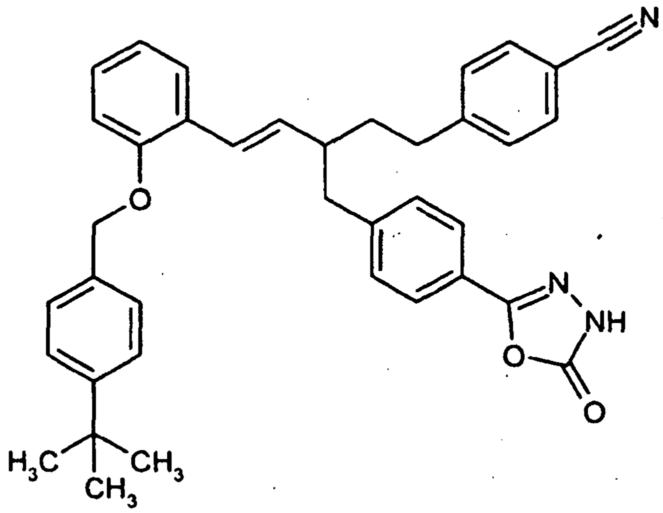

Zu einer Lösung von 87.3 mg (0.44 mmol) Chlorameisensäuretrichlormethylester in 0.5 ml Dioxan wird eine Lösung von 185 mg (0.29 mmol, 89% Reinheit) 4-{(3*E*)-4-{2-[(4-*tert*.-Butylbenzyl)-oxy]phenyl}-2-[2-(4-cyanophenyl)ethyl] but-3-en-1-yl}benzohydrazid aus Beispiel 103A in 1 ml Dioxan langsam zugetropft. Die Reaktionsmischung wird zwei Stunden unter Rückfluss gerührt. Nach dem Abkühlen wird der Ansatz im Vakuum eingeengt, direkt auf Kieselgel aufgenommen und durch Flash-Chromatographie (Laufmittel: Dichlormethan/Methanol/aq. Ammoniak 10:1:0.1) gereinigt. Es werden 166 mg (0.28 mmol, 96% Ausbeute) eines Öls isoliert.

$^1$H-NMR (400 MHz, $CDCl_3$, δ/ppm): 8,37 (1H, s), 7,77-7,65 (2H, d), 7,49-7,44 (2H, d), 7,42-7,11 (10H, m), 6,98-6,88 (2H, m), 6,69-6,60 (1H, d), 6,04-5,92 (1H, dd), 5,09-4,96 (2H, m), 2,81-2,71 (3H, m), 2,66-2,55 (1H, m), 2,52-2,41 (1H, m), 1,86-1,75 (1H, m), 1,72-1,60 (1H, m), 1,32 (9H, s).

LC-MS (Methode 4): $R_t$ 3.39 min; m/z 583 ($M^+$).

**Beispiel 105A**

4-{(3*E*)-4-{2-[(4-*tert*.-Butylbenzyl)oxy]phenyl}-2-[2-(4-cyanophenyl)ethyl]but-3-en-1-yl}benzoesäure

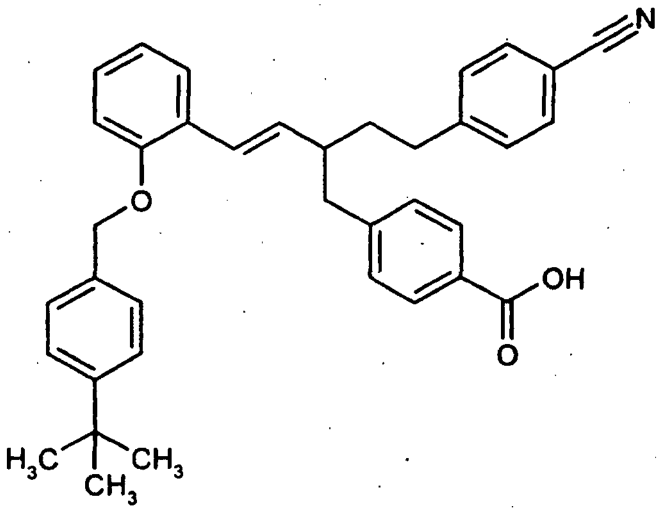

Eine Lösung von 1.1 g (1.97 mmol) Methyl-4-{(3*E*)-4-{2-[(4-*tert*.-Butylbenzyl)oxy]phenyl}-2-[2-(4-cyanophenyl) ethyl]but-3-en-1-yl}benzoat aus Beispiel 102A in 7.20 ml THF und 3.60 ml Wasser wird mit 189 mg (7.89 mmol) Lithiumhydroxid versetzt und 12 h bei 60°C gerührt. Die Reaktionsmischung wird mit 1 M Salzsäure auf pH 3-4 eingestellt und eingeengt. Der Rückstand wird auf Kieselgel aufgenommen und chromatographisch (Laufmittel: Cyclohexan/Essigsäure-ethylester 1:1, mit 0.1% Ameisensäure) aufgereinigt. Es werden 990 mg (1.77 mmol, 97% Reinheit, 90% Ausbeute) eines Feststoffs isoliert.

$^1$H-NMR (400 MHz, DMSO-$d_6$, δ/ppm): 12,88-12,64 (1H, breit), 7,82-7,80 (2H, d), 7,66-7,63 (2H, d), 7,42-7,23 (9H, m), 7,21-7,13 (1H, m), 7,07-7,01 (1H, d), 6,93-6,86 (1H, t), 6,49-6,44 (1H, d), 6,12-6,04 (1H, dd), 5,08-4,99 (2H, m), 2,90-2,57 (4H, m), 2,57-2,41 (1H, m), 1,86-1,58 (2H, m), 1,26 (9H, s).

LC-MS (Methode 1): $R_t$ 3.39 min; m/z 543 $(M)^+$.

**Beispiel 106A**

2-(4-{(3*E*)-4-{2-[(4-*tert*.-Butylbenzyl)oxy]phenyl}-2-[2-(4-cyanophenyl)ethyl]but-3-en-1-yl}-benzoyl)hydrazincarboxamid

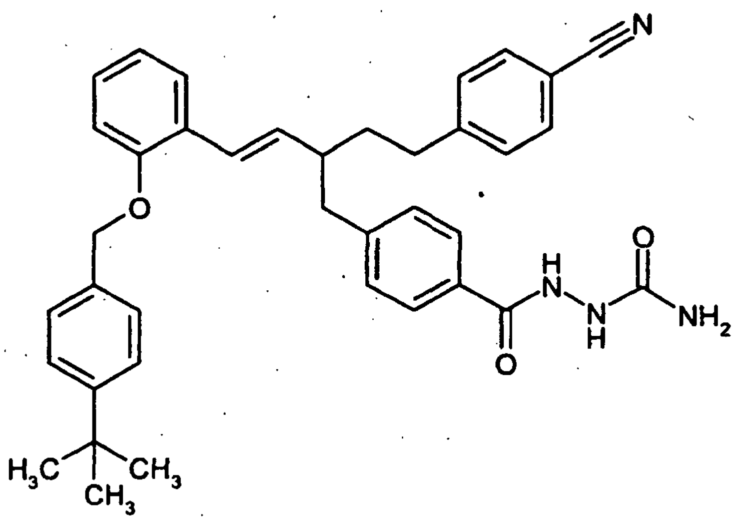

Zu einer Lösung von 600 mg (1.10 mmol) 4-{(3*E*)-4-{2-[(4-*tert*.-Butylbenzyl)oxy]phenyl}-2-[2-(4-cyanophenyl) ethyl]but-3-en-1-yl}benzoesäure aus Beispiel 105A in 6 ml trockenem Toluol mit 4 Tropfen DMF werden 836 mg (6.62 mmol) Oxalylchlorid unter Eiskühlung langsam zugetropft. Die Reaktionsmischung wird 10 Minuten bei RT gerührt und anschließend 1 Stunde zum Rückfluss erhitzt. Nach Abkühlen wird der Ansatz im Vakuum eingeengt, der Rückstand zweimal mit Toluol co-evaporiert und dann in 6 ml THF aufgenommen. Diese Lösung wird bei 0°C zu einer zuvor

hergestellten Lösung aus 135.4 mg (1.21 mmol) Semicarbazid-Hydrochlorid und 88.3 mg (2.21 mmol) Natriumhydroxid in 1 ml THF und 0.25 ml Wasser getropft. Die Mischung wird 2 Stunden bei 0°C und anschließend 30 Minuten bei RT gerührt, dann eingeengt und der Rückstand ohne weitere Aufreinigung in der Folgestufe umgesetzt.

LC-MS (Methode 1): $R_t$ 3.17 min, m/z 600 $(M)^+$.

**Beispiel 107A**

2-(4-{(3*E*)-4-{2-[(4-*tert*.-Butylbenzyl)oxy]phenyl}-2-[2-(4-cyanophenyl)ethyl]but-3-en-1-yl}-benzoyl)hydrazincarboximidamid

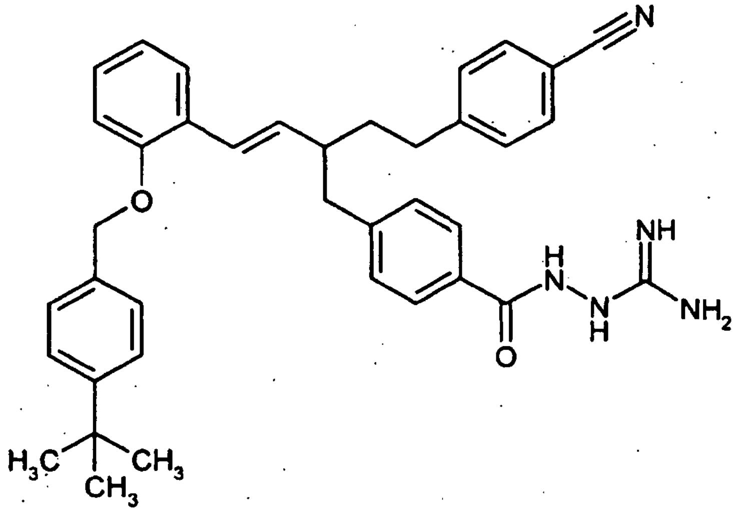

Zu einer Lösung von 150 mg (0.28 mmol) 4-{(3*E*)-4-{2-[(4-*tert*.-Butylbenzyl)oxy]phenyl}-2-[2-(4-cyanophenyl) ethyl]but-3-en-1-yl}benzoesäure aus Beispiel 105A in 1.5 ml trockenem Toluol mit 2 Tropfen DMF werden 210 mg (1.66 mmol) Oxalylchlorid unter Eiskühlung langsam zugetropft. Die Reaktionsmischung wird 5 Minuten bei RT gerührt und anschließend 1 Stunde zum Rückfluss erhitzt. Nach Abkühlen wird der Ansatz im Vakuum eingeengt, der Rückstand zweimal mit Toluol co-evaporiert und dann in 3 ml THF aufgenommen. Diese Lösung wird bei 0°C zu einer zuvor hergestellten Lösung aus 33.5 mg (0.30 mmol) Aminoguanidin-Hydrochlorid und 22 mg (0.55 mmol) Natriumhydroxid in 1 ml THF und 0.25 ml Wasser getropft. Die Mischung wird 2 Stunden bei 0°C gerührt, dann eingeengt und der Rückstand ohne weitere Aufreinigung in der Folgestufe umgesetzt.

LC-MS (Methode 5): $R_t$ 3.91 min; m/z 599 $(M^+)$.

**Beispiel 108A**

2-Allyloxycarbonyl-2-(4-cyanobenzyl)-heptandisäure-1-allylester-7-ethylester

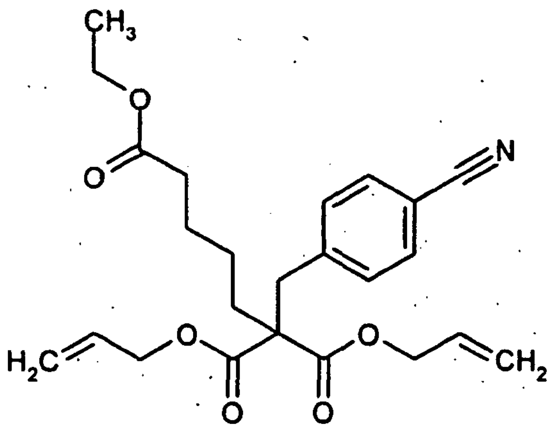

Eine Lösung von 20 g (64.03 mmol) 2-Allyloxycarbonyl-heptandisäure-1-allylester-7-ethylester aus Beispiel 82A in 140 ml Dimethylformamid wird bei 0°C portionsweise mit 1.69 g (70.43 mmol) Natriumhydrid versetzt. Anschließend lässt man die Reaktionslösung auf Raumtemperatur kommen und rührt 30 min lang nach. Die Reaktionslösung wird wieder auf 0°C abgekühlt, mit 16.32 g (83.24 mmol) 4-Brommethyl-benzonitril in 140 ml DMF versetzt und 30 min bei dieser Temperatur nachgerührt. Der Ansatz wird danach bei Raumtemperatur über Nacht gerührt. Das Reaktionsgemisch wird tropfenweise mit Wasser versetzt, mit Essigsäureethylester dreimal extrahiert, die vereinigten organischen Phasen mit gesättigter Kochsalz-Lösung gewaschen und über Natriumsulfat getrocknet. Nach der Filtration wird das Lösungsmittel im Vakuum bis zur Trockene eingeengt. Das erhaltene Rohprodukt wird durch Flash-Chromatographie an Kieselgel (Laufmittel: Cyclohexan/Essigsäureethylester 10:1 → 4:1 → 1:1) gereinigt. Es werden 20.08 g (46.97 mmol, 73% Ausbeute) eines farblosen Feststoffes erhalten.

$^1$H-NMR (400 MHz, $CDCl_3$, $\delta$/ppm): 7,56 (2H, d), 7,22 (2H, d), 5,91-5,78 (2H, m), 5,37-5,18 (4H, m), 4,66-4,54 (4H, m), 4,13 (2H, q), 3,29 (2H, s), 2,31 (2H, t), 1,87-1,77 (2H, m), 1,69-1,57 (2H, m), 1,39-1,28 (2H, m), 1,26 (3H, t).

MS (DCI): 445 ($M+NH_4^+$).

## Beispiel 109A

2-(4-Cyanobenzyl)-heptandisäure-7-ethylester

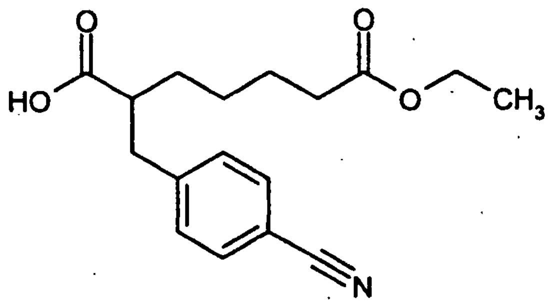

Eine Lösung von 22.5 g (52.63 mmol) 2-Allyloxycarbonyl-2-(4-cyanobenzyl)-heptandisäure-1-allylester-7-ethylester aus Beispiel 108A, 970 mg (3.68 mmol) Triphenylphosphin und 240 mg (1.05 mmol) Palladiumacetat in 500 ml Dioxan wird bei Raumtemperatur mit einer Lösung von 24.21 ml (173.69 mmol) Triethylamin und 4.96 ml (131.58 mmol) Ameisensäure in 500 ml Dioxan versetzt. Das Reaktionsgemisch wird anschließend bei 100°C 2 Stunden lang gerührt. Nach vollständigem Umsatz wird die Reaktionslösung abgekühlt und das Lösungsmittel im Vakuum entfernt. Anschließend wird der Rückstand in Essigsäureethylester und Wasser aufgenommen, mit 1 N Salzsäure angesäuert und die organische Phase abgetrennt. Die wässrige Phase wird noch dreimal mit Essigsäureethylester extrahiert, die organischen Phasen anschließend vereinigt, mit gesättigter Kochsalz-Lösung gewaschen und über Natriumsulfat getrocknet. Nach Filtration wird die Lösung im Vakuum eingeengt. Es werden 17.5 g (87% Ausbeute, 80% Reinheit) eines farblosen Feststoffes erhalten.

LC-MS (Methode 2): $R_t$ 1.97 min; m/z 304 ($M+H^+$).

**Beispiel 110A**

6-(4-Cyanobenzyl)-7-hydroxy-heptansäureethylester

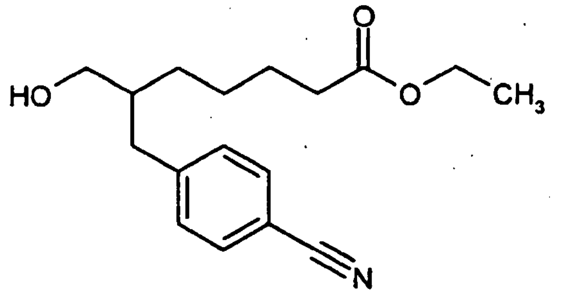

Zu einer Lösung von 6.64 g (22.94 mmol) 2-(4-Cyanobenzyl)-heptandisäure-7-ethylester aus Beispiel 109A in 260 ml THF werden 34.42 ml einer 1 M Boran-THF-Komplex-Lösung (34.42 mmol) bei -10°C zugetropft. Nach Erwärmen auf 0°C wird bei dieser Temperatur noch 2 Stunden nachgerührt. Nach vollständiger Umsetzung wird das Reaktionsgemisch mit gesättigter Natriumhydrogencarbonat-Lösung versetzt und das Lösungsmittel bis zur Trockene eingeengt. Der Rückstand wird in Dichlormethan aufgenommen, über Natriumsulfat getrocknet und wiederum vom Lösungsmittel befreit. Das erhaltene Rohprodukt wird durch Flash-Chromatographie an Kieselgel (Laufmittel: Essigsäureethylester/ Cyclohexan 1:8 $\rightarrow$ 1:2) gereinigt. Es werden 5.84 g (88% Ausbeute, 20.19 mmol) eines farblosen Feststoffes erhalten.

$^1$H-NMR (400 MHz, DMSO-$d_6$, $\delta$/ppm): 8,14 (2H, d), 7,80 (2H, d), 4,91 (1H, t), 4,44 (2H, q), 3,69-3,58 (2H, m), 3,16-3,06 (1H, m), 3,01-2,92 (1H, m), 2,62 (2H, t), 2,14-2,01 (1H, m), 1,92-1,78 (2H, m), 1,77-1,60 (3H, m), 1,59-1,48 (1H, m), 1,57 (3H, t).

MS (DCI): 307 ($M+NH_4^+$).

**Beispiel 111A**

6-(4-Cyanobenzyl)-7-oxo-heptansäureethylester

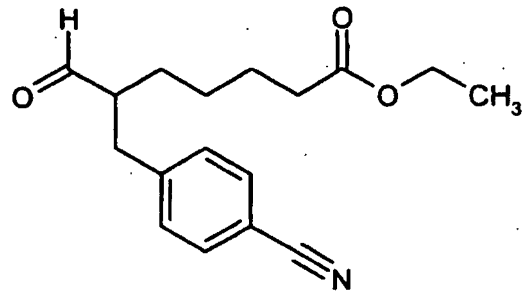

Eine Lösung von 4.6 g (15.90 mmol) 6-(4-Cyanobenzyl)-7-hydroxyheptansäureethylester aus Beispiel 110A in 250 ml Dichlormethan wird mit 4.11 g (19.08 mmol) Pyridiniumchlorochromat (PCC) versetzt und 12 Stunden bei Raumtemperatur gerührt. Nach vollständigem Umsatz werden 10 g Kieselgel zugegeben und das Lösungsmittel vorsichtig im Vakuum bis zur Trockene eingeengt. Der erhaltene Rückstand wird durch Flash-Chromatographie an Kieselgel (Laufmittel: Cyclohexan/Essigsäureethylester 1:1) gereinigt. Es werden 4.09 g (14.23 mmol, 89% Ausbeute) eines farblosen Feststoffes erhalten.

$^1$H-NMR (400 MHz, DMSO-$d_6$, $\delta$/ppm): 9,60 (1H, s), 7,75 (2H, d), 7,41 (2H, d), 4,03 (2H, q), 3,08-2,97 (1H, m), 2,82-2,64 (2H, m), 2,24 (2H, t), 1,63-1,19 (6H, m), 1,17 (3H, t).

MS (DCI): 305 ($M+NH_4^+$).

**Beispiel 112A**

*E*-6-(4-Cyanobenzyl)-8-(2-hydroxyphenyl)-oct-7-ensäureethylester

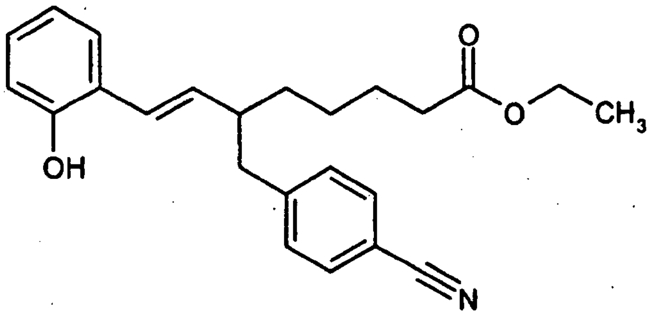

Zu einer Lösung von 6.411 g (14.27 mmol) (2-Hydroxybenzyl)-triphenylphosphoniumbromid in 300 ml wasserfreiem THF werden bei 0°C 15.98 ml (39.95 mmol) einer 2.5 M Lösung von n-Butyllithium in Hexan langsam hinzugefügt. Anschließend werden bei dieser Temperatur 4.1 g (14.27 mmol) 6-(4-Cyanobenzyl)-7-oxo-heptansäureethylester aus Beispiel 111A langsam zudosiert. Nach Erwärmung auf Raumtemperatur wird die Reaktionslösung 12 Stunden nachgerührt, dann mit gesättigter Ammoniumchlorid-Lösung versetzt und bis zur Trockene eingeengt. Der Rückstand wird in Essigsäureethylester aufgenommen, mit Wasser und gesättigter Kochsalz-Lösung gewaschen und über Natriumsulfat getrocknet. Nach Filtration wird das Lösungsmittel bis zur Trockene eingeengt. Das erhaltene Rohprodukt wird durch Flash-Chromatographie an Kieselgel (Laufmittel: Cyclohexan/Essigsäureethylester 4:1) gereinigt. Es werden 1.75 g (4.64 mmol, 32% Ausbeute) eines farblosen Feststoffes erhalten.

$^1$H-NMR (400 MHz, DMSO-$d_6$, $\delta$/ppm): 9,42 (1H, s), 7,72 (2H, d), 7,40 (2H, d), 7,29 (1H, d), 7,00 (1H, t), 6,79-6,67 (2H, m), 6,39 (1H, d), 6,04-5,94 (1H, m), 4,01 (2H, q), 2,87-2,77 (1H, m), 2,76-2,66 (1H, m), 2,48-2,38 (1H, m), 2,25 (2H, t), 1,57-1,39 (3H, m), 1,38-1,19 (3H, m), 1,13 (3H, t).

MS (DCI): 395 ($M+NH_4^+$).

**Beispiel 113A**

*E*-8-[2-(4-*tert*.-Butylbenzyloxy)phenyl]-6-(4-cyanobenzyl)-oct-7-ensäureethylester

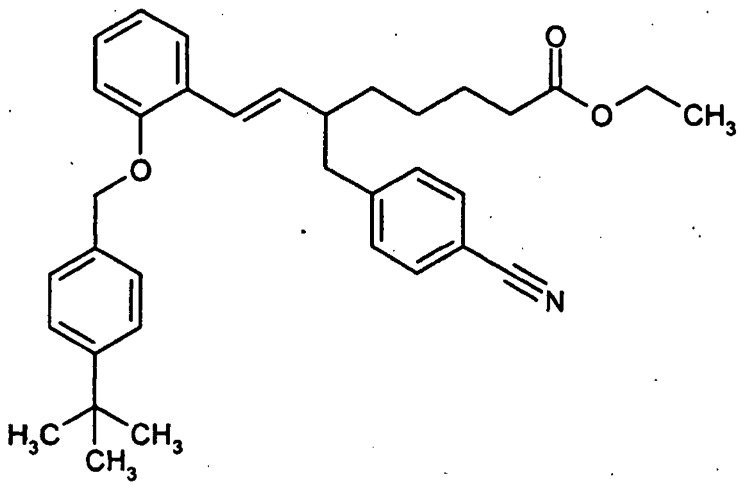

Eine Lösung von 1.75 g (4.64 mmol) *E*-6-(4-Cyanobenzyl)-8-(2-hydroxyphenyl)-oct-7-ensäure-ethylester aus Beispiel 112A in 50 ml trockenem Acetonitril wird mit 1579 mg (6.95 mmol) 4-(*tert*.-Butyl)benzylbromid und 961 mg (6.95 mmol) wasserfreiem Kaliumcarbonat versetzt und 12 Stunden zum Rückfluss erhitzt. Anschließend wird der Ansatz zur

Trockene eingeengt: Der Rückstand wird in Essigsäureethylester aufgenommen, mit Wasser und gesättigter Kochsalz-Lösung gewaschen und über Natriumsulfat getrocknet. Die organische Phase wird eingeengt. Das erhaltene Rohprodukt wird durch Flash-Chromatographie an Kieselgel (Laufmittel: Cyclohexan/ Essigsäureethylester 8:1 → 4:1) gereinigt. Es werden 2.24 g (4.28 mmol, 92% Ausbeute) eines Feststoffs erhalten.

$^1$H-NMR (300 MHz, DMSO-$d_6$, $\delta$/ppm): 7,68 (2H, d), 7,44-7,32 (5H, m), 7,28 (2H, d), 7,14 (1H, t), 7,01 (1H, d), 6,88 (1H, t), 6,42 (1H, d), 6,08-5,95 (1H, m), 5,04 (2H, s), 4,00 (2H, q), 2,89-2,78 (1H, m), 2,75-2,60 (2H, m), 2,54-2,40 (1H, m), 2,23 (2H, t), 1,60-1,21 (5H, m), 1,28 (9H, s), 1,13 (3H, t).

MS (DCI): 541 ($M+NH_4^+$).

**Beispiel 114A**

*E*-8-[2-(4-*tert*.-Butylbenzyloxy)phenyl]-6-[4-(5-oxo-4,5-dihydro-[1,2,4]oxadiazol-3-yl)benzyl]-oct-7-ensäureethylester

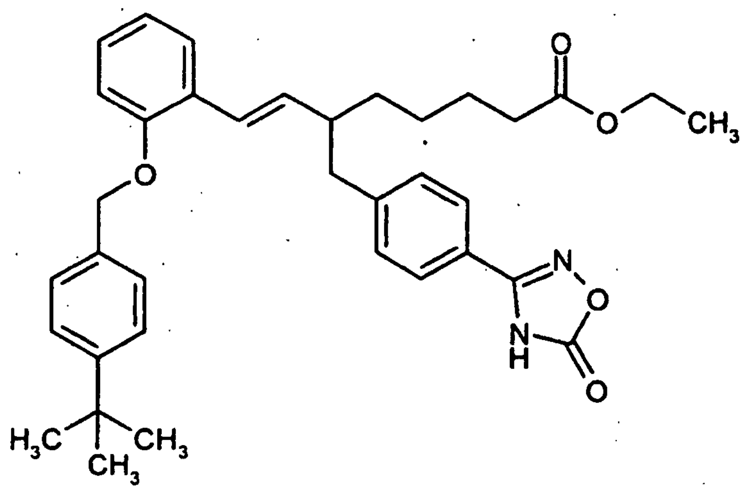

Eine Lösung von 823 mg (11.84 mmol) Hydroxylaminhydrochlorid in 10 ml DMSO wird mit 1.65 ml (11.84 mmol) Triethylamin versetzt und 10 min bei Raumtemperatur gerührt. Der anfallende Niederschlag wird abfiltriert. In das Filtrat werden anschließend 1.24 g (2.37 mmol) *E*-8-[2-(4-*tert*.-Butylbenzyloxy)phenyl]-6-(4-cyanobenzyl)-oct-7-ensäureethylester aus Beispiel 113A zudosiert. Die Reaktionslösung wird 12 Stunden bei 75°C gerührt. Nach vollständigem Umsatz und Abkühlung werden zu der Reaktionslösung 10 ml Wasser gegeben, mit Essigsäureethylester dreimal extrahiert und die vereinigten organischen Phasen mit gesättigter Kochsalz-Lösung gewaschen. Anschließend wird die organische Phase über Natriumsulfat getrocknet und bis zur Trockene eingeengt. Es werden 1380 mg eines farblosen Öls erhalten, das ohne weitere Reinigung direkt weiter umgesetzt wird.

Das oben erhaltene Öl wird in 15 ml DMF gelöst und mit 0.22 ml (2.77 mmol) Pyridin versetzt. Anschließend wird die Lösung auf 0°C abgekühlt und langsam mit 412 mg (2.31 mmol) Chlor-ameisensäure-2-ethylhexylester versetzt. Der Ansatz wird ca. 30 min bei 0°C nachgerührt, dann in Wasser aufgenommen und mit Essigsäureethylester extrahiert. Die organische Phase wird über Natriumsulfat getrocknet und bis zur Trockene eingeengt. Der erhaltene Rückstand wird in 15 ml Xylol aufgenommen und 4 Stunden zum Rückfluss erhitzt. Nach vollständiger Umsetzung wird die Reaktionslösung abgekühlt und bis zur Trockene eingeengt. Es werden 1.38 g (90% Reinheit, 92% Ausbeute) eines Feststoffs erhalten.

LC-MS (Methode 2): $R_t$ 3.18 min; m/z 583 ($M+H^+$).

**Beispiel 115A**

2-(4-Cyanobutyl)-2-(4-methoxycarbonyl-benzyl)-malonsäurediallylester

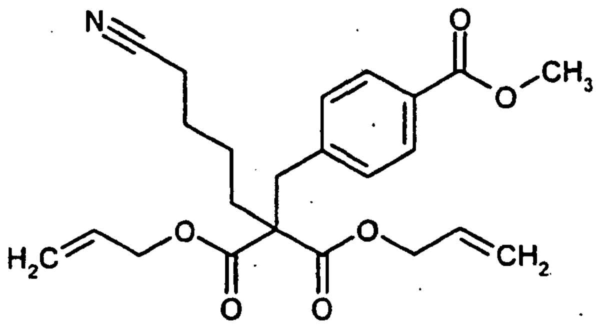

Eine Lösung von 20 g (60.18 mmol) 2-(4-Methoxycarbonyl-benzyl)-malonsäurediallylester aus Beispiel 12A in 140 ml DMF wird bei 0°C portionsweise mit 1.88 g (78.23 mmol) Natriumhydrid versetzt. Anschließend erwärmt man die Reaktionslösung auf 40°C und lässt 30 min lang bei dieser Temperatur nachrühren. Die Reaktionslösung wird dann wieder auf 0°C abgekühlt, mit 10.73 g (66.20 mmol) Bromvaleriansäurenitril in 140 ml DMF versetzt und 30 min bei dieser Temperatur nachgerührt. Der Ansatz wird danach bei Raumtemperatur über Nacht gerührt. Das Reaktionsgemisch wird tropfenweise mit Wasser versetzt, mit Essigsäureethylester dreimal extrahiert, die vereinigten organischen Phasen mit gesättigter Kochsalz-Lösung gewaschen und über Natriumsulfat getrocknet. Nach der Filtration wird das Lösungsmittel im Vakuum bis zur Trockene eingeengt. Das erhaltene Rohprodukt wird durch Flash-Chromatographie an Kieselgel (Laufmittel: Cyclohexan/Essigsäureethylester 9:1 $\rightarrow$ 4:1) gereinigt. Es werden 15.5 g (37.49 mmol, 62% Ausbeute) eines farblosen Feststoffes erhalten.

$^1$H-NMR (400 MHz, $CDCl_3$, $\delta$/ppm): 7,94 (2H, d), 7,17 (2H, d), 5,95-5,80 (2H, m), 5,38-5,20 (4H, m), 4,69-4,56 (4H, m), 3,91 (3H, s), 3,32 (2H, s), 2,34 (2H, t), 1,85-1,74 (2H, m), 1,71-1,59 (2H, m), 1,53-1,40 (2H, m).

MS (DCI): 431 ($M+NH_4^+$).

**Beispiel 116A**

4-(2-Carboxy-6-cyanohexyl)-benzoesäuremethylester

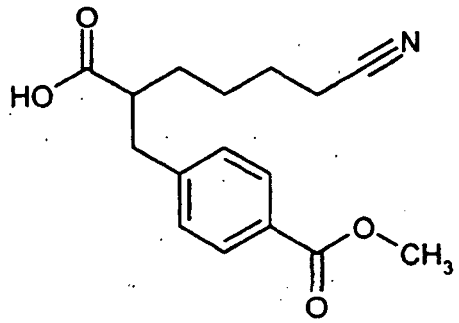

Eine Lösung von 15.5 g (37.49 mmol) 2-(4-Cyanobutyl)-2-(4-methoxycarbonyl-benzyl)-malon-säurediallylester aus Beispiel 115A, 690 mg (2.62 mmol) Triphenylphosphin und 170 mg (0.75 mmol) Palladiumacetat in 500 ml Dioxan wird bei Raumtemperatur mit einer Lösung von 17.24 ml (123.71 mmol) Triethylamin und 4.24 ml (112.46 mmol) Ameisensäure in 500 ml Dioxan versetzt. Das Reaktionsgemisch wird anschließend bei 100°C 12 Stunden gerührt. Nach vollständigem Umsatz wird die Reaktionslösung abgekühlt und das Lösungsmittel im Vakuum entfernt. Anschließend wird der Rückstand in Essigsäureethylester und Wasser aufgenommen, mit 1 N Salzsäure angesäuert und die organische Phase abgetrennt. Die wässrige Phase wird noch dreimal mit Essigsäureethylester extrahiert, die organischen Phasen anschließend vereinigt, mit gesättigter Kochsalz-Lösung gewaschen und über Natriumsulfat getrocknet. Nach Filtration wird die Lösung im Vakuum eingeengt. Es werden 8.1 g (74% Ausbeute) eines farblosen Feststoffes erhalten.

LC-MS (Methode 2): R, 1.76 min; m/z 290 ($M+H^+$).

**Beispiel** 117A

4-(6-Cyano-2-hydroxymethyl-hexyl)-benzoesäuremethylester

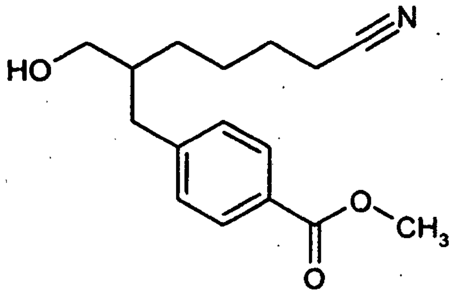

Zu einer Lösung von 3.835 g (13.83 mmol) 4-(2-Carboxy-6-cyano-hexyl)-benzoesäuremethylester aus Beispiel 116A in 200 ml THF werden 27.65 ml einer 1 M Boran-THF-Komplex-Lösung bei -10°C zugetropft. Nach Erwärmen auf 0°C wird bei dieser Temperatur noch 2 Stunden nachgerührt. Nach vollständiger Umsetzung wird das Reaktionsgemisch mit gesättigter Natriumhydrogencarbonat-Lösung versetzt und das Lösungsmittel bis zur Trockene eingeengt. Der Rückstand wird in Dichlormethan aufgenommen, über Natriumsulfat getrocknet und wiederum vom Lösungsmittel befreit. Das erhaltene Rohprodukt wird durch Flash-Chromatographie an Kieselgel (Laufmittel: Essigsäureethylester/Cyclohexan 1:8 → 1:2) gereinigt. Es werden 2.95 g (77% Ausbeute) eines farblosen Feststoffes erhalten.

$^1$H-NMR (400 MHz, DMSO-$d_6$, δ/ppm): 7,88 (2H, d), 7,33 (2H, d), 4,50 (1H, t), 3,84 (3H, s), 3,28 (2H, t), 2,75-2,64 (1H, m), 2,59-2,52 (1H, m), 2,45 (2H, t), 1,76-1,62 (1H, m), 1,54-1,43 (2H, m), 1,43-1,26 (3H, m), 1,24-1,10 (1H, m).

MS (DCI): 293 ($M+NH_4^+$).

**Beispiel 118A**

4-(6-Cyano-2-formyl-hexyl)-benzoesäuremethylester

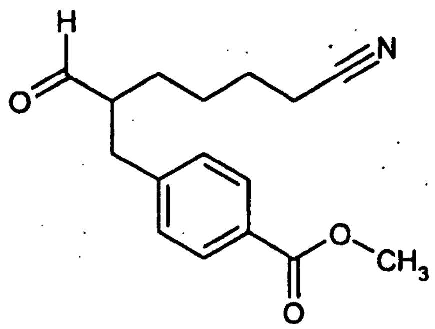

Eine Lösung von 3.8 g (13.80 mmol) 4-(6-Cyano-2-hydroxymethyl-hexyl)-benzoesäuremethylester aus Beispiel 117A in 250 ml Dichlormethan wird mit 3.57 g (16.56 mmol) Pyridiniumchlorochromat (PCC) versetzt und 12 Stunden bei Raumtemperatur gerührt. Nach vollständigem Umsatz werden 10 g Kieselgel zugegeben und das Lösungsmittel vorsichtig im Vakuum bis zur Trockene eingeengt. Der erhaltene Rückstand wird durch Flash-Chromatographie an Kieselgel (Laufmittel: Cyclohexan/Essigsäureethylester 1:1) gereinigt. Es werden 3.49 g (92% Ausbeute) eines farblosen Feststoffes erhalten.

$^1$H-NMR (400 MHz, DMSO-$d_6$, δ/ppm): 9,64 (1H, s), 7,87 (2H, d), 7,36 (2H, d), 3,84 (3H, s), 3,09-2,99 (1H, m), 2,84-2,66 (2H, m), 2,47 (2H, t), 1,68-1,22 (6H, m).

MS (DCI): 291 ($M+NH_4^+$).

**Beispiel 119A**

*E*-4-{6-Cyano-2-[2-(2-hydroxyphenyl)vinyl]hexyl}-benzoesäuremethylester

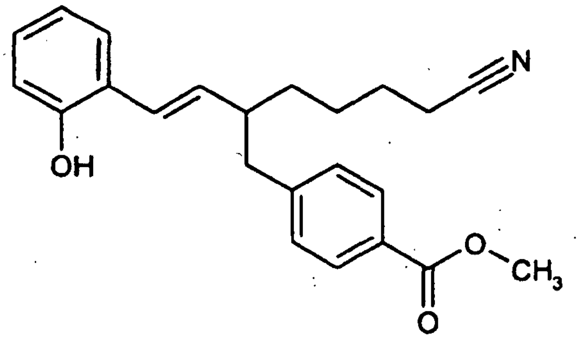

Wasser und gesättigter Kochsalz-Lösung gewaschen und über Natriumsulfat getrocknet. Die organische Phase wird eingeengt. Das erhaltene Rohprodukt wird durch Flash-Chromatographie an Kieselgel (Laufmittel: Cyclohexan/ Essigsäureethylester 8:1 → 4: 1) gereinigt. Es werden 3.85 g (92% Ausbeute) eines Feststoffs erhalten.
$^1$H-NMR (300 MHz, DMSO-$d_6$, δ/ppm): 7,83 (2H, d), 7,43-7,35 (3H, m), 7,34-7,23 (4H, m), 7,16 (1H, t), 7,00 (1H, d), 6,88 (1H, t), 6,45 (1H, d), 6,09-5,98 (1H, m), 5,02 (2H, s), 3,80 (3 H, s), 2,89-2,76 (1H, m), 2,75-2,61 (1H, m), 2,55-2,47 (1H, m), 2,44 (2H, t), 1,60-1,31 (6H, m), 1,28 (9H, s).
MS (DCI): 527 (M+$NH_4^+$).

**Beispiel 121A**

*E*-8-[2-(4-*tert*.-Butylbenzyloxy)phenyl]-6-[4-(5-oxo-4,5-dihydro-[1,3,4]oxadiazol-2-yl)benzyl]-oct-7-ennitril

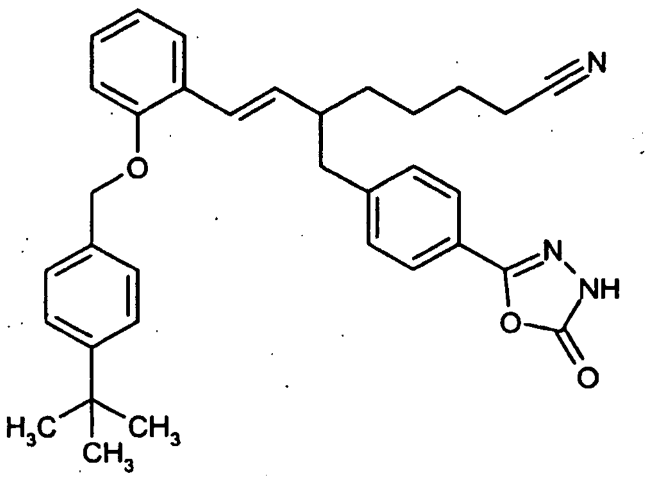

Eine Lösung von 200 mg (0.39 mmol) *E*-4-(2-{2-[2-(4-*tert*.-Butylbenzyloxy)phenyl]vinyl}-6-cyano-hexyl)-benzoesäuremethylester aus Beispiel 120A in 2 ml Methanol und 1 ml THF wird mit 785 mg (15.70 mmol) Hydrazinmonohydrat versetzt und 12 Stunden lang bei 70°C gerührt. Das Reaktionsgemisch wird zur Trockene eingeengt und der Rückstand nochmals mit Dichlormethan co-evaporiert. Das erhaltene Rohprodukt (166 mg) wird direkt in der Folgereaktion umgesetzt.
Eine Lösung von 96.65 mg (0.49 mmol) Chlorameisensäuretrichlormethylester in 0.5 ml trockenem Dioxan wird mit 166 mg der oben erhaltenen Zwischenstufe (Rohprodukt) in 1 ml Dioxan versetzt und 1 Stunde zum Rückfluss erhitzt. Anschließend wird der Ansatz bis zur Trockene eingeengt und der erhaltene Rückstand mittels präparativer

HPLC gereinigt. Es werden 47 mg (22% Ausbeute bezogen auf beide Stufen) eines Feststoffes erhalten.

LC-MS (Methode 1): $R_t$ 3.26 min; m/z 534 $(M-H)^-$.

Beispiel 122A

(7*E*)-8-{2-[(4-*tert*.-Butylbenzyl)oxy]phenyl}-6-[2-(4-cyanophenyl)ethyl]oct-7-ensäure

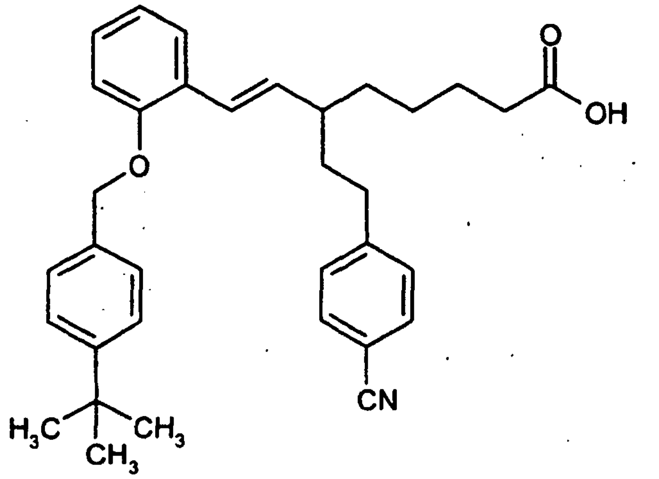

Eine Lösung von 798 mg (1.48 mmol) 7*E*-8-[2-(4-*tert*.-Butylbenzyloxy)phenyl]-6-[2-(4-cyano-phenyl)ethyl]oct-7-ensäureethylester in 20 ml THF und 20 ml Wasser wird mit 71 mg (2.97 mmol) Lithiumhydroxid versetzt und 12 Stunden bei 50°C gerührt. Nach dem Abkühlen wird mit Wasser und Diethylether verdünnt und die Phasen getrennt Die wässrige Phase wird mit 1 M Salzsäure sauer gestellt und mit Diethylether extrahiert. Die organische Phase wird mit gesättigter Natriumchlorid-Lösung gewaschen, über Natriumsulfat getrocknet und eingeengt. Es werden 700 mg (1.4 mmol, 93% d. Th.) der Titelverbindung erhalten.

HPLC (Methode 3): $R_t$ = 5.69 min

MS (ESIneg): m/z = 508 $(M-H)^-$

$^1$H-NMR (400 MHz, DMSO-$d_6$, $\delta$/ppm): 11.96 (s, 1H), 7.65 (d, 2H), 7.47 (d, 1H), 7.4-7.3 (m, 6H), 7.2 (t, 1H), 7.1 (d, 1H), 6.92 (t, 1H), 6.6 (d, 1H), 6.02 (dd, 1H), 5.1 (s, 2H), 2.75-2.57 (m, 2H), 2.18 (t, 2H), 2.11-2.0 (m, 1H), 1.78-1.54 (m, 2H), 1.5-1.38 (m, 3H), 1.38-1.15 (m, 12H).

**Beispiel 123A**

2-{(7*E*)-8-{2-[(4-*tert*.-Butylbenzyl)oxy]phenyl}-6-[2-(4-cyanophenyl)ethyl]oct-7-enoyl}hydrazin-carboxamid

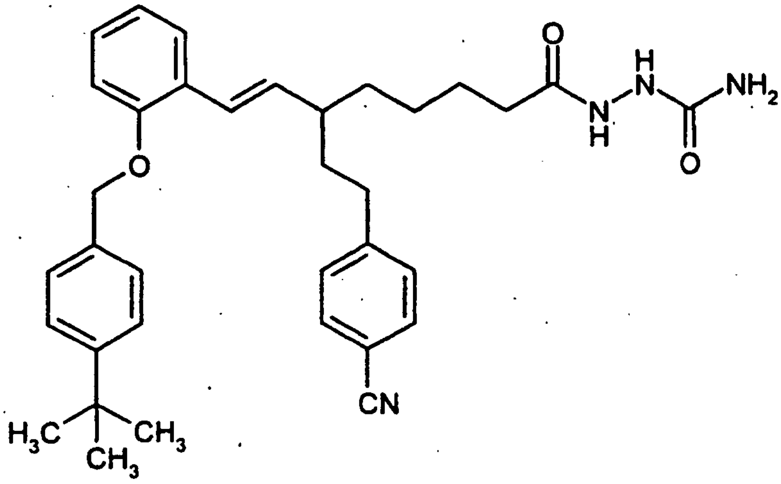

Eine Lösung von 700 mg (1.37 mmol) (7*E*)-8-{2-[(4-*tert*.-Butylbenzyl)oxy]phenyl}-6-[2-(4-cyano-phenyl)ethyl] oct-7-ensäure in 8 ml Toluol wird mit 4 Tropfen DMF versetzt. Anschließend werden 0.72 ml (8.24 mmol) Oxalylchlorid zugetropft und die Mischung 10 min bei Raumtemperatur gerührt. Danach wird die Reaktionslösung auf 80°C erwärmt und 1 h bei dieser Temperatur nachgerührt. Nach dem Abkühlen wird das Lösungsmittel abgezogen, der Rückstand noch zweimal mit Toluol versetzt und jeweils wieder zur Trockene eingedampft (Zwischenprodukt).

168.5 mg (1.5 mmol) Semicarbazid-Hydrochlorid werden in 5 ml THF und 2 ml Wasser gelöst und mit 110 mg (2.75 mmol) Natriumhydroxid versetzt. Die Lösung wird auf 0°C abgekühlt und das oben erhaltene Zwischenprodukt, in 10 ml THF gelöst, langsam zugetropft. Der Ansatz wird 2 h bei 0°C gerührt und dann eingeengt. Es werden 778 mg (1.3 mmol, 100% d. Th.) der Titelverbindung erhalten, welche ohne weitere Reinigung und Charakterisierung umgesetzt wird.

**Beispiel 124A**

4-((3*E*)-2-(2-{4-[(Amino(hydroxyimino)methyl]phenyl}ethyl)-4-{2-[(4-*tert*.-butylbenzyl)oxy]-phenyl}but-3-en-1-yl)benzoesäuremethylester

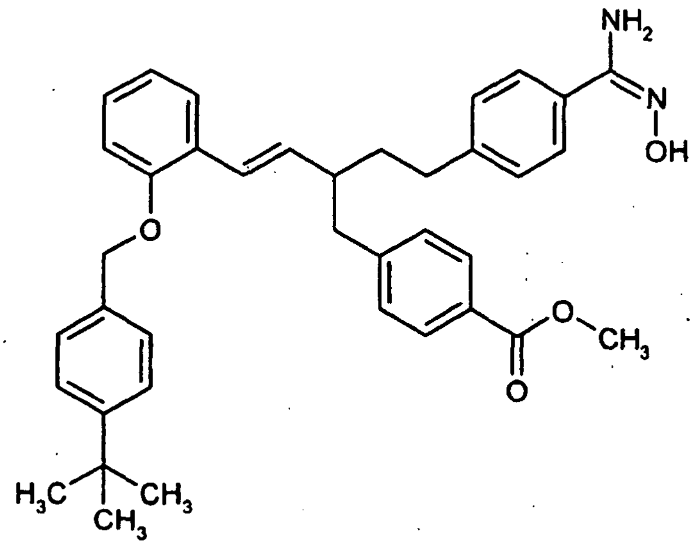

1.25 g (17.9 mmol) Hydroxylammoniumchlorid werden in 20 ml DMSO vorgelegt und mit 2.5 ml Triethylamin versetzt. Es wird 10 min bei Raumtemperatur nachgerührt und der ausgefallene Feststoff abgesaugt. Die Mutterlauge wird zu 2 g (3.59 mmol) 4-{(3*E*)-4-{2-(4-*tert*.-Butylbenzyl)oxy]-phenyl}-2-[2-(4-cyanophenyl)ethyl]but-3-en-1-yl}benzoesäuremethylester gegeben und die Mischung dann 12 h bei 75°C gerührt. Nach vollständigem Umsatz wird die Reaktionslösung auf Eiswasser gegeben. Die erhaltenen Kristalle werden abgesaugt, mit Wasser gewaschen, dann in Diethylether aufgenommen und mit Natriumchlorid-Lösung gewaschen. Die organische Phase wird über Natriumsulfat getrocknet und eingeengt. Es werden 1.9 g (83% Reinheit, 2.67 mmol, 74% d. Th.) der Titelverbindung erhalten.

LC-MS (Methode 4): $R_t$ = 2.77 min

MS (ESIpos): m/z = 591 $(M+H)^+$.

**Beispiel 125A**

4-((3*E*)-4-{2-[(4-*tert.*-Butylbenzyl)oxy]phenyl}-2-{2-[4-(5-oxo-4,5-dihydro-1,2,4-oxadiazol-3-yl)-phenyl]ethyl}but-3-en-1-yl)benzoesäuremethylester

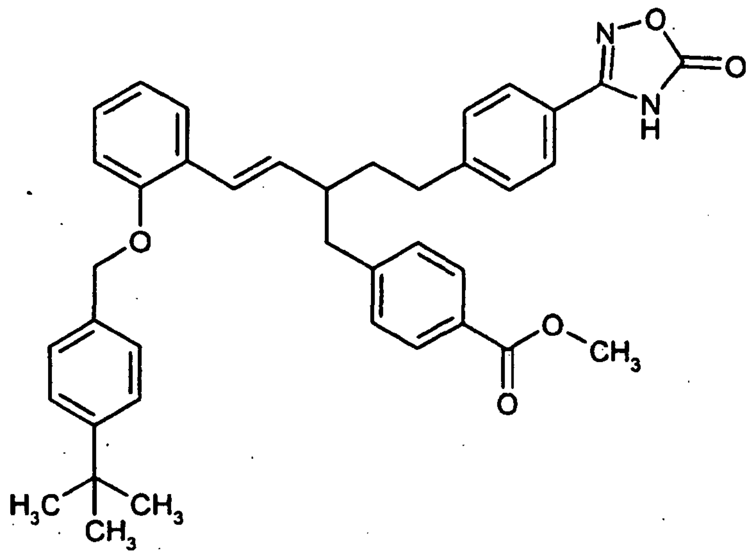

Eine Lösung von 1.9 g (83% Gehalt, 2.67 mmol) 4-((3*E*)-2-(2-{4-[(Amino(hydroxyimino)methyl]-phenyl}ethyl)-4-{2-[(4-*tert.*-butylbenzyl)oxy]phenyl}but-3-en-1-yl)benzoesäuremethylester in 20 ml DMF wird mit 0.31 ml (3.86 mmol) Pyridin versetzt und im Eisbad abgekühlt. Anschließend werden 0.63 ml (3.2 mmol) 2-Ethylhexylchlorformiat zugetropft und die Mischung 30 min bei 0°C nachgerührt. Nach vollständigem Umsatz wird mit Wasser versetzt und mit Diethylether extrahiert. Die organische Phase wird über Natriumsulfat getrocknet und eingeengt. Der erhaltene Rückstand wird über Flash-Chromatographie an Kieselgel aufgereinigt (Laufmittel: Cyclohexan/Essigsäureethylester 4:1). Das so erhaltene Produkt wird in 20 ml Xylol aufgenommen und 2 h unter Rückfluss erhitzt. Danach wird Wasser zugegeben und mit Diethylether extrahiert. Die organische Phase wird mit Natriumchlorid-Lösung gewaschen, über Natriumsulfat getrocknet und eingeengt. Der erhaltene Rückstand wird über Flash-Chromatographie an Kieselgel gereinigt (Laufmittel: Cyclohexan/Essigsäureethylester 4:1 → 1:1). Es werden 503 mg (0.82 mmol, 24% d. Th.) der Titelverbindung erhalten.

HPLC (Methode 2): $R_t$ = 3.31 min

MS (ESIpos): m/z = 617 $(M+H)^+$.

**Beispiel 126A**

Triphenyl-[2-(trifluormethoxy)benzyl]phosphoniumbromid

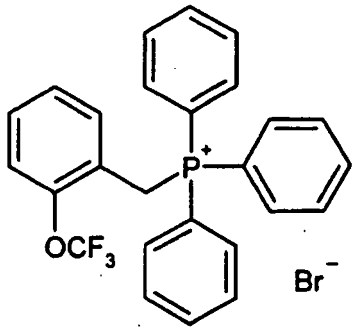

5 g (26 mmol) 2-Trifluormethoxybenzylalkohol und 8.5 g (24.7 mmol) Triphenylphosphonium-bromid werden in 100 ml Acetonitril 3 h unter Rückfluss gerührt. Nach dem Abkühlen wird der entstandene Niederschlag abgesaugt und getrocknet. Es werden 13.5 g (quant.) der Titelverbindung erhalten.
LC-MS (Methode 3): $R_t$ = 1.91 min; MS (ESIpos): m/z = 437 (M-Br)$^+$.

**Beispiel 127A**

*E*-4-[3-(4-Cyanobenzyl)-5-(2-hydroxyphenyl)-pent-4-enyl]-benzoesäuremethylester *(Enantiomer 1)*

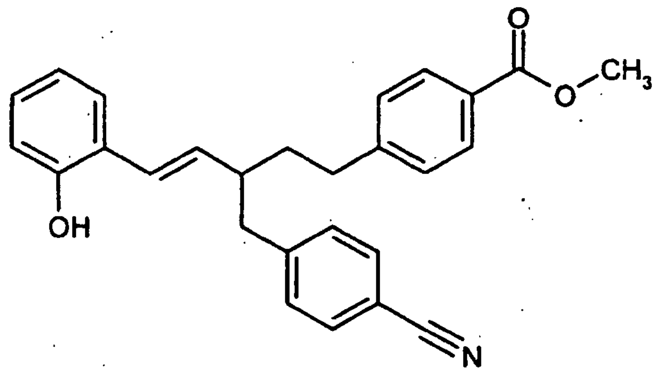

11.3 g (27.5 mmol) des unter Beispiel 75A erhaltenen racemischen *E*-4-[3-(4-Cyanobenzyl)-5-(2-hydroxyphenyl)-pent-4-enyl]-benzoesäuremethylesters werden mittels präparativer HPLC an chiraler Phase weiter aufgetrennt. Es werden jeweils enantiomerenrein 4.14 g bzw. 3.69 g der beiden *E*-Isomere als farblose Feststoffe erhalten (Beispiel 127A bzw. 128A).

Methode Enantiomerentrennung:

Säule: Daicel Chiralpak AD-H 250 mm x 4.6 mm, 5 μm; Elutionsmittel: Isohexan / Isopropanol 50:50 (v/v); Fluss: 1 ml/min; UV-Detektion: 210 nm; Temperatur: 25°C.
$R_t$ 6.77 min; Reinheit 96.85%; >99.5% ee
Ausbeute: 4.14 g
LC-MS (Methode 6): $R_t$ = 3.03 min; MS (ESIpos): m/z = 412 (M+H)$^+$.

**Beispiel 128A**

*E*-4-[3-(4-Cyanobenzyl)-5-(2-hydroxyphenyl)-pent-4-enyl]-benzoesäuremethylester (*Enantiomer 2)*

Methode Enantiomerentrennung: siehe Beispiel 127A.
$R_t$ 7.82 min; Reinheit 96%; >99% ee
Ausbeute: 3.69 g
LC-MS (Methode 6): $R_t$ = 3.03 min; MS (ESIpos): m/z = 412 (M+H)$^+$.

**Ausführungsbeispiele:**

**Beispiel 1**

4-[({2-[4-(5-Oxo-4,5-dihydro-[1,2,4]oxadiazol-3-yl)phenyl]ethyl}-{2-[2-(4'-trifluormethyl-biphenyl-4-ylmethoxy)phenyl] ethyl}amino)methyl]benzoesäure

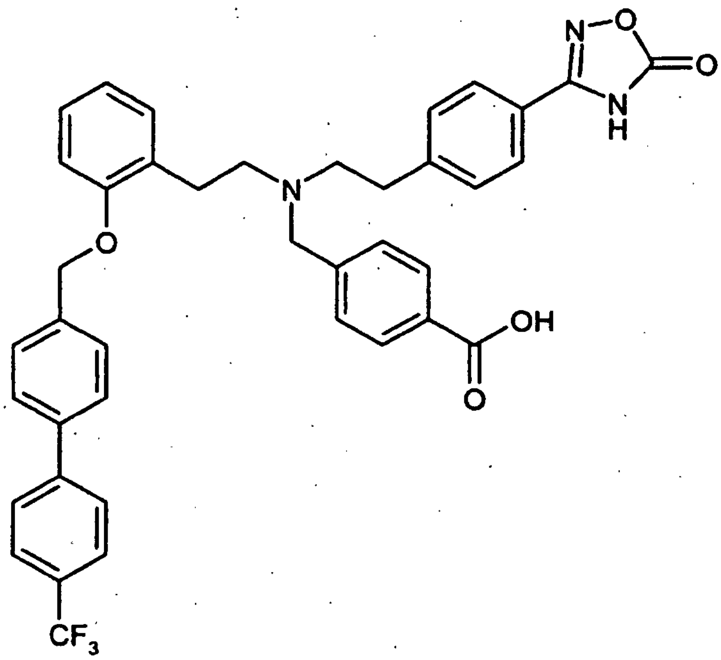

Eine Lösung von 32.7 mg (0.05 mmol) 4-[({2-[4-(5-Oxo-4,5-dihydro-[1,2,4]oxadiazol-3-yl)-phenyl)ethyl}-{2-[2-(4'-trifluormethyl-biphenyl-4-ylmethoxy)phenyl)ethyl}amino)methyl]benzoesäuremethylester aus Beispiel 11A in 2 ml THF und 2 ml Wasser wird mit 2.2 mg (0.09 mmol) Lithiumhydroxid versetzt und 12 h bei 50°C gerührt. Nach dem Abkühlen wird das THF abgezogen und die wässrige Phase mit 1 M Salzsäure auf pH 4-5 eingestellt. Anschließend wird mit Essigsäureethylester dreimal extrahiert und die vereinigten organischen Phasen über Natriumsulfat getrocknet. Nach Abfiltrieren und Einengen wird der erhaltene Rückstand mittels präparativer HPLC gereinigt. Es werden 7.4 mg (0.01 mmol, 23% Ausbeute) eines farblosen Ols erhalten.

$^1$H-NMR (400 MHz, DMSO-$d_6$, $\delta$/ppm): 13,0-12,5 (2H, breit), 7,79 (2H, d), 7,62 (1H, d), 7,52 (2H, d), 7,49-7,39 (4H, m), 7,31-7,13 (8H, m), 7,12-7,00 (2H, m), 6,86 (1H, t), 5,08 (2H, s), 3,71 (2H, s), 2,82-2,61 (8H, m).

LC-MS (Methode 3): $R_t$ 2.51 min; m/z 694 (M+H$^+$).

Auf analoge Weise werden die in der folgenden Tabelle aufgeführten Beispiele erhalten:

| Beispiel | Struktur | Analytische Daten |
|---|---|---|
| **2** (ausgehend von (5-Brom-pentyl)benzol und Bsp. 3A) | 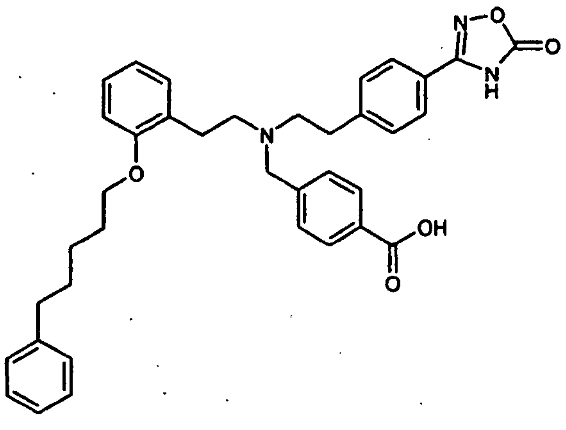 | $^1$H-NMR (300 MHz, DMSO-$d_6$, δ/ppm): 12,97-12,51 (2H, breit), 7,81-7,78 (2H, d), 7,70-7,67 (2H, d), 7,32-7,03 (11H, m), 6,88-6,78 (2H, m), 3,98-3,11 (m), 2,56-2,45 (m), 1,66-1,47 (4H, m), 1,42-1,29 (3H, s). LC-MS (Methode 2): $R_t$ 2.05 min; m/z 606 ($M+H^+$). |
| **3** (ausgehend von Bsp. 9A) | 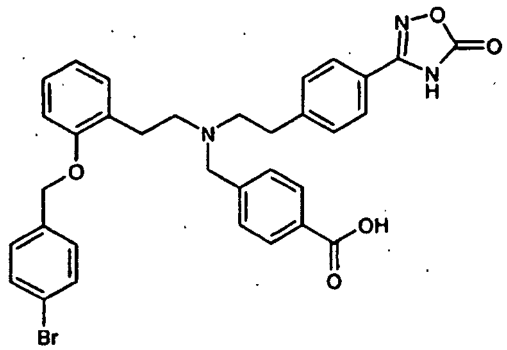 | $^1$H-NMR (300 MHz, DMSO-$d_6$, δ/ppm): 13,0-12,5 (2H, breit), 7,79 (2H, $m_c$), 7,67 (2H, $m_c$), 7,48 (2H, $m_c$), 7,32-7,12 (8H, m), 6,98 (1H, $m_c$), 6,85 (2H, $m_c$), 5,02 (2H, s), 2,42 (2H, $m_c$), 2,23 (2H, $m_c$). HPLC (Methode 1): 4.5 min. MS: 628 ($M+H^+$ $^{79}$Br), 630 ($M+H^+$ $^{81}$Br). |
| **4** (ausgehend von Bsp. 9A und 4-Methoxybenzolboronsäure) | 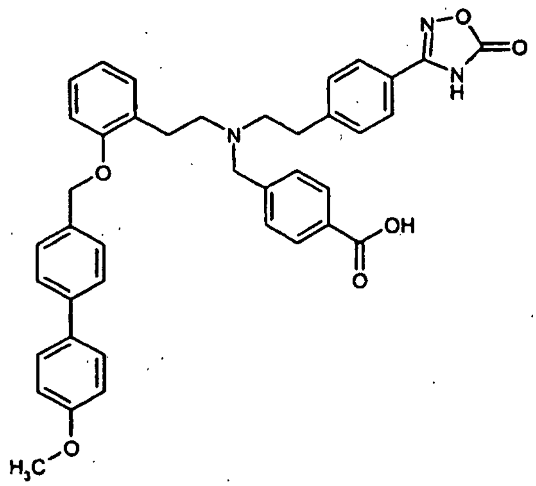 | $^1$H-NMR (300 MHz, DMSO-$d_6$, δ/ppm): 13,0-12,5 (2H, breit), 7,78 (2H, $d_{AB}$), 7,63 (2H, $d_{AB}$), 7,53-7,45 (4H, m), 7,41 (2H, $d_{AB}$), 7,29-7,13 (6H, m), 7,07 (1H, $m_c$), 6,97 (2H, $m_c$), 6,85 (1H, $m_c$), 5,06 (2H, s), 3,28 (3H, s), 2,73 (4H, $m_c$), 2,28 (2H, $m_c$). HPLC (Methode 1): 4.7 min. MS: 656 ($M+H^+$). |

(fortgesetzt)

| Beispiel | Struktur | Analytische Daten |
|---|---|---|
| **5** (ausgehend von Bsp. 9A und 4-Fluorbenzolboronsäure) | 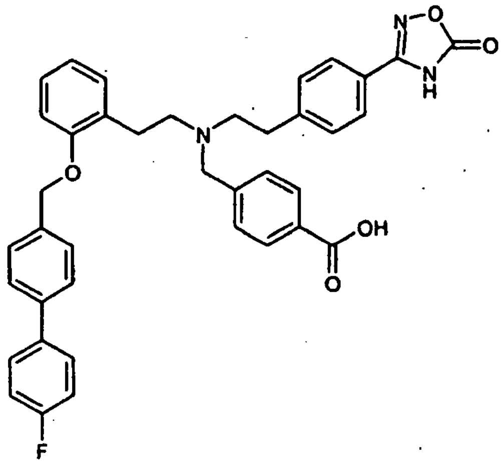 | LC-MS (Methode 2): R, 2.03 min; m/z 644 $(M+H^+)$. |
| **6** (ausgehend von Bsp. 9A und Benzolboronsäure) | 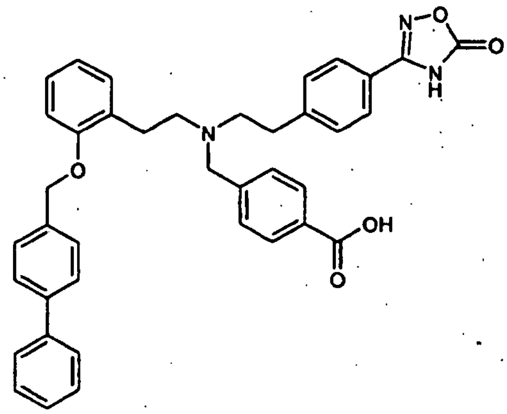 | LC-MS (Methode 3): $R_t$ 2.00 min; m/z 626 $(M+H^+)$. |
| **7** (ausgehend von Bsp. 9A und 4-Methylbenzolboronsäure) | 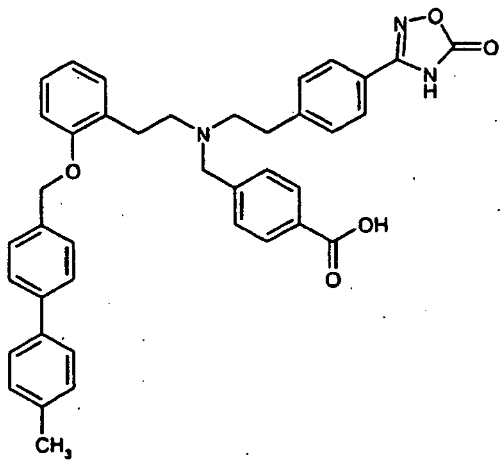 | $^1$H-NMR (400 MHz, DMSOd6, δ/ppm): 13,00-12,49 (2H, breit), 7,79 (2H, d), 7,63 (1H, d), 7,59-7,38 (6H, m), 7,34-7,13 (8H, m), 7,12-6,98 (2H, m), 6,84 (1H, t), 5,06 (2H, s), 3,73 (2H, s), 2,86-2,62 (8H, m), 2,33 (3H, s). LC-MS (Methode 3): R, 2.01 min; m/z 640 $(M+H^+)$. |

(fortgesetzt)

| Beispiel | Struktur | Analytische Daten |
|---|---|---|
| **8** (ausgehend von Bsp. 9A und 4-Chlorbenzolboronsäure) | 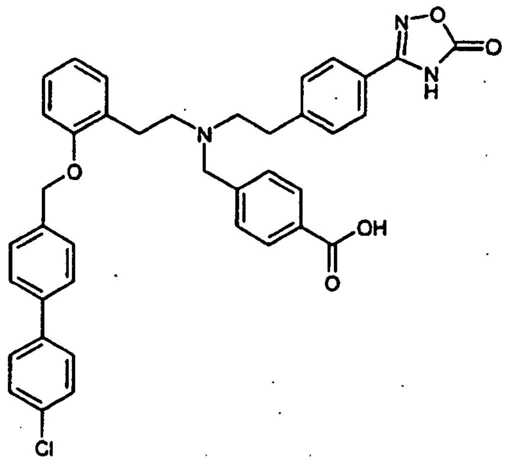 | LC-MS (Methode 3): $R_t$ 2.01 min; m/z 660 ($M^+$). |

**Beispiel 9**

rac-*E*-4-{2-{2-{4-(5-Oxo-4,5-dihydro-[1,2,4]oxadiazol-3-yl)pheny]ethyl}-4-[2-(4'-trifluormethylbiphenyl-4-yl-methoxy) phenyl]-but-3-enyl}-benzoesäure

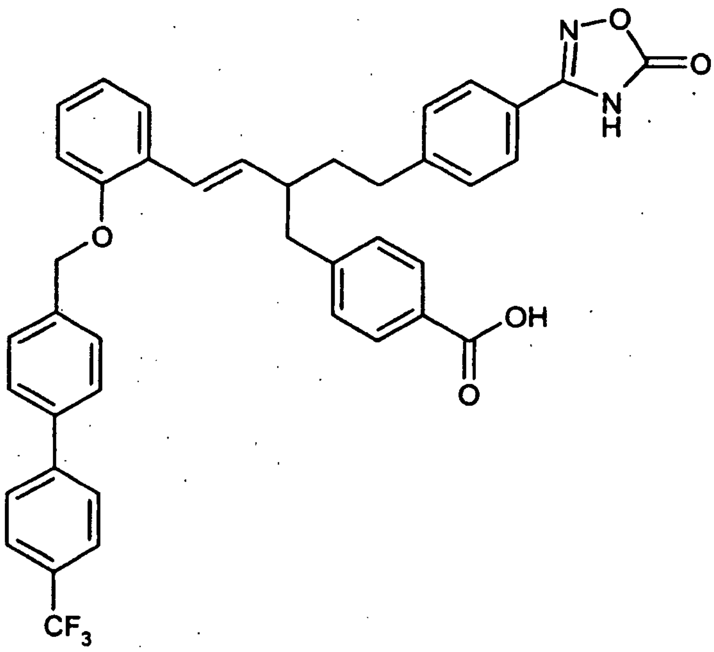

Eine Lösung von 35 mg (0.05 mmol) *E*-4-{2-{2-[4-(5-Oxo-4,5-dihydro-[1,2,4]oxadiazol-3-yl)-phenyl]ethyl}-4-[2-(4'-trifluormethyl-biphenyl-4-yl-methoxy)phenyl]-but-3-enyl}-benzoesäuremethylester aus Beispiel 20A in 2 ml THF und 2 ml Wasser wird mit 2.38 mg (0.1 mmol) Lithiumhydroxid versetzt und 12 h bei 50°C gerührt. Nach dem Abkühlen wird das THF abgezogen und die wässrige Phase mit 1 M Salzsäure auf pH 4-5 eingestellt: Anschließend wird mit Essigsäureethylester dreimal extrahiert und die vereinigten organischen Phasen über Natriumsulfat getrocknet. Nach Abfiltrieren und Einengen wird der erhaltene Rückstand mittels präparativer HPLC gereinigt. Es werden 19.8 mg (0.028 mmol, 57% Ausbeute) eines farblosen Feststoffes erhalten.

$^1$H-NMR (400 MHz, DMSO-$d_6$, $\delta$/ppm): 12,92-12,68 (2H, breit), 7,90-7,77 (6H, m), 7,70 (2H, d), 7,68 (2H, d), 7,50 (2H, d), 7,43 (1H, d), 7,35 (2H, d), 7,28 (2H, d), 7,19 (1H, t), 7,06 (1H, d), 6,92 (1H, t), 6,51 (1H, d), 6,12 (1H, dd),

5,16 (2H, s), 2,94-2,82 (1H, m), 2,79-2,69 (2H, m), 2,68-2,57 (1H, m), 2,56-2,44 (1H, m), 1,89-1,76 (1H, m), 1,75-1,61 (1H, m).

LC-MS (Methode 4): $R_t$ 3.23 min; m/z 690 ($M^+$).

## Beispiel 10

*E*/*Z*-4-(4-{5-Fluor-2-[2-(4-methoxyphenyl)ethyl]phenyl}-2-{2-[4-(5-oxo-4,5-dihydro-[1,2,4]oxadiazol-3-yl)phenyl] ethyl}-but-3-enyl)-benzoesäure

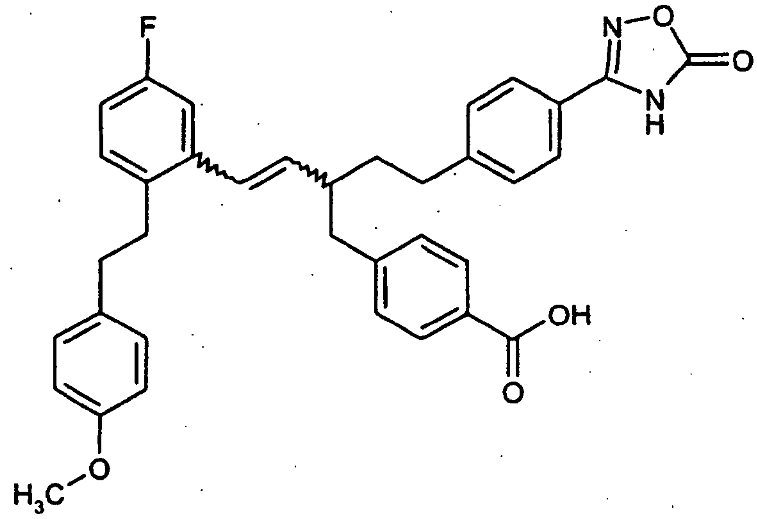

Eine Lösung von 447 mg (0.74 mmol) *E*/*Z*-4-(4-{5-Fluor-2-(2-(4-methoxyphenyl)ethyl]phenyl}-2-{2-[4-(5-oxo-4,5-dihydro-[1,2,4]oxadiazol-3-yl)phenyl]ethyl}-but-3-enyl)-benzoesäuremethylester aus Beispiel 69A in 10 ml THF und 10 ml Wasser wird mit 35.3 mg (1.47 mmol) Lithiumhydroxid versetzt und 12 h bei 50°C gerührt. Nach dem Abkühlen wird das THF abgezogen und die wässrige Phase mit 1 M Salzsäure auf pH 4-5 eingestellt. Anschließend wird mit Essigsäureethylester dreimal extrahiert und die vereinigten organischen Phasen über Natriumsulfat getrocknet. Nach Abfiltrieren und Einengen wird der erhaltene Rückstand durch Flashchromatographie an Kieselgel gereinigt (Laufmittel: Cyclohexan/Essigsäureethylester 4:1 → 2:1 → 1:1). Es werden 291 mg (0.49 mmol, 63% Ausbeute) eines farblosen Feststoffes erhalten.

$^1$H-NMR (300 MHz, DMSO-$d_6$, δ/ppm): 2:1-*E*/*Z*-Gemisch: 13,03-12,59 (2H, breit), 7,80 (2H, t), 7,71 (0,66H, d), 7,63 (0,33H, d), 7,42-6,72 (12H, m), 6,45 (0,33H, d), 6,36 (0,66H, d), 6,18-6,02 (1H, m), 5,67 (0,33H, t), 3,69 (3H, s), 2,99-2,42 (9H, m), 1,93-1,57 (2H, m).

MS (DCI): 593 ($M+H^+$).

291 mg (0.49 mmol) der so erhaltenen *E*/*Z*-4-(4-{5-Fluor-2-[2-(4-methoxyphenyl)ethyl]phenyl}-2-{2-[4-(5-oxo-4,5-dihydro-[1,2,4]oxadiazol-3-yl)phenyl]ethyl}-but-3-enyl)-benzoesäure werden mittels präparativer HPLC an chiraler Phase weiter aufgetrennt. Es werden jeweils enantiomerenrein 53 mg bzw. 64.5 mg der beiden E-Isomere und 72.4 mg des racemischen Z-Isomers als farblose Feststoffe erhalten (siehe Beispiele 11-13).

## Beispiel 11

*E*-4-(4-{5-Fluor-2-[2-(4-methoxyphenyl)ethyl]phenyl}-2-{2-[4-(5-oxo-4,5-dihydro-[1,2,4]oxadiazol-3-yl)phenyl] ethyl}-but-3-enyl)-benzoesäure *(Enantiomer 1)*

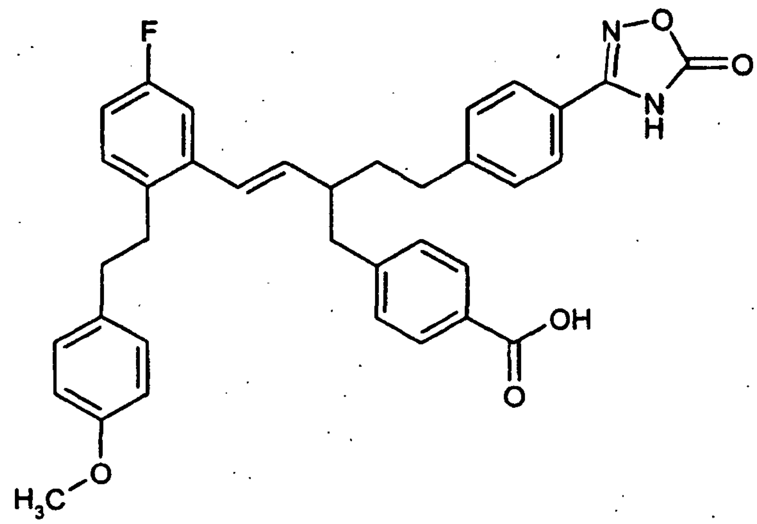

Methode Enantiomerentrennung:

Säule: Daicel Chiralpak AD-H 250 min x 20 mm; Elutionsmittel: Isopropanol (mit 1% Wasser und 0.2% Trifluoressigsäure) / Isohexan 30:70 (v/v); Fluss: 15 ml/min; UV-Detektion: 220 nm; Temperatur: 24°C.
$R_t$ 14.87 min; Reinheit 95%; >99% ee
Ausbeute: 53 mg
$^1$H-NMR (300 MHz, DMSO-$d_6$, δ/ppm): 13,02-12,49 (2H, breit), 7,81 (2H, d), 7,70 (2H, d), 7,39 (2H, d), 7,30 (2H, d), 7,26-7,15 (1H, m), 7,14-7,06 (1H, m), 6,99-6,88 (3H, m), 6,79 (2H, d), 6,37 (1H, d), 6,15-6,00 (1H, m), 3,69 (3H, s), 2,99-2,39 (9H, m), 1,94-1,67 (2H, m).
MS (DCI): 593 (M+H$^+$).

## Beispiel 12

*E*-4-(4-{5-Fluor-2-[2-(4-methoxyphenyl)ethyl]phenyl}-2-{2-[4-(5-oxo-4,5-dihydro-[1,2,4]oxadiazol-3-yl)phenyl] ethyl}-but-3-enyl)-benzoesäure *(Enantiomer 2)*

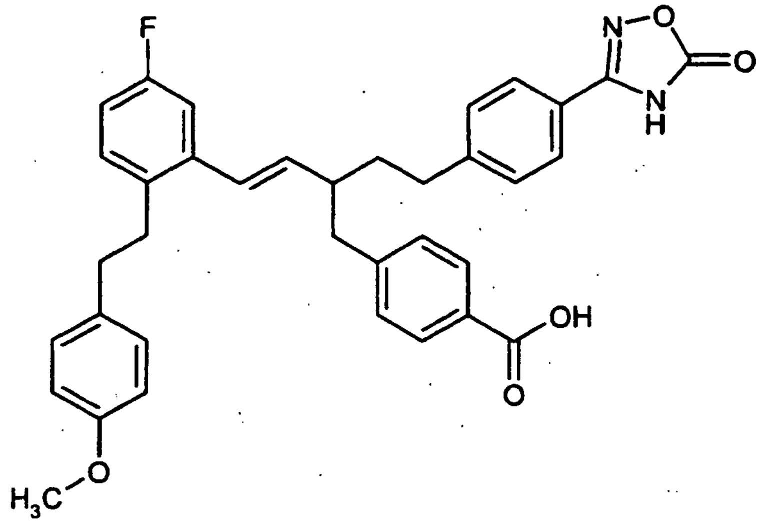

Methode Enantiomerentrennung: siehe Beispiel 11.
$R_t$ 16.66 min; Reinheit 92%; >94% ee
Ausbeute: 64.5 mg
$^1$H-NMR (300 MHz, DMSO-$d_6$, δ/ppm): 13,02-12,49 (2H, breit), 7,81 (2H, d), 7,70 (2H, d), 7,39 (2H, d), 7,30

(2H, d), 7,26-7,15 (1H, m), 7,14-7,06 (1H, m), 6,99-6,88 (3H, m), 6,79 (2H, d), 6,37 (1H, d), 6,15-6,00 (1H, m), 3,69 (3H, s), 2,99-2,39 (9H, m), 1,94-1,67 (2H, m).

MS) (DCI): 593 (M+H$^+$).

## Beispiel 13

rac-*Z*-4-(4-{5-Fluor-2-[2-(4-methoxyphenyl)ethyl]phenyl}-2-{2-[4-(5-oxo-4,5-dihydro-[1,2,4]oxadiazol-3-yl)phenyl] ethyl}-but-3-enyl)-benzoesäure

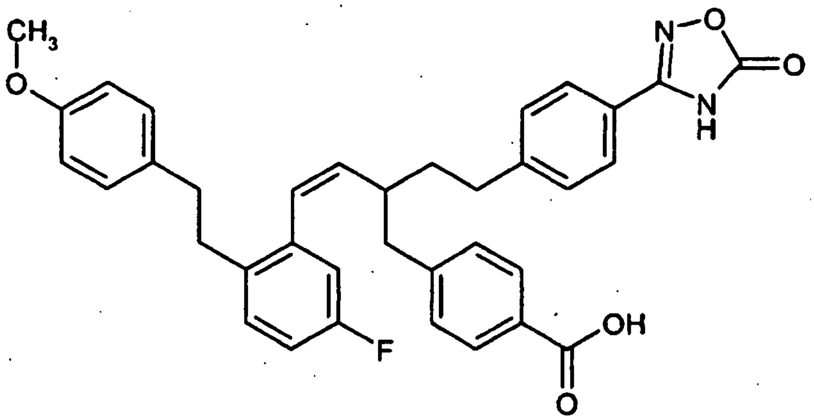

Methode Isomerentrennung: siehe Beispiel 11.

$R_t$ 13.23 min; Reinheit 97%

Ausbeute: 72.4 mg

$^1$H-NMR (300 MHz, DMSO-$d_6$, $\delta$/ppm): 13,05-12,61 (2H, breit), 7,79 (2H, d), 7,62 (2H, d), 7,24 (2H, d), 7,18 (2H, d), 7,14-7,06 (1H, m), 6,99 (2H, d), 6,98-6,88 (1H, m), 6,79 (2H, d), 6,46 (1H, d), 6,18-6,08 (1H, m), 5,68 (1H, t), 3,69 (3H, s), 2,95-2,80 (1H, m), 2,77-2,41 (8H, m), 1,83-1,54 (2H, m).

MS (DCI): 593 (M+H$^+$).

Auf analoge Weise werden die in der folgenden Tabelle aufgeführten Beispiele erhalten:

| Beispiel | Struktur | Analytische Daten |
|---|---|---|
| **14**<br>(ausgehend von Bsp. 28A und Bsp. 16A) | 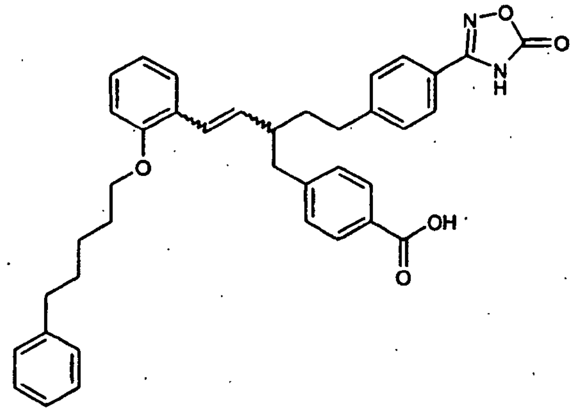 | LC-MS (Methode 2): $R_t$ 3.01 min; m/z 603 (M+H$^+$). |

(fortgesetzt)

| Beispiel | Struktur | Analytische Daten |
|---|---|---|
| **15**<br><br>(*E*-Enantiomer 1, ausgehend von Bsp. 14 nach Trennung) | 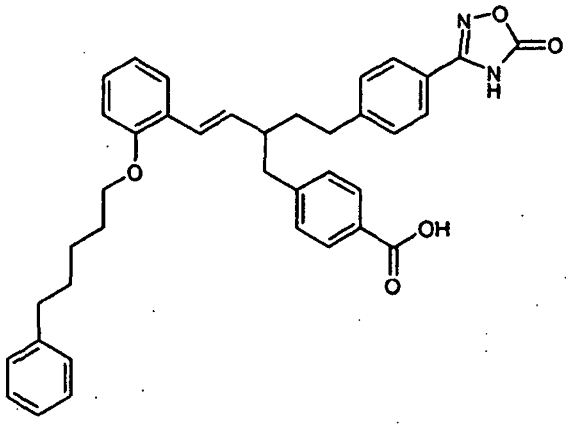 | $^1$H-NMR (300 MHz, DMSO-$d_6$, δ/ppm): 12,95-12,60 (2H, breit), 7,81 (2H, d), 7,70 (2H, d), 7,38 (3H, d), 7,32-7,18 (4H, m), 7,17-7,08 (4H, m), 6,97-6,82 (2H, m), 6,42 (1H, d), 6,16-6,01 (1H, m), 3,96-3,83 (2H, m), 2,94-2,40 (7H, m), 1,90-1,51 (6H, m), 1,48-1,29 (2H, m).<br>LC-MS (Methode 2): $R_t$ 3.01 min; m/z 603 (M+H$^+$).<br>chirale HPLC: $R_t$ 17.35 min;<br>Reinheit 98%; >99.5% ee. |
| **16**<br><br>(*E*-Enantiomer 2, ausgehend von Bsp. 14 nach Trennung) | 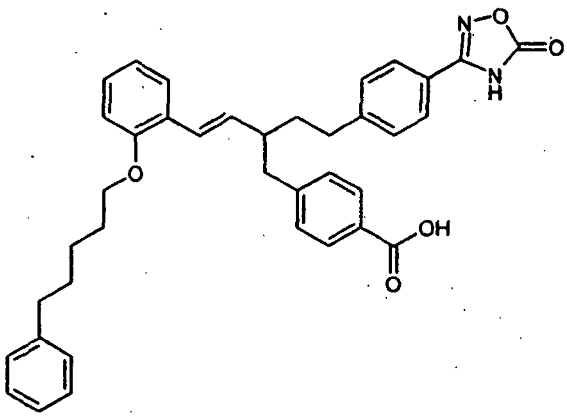 | $^1$H-NMR und LC-MS siehe Beispiel 15;<br>chirale HPLC: $R_t$ 12.12 min;<br>Reinheit 94%; >99.5% ee. |
| **17**<br>(ausgehend von Bsp. 25A und Bsp. 16A) | 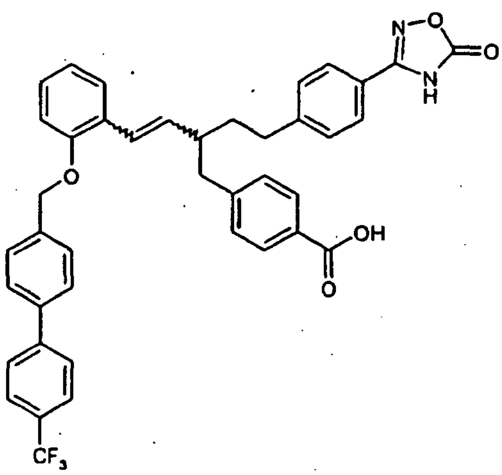 | LC-MS (Methode 1): $R_t$ 3.24 min; m/z 691 (M+H$^+$). |

(fortgesetzt)

| Beispiel | Struktur | Analytische Daten |
|---|---|---|
| **18** (*E*-Enantiomer 1, ausgehend von Bsp. 17 nach Trennung) | 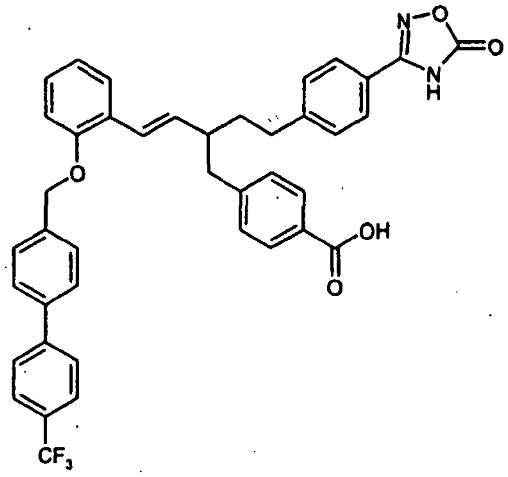 | $^1$H-NMR (400 MHz, DMSO-$d_6$, δ/ppm): 12,92-12,68 (2H, breit), 7,90-7,77 (6H, m), 7,70 (2H, d), 7,68 (2H, d), 7,50 (2H, d), 7,43 (1H, d), 7,35 (2H, d), 7,28 (2H, d), 7,19 (1H, t), 7,06 (1H, d), 6,92 (1H, t), 6,51 (1H, d), 6,12 (1H, dd), 5,16 (2H, s), 2,94-2,82 (1H, m), 2,79-2,69 (2H, m), 2,68-2,57 (1H, m), 2,56-2,44 (1H, m), 1,89-1,76 (1H, m), 1,75-1,61 (1H, m). LC-MS (Methode 1): $R_t$ 3.24 min; m/z 691 (M+H$^+$). chirale HPLC: $R_t$ 9.79 min; Reinheit >99.5%; >99.5% ee. |
| **19** (*E*-Enantiomer 2,ausgehend von Bsp. 17 nach Trennung) | 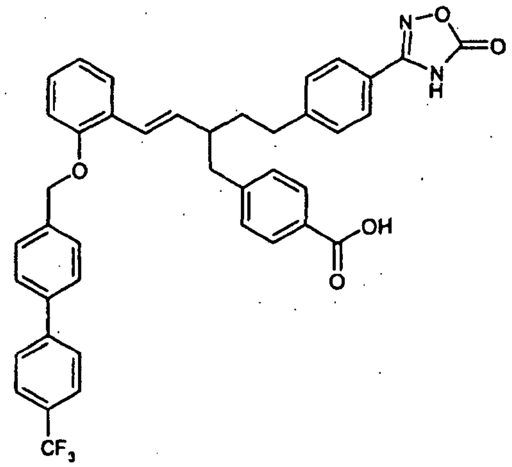 | $^1$H-NMR und LC-MS siehe Beispiel 18; chirale HPLC: $R_t$ 7.46 min; Reinheit 98.5%; >99.5% ee. |
| **20** (rac-Z, ausgehend von Bsp. 17 nach Trennung) | 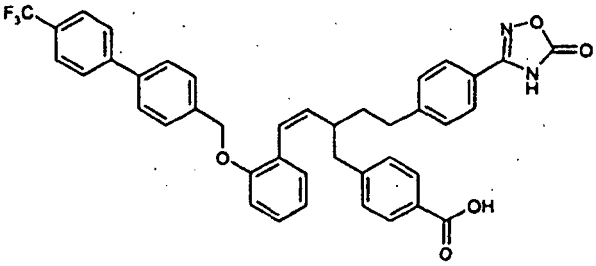 | LC-MS (Methode 1): $R_t$ 3.24 min; m/z 691 (M+H$^+$). |

(fortgesetzt)

| Beispiel | Struktur | Analytische Daten |
|---|---|---|
| **21** (ausgehend von Bsp. 50A und Bsp. 16A) | 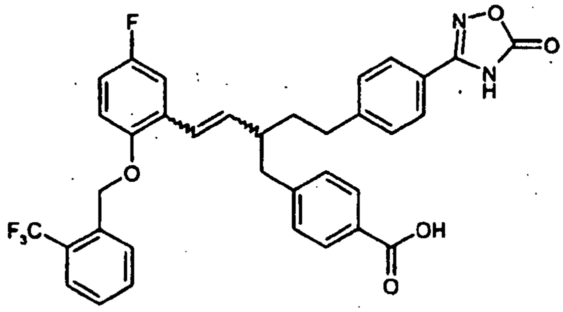 | $^1$H-NMR (400 MHz, DMSO-$d_6$, $\delta$/ppm): 12,87-12,76 (2H, m), 7,79-7,75 (3H, m), 7,70-7,52 (5H, m), 7,32-7,16 (5H, m), 7,04-6,93 (2H, m), 6,46-6,39 (1H, m), 6,23-6,14 (1H, m), 5,61-5,55 (0,5H, m), 5,17-5,07 (2H, m), 2,89-2,81 (1H, m), 2,73-2,53 (3H, m), 2,52-2,40 (1H, m), 1,83-1,52 (2H, m). LC-MS (Methode 4): $R_t$ 3.01 min; m/z 633 (M+H$^+$). |
| **22** (*E*-Enantiomer 1, ausgehend von Bsp. 21 nach Trennung) | 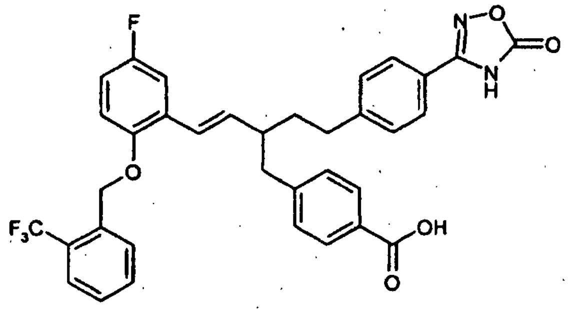 | LC-MS (Methode 4): $R_t$ 3.01 min; m/z 633 (M+H$^+$). |
| **23** (*E*-Enantiomer 2, ausgehend von Bsp. 21 nach Trennung) | 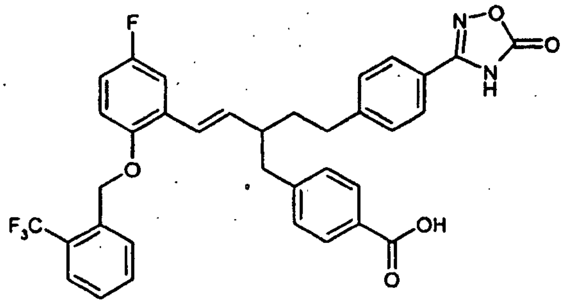 | LC-MS (Methode 2): $R_t$ 2.82 min; m/z 633 (M+H$^+$). |

(fortgesetzt)

| Beispiel | Struktur | Analytische Daten |
|---|---|---|
| **24** (*Z*-Enantiomer 1, ausgehend von Bsp. 21 nach Trennung) | 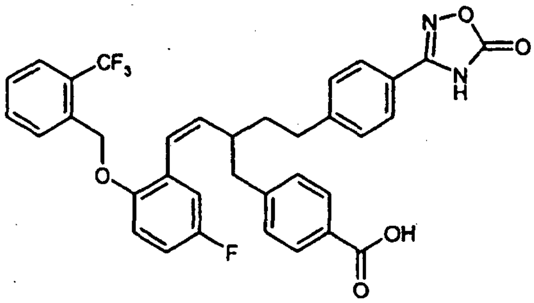 | $^1$H-NMR (400 MHz, $CDCl_3$, δ/ppm): 10,91-10,88 (1H, breit), 7,88-7,86 (2H, m), 7,69-7,67 (2H, m), 7,55-7,51 (1H, m), 7,45-7,39 (3H, m), 7,07-7,05 (2H, m), 6,94-6,87 (3H, m), 6,83-6,79 (1H, m), 6,63-6,60 (2H, m), 5,66-5,61 (1H, m), 5,24-5,16 (2H, m), 2,78-2,62 (4H, m), 2,41-2,33 (1H, m), 1,91-1,47 (2H, m). |
| **25** (*Z*-Enantiomer 2,ausgehend von Bsp. 21 nach Trennung) | 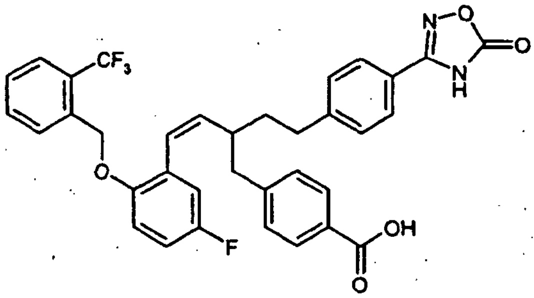 | LC-MS (Methode 2): $R_t$ 2.81 min; m/z 633 (M+H$^+$). |
| **26** (ausgehend von Bsp. 53A und Bsp. 16A) | 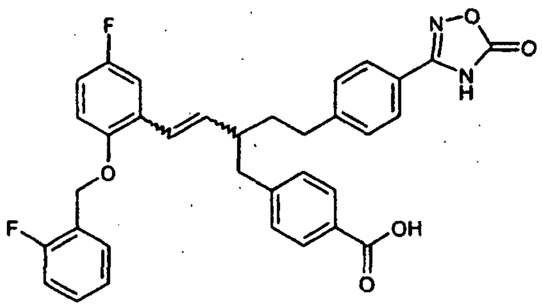 | $^1$H-NMR (400 MHz, DMSO-$d_6$, δ/ppm): 12,86-12,76 (2H, breit), 7,80-7,77 (2H, m), 7,69-7,66 (1H, m), 7,61-7,59 (1H, m), 7,48-7,14 (9H, m), 7,11-6,96 (2H, m), 6,44-6,38 (1H, m), 6,23-6,10 (1H, m), 5,60-5,54 (0,5H, m), 5,09-5,0 (2H, m), 2,86-2,80 (1H, m), 2,73-2,56 (3H, m), 2,52-2,38 (1H, m), 1,82-1,54 (2H, m). LC-MS (Methode 1): $R_t$ 2.91 min; m/z 583 (M+H$^+$). |

(fortgesetzt)

| Beispiel | Struktur | Analytische Daten |
|---|---|---|
| **27**<br>(*E*-Enantiomer 1, ausgehend von Bsp. 26 nach Trennung) | 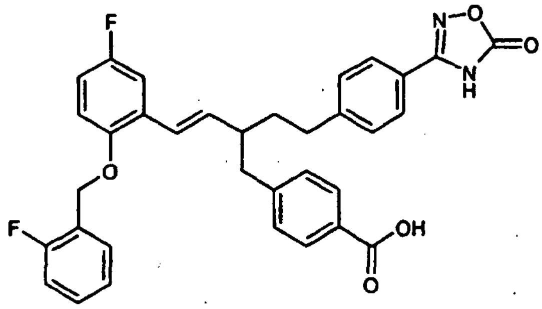 | LC-MS (Methode 1): $R_t$ 2.91 min; m/z 583 (M+H$^+$). |
| **28**<br>(*E*-Enantiomer 2, ausgehend von Bsp. 26 nach Trennung) | 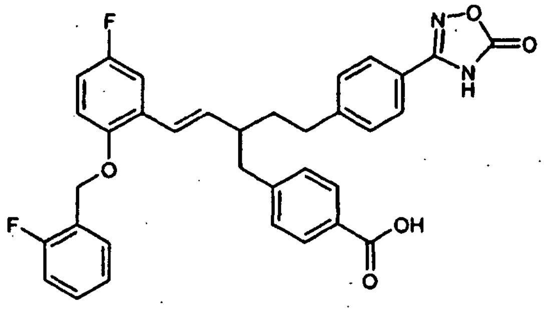 | $^1$H-NMR (400 MHz, DMSO-$d_6$, $\delta$/ppm): 12,92-12,72 (2H, m), 7,81-7,77 (2H, m), 7,70-7,64 (2H, m), 7,47-7,35 (2H, m), 7,33-7,16 (7H, m), 7,12-6,98 (2H, m), 6,43-6,35 (1H, m), 6,24-6,15 (1H, m), 5,10-5,07 (2H, m), 2,88-2,80 (1H, m), 2,75-2,58 (3H, m), 2,54-2,42 (1H, m), 1,82-1,57 (2H, m).<br>LC-MS (Methode 1): $R_t$ 2.91 min; m/z 583 (M+H$^+$). |
| **29**<br>(*Z*-Enantiomer 1, ausgehend von Bsp. 26 nach Trennung) | 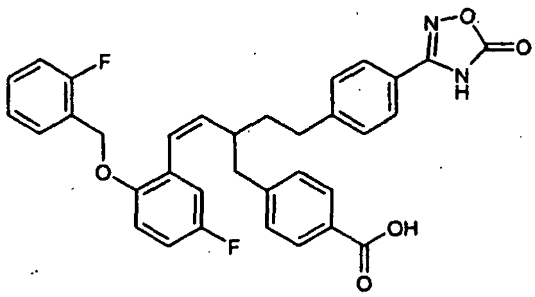 | $^1$H-NMR (400 MHz, DMSO-$d_6$, $\delta$/ppm): 12,93-12,75 (2H, breit), 7,82-7,77 (2H, m), 7,62-7,56 (2H, m), 7,48-7,43 (1H, m), 7,41-7,34 (1H, m), 7,24-7,12 (6H, m), 7,07-6,96 (2H, m), 6,44-6,40 (1H, m), 6,11-6,06 (1H, m), 5,60-5,53 (1H, m), 5,11-4,98 (2H, m), 2,85-2,60 (4H, m), 2,46-2,41 (1H, m), 1,76-1,52 (2H, m).<br>LC-MS (Methode 1): $R_t$ 2.89 min; m/z 583 (M+H$^+$). |

(fortgesetzt)

| Beispiel | Struktur | Analytische Daten |
|---|---|---|
| **30** (*Z*-Enantiomer 2, ausgehend von Bsp. 26 nach Trennung) | 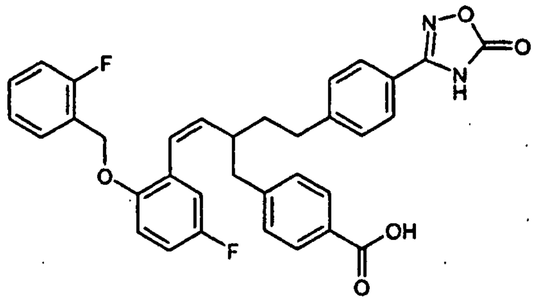 | LC-MS (Methode 1): $R_t$ 2.89 min; m/z 583 (M+H$^+$). |
| **31** (ausgehend von Bsp. 42A und Bsp. 16A) | 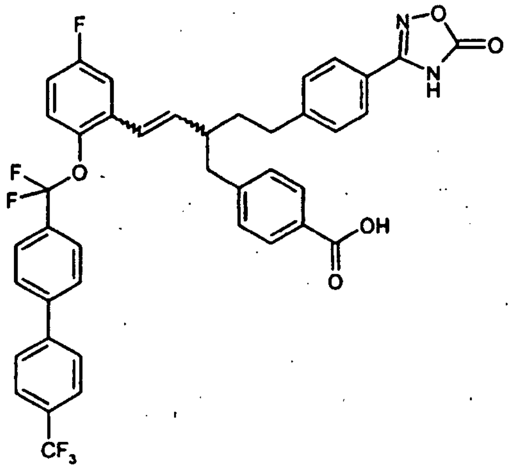 | LC-MS (Methode 4): $R_t$ 3.27 min; m/z 745 (M+H$^+$). |
| **32** (*E*-Enantiomer 1, ausgehend von Bsp. 31 nach Trennung) | 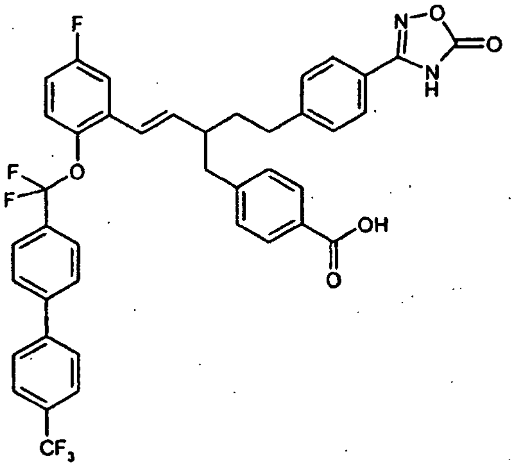 | $^1$H-NMR (400 MHz, $CDCl_3$, δ/ppm): 11,23 (1H, breit), 8,00-7,88 (2H, m), 7,82-7,75 (2H, m), 7,74-7,64 (6H, m), 7,56-7,43 (3H, m), 7,34-7,27 (1H, m), 7,25-7,21 (1H, m), 7,20-7,06 (3H, m), 7,00-6,94 (1H, m), 6,63-6,55 (1H, m), 6,13-6,04 (1H, m), 2,87-2,64 (3H, m), 2,58-2,36 (2H, m), 1,84-1,44 (2H, m). LC-MS (Methode 4): $R_t$ 3.27 min; m/z 745 (M+H$^+$). |

(fortgesetzt)

| Beispiel | Struktur | Analytische Daten |
|---|---|---|
| **33** (*E*-Enantiomer 2,ausgehend von Bsp. 31 nach Trennung) | 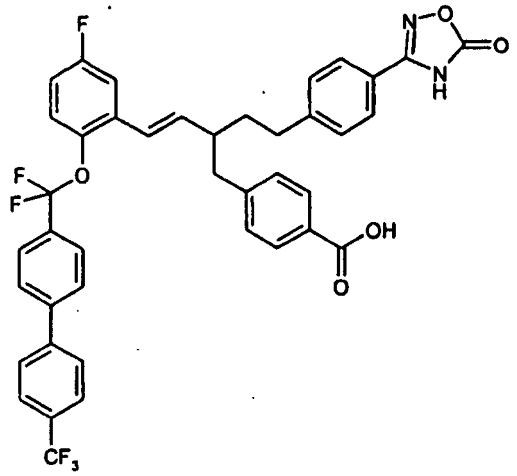 | LC-MS (Methode 4): $R_t$ 3.27 min; m/z 745 (M+H$^+$). |
| **34** (rac-Z, ausgehend von Bsp. 31 nach Trennung) | 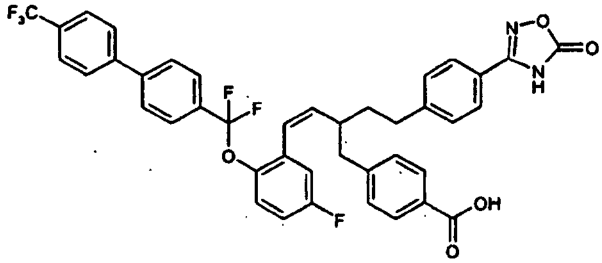 | $^1$H-NMR (400 MHz, $CDCl_3$, $\delta$/ppm): 11,44 (1H, breit), 8,01-7,89 (2H, m), 7,80-7,63 (8H, m), 7,57-7,46 (2H, m), 7,34-7,27 (1H, m), 7,24-7,09 (5H, m), 7,00-6,92 (1H, m), 6,60-6,52 (1H, m), 6,11-6,01 (1H, m), 2,87-2,64 (3H, m), 2,62-2,37 (2H, m), 1,90-1,63 (2H, m). LC-MS (Methode 4): $R_t$ 3.26 min; m/z 745 (M+H$^+$). |
| **35** (ausgehend von Bsp. 54A und Bsp. 16A) | 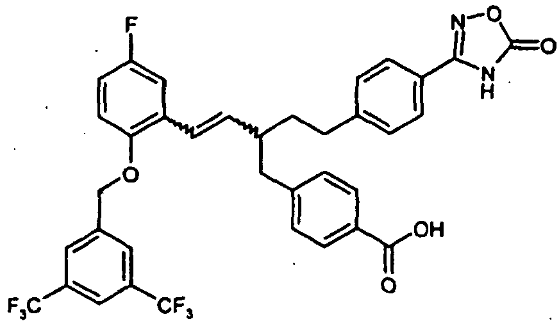 | $^1$H-NMR (400 MHz, DMSO-$d_6$, $\delta$/ppm): 12,91-12,68 (2H, breit), 8,10-8,04 (3H, m), 7,79-7,56 (4H, m), 7,36-6,97 (6H, m), 6,52-6,43 (1H, m), 6,33-6,14 (1H, m), 5,66-5,57 (0,5H, m), 5,29-5,09 (2H, m), 2,91-2,81 (1H, m), 2,78-2,57 (3H, m), 2,54-2,43 (1H, m), 1,84-1,56 (2H, m). LC-MS (Methode 2): $R_t$ 2.92 und 2.96 min; m/z 701 (M+H$^+$). |

(fortgesetzt)

| Beispiel | Struktur | Analytische Daten |
|---|---|---|
| **36** (*E*-Enantiomer 1, ausgehend von Bsp. 35 nach Trennung) | 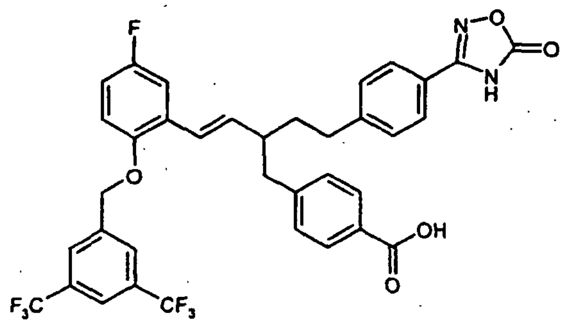 | LC-MS (Methode 1): $R_t$ 3.09 min; m/z 701 ($M+H^+$). |
| **37** (*E*-Enantiomer 2, ausgehend von Bsp. 35 nach Trennung) | 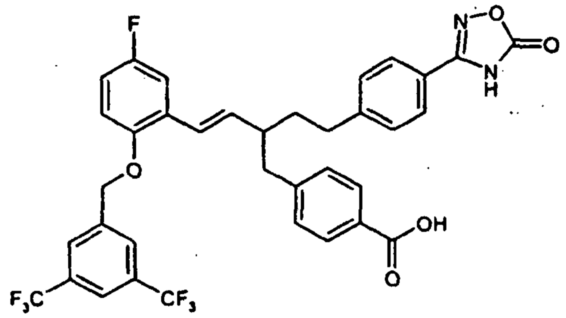 | $^1$H-NMR (400 MHz, DMSO-$d_6$, $\delta$/ppm): 12,76 (2H, breit), 8,12-8,05 (4H, m), 7,87-7,67 (3H, m), 7,36-7,19 (5H, m), 7,09-7,00 (2H, m), 6,53-6,42 (1H, m), 6,32-6,20 (1H, m), 5,32-5,22 (2H, m), 2,91-2,33 (5H, m), 1,97-1,57 (2H, m). LC-MS (Methode 1): $R_t$ 3.10 min; m/z 701 ($M+H^+$). |
| **38** (*Z*-Enantiomer 1, ausgehend von Bsp. 35 nach Trennung) | 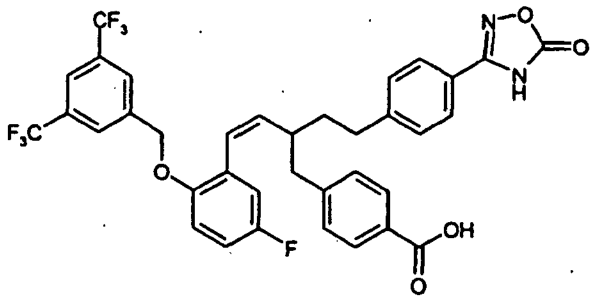 | $^1$H-NMR (400 MHz, DMSO-$d_6$, $\delta$/ppm): 12,72 (2H, breit), 8,11-8,00 (3H, m), 7,87-7,62 (3H, m), 7,26-6,95 (7H, m), 6,51-6,40 (1H, m), 6,25-6,07 (1H, m), 5,67-5,51 (1H, m), 5,30-5,06 (2H, m), 2,89-2,48 (4H, m), 2,46-2,34 (1H, m), 1,81-1,47 (2H, m). LC-MS (Methode 1): $R_t$ 3.13 min; m/z 701 ($M+H^+$). |
| **39** (*Z*-Enantiomer 2,ausgehend von Bsp. 35 nach Trennung) | 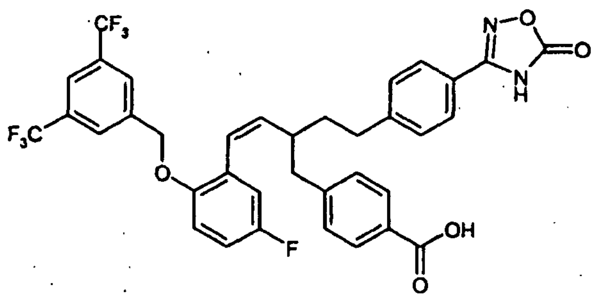 | LC-MS (Methode 1): $R_t$ 3.13 min; m/z 701 ($M+H^+$). |

(fortgesetzt)

| Beispiel | Struktur | Analytische Daten |
|---|---|---|
| **40** (ausgehend von Bsp. 46A und Bsp. 16A) | 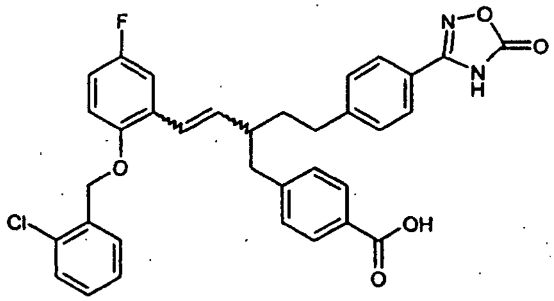 | $^1$H-NMR (300 MHz, $CDCl_3$, δ/ppm): 11.21 (1H, br. s); 11.16 (1H, br. s), 7.93 (4H, dd), 7.56 (2H, d), 7.47 (2H, d), 7.41-7.36 (2H, m), 7.18 (2H, d), 7.09 (2H, d), 6.95-6.19 (4H, m), 6.71 (1H, d), 6.62 (1H, d), 6.57-6.53 (2H, m), 6.06 (1H, dd), 5.60 (2H, $m_c$), 5.14 (2H, s), 5.07 (2H, d), 2.77-2.59 (6H, m). HPLC (Methode 2): $R_t$ 5.3 min. |
| **41** (*E*-Enantiomer 1, ausgehend von Bsp. 40 nach Trennung) | 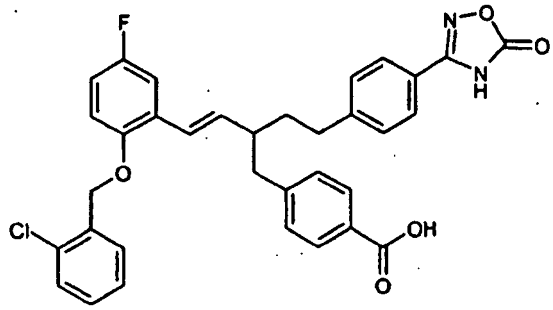 | $^1$H-NMR (300 MHz, DMSO-$d_6$, δ/ppm): 12.88 (1H, br. s), 12.68 (1H, br. s), 7.79 (2H, d), 7.67 (2H, d), 7.51-7.47 (2H, m), 7.38-7.24 (7H, m), 7.10-6.97 (2H, m), 6.43 (1H, d), 6.21 (1H, dd), 5.10 (2H, s), 2.84 (1H, dd), 2.75-2.57 (6H, m), 1.79-1.75 (1H, m), 1.70-1.62 (1H, m). LC-MS (Methode 2): $R_t$ 2.8 min; m/z 599 $(M+H)^+$. spez. Drehwert: $\alpha_D^{20}$ (Aceton): -14.8° |
| **42** (*E*-Enantiomer 2,ausgehend von Bsp. 40 nach Trennung) | 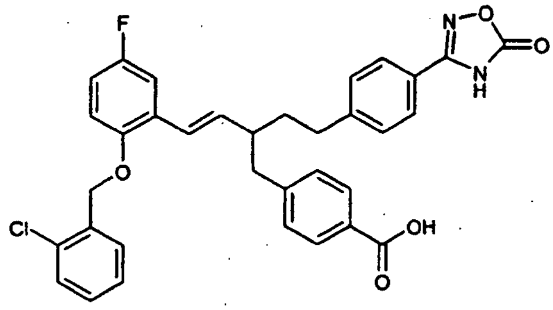 | $^1$H-NMR (300 MHz, DMSO-$d_6$, δ/ppm): 12.88 (1H, br. s), 12.74 (1H, br. s), 7.78 (2H, d), 7.67 (2H, d), 7.51-7.47 (2H, m), 7.38-7.24 (7H, m), 7.10-6.98 (2H, m), 6.43 (1H, d), 6.20 (1H, dd), 5.10 (2H, s), 2.84 (1H, dd), 2.74-2.58 (6H, m), 1.79-1.75 (1H, m), 1.70-1.62 (1H, m). LC-MS (Methode 2): $R_t$ 2.8 min; m/z 599 $(M+H)^+$. |

(fortgesetzt)

| Beispiel | Struktur | Analytische Daten |
|---|---|---|
| **43**<br><br>(*Z*-Enantiomer 1, ausgehend von Bsp. 40 nach Trennung) | 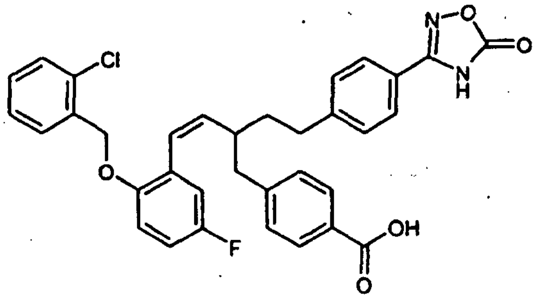 | $^1$H-NMR (300 MHz, DMSO-$d_6$, δ/ppm): 12.86 (1H, br. s), 12.77 (1H, br. s), 7.78 (2H, d), 7.59 (2H, d), 7.52-7.46 (2H, m), 7.38-7.29 (2H, m), 7.20-7.16 (4H, m), 7.01-6.99 (2H, m), 6.46 (1H, d), 6.13 (1H, d), 5.59 (1H, t), 5.04 (2H, $d_{AB}$), 2.84 (1H, d), 2.73-2.60 (3H, m), 1.76-1.71 (1H, m), 1.63-1.55 (1H, m), 1.23 (1H, br. s).<br>LC-MS (Methode 1): $R_t$ 2.8 min; m/z 599 $(M+H)^+$.<br>spez. Drehwert:<br>$\alpha_D^{20}$ (Aceton): +89.0° |
| **44**<br><br>(*Z*-Enantiomer 2, ausgehend von Bsp. 40 nach Trennung) | 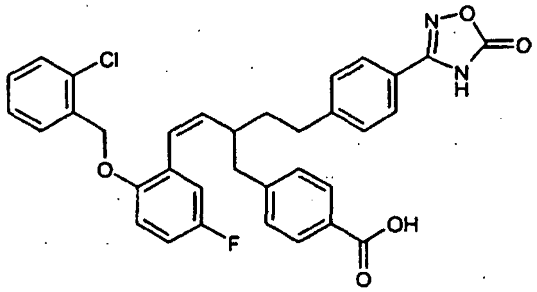 | $^1$H-NMR (300 MHz, DMSO-$d_6$, δ/ppm): 12.86 (2H, br. s), 7.78 (2H, d), 7.59 (2H, d), 7.52-7.46 (2H, m), 7.38-7.29 (2H, m), 7.21-7.15 (4H, m), 7.01-6.99 (2H, m), 6.46 (1H, d), 6.13 (1H, d), 5.58 (1H, t), 5.04 (2H, $d_{AB}$), 2.84 (1H, d), 2.76-2.58 (3H, m), 1.78-1.67 (1H, m), 1.63-1.52 (1H, m), 1.23 (1H, br. s).<br>LC-MS (Methode 2): $R_t$ 2.8 min; m/z 599 $(M+H)^+$. |

(fortgesetzt)

| Beispiel | Struktur | Analytische Daten |
|---|---|---|
| **45**<br><br>(ausgehend von Bsp. 63A und Bsp. 16A) | 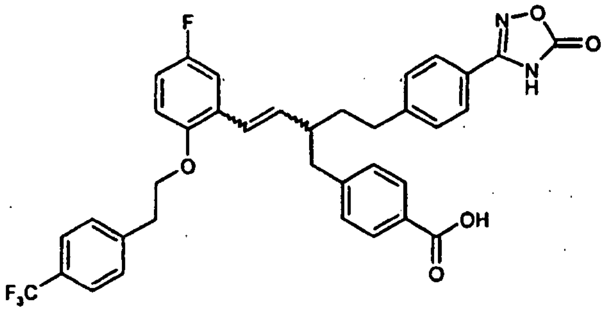 | $^1$H-NMR (400 MHz, DMSO-$d_6$, $\delta$/ppm): (*E*/*Z* = 3:1) 12,97-12,67 (2H, breit), 7,90-6,84 (16H, m), 6,32 (0,33H, d), 6,27 (1H, d), 6,19-6,05 (1,33H, m), 5,52 (0,33H, t), 4,22-4,11 (2H, m), 3,16-3,00 (3H, m), 2,90-2,31 (4H, m), 1,84-1,48 (4H, m).<br>LC-MS (Methode 2): $R_t$ 2.82 und 2.87 min; m/z 647 ($M+H^+$). |
| **46**<br>(ausgehend von Bsp. 67A und Bsp. 16A) | 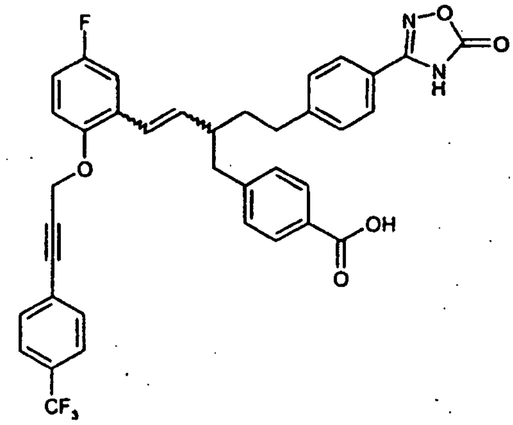 | LC-MS (Methode 4): $R_t$ 3.09 min; m/z 657 ($M+H^+$). |
| **47**<br>(ausgehend von Bsp. 51 A und Bsp. 16A) | 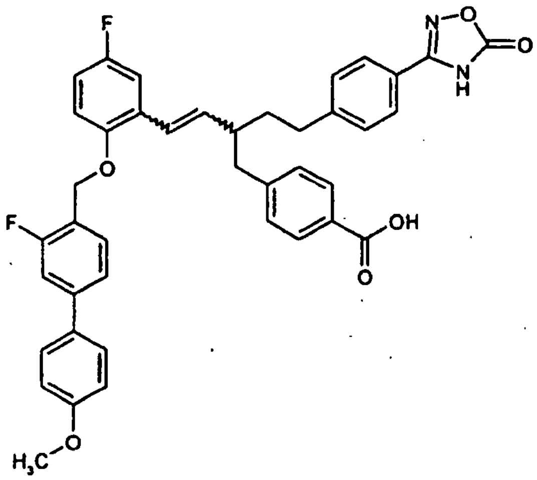 | LC-MS (Methode 4): R, 3.13 min; m/z 689 ($M+H^+$). |

(fortgesetzt)

| Beispiel | Struktur | Analytische Daten |
|---|---|---|
| **48** (ausgehend von Bsp. 55A und Bsp. 16A) | 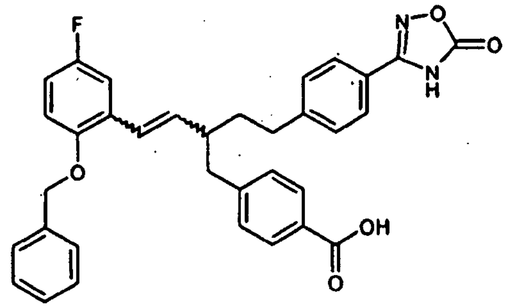 | $^1$H-NMR (300 MHz, DMSO-$d_6$, δ/ppm): 12,82 (2H, breit), 7,83-7,78 (2H, m), 7,70-7,60 (2H, m), 7,35-7,26 (8H, m), 7,21-7,18 (2H, m), 7,06-6,96 (2H, m), 6,49-6,41 (1H, m), 6,26-6,11 (1H, m), 5,05-4,98 (2H, m), 2,92-2,80 (1H, m), 2,76-2,63 (3H, m), 2,56-2,39 (1H, m), 1,86-1,54 (2H, m). LC-MS (Methode 4): $R_t$ 2.87 min; m/z 565 (M+H$^+$). |
| **49** (ausgehend von Bsp. 59A und Bsp. 16A) | 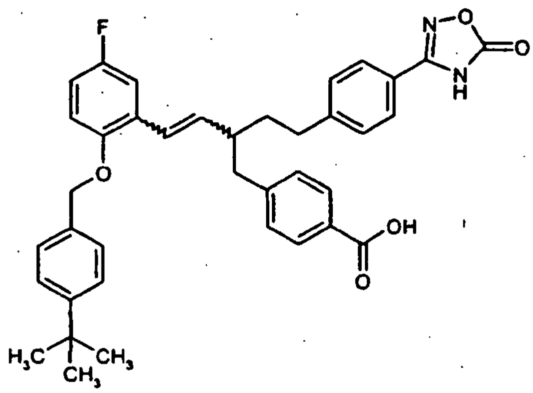 | LC-MS (Methode 4): $R_t$ 3.17 min; m/z 621 (M+H$^+$). |

(fortgesetzt)

| Beispiel | Struktur | Analytische Daten |
|---|---|---|
| **50**<br><br>(*E*-Enantiomer 1, ausgehend von Bsp. 49 nach Trennung) | 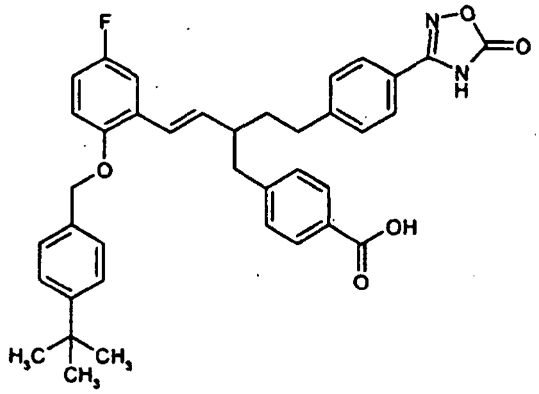 | $^1$H-NMR (300 MHz, DMSO-$d_6$, δ/ppm): 12,99-12,64 (2H, breit), 7,81 (2H, d), 7,69 (2H, d), 7,37 (4H, d), 7,31-7,21 (5H, m), 7,09-6,94 (2H, m), 6,44 (1H, d), 6,28 (1H, m), 5,0 (2H, s), 2,92-2,45 (5H, m), 1,89-1,60 (2H, m), 1,26 (9H, s).<br>LC-MS (Methode 4): $R_t$ 2.80 min; m/z 621 (M+H$^+$).<br>chirale HPLC: $R_t$ 8.24 min;<br>Reinheit >96.5%; >99.5% ee. |
| **51**<br><br>(*E*-Enantiomer 2, ausgehend von Bsp. 49 nach Trennung) | 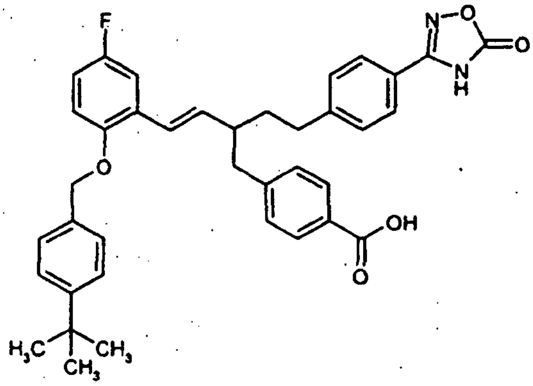 | $^1$H-NMR und LC-MS siehe Beispiel 50;<br>chirale HPLC: $R_t$ 13.84 min;<br>Reinheit >99.5%; >99.5% ee. |

(fortgesetzt)

| Beispiel | Struktur | Analytische Daten |
|---|---|---|
| **52** (*Z*-Enantiomer 1, ausgehend von Bsp. 49 nach Trennung) | 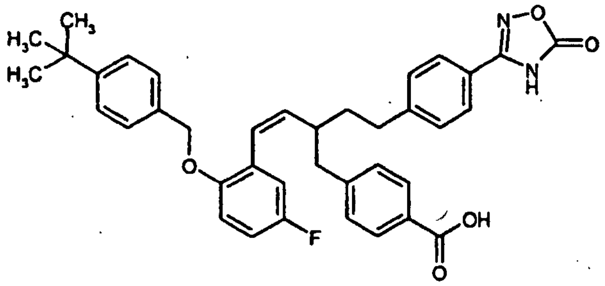 | $^1$H-NMR (300 MHz, DMSO-$d_6$, $\delta$/ppm): 12,96-12,79 (2H, breit), 7,80 (2H, d), 7,61 (2H, d), 7,35-7,25 (5H, m), 7,24-7,14 (4H, m), 6,98 (2H, d), 6,47 (1H, d), 6,10 (1H, d), 5,59 (1H, t), 4,98 (2H, d), 2,91-2,79 (1H, m), 2,77-2,56 (4H, m), 1,82-1,68 (1H, m), 1,67-1,51 (1H, m), 1,23 (9H, s). LC-MS (Methode 4): $R_t$ 2.85 min; m/z 621 (M+H$^+$). chirale HPLC: $R_t$ 5.13 min; Reinheit >92%; >99.5% ee. |
| **53** (Z-Enantiomer 2, ausgehend von Bsp. 49 nach Trennung) | 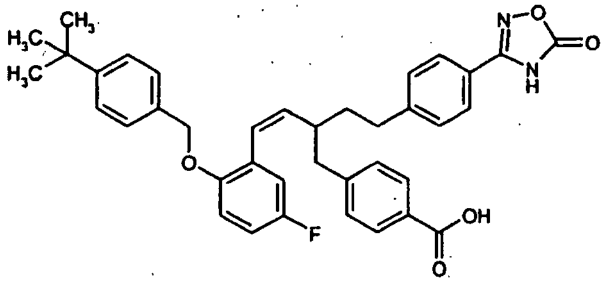 | $^1$H-NMR und LC-MS siehe Beispiel 52; chirale HPLC: $R_t$ 6.52 min; Reinheit >95%; >99.5% ee. |

[Methode *E*/*Z*-Isomeren- und Enantiomerentrennung (chirale HPLC): Säule: Daicel Chiralpak AD-H 250 mm x 20 mm; Elutionsmittel: Isopropanol (mit 1% Wasser und 0.2% Trifluoressigsäure) / Isohexan 30:70 (v/v); Fluss: 15 ml/min; UV-Detektion: 220 nm; Temperatur: 24°C].

**<u>Beispiel 54</u>**

*E*-4-{3-[4-(5-Oxo-4,5-dihydro-[1,2,4]oxadiazol-3-yl)benzyl]-5-[2-(4'-trifluormethyl-biphenyl-4-yl-methoxy)phenyl]-pent-4-enyl}-benzoesäure *(Racemat)*

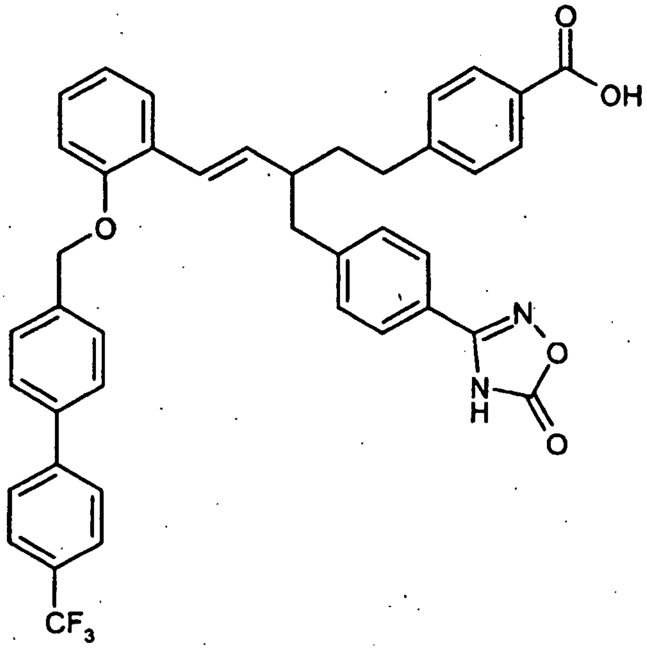

Eine Lösung von 1000 mg (1.42 mmol) *E*-4-{3-[4-(5-Oxo-4,5-dihydro-[1,2,4]oxadiazol-3-yl)-benzyl]-5-[2-(4'-trifluormethyl-biphenyl-4-ylmethoxy)phenyl]-pent-4-enyl}-benzoesäuremethyl-ester aus Beispiel 77A in 20 ml THF und 20 ml Wasser wird mit 68 mg (2.84 mmol) Lithiumhydroxid versetzt und 12 h bei 50°C gerührt. Nach dem Abkühlen wird das THF abgezogen und die wässrige Phase mit 1 M Salzsäure auf pH 4-5 eingestellt. Anschließend wird mit Essigsäureethylester dreimal extrahiert und die vereinigten organischen Phasen über Natriumsulfat getrocknet. Nach Abfiltrieren und Einengen wird der erhaltene Rückstand mittels präparativer HPLC gereinigt. Es werden 870 mg (1.26 mmol, 89% Ausbeute) eines farblosen Feststoffes erhalten.

LC-MS (Methode 1): $R_t$ 3.32 min; m/z 691 ($M+H^+$).

500 mg (0.72 mmol) der so erhaltenen racemischen *E*-4-{3-[4-(5-Oxo-4,5-dihydro-[1,2,4]oxadiazol-3-yl)benzyl]-5-[2-(4'-trifluormethyl-biphenyl-4-ylmethoxy)phenyl]-pent-4-enyl}-benzoesäure werden mittels präparativer HPLC an chiraler Phase weiter aufgetrennt. Es werden jeweils enantiomerenrein 288 mg bzw. 160 mg der beiden *E*-Isomere erhalten (siehe Beispiele 55 und 56).

**Beispiel 55**

*E*-4-{3-[4-(5-Oxo-4,5-dihydro-[1,2,4]oxadiazol-3-yl)benzyl]-5-[2-(4'-trifluormethyl-biphenyl-4-yl-methoxy)phenyl]-pent-4-enyl}-benzoesäure (*Enantiomer 1*)

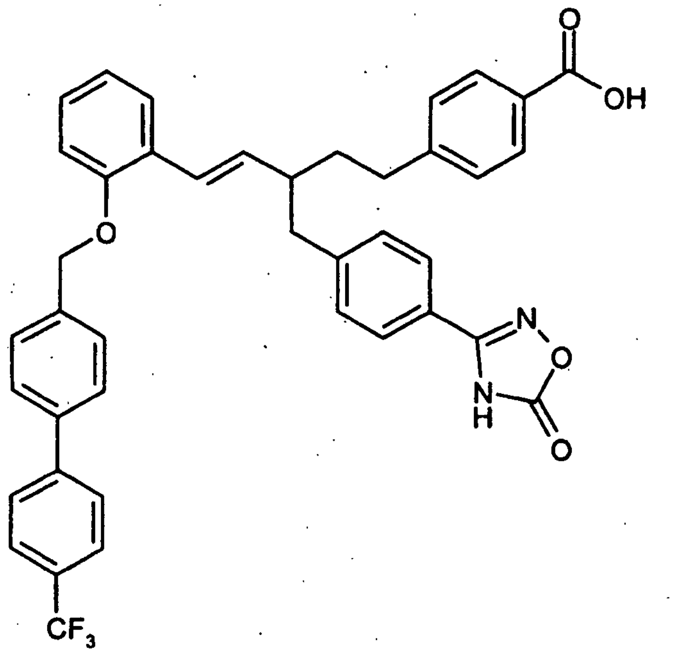

Methode Enantiomerentrennung:

Säule: Daicel Chiralpak AD-H 250 mm x 20 mm; Elutionsmittel: Isopropanol (mit 1% Wasser und 0.2% Trifluoressigsäure) / Isohexan 30:70 (v/v); Fluss: 15 ml/min; UV-Detektion: 220 nm; Temperatur: 24°C.

$R_t$ 7.33 min; Reinheit >98%; >99.5% ee

Ausbeute: 160 mg

$^1$H-NMR (400 MHz, DMSO-$d_6$, $\delta$/ppm): 12,92-12,62 (2H, breit), 7,91-7,77 (6H, m), 7,68 (4H, t), 7,50 (2H, d), 7,44 (1H, d), 7,34 (2H, d), 7,27 (2H, d), 7,19 (1H, t), 7,06 (1H, d), 6,91 (1H, t), 6,53 (1H, d), 6,19-6,09 (1H, m), 5,18 (2H, s), 2,97-2,81 (1H, m), 2,80-2,70 (2H, m), 2,69-2,57 (2H, m), 1,88-1,76 (1H, m), 1,75-1,61 (1H, m).

LC-MS (Methode 1): $R_t$ 3.24 min; m/z 691 (M+H$^+$).

**Beispiel 56**

*E*-4-{3-[4-(5-Oxo-4,5-dihydro-[1,2,4]oxadiazol-3-yl)benzyl]-5-[2-(4'-trifluormethyl-biphenyl-4-yl-methoxy)phenyl]-pent-4-enyl}-benzoesäure (*Enantiomer 2*)

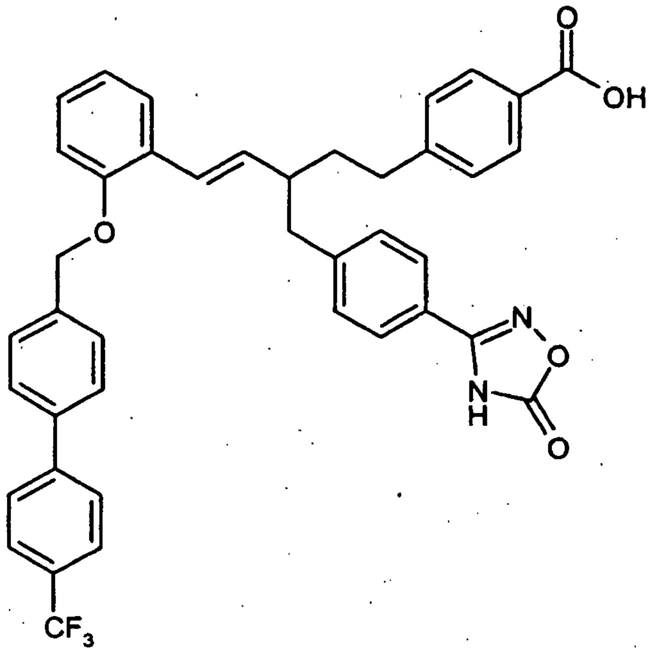

Methode Enantiomerentrennung: siehe Beispiel 55.

$R_t$ 6.13 min; Reinheit 90%; >99.5% ee

Ausbeute: 288 mg

$^{1}$H-NMR (400 MHz, DMSO-$d_6$, δ/ppm): 12,92-12,62 (2H, breit), 7,91-7,77 (6H, m), 7,68 (4H, t), 7,50 (2H, d), 7,44 (1H, d), 7,34 (2H, d), 7,27 (2H, d), 7,19 (1H, t), 7,06 (1H, d), 6,91 (1H, t), 6,53 (1 H, d), 6,19-6,09 (1 H, m), 5,18 (2H, s), 2,97-2,81 (1H, m), 2,80-2,70 (2H, m), 2,69-2,57 (2H, m), 1,88-1,76 (1H, m), 1,75-1,61 (1H, m).

LC-MS (Methode 1): $R_t$ 3.24 min; m/z 691 ($M+H^+$).

Auf analoge Weise werden die in der folgenden Tabelle aufgeführten Beispiele erhalten:

| Beispiel | Struktur | Analytische Daten |
|---|---|---|
| **57**<br><br>(Z-Enantiomer 1, ausgehend von Bsp. 74A und Bsp. 25A) | 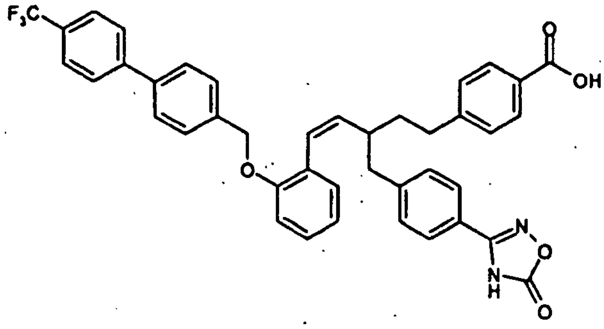 | $^{1}$H-NMR (300 MHz, DMSO-$d_6$, δ/ppm): 12,93-12,67 (2H, breit), 7,91-7,70 (6H, m), 7,69-7,59 (4H, m), 7,48 (2H, d), 7,26 (2H, d), 7,18 (1H, d), 7,11 (2H, d), 7,01 (1H, d), 6,76 (1H, t), 6,68 (1H, d), 6,53 (1H, d), 5,56 (1H, t), 5,10 (2H, q), 2,95-2,38 (5H, m), 1,81-1,51 (2H, m).<br>MS (EI): 689 ($M-H^-$), 691 ($M+H^+$).<br>chirale HPLC: $R_t$ 5.36 min; >99% ee. |

(fortgesetzt)

| Beispiel | Struktur | Analytische Daten |
|---|---|---|
| **58**<br><br>(Z-Enantiomer 2,ausgehend von Bsp. 74A und Bsp. 25A) | 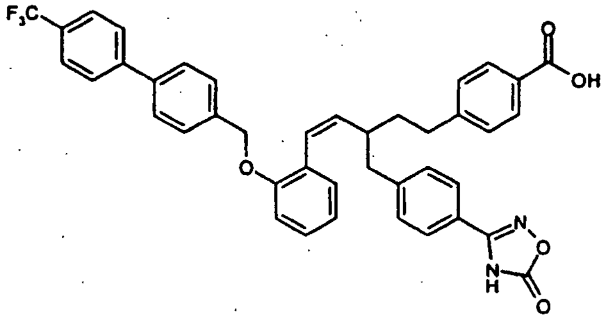 | $^1$H-NMR und MS siehe Beispiel 57; chirale HPLC: $R_t$ 9.96 min; >95% ee. |
| **59**<br><br>(E-Enantiomer 1, ausgehend von Bsp. 74A und Bsp. 79A) | 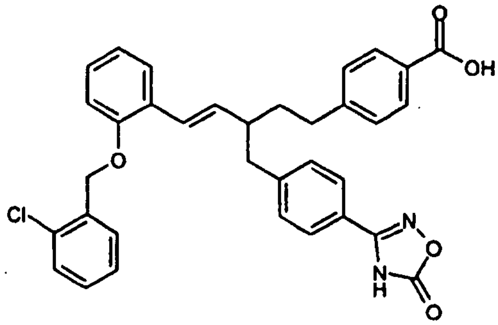 | $^1$H-NMR (300 MHz, DMSO-$d_6$, δ/ppm): 12,89-12,65 (2H, breit), 7,79 (2H, d), 7,66 (2H, d), 7,49 (2H, d), 7,42 (1H, d), 7,39-7,28 (4H, m), 7,22 (2H, d), 7,18 (1H, d), 7,06 (1H, d), 6,92 (1H, t), 6,48 (1H, d), 6,17-6,06 (1H, m), 5,12 (2H, s), 2,90-2,80 (1H, m), 2,78-2,65 (2H, m), 2,64-2,54 (1H, m), 2,53-2,40 (1H, m), 1,86-1,72 (1H, m), 1,71-1,57 (1H, m). LC-MS (Methode 2): $R_t$ 2.79 min; m/z 581 (M+H$^+$). chirale HPLC: $R_t$ 16.17 min; >99.5% ee. |
| **60**<br><br>(*E*-Enantiomer 2, ausgehend von Bsp. 74A und Bsp. 79A) | 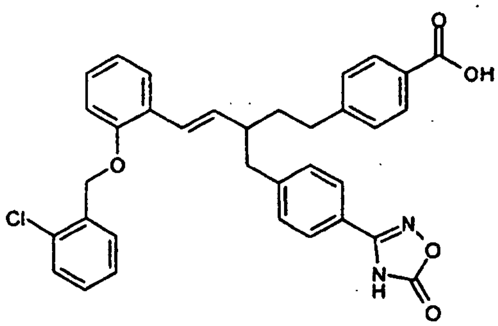 | $^1$H-NMR und LC-MS siehe Beispiel 59; chirale HPLC: $R_t$ 17.91 min; >97% ee. |

(fortgesetzt)

| Beispiel | Struktur | Analytische Daten |
|---|---|---|
| **61**<br><br>(*E*-Enantiomer 1, ausgehend von Bsp. 74A und Bsp. 81 A) | 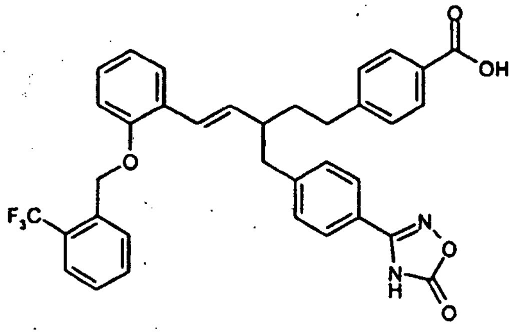 | $^1$H-NMR (400 MHz, DMSO-$d_6$, δ/ppm): 13,10-12,50 (2H, breit), 7,80 (3H, d), 7,71-7,61 (4H, m), 7,60-7,52 (1H, m), 7,46 (1H, d), 7,32 (2H, d), 7,27-7,16 (3H, m), 7,02 (1H, d), 6,95 (1H, t), 6,46 (1H, d), 6,14-6,02 (1H, m), 5,20 (2H, s), 2,90-2,80 (1H, m), 2,76-2,64 (2H, m), 2,62-2,38 (2H, m), 1,85-1,71 (1H, m), 1,70-1,58 (1H, m).<br>LC-MS (Methode 2): $R_t$ 2.81 min; m/z 615 (M+H$^+$).<br>chirale HPLC: $R_t$ 13.02 min; 98.9% ee. |
| **62**<br><br>(*E*-Enantiomer 2, ausgehend von Bsp. 74A und Bsp. 81A) | 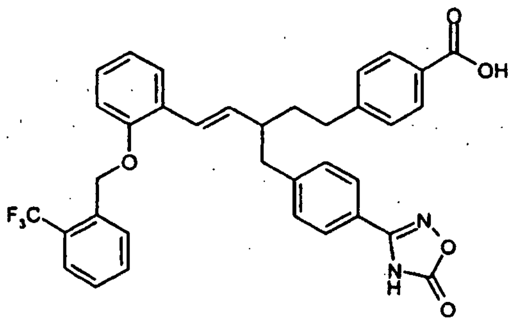 | $^1$H-NMR und LC-MS siehe Beispiel 61;<br>chirale HPLC: $R_t$ 11.08 min; >99.5% ee. |

(fortgesetzt)

| Beispiel | Struktur | Analytische Daten |
|---|---|---|
| **63**<br><br>(*E*-Enantiomer 1, ausgehend von Bsp. 74A und Bsp. 28A) | 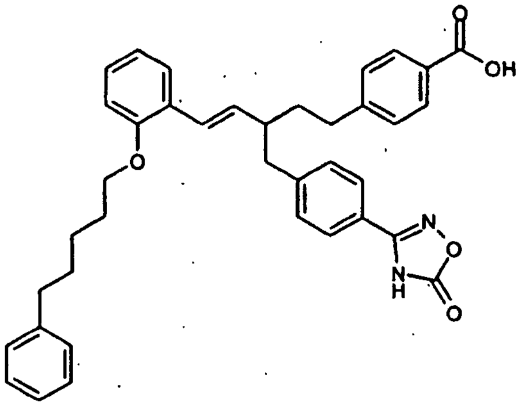 | $^1$H-NMR (400 MHz, DMSO-$d_6$, $\delta$/ppm): 12,91-12,81 (1H, breit), 12,80-12,69 (1H, breit), 7,86 (2H, d), 7,68 (2H, d), 7,40-7,32 (3H, m), 7,29 (2H, d), 7,21 (2H, t), 7,19-7,08 (4H, m), 6,97-6,83 (2H, m), 6,42 (1H, d), 6,15-6,03 (1H, m), 3,99-3,85 (2H, m), 2,92-2,81 (1H, m), 2,79-2,69 (2H, m), 2,68-2,41 (3H, m), 1,89-1,76 (1H, m), 1,75-1,64 (3H, m), 1,63-1,52 (2H, m), 1,47-1,28 (3H, m).<br>MS (EI): 603 (M+H$^+$).<br>chirale HPLC: $R_t$ 9.95 min; >99% ee. |
| **64**<br><br>(*E*-Enantiomer 2, ausgehend von Bsp. 74A und Bsp. 28A) | 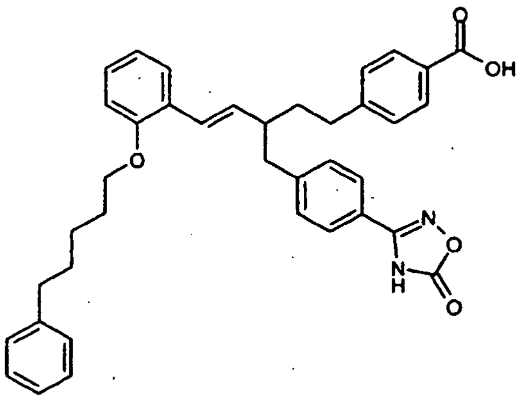 | $^1$H-NMR und MS siehe Beispiel 63;<br>chirale HPLC: R, 10.88 min; >93% ee. |

(fortgesetzt)

| Beispiel | Struktur | Analytische Daten |
|---|---|---|
| **65**<br><br>(*E*-Isomere, ausgehend von Bsp. 75A und *4-tert.*-Butylbenzylbromid) | 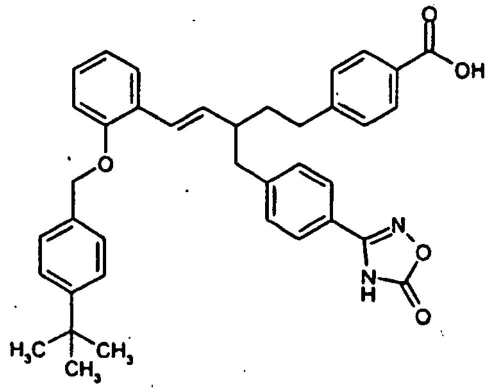 | $^1$H-NMR (300 MHz, DMSO-$d_6$, δ/ppm): 13,11-12,61 (2H, breit), 7,84-7,81 (2H, d), 7,69-7,66 (2H, d), 7,44-7,40 (1H, m), 7,36-7,25 (8H, m), 7,20-7,15 (1H, m), 7,06-7,02 (1H, m), 6,93-6,87 (1H, t), 6,51-6,44 (1H, d), 6,16-6,07 (1H, dd), 5,09-4,99 (2H, m), 2,92-2,58 (5H, m), 1,92-1,60 (2H, m), 1,24 (9H, s).<br>LC-MS (Methode 4): $R_t$ 3.19 min; m/z 603 (M+H$^+$). |
| **66**<br><br>(*E*-Enantiomer 1, ausgehend von Bsp. 65) | 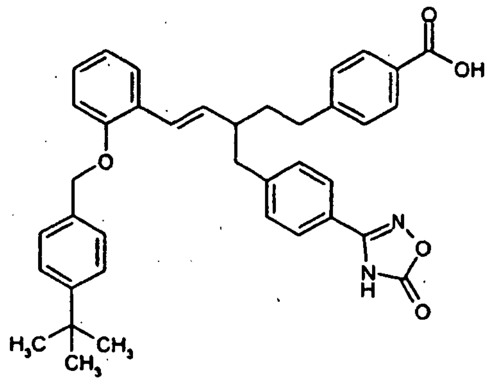 | chirale HPLC: $R_t$ 6.35 min; >99.5% ee.<br>LC-MS (Methode 1): $R_t$ 3.27 min; m/z 603 (M+H$^+$). |
| **67**<br><br>(*E*-Enantiomer 2,ausgehend von Bsp. 65) | 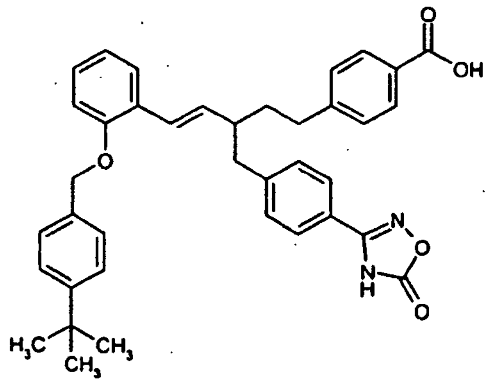 | chirale HPLC: $R_t$ 9.09 min; >99.5% ee.<br>LC-MS (Methode 2): $R_t$ 3.00 min; m/z 603 (M+H$^+$). |

(fortgesetzt)

| Beispiel | Struktur | Analytische Daten |
|---|---|---|
| **68**<br><br>(E-Enantiomer 1, ausgehend von Bsp. 74A und Bsp. 32A) | 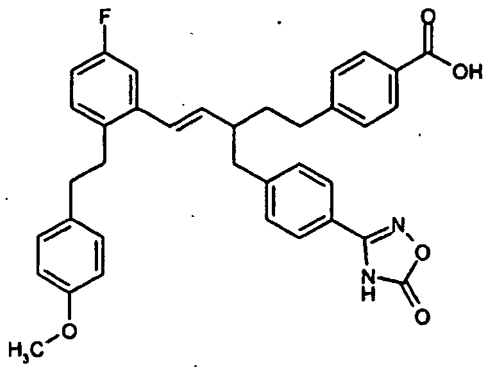 | $^1H$-NMR (400 MHz, DMSO-$d_6$, δ/ppm): 12,87-12,61 (2H, breit), 7,85 (2H, d), 7,64 (2H, d), 7,37 (2H, d), 7,32 (2H, d), 7,21 (1H, d), 7,09 (1H, t), 6,94 (1H, t), 6,90 (2H, d), 6,75 (2H, d), 6,35 (1H, d), 6,13-6,01 (1H, m), 3,69 (3H, s), 3,00-2,87 (1H, m), 2,82-2,47 (6H, m), 2,43 (2H, t), 1,94-1,81 (1H, m), 1,80-1,69 (1H, m).<br>LC-MS (Methode 2): $R_t$ 2.81 min; m/z 593 ($M+H^+$).<br>chirale HPLC: $R_t$ 6.22 min; >99.5% ee. |
| **69**<br><br>(E-Enantiomer 2, ausgehend von Bsp. 74A und Bsp. 32A) | 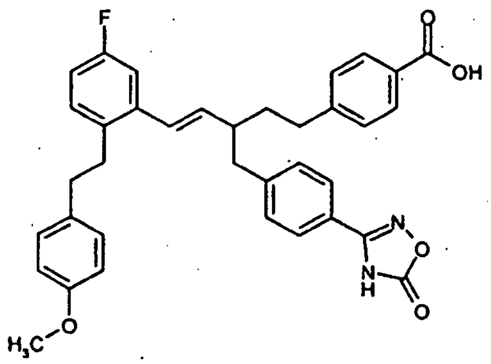 | $^1H$-NMR und LC-MS siehe Beispiel 68; chirale HPLC: $R_t$ 7.28 min; 97.94% ee. |

[Methode *E*/*Z*-Isomeren- und Enantiomerentrennung (chirale HPLC:): Säule: Daicel Chiralpak AD-H 250 mm x 20 mm; Elutionsmittel: Isopropanol (mit 1% Wasser und 0.2% Trifluoressigsäure) / Isohexan 30:70 (v/v); Fluss: 15 ml/min; UV-Detektion: 220 nm; Temperatur: 24°C].

<u>**Beispiel 70**</u>

*E*-8-[2-(4-*tert*.-Butylbenzyloxy)phenyl]-6-{2-[4-(5-oxo-4,5-dihydro-[1,2,4]oxadiazol-3-yl)phenyl]-ethyl}-oct-7-ensäure *(Racemat)*

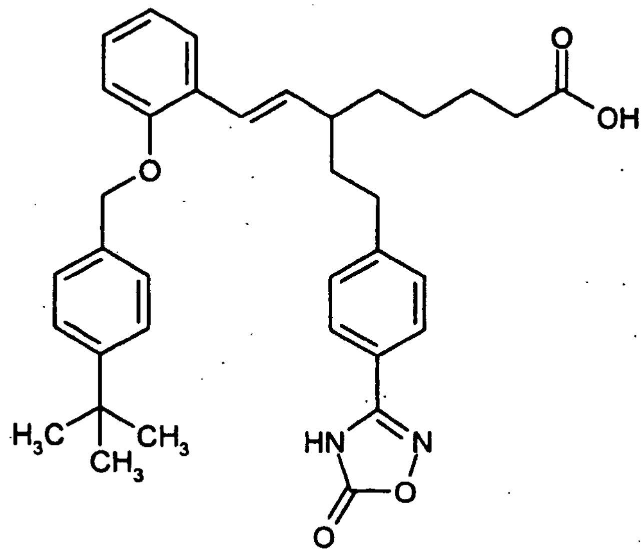

Eine Lösung von 1150 mg (1.93 mmol) 8-[2-(4-*tert*.-Butylbenzyloxy)phenyl]-6-{2-[4-(5-oxo-4,5-dihydro-[1,2,4]oxadiazol-3-yl)phenyl]ethyl}-oct-7-ensäureethylester aus Beispiel 89A in 10 ml THF und 10 ml Wasser wird mit 93.30 mg (3.85 mmol) Lithiumhydroxid versetzt und 12 h bei 50°C gerührt. Nach dem Abkühlen wird das THF abgezogen und die wässrige Phase mit 1 M Salzsäure auf pH 4-5 eingestellt. Anschließend wird mit Essigsäureethylester dreimal extrahiert und die vereinigten organischen Phasen über Natriumsulfat getrocknet. Nach Filtrieren und Einengen wird der erhaltene Rückstand mittels präparativer HPLC gereinigt. Es werden 1.04 g (93% Ausbeute) eines farblosen Feststoffes erhalten.

MS (EI): 569 ($M+H^+$), 591 ($M+Na^+$).

800 mg (1.41 mmol) der so erhaltenen racemischen *E*-8-[2-(4-*tert*.-Butylbenzyloxy)phenyl]-6-{2-[4-(5-oxo-4,5-dihydro-[1,2,4]oxadiazol-3-yl)phenyl]ethyl}-oct-7-ensäure werden mittels präparativer HPLC an chiraler Phase weiter aufgetrennt. Es werden jeweils enantiomerenrein 381 mg bzw. 412 mg der beiden E-Isomere erhalten (siehe Beispiele 71 und 72).

__Beispiel 71__

*E*-8-[2-(4-*tert*.-Butylbenzyloxy)phenyl]-6-{2-[4-(5-oxo-4,5-dihydro-[1,2,4]oxadiazol-3-yl)phenyl]-ethyl}-oct-7-ensäure *(Enantiomer 1)*

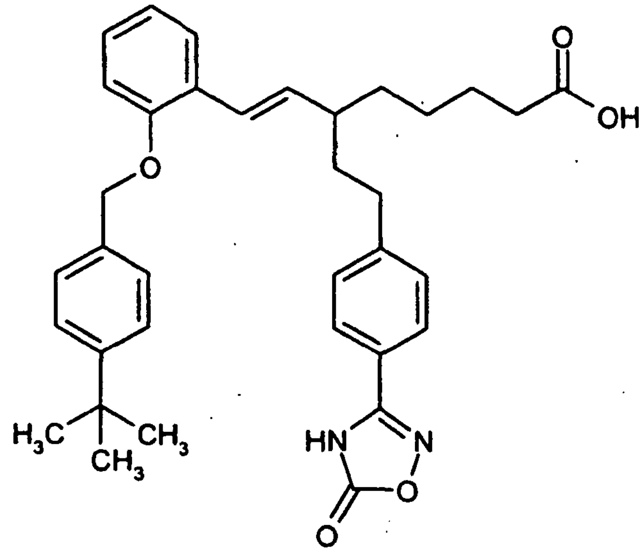

Methode Enantiomerentrennung:

Säule: Daicel Chiralpak AD-H 250 mm x 20 mm; Elutionsmittel: Isopropanol (mit 1% Wasser und 0.2% Trifluoressigsäure) / Isohexan 30:70 (v/v); Fluss: 15 ml/min; UV-Detektion: 220 nm; Temperatur: 24°C.
$R_t$ 5.48 min; Reinheit>99.5%; >99.5% ee
Ausbeute: 381 mg
$^1$H-NMR (300 MHz, DMSO-$d_6$, δ/ppm): 13,40-12,40 (1H, breit), 12,10-11,80 (1H, breit), 7,70 (2H, d), 7,47(1H, d), 7,41-7,34 (6H, m), 7,19 (1H, t), 7,09 (1H, d), 6,91 (1H, t), 6,64 (1H, d), 6,12-5,98 (1H, m), 5,12 (2H, s), 2,78-2,54 (2H, m), 2,21-2,02 (3H, m), 1,82-1,68 (1H, m), 1,67-1,56 (1H, m), 1,55-1,39 (3H, m), 1,38-1,28 (3H, m), 1,26 (9H, s).
MS (EI): 567 $(M-H)^-$.

**Beispiel 72**

*E*-8-[2-(4-*tert*.-Butylbenzyloxy)phenyl]-6-{2-[4-(5-oxo-4,5-dihydro-[1,2,4]oxadiazol-3-yl)phenyl]-ethyl}-oct-7-ensäure (*Enantiomer 2)*

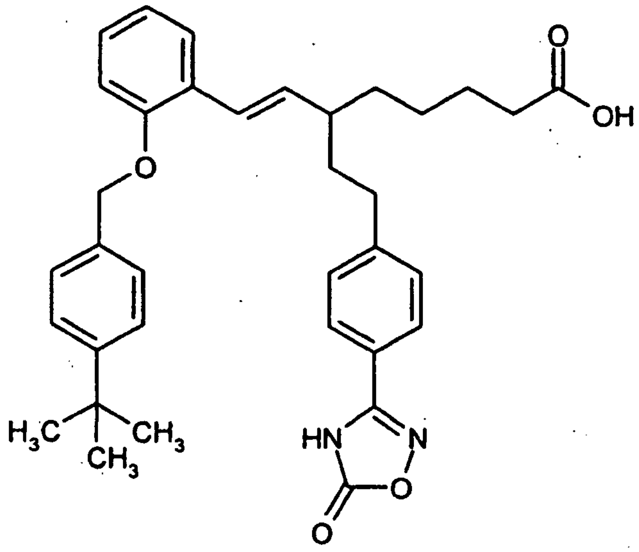

Methode Enantiomerentrennung: siehe Beispiel 71.
R, 6.25 min; Reinheit >99.5%; >98.5% ee
Ausbeute: 412 mg
$^1$H-NMR (300 MHz, DMSO-$d_6$, δ/ppm): 13,40-12,40 (1H, breit), 12,10-11,80 (1H, breit), 7,70 (2H, d), 7,47 (1H, d), 7,41-7,34 (6H, m), 7,19 (1H, t), 7,09 (1H, d), 6,91 (1H, t), 6,64 (1H, d), 6,12-5,98 (1H, m), 5,12 (2H, s), 2,78-2,54 (2H, m), 2,21-2,02 (3H, m), 1,82-1,68 (1H, m), 1,67-1,56 (1H, m), 1,55-1,39 (3H, m), 1,38-1,18 (3H, m), 1,26 (9H, s).
MS (EI): 567 $(M-H)^-$.

**Beispiel 73**

*E*-8-[2-(4-*tert*.-Butylbenzyloxy)phenyl]-6-{2-[4-(5-oxo-4,5-dihydro-[1,3,4]oxadiazol-3-yl)phenyl]-ethyl}-oct-7-ensäure *(Racemat)*

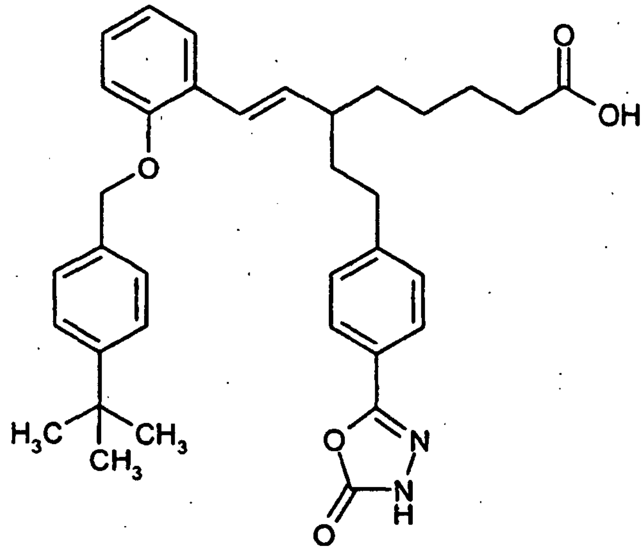

220 mg (0.4 mmol) (7*E*)-8-{2-[(4-*tert.*-Butylbenzyl)oxy]phenyl}-6-{2-[4-(5-oxo-4,5-dihydro-[1,3,4]oxadiazol-2-yl) phenyl]ethyl}-oct-7-ennitril aus Beispiel 98A in 8 ml n-Propanol werden mit 898 mg (16 mmol) Kaliumhydroxid versetzt und 12 Stunden zum Rückfluss erhitzt. Anschließend wird der Ansatz mit 1 N Salzsäure auf pH 4 gestellt und im Vakuum bis zur Trockene eingeengt. Der Rückstand wird in Dichlormethan/Methanol (10:1) aufgenommen, filtriert, das Filtrat erneut eingeengt und der Rückstand durch Flash-Chromatographie an Kieselgel (Laufmittel: Essigsäureethylester/Cyclohexan 1:1, mit 0.1% Ameisensäure) gereinigt. Es werden 95 mg (0.167 mmol, 42% Ausbeute) eines Feststoffes erhalten.

$^{1}$H-NMR (400 MHz, DMSO-$d_6$, δ/ppm): 12,80-11,67 (2H, breit), 7,68-7,64 (2H, d), 7,48-7,45 (1H, m), 7,40-7,31 (6H, m), 7,23-7,17 (1H, m); 7,11-7,06 (1H, m), 6,94-6,89 (1H, t), 6,66-6,59 (1H, d), 6,08-6,02 (1H, dd), 5,13-5,07 (2H, m), 2,73-2,53 (2H, m), 2,18-2,14 (2H, t), 2,13-2,05 (1H, m), 1,79-1,69 (1H, m), 1,66-1,53 (1H, m), 1,53-1,39 (3H, m), 1,37-1,19 (3H, m), 1,25 (9H, s).

LC-MS (Methode 4): $R_t$ 3.01 min; m/z 569 $(M+H)^+$.

95 mg (0.17 mmol) der so erhaltenen racemischen *E*-8-[2-(4-*tert.*-Butylbenzyloxy)phenyl]-6-{2-[4-(5-oxo-4,5-dihydro-[1,3,4]oxadiazol-3-yl)phenyl]ethyl}-oct-7-ensäure werden mittels präparativer HPLC an chiraler Phase weiter aufgetrennt. Es werden jeweils enantiomerenrein 35 mg bzw. 39 mg der beiden E-Isomere erhalten (siehe Beispiele 74 und 75).

## Beispiel 74

*E*-8-[2-(4-*tert.*-Butylbenzyloxy)phenyl]-6-{2-[4-(5-oxo-4,5-dihydro-[1,3,4]oxadiazol-3-yl)phenyl]-ethyl}-oct-7-ensäure (*Enantiomer 1*)

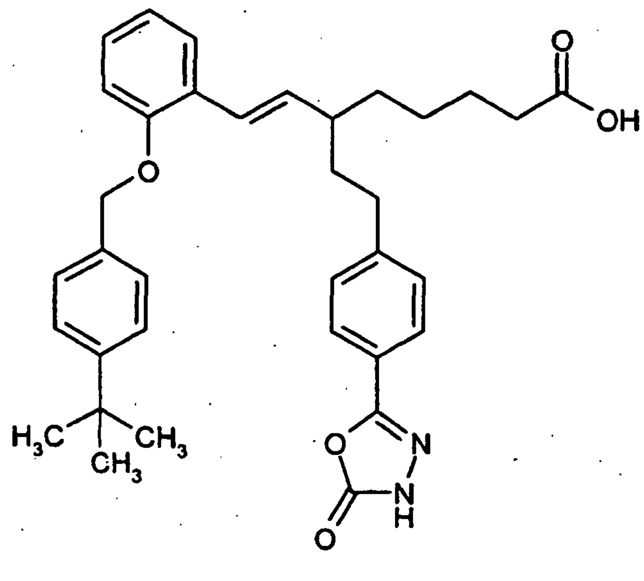

Methode Enantiomerentrennung:

Säule: Daicel Chiralpak AD-H 250 mm x 20 mm; Elutionsmittel: Isopropanol (mit 1% Wasser und 0.2% Trifluoressigsäure) / Isohexan 60:40 (v/v); Fluss: 15 ml/min; UV-Detektion: 220 nm; Temperatur: 25°C.
$R_t$ 5.53 min; Reinheit >99.5%; >99.5% ee
Ausbeute: 35 mg
LC-MS (Methode 1): $R_t$ 3.22 min; m/z 568 ($M^+$).

**Beispiel 75**

*E*-8-[2-(4-*tert.*-Butylbenzyloxy)phenyl]-6-{2-[4-(5-oxo-4,5-dihydro-[1,3,4]oxadiazol-3-yl)phenyl]-ethyl}-oct-7-ensäure *(Enantiomer 2)*

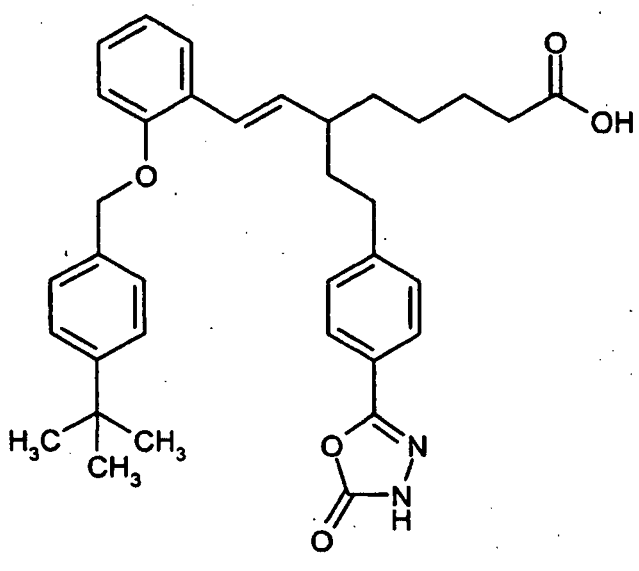

Methode Enantiomerentrennung: siehe Beispiel 74.
$R_t$ 6.02 min; Reinheit >99.0%; >94% ee
Ausbeute: 39 mg
LC-MS (Methode 1): $R_t$ 3.22 min; m/z 568 ($M^+$).

**Beispiel** 76

4-((3*E*)-4-{2-[(4-*tert*.-Butylbenzyl)oxy]phenyl}-2-{2-[4-(5-thioxo-4,5-dihydro-1*H*-1,2,4-triazol-3-yl)phenyl]ethyl}but-3-en-1-yl)-benzoesäure

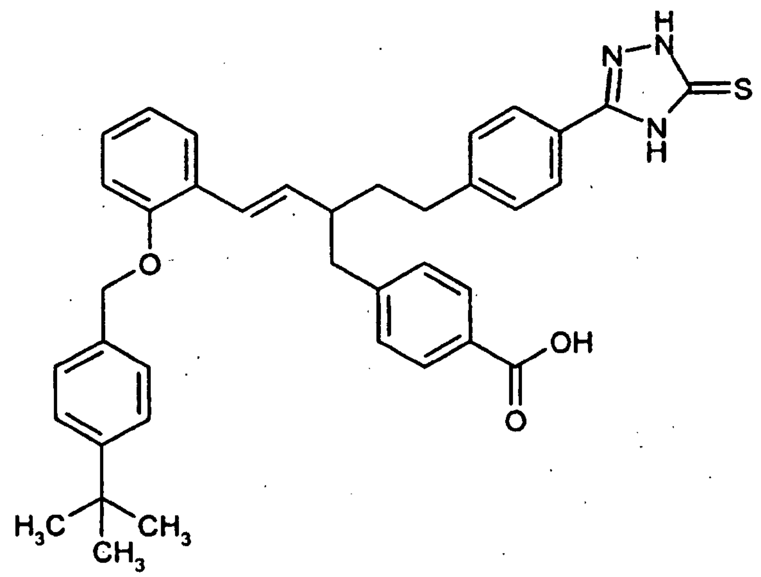

Das Rohprodukt aus Beispiel 101A und 64.52 mg (1.15 mmol) Kaliumhydroxid werden in 1 ml n-Propanol 12 Stunden bei 110°C gerührt. Nach dem Abkühlen wird die Reaktionsmischung mit 1 N Salzsäure auf pH 2-3 eingestellt, eingeengt und der Rückstand mittels präparativer HPLC aufgereinigt. Es werden 7 mg der Titelverbindung isoliert.
LC-MS (Methode 2): $R_t$ 2.90 min; m/z 617 ($M^+$).

**Beispiel 77**

4-{(4*E*)-5-{2-[(4-*tert*.-Butylbenzyl)oxy]phenyl}-3-[4-(5-oxo-4,5-dihydro-1,3,4-oxadiazol-2-yl)-benzyl]pent-4-en-1-yl}-benzoesäure *(Racemat)*

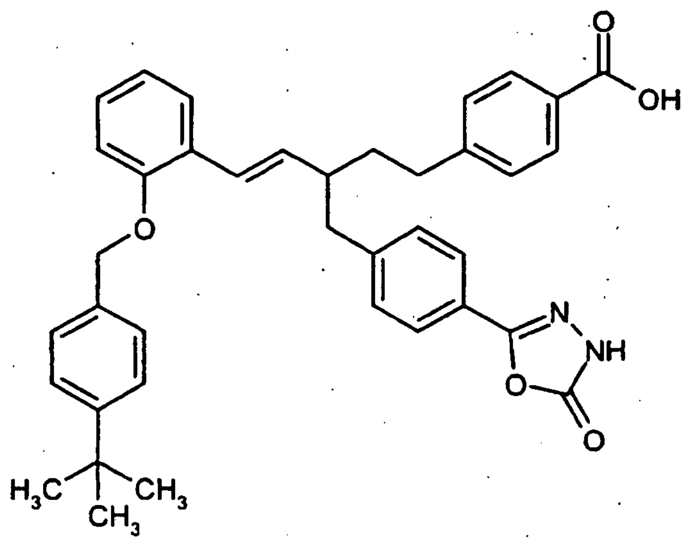

143 mg (0.22 mmol) 4-{(4*E*)-5-{2-[(4-*tert*.-Butylbenzyl)oxy]phenyl}-3-[4-(5-oxo-4,5-dihydro-1,3,4-oxadiazol-2-yl)benzyl]-pent-4-en-1-yl}-benzonitril aus Beispiel 104A in 5.7 ml n-Propanol werden mit 489 mg (8.72 mmol) Kaliumhydroxid versetzt und 15 Stunden bei 110°C gerührt. Anschließend wird der Ansatz mit 1 N Salzsäure auf pH 3-4 eingestellt und im Vakuum eingeengt. Der erhaltene Rückstand wird mittels präparativer HPLC aufgereinigt. Es werden 136 mg (0.21 mmol, 91.5% Reinheit, 94.7% Ausbeute) eines Feststoffes erhalten.

$^1$H-NMR (400 MHz, DMSO-$d_6$, δ/ppm): 13,0-12,0 (breit), 7,83-7,79 (2H, d), 7,67-7,62 (2H, m), 7,44-7,39 (1H, m), 7,36-7,23 (8H, m), 7,05-7,02 (1H, m), 6,92-6,86 (1H, m), 6,51-6,45 (1H, d), 6,15-6,07 (1 H, dd), 5,07-4,99 (2H, m), 2,90-2,57 (5H, m), 1,86-1,62 (2H, m), 1,25 (9H, s).

LC-MS (Methode 2): $R_t$ 3.06 min; m/z 602 ($M^+$).

135 mg (0.21 mmol) der so erhaltenen racemischen 4-{(4*E*)-5-{2-[(4-*tert*.-Butylbenzyl)oxy]-phenyl}-3-[4-(5-oxo-4,5-dihydro-1,3,4-oxadiazol-2-yl)benzyl]-pent-4-en-1-yl}-benzoesäure werden mittels präparativer HPLC an chiraler Phase weiter aufgetrennt. Es werden jeweils enantiomerenrein 68 mg bzw. 64 mg der beiden *E*-Isomere erhalten (siehe Beispiele 78 und 79).

**Beispiel 78**

4-{(4*E*)-5-{2-[(4-*tert*.-Butylbenzyl)oxy]phenyl}-3-[4-(5-oxo-4,5-dihydro-1,3,4-oxadiazol-2-yl)-benzyl]pent-4-en-1-yl}-benzoesäure *(Enantiomer 1)*

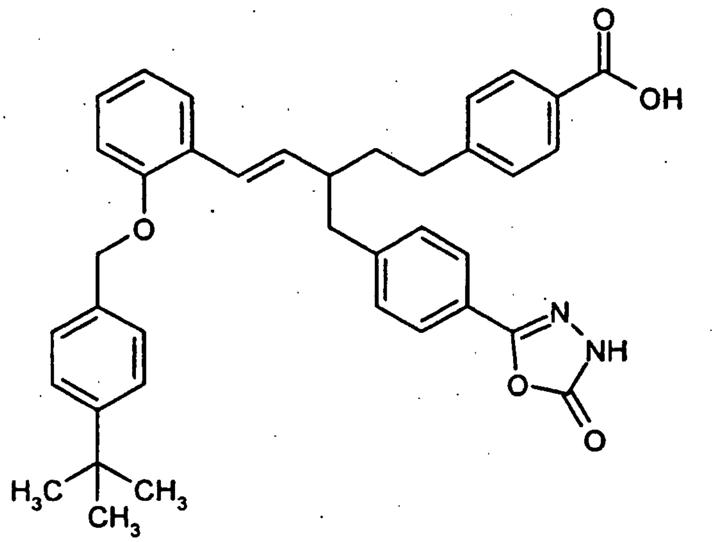

**Methode Enantiomerentrennung:**

Säule: Daicel Chiralpak AD-H 250 mm x 20 mm; Elutionsmittel: Ethanol (mit 1% Wasser und 0.2% Trifluoressigsäure) / Isohexan 20:80 (v/v); Fluss: 15 ml/min; UV-Detektion: 220 nm; Temperatur: 30°C.

$R_t$ 16.56 min; Reinheit >99%; >99.5% ee

Ausbeute: 68 mg

LC-MS (Methode 2): $R_t$ 3.05 min; m/z 602 ($M^+$).

**Beispiel 79**

4-{(4*E*)-5-{2-[(4-*tert*.-Butylbenzyl)oxy]phenyl}-3-[4-(5-oxo-4,5-dihydro-1,3,4-oxadiazol-2-yl)-benzyl]pent-4-en-1-yl}-benzoesäure *(Enantiomer 2)*

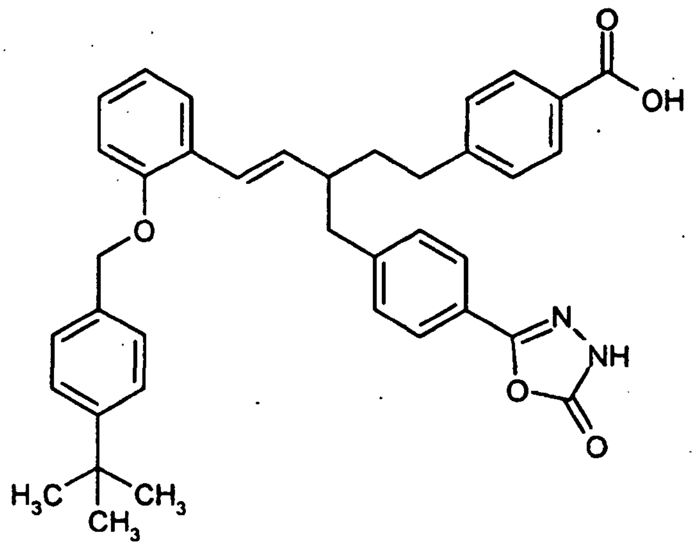

Methode Enantiomerentrennung: siehe Beispiel 78.

$R_t$ 18.25 min; Reinheit >99%; >99% ee

Ausbeute: 64 mg

LC-MS (Methode 2): $R_t$ 3.05 min; m/z 602 ($M^+$).

## Beispiel 80

4-{(4*E*)-5-{2-[(4-*tert*.-Butylbenzyl)oxy]phenyl}-3-[4-(5-oxo-4,5-dihydro-1*H*-1,2,4-triazol-3-yl)-benzyl]pent-4-en-1-yl}-benzoesäure

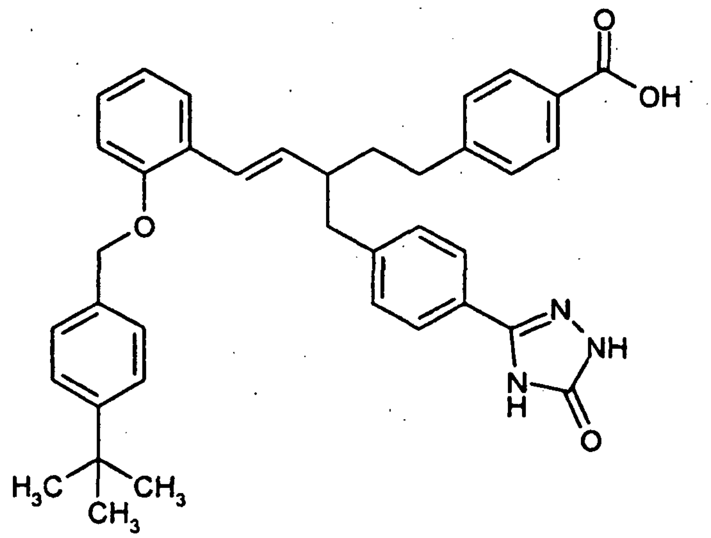

Das Rohprodukt aus Beispiel 106A wird in 10 ml 2 M Natronlauge suspendiert und 12 Stunden zum Rückfluss erhitzt. Der Ansatz wird dann mit 1 N Salzsäure auf pH 3-4 gestellt und eingeengt. Der Rückstand wird in Dichlormethan/Methanol (10:1) aufgenommen, filtriert und das Filtrat erneut eingeengt. Das erhaltene Rohprodukt wird mittels präparativer HPLC aufgereinigt. Es werden 268 mg (64% Reinheit, 34% Ausbeute) eines Feststoffes isoliert. Eine kleine Menge hiervon wird für die analytische Charakterisierung mittels nochmaliger präparativer HPLC nachgereinigt.

$^1$H-NMR (300 MHz, DMSO-$d_6$, δ/ppm): 11,91 (1H, s), 11,58 (1H, s), 7,82-7,80 (2H, d), 7,66-7,63 (2H, d), 7,45-7,40 (1H, m), 7,36-7,13 (9H, m), 7,07-7,01 (1H, m), 6,93-6,87 (1H, t), 6,52-6,47 (1H, d), 6,16-6,08 (1H, dd), 5,09-5,00 (2H, m), 2,89-2,58 (5H, m), 1,85-1,58 (2H, m), 1,24 (9H, s).

LC-MS (Methode 2): $R_t$ 2.72 min; m/z 602 (M+H$^+$).

## Beispiel 81

4-((4*E*)-3-[4-(5-Amino-1*H*-1,2,4-triazol-3-yl)benryl]-5-{2-[(4-*tert*.-butylbenzyl)oxy]phenyl}pent-4-en-1-yl)-benzoesäure

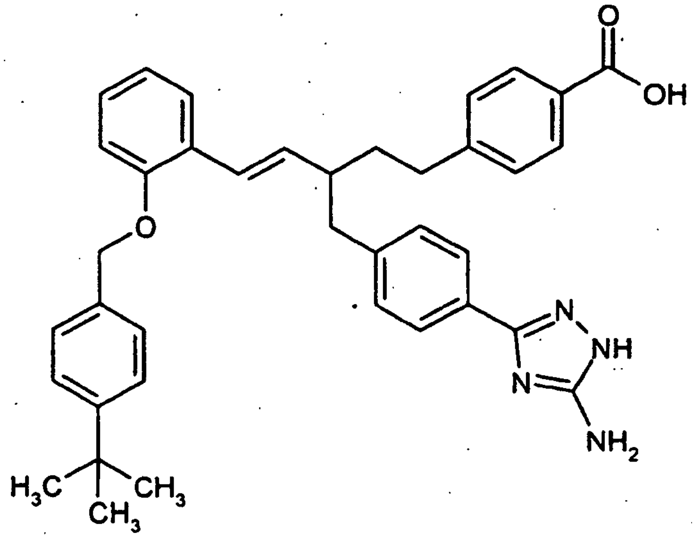

Das Rohprodukt aus Beispiel 107A wird in 2 ml 2 M Natronlauge suspendiert und 12 Stunden zum Rückfluss erhitzt. Der Ansatz wird dann mit 1 N Salzsäure auf pH 3-4 gestellt und eingeengt. Der Rückstand wird in Dichlormethan/Methanol (10:1) aufgenommen, filtriert, das Filtrat erneut eingeengt und das Rohprodukt mittels präparativer HPLC aufgereinigt. Es werden 17 mg eines Feststoffes isoliert.

$^1$H-NMR (300 MHz, DMSO-$d_6$, $\delta$/ppm): 12,77 (1H, breit), 11,97 (1H, s), 7,82-7,80 (2H, d), 7,76-7,74 (2H, d), 7,44-7,40 (1H, m), 7,37-7,27 (5H, m), 7,26-7,22 (2H, m), 7,20-7,13 (3H, m), 7,07-7,02 (1H, m), 6,93-6,87 (1H, t), 6,16-6,10 (1H, dd), 6,05-5,89 (1H, breit), 5,08-5,02 (2H, m), 2,81-2,54 (5H, m), 1,84-1,58 (2H, m), 1,24 (9H, s).

LC-MS (Methode 1): $R_t$ 2.89 min; m/z 601 (M+H$^+$).

## Beispiel 82

*E*-8-[2-(4-*tert*.-Butylbenzyloxy)phenyl]-6-[4-(5-oxo-4,5-dihydro-[1,2,4]oxadiazol-3-yl)benzyl]-oct-7-ensäure *(Racemat)*

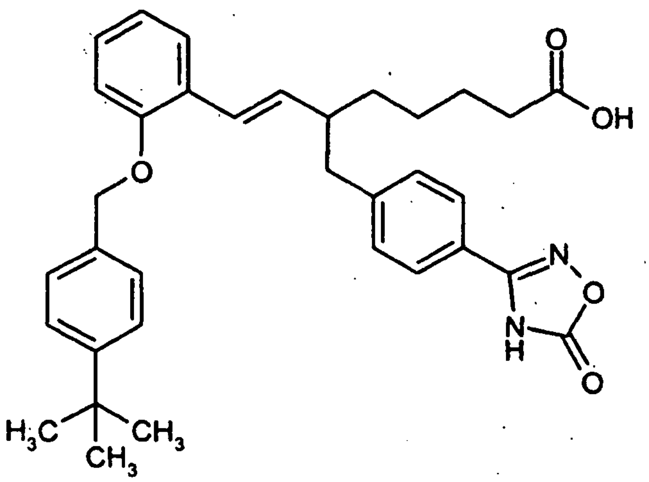

Eine Lösung von 1.38 g (2.25 mmol) *E*-8-[2-(4-*tert.*-Butylbenzyloxy)phenyl]-6-[4-(5-oxo-4,5-dihydro-[1,2,4] oxadiazol-3-yl)benzyl]-oct-7-ensäureethylester aus Beispiel 114A in 10 ml THF und 10 ml Wasser wird mit 107.7 mg (4.5 mmol) Lithiumhydroxid versetzt und 12 h bei 60°C gerührt. Nach dem Abkühlen wird das THF abgezogen und die wässrige Phase mit 1 M Salzsäure auf pH 4-5 eingestellt. Anschließend wird mit Essigsäureethylester dreimal extrahiert und die vereinigten organischen Phasen über Natriumsulfat getrocknet. Nach Filtrieren und Einengen wird der erhaltene Rückstand mittels präparativer HPLC gereinigt. Es werden 867 mg (69% Ausbeute) eines farblosen Feststoffes erhalten.

LC-MS (Methode 2): $R_t$ 2.84 min; m/z 555 ($M+H^+$).

800 mg (1.44 mmol) der so erhaltenen racemischen *E*-8-[2-(4-*tert.*-Butylbenzyloxy)phenyl]-6-[4-(5-oxo-4,5-dihydro-[1,2,4]oxadiazol-3-yl)benzyl]-oct-7-ensäure werden mittels präparativer HPLC an chiraler Phase weiter aufgetrennt. Es werden jeweils enantiomerenrein 390 mg bzw. 357 mg der beiden E-Isomere erhalten (siehe Beispiele 83 und 84).

__Beispiel 83__

*E*-8-[2-(4-*tert.*-Butylbenzyloxy)phenyl]-6-[4-(5-exo-4,5-dihydro-[1,2,4]oxadiazo1-3-yl)benzyl]-oct-7-ensäure *(Enantiomer 1)*

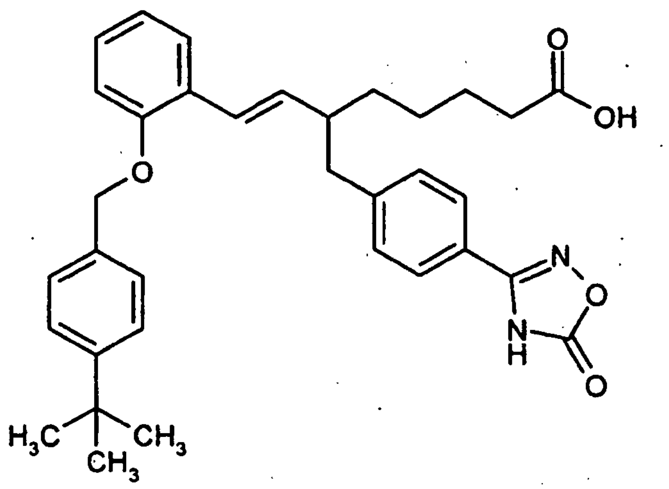

__Methode Enantiomerentrennung:__

Säule: Daicel Chiralpak AD-H 250 mm x 20 mm; Elutionsmittel: Isopropanol (mit 1% Wasser und 0.2% Trifluoressigsäure) / Isohexan 30:70 (v/v); Fluss: 15 ml/min; UV-Detektion: 220 nm; Temperatur: 24°C.

$R_t$ 3.87 min; Reinheit >99%; >99.5% ee

Ausbeute: 390 mg

$^1$H-NMR (400 MHz, DMSO-$d_6$, $\delta$/ppm): 12,91-12,77 (1H, breit), 11,96 (1H, s), 7,69 (2H, d), 7,41-7,32 (5H, m), 7,27 (2H, d), 7,15 (1H, t), 7,01 (1H, d), 6,88 (1H, t), 6,44 (1H, d), 6,10-5,99 (1H, m), 5,03 (2H, s), 2,88-2,76 (1H, m), 2,71-2,61 (1H, m), 2,56-2,41 (1H, m), 2,18 (2H, t), 1,56-1,41 (3H, m), 1,41-1,20 (3H, m), 1,28 (9H, s).

MS (EI): 553 $(M-H)^-$.

__Beispiel 84__

*E*-8-[2-(4-*tert.*-Butylbenzyloxy)phenyl]-6-[4-(5-oxo-4,5-dihydro-[1,2,4]oxadiazol-3-yl)benzyl]-oct-7-ensäure *(Enantiomer 2)*

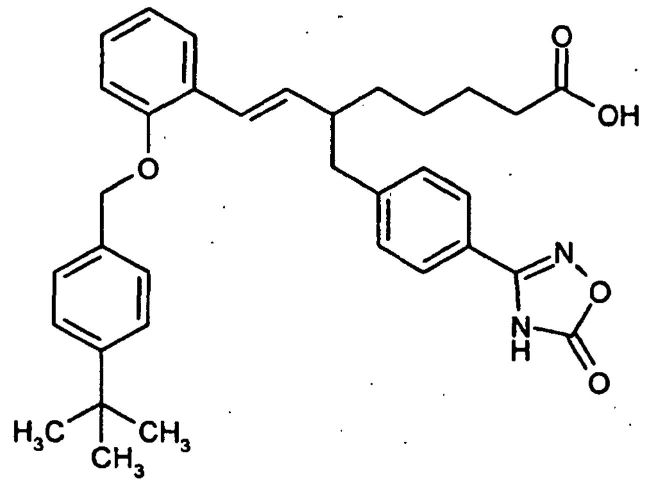

Methode Enantiomerentrennung: siehe Beispiel 83.
$R_t$ 4.55 min; Reinheit >99.5%; >98.8% ee
Ausbeute: 357 mg
$^1$H-NMR und MS siehe Beispiel 83.

**Beispiel 85**

*E*-8-[2-(4-*tert.*-Butylbenzyloxy)phenyl]-6-[4-(5-oxo-4,5-dihydro-[1,3,4]oxadiazol-2-yl)benzyl]-oct-7-ensäure

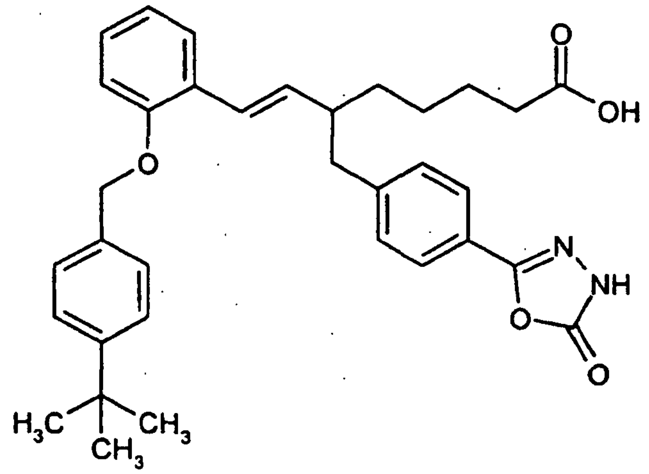

47 mg (0.09 mmol) *E*-8-[2-(4-*tert.*-Butylbenzyloxy)phenyl]-6-[4-(5-oxo-4,5-dihydro-[1,3,4]oxadiazol-2-yl)benzyl]-oct-7-ennitril aus Beispiel 121A in 10 ml n-Propanol werden mit 196 mg (3.51 mmol) Kaliumhydroxid versetzt und 15 Stunden bei 110°C gerührt. Anschließend wird der Ansatz mit 1 N Salzsäure auf pH 4 gestellt und dreimal mit Diethylether extrahiert. Die vereinigten organischen Phasen werden über Natriumsulfat getrocknet und anschließend im Vakuum eingeengt. Der erhaltene Rückstand wird mittels präparativer HPLC gereinigt. Es werden 12 mg (0.02 mmol, 24% Ausbeute) eines Feststoffes erhalten.
$^1$H-NMR (400 MHz, DMSO-$d_6$, $\delta$/ppm): 12,56-12,43 (1H, breit), 12,03-12,91 (1H, breit), 7,66 (2H, d), 7,41-7,29 (5H, m), 7,27 (2H, d), 7,14 (1H, t), 7,00 (1H, d), 6,88 (1H, t), 6,44 (1H, d), 6,10-5,98 (1H, m), 5,02 (2H, s), 2,88-2,77 (1H, m), 2,76-2,59 (2H, m), 2,18 (2H, t), 1,56-1,20 (8H, m), 1,27 (9H, s).
LC-MS (Methode 2): $R_t$ 2.88 min; m/z 554 (M-H)$^-$.

**Beispiel 86**

4-[3-((7*E*)-2-{2-[(4-*tert*.-Butylbenzyl)oxy]phenyl}vinyl)-7-(5-oxo-4,5-dihydro-1*H*-1,2,4-triazol-3-yl)heptyl]benzoesäure

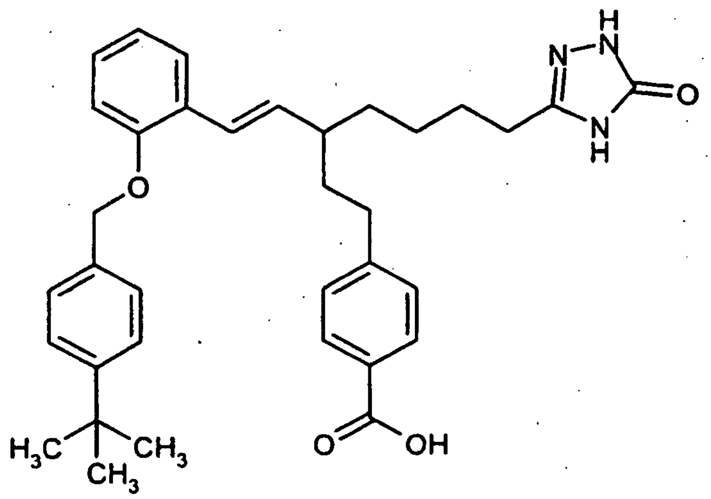

778 mg (1.3 mmol) 2-{(7*E*)-8-{2-[(4-*tert*.-Butylbenzyl)oxy]phenyl}-6-[2-(4-cyanophenyl)ethyl]-oct-7-enoyl}hydrazincarboxamid werden in 10 ml 2 M Natronlauge suspendiert und die Mischung 12 h bei 100°C gerührt. Nach dem Abkühlen wird mit 1 M Salzsäure sauer gestellt und mit Essigsäureethylester extrahiert. Die organische Phase wird über Natriumsulfat getrocknet und eingeengt. Der erhaltene Rückstand wird über präparative HPLC gereinigt. Es werden 580 mg (85% Reinheit, 0.87 mmol, 56% d. Th.) der Titelverbindung erhalten.

LC-MS (Methode 4): $R_t$ = 2.81 min

MS (ESIpos): m/z = 568 $(M+H)^+$.

580 mg (Gehalt 85%, 0.87 mmol) 4-[3-((7*E*)-2-{2-[(4-*tert*.-Butylbenzyl)oxy]phenyl}vinyl)-7-(5-oxo-4,5-dihydro-1*H*-1,2,4-triazol-3-yl)heptyl]benzoesäure werden mittels präparativer HPLC an chiraler Phase weiter aufgetrennt. Es werden jeweils enantiomerenrein 139 mg bzw. 143 mg der beiden *E*-Isomere erhalten (siehe Beispiele 87 und 88).

**Beispiel 87**

4-[3-((7*E*)-2-{2-[(4-*tert*.-Butylbenzyl)oxy]phenyl}vinyl)-7-(5-oxo-4,5-dihydro-1*H*-1,2,4-triazol-3-yl)heptyl]benzoesäure (*Enantiomer 1*)

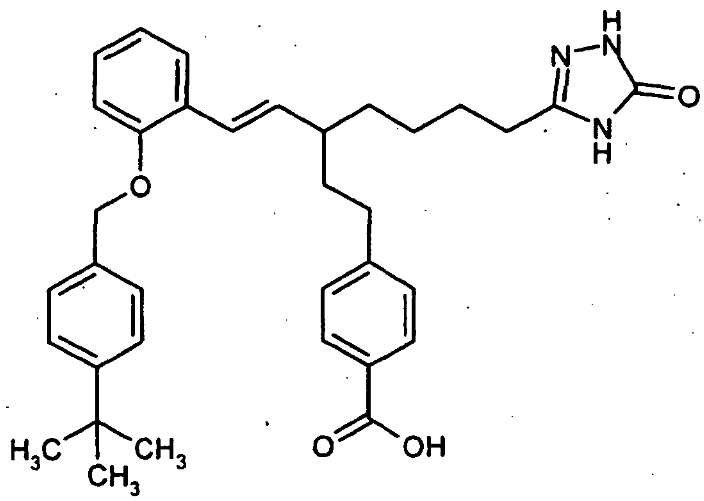

**Methode Enantiomerentrennung:**

Säule: Daicel Chiralcel OD-H 250 mm x 20 mm; Elutionsmittel: Isopropanol (mit 1% Wasser und 0.2% Trifluoressigsäure) / Isohexan 20:80 (v/v); Fluss: 15 ml/min; UV-Detektion: 220 nm; Temperatur: 30°C.

$R_t$ 9.17 min; Reinheit >98%; >98.5% ee

Ausbeute: 139 mg

MS (ESIpos): m/z= 568 $(M-H)^-$

$^1H$-NMR (400 MHz, DMSO-$d_6$, $\delta$/ppm): 12.85-12.68 (1H, breit), 11.13 (1H, s), 11.04 (1H, s), 7.82 (2H, d), 7.47 (1H, dd), 7.38 (4H, s), 7.28 (2H, d), 7.20 (1H, t), 7.09 (1H, d), 6.91 (1H, t), 6.64 (1H, d), 6.10-5.99 (1H, m), 5.10 (2H, s), 2.72-2.61 (1H, m), 2.60-2.51 (1H, m), 2.31 (2H, t), 2.16-2.03 (1H, m), 1.80-1.66 (1H, m), 1.65-1.38 (4H, m), 1.37-1.18 (12H, m, darin 9H, s).

**Beispiel 88**

4-[3-((7*E*)-2-{2-[(4-*tert.*-Butylbenzyl)oxy]phenyl}vinyl)-7-(5-oxo-4,5-dihydro-1*H*-1,2,4-triazol-3-yl)heptyl]benzoesäure (*Enantiomer 2)*

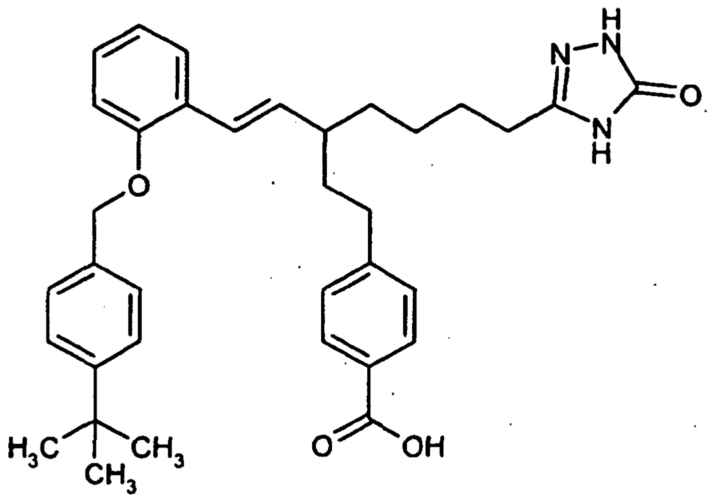

Methode Enantiomerentrennung: siehe Beispiel 87.

$R_t$ 11.13 min; Reinheit >99%; >95% ee

Ausbeute: 143 mg.

**Beispiel 89**

4-((3*E*)-4-{2-[(4-*tert.*-Butylbenzyl)oxy]phenyl}-2-{2-[4-(5-oxo-4,5-dihydro-1,2,4-oxadiazol-3-yl)-phenyl]ethyl}but-3-en-1-yl)benzoesäure

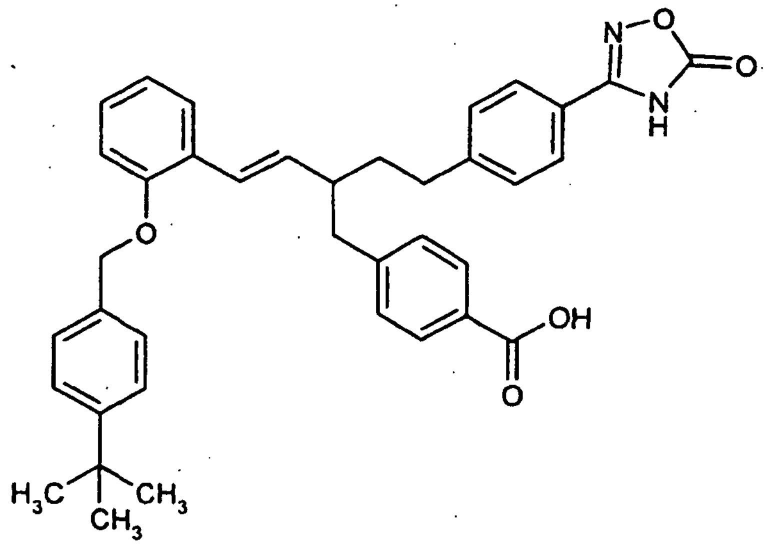

Eine Lösung von 500 mg (0.81 mmol) 4-((3*E*)-4-{2-[(4-*tert*.-Butylbenzyl)oxy]phenyl}-2-{2-[4-(5-oxo-4,5-dihydro-1,2,4-oxadiazol-3-yl)phenyl]ethyl}but-3-en-1-yl)benzoesäuremethylester in 5 ml THF und 5 ml Wasser wird mit 38.8 mg (1.62 mmol) Lithiumhydroxid versetzt und 12 h bei 50°C gerührt. Nach dem Abkühlen wird Wasser zugegeben und mit Diethylether extrahiert. Die wässrige Phase wird mit 1 M Salzsäure sauer gestellt und mit Diethylether ausgeschüttelt. Die erhaltene organische Phase wird mit Natriumchlorid-Lösung gewaschen, über Natriumsulfat getrocknet und eingeengt. Es werden 420 mg (0.7 mmol, 79% d. Th.) der Titelverbindung erhalten.

LC-MS (Methode 1): $R_t$ = 3.31 min

MS (ESIpos): m/z = 603 $(M+H)^+$

$^1$H-NMR (400 MHz, DMSO-$d_6$, $\delta$/ppm): 12.8 (2H, breit), 7.8 (d, 2H), 7.69 (d, 2H), 7.4 (d, 1H), 7.35 (d, 4H), 7.3-7.22 (m, 4H), 7.18 (t, 1H), 7.03 (d, 1H), 6.9 (t, 1H), 6.5 (d, 1H), 6.12 (dd, 1H), 5.05 (s, 2H), 2.9-2.82 (m, 1H), 2.78-2.58 (m, 3H), 1.88-1.5 (m, 3H).

400 mg (0.66 mmol) 4-((3*E*)-4-{2-[(4-*tert*.-Butylbenzyl)oxy]phenyl}-2-{2-[4-(5-oxo-4,5-dihydro-1,2,4-oxadiazol-3-yl)phenyl]ethyl}but-3-en-1-yl)benzoesäure werden mittels präparativer HPLC an chiraler Phase weiter aufgetrennt. Es werden enantiomerenrein jeweils 155 mg der beiden *E*-Isomere erhalten (siehe Beispiele 90 und 91).

## Beispiel 90

4-((3*E*)-4-{2-[(4-*tert*.-Butylbenzyl)oxy]phenyl}-2-{2-[4-(5-oxo-4,5-dihydro-1,2,4-oxadiazol-3-yl)-phenyl]ethyl}but-3-en-1-yl)benzoesäure *(Enantiomer 1)*

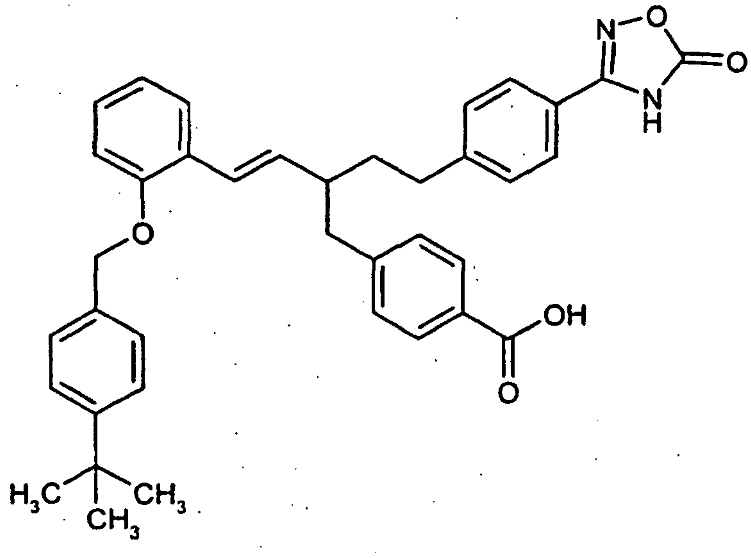

Methode Enantiomerentrennung:

Säule: Daicel Chiralpak AD-H 250 mm x 20 mm; Elutionsmittel: Isopropanol (mit 1% Wasser und 0.2% Trifluoressigsäure)/ Isohexan 50:50 (v/v); Fluss: 15 ml/min; UV-Detektion: 220 nm; Temperatur: 29°C.
$R_t$ 7.5 min; Reinheit >99.5%; >99.5% ee
Ausbeute: 155 mg.

**Beispiel 91**

4-((3*E*)-4-{2-((4-*tert.*-Butylbenzyl)oxy]phenyl}-2-{2-[4-(5-oxo-4,5-dihydro-1,2,4-oxadiazol-3-yl)-phenyl]ethyl}but-3-en-1-yl)benzoesäure *(Enantiomer 2)*

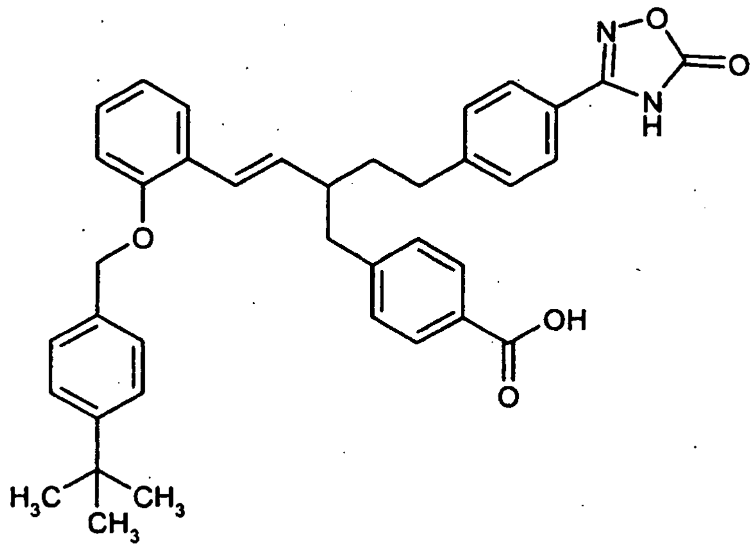

Methode Enantiomerentrennung: siehe Beispiel 90.
$R_t$ 11.2 min; Reinheit >99%; >98.5% ee
Ausbeute: 155 mg.
Auf analoge Weise werden die in der folgenden Tabelle aufgeführten Beispiele erhalten:

| Beispiel Nr. | Beispiel-Struktur *[Edukte]* | Analytische Daten |
|---|---|---|
| **92** | 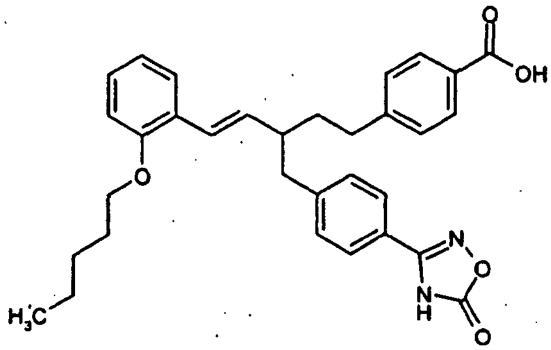<br>*(Enantiomer 1)*<br>*[ausgehend von Bsp. 75A und 1-Brompentan, sowie Enantiomerentrennung]* | $R_t$ 7.17 min; Reinheit >99.5%; >99.5% ee.<br>$^1$H-NMR (400 MHz, DMSO-$d_6$, δ/ppm): 12.86 (1H, br. s), 12.78 (1H, br. s), 7.85 (2H, d), 7.69 (2H, d), 7.43-7.34 (3H, m), 7.30 (2H, d), 7.17 (1H, t), 6.97-6.83 (2H, m), 6.43 (1H, d), 6.18-6.05 (1H, m), 3.98-3.84 (2H, m), 2.94-2.84 (1H, m), 2.81-2.69 (2H, m), 2.68-2.57 (1H, m), 2.56-2.41 (1H, m), 1.89-1.75 (1H, m), 1.74-1.58 (3H, m), 1.40-1.21 (4H, m), 0.83 (3H, t).<br>LC-MS (Methode 1): $R_t$ = 3.09 min; m/z = 527 (M+H$^+$). |
| **93** | 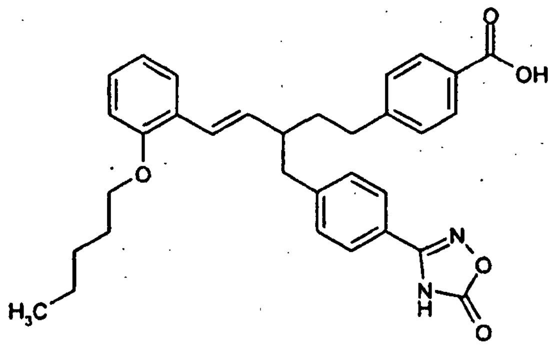<br>*(Enantiomer 2)*<br>*[ausgehend von Bsp. 75A und 1-Brompentan, sowie Enantiomerentrennung]* | $R_t$ 8.74 min; Reinheit >99%; >98.5% ee.<br>$^1$H-NMR: siehe Beispiel 92<br>LC-MS (Methode 1): $R_t$ = 3.09 min; m/z = 527 (M+H$^+$). |
| **94** | 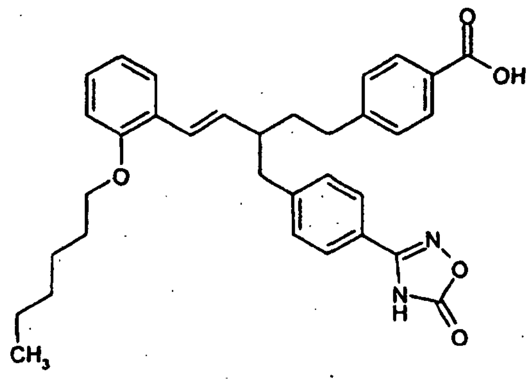<br>*(Enantiomer 1)*<br>*[ausgehend von Bsp. 75A und 1-Bromhexan, sowie Enantiomerentrennung]* | $R_t$ 9.06 min; >98.2% ee.<br>$^1$H-NMR (400 MHz, DMSO-$d_6$, δ/ppm): 12.82 (2H, br. s), 7.84 (2H, d), 7.68 (2H, d), 7.43-7.32 (3H, m), 7.29 (2H, d), 7.16 (1H, t), 6.97-6.82 (2H, m), 6.43 (1H, d), 6.17-6.05 (1H, m), 3.98-3.84 (2H, m), 2.94-2.83 (1H, m), 2.81-2.69 (2H, m), 2.68-2.58 (1H, m), 2.57-2.41 (1H, m), 1.89-1.76 (1H, m), 1.75-1.59 (3H, m), 1.42-1.30 (2H, m), 1.30-1.15 (4H, m), 0.81 (3H, t).<br>LC-MS (Methode 2): $R_t$ = 2.98 min; m/z = 541 (M+H$^+$). |

(fortgesetzt)

| Beispiel Nr. | Beispiel-Struktur *[Edukte]* | Analytische Daten |
|---|---|---|
| **95** | 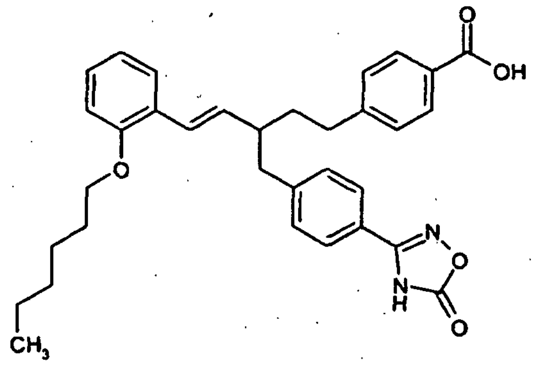 (*Enantiomer 2*) *[ausgehend von Bsp. 75A und 1-Bromhexan, sowie Enantiomerentrennung]* | $R_t$ 16.57 min; >99.5% ee. $^1$H-NMR: siehe Beispiel 94<br>LC-MS (Methode 2): $R_t$ = 2.98 min; m/z = 541 (M+H$^+$). |
| **96** | 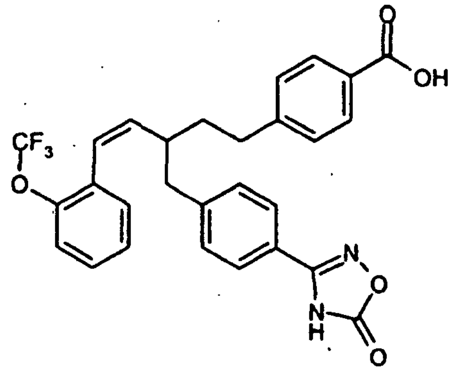 (*cis-Enantiomer 1*) *[ausgehend von Bsp. 74A und Bsp. 126A, sowie Enantiomerentrennung]* | $R_t$ 7.69 min; >99.5% ee. $^1$H-NMR (400 MHz, DMSO-$d_6$, δ/ppm): 12.88 (2H, br. s), 7.76 (2H, d), 7.67 (2H, d), 7.37 (1H, t), 7.30 (1H, d), 7.25 (2H, d), 7.18 (1H, t), 7.13 (2H, d), 6.80 (1H, d), 6.45 (1H, d), 5.79-5.67 (1H, m), 2.90-2.79 (1H, m), 2.75-2.59 (3H, m), 2.58-2.39 (1H, m), 1.78-1.65 (1H, m), 1.65-1.52 (1H, m).<br>LC-MS (Methode 4): $R_t$ = 2.77 min; m/z = 525 (M+H$^+$). |
| **97** | 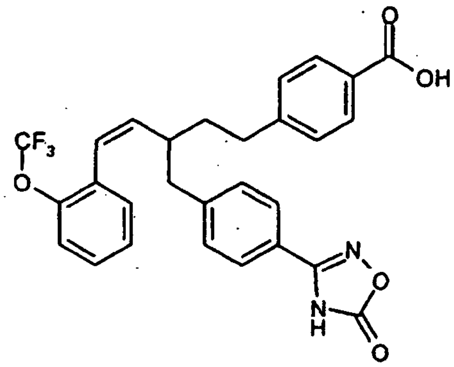 (*cis-Enantiomer 2*) *[ausgehend von Bsp. 74A und Bsp. 126A, sowie Enantiomerentrennung]* | $R_t$ 13.00 min; >99.5% ee. $^1$H-NMR: siehe Beispiel 96.<br>LC-MS (Methode 4): $R_t$ = 2.77 min; m/z = 525 (M+H$^+$). |

(fortgesetzt)

| Beispiel Nr. | Beispiel-Struktur *[Edukte]* | Analytische Daten |
|---|---|---|
| **98** | 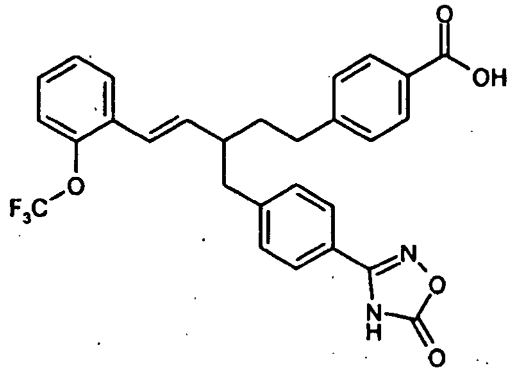<br>*(trans-Enantiomer 1)*<br>*[ausgehend von Bsp. 74A und Bsp. 126A, sowie Enantiomerentrennung]* | $R_t$ 8.75 min; >99% ee.<br>$^1$H-NMR (400 MHz, DMSO-$d_6$, $\delta$/ppm): 12.85 (2H, br. s), 7.84 (2H, d), 7.68 (3H, d), 7.40-7.32 (4H, m), 7.29 (3H, d), 6.36 (1H, d), 6.31-6.21 (1H, m), 2.95-2.86 (1H, m), 2.82-2.68 (2H, m), 2.67-2.45 (1H, m), 1.91-1.78 (1H, m), 1.77-1.65 (1H, m).<br>LC-MS (Methode 4): $R_t$ = 2.77 min; m/z = 525 ($M+H^+$). |
| **99** | 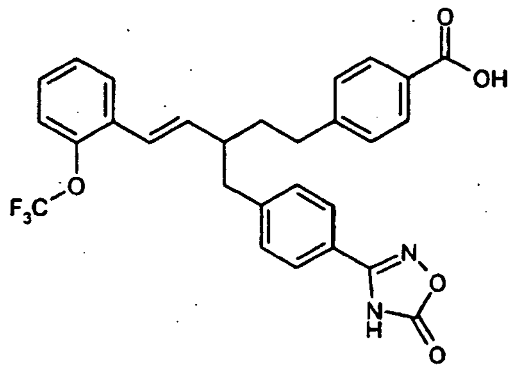<br>(*trans-Enantiomer 2*)<br>*[ausgehend von Bsp. 74A und Bsp.126A, sowie Enantiomerentrennung]* | $R_t$ 10.34 min; >99% ee.<br>$^1$H-NMR: siehe Beispiel 98<br>LC-MS (Methode 4): $R_t$ = 2.77 min; m/z = 525 ($M+H^+$). |
| **100** | 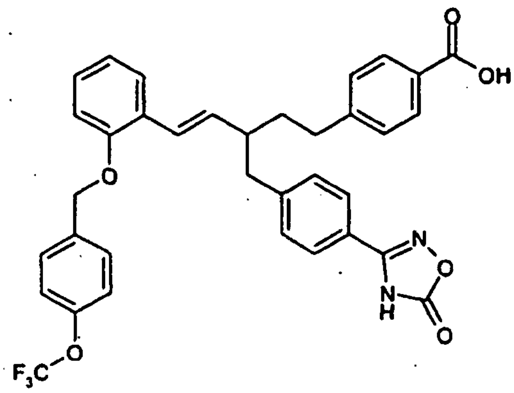<br>*(Enantiomer 1)*<br>*[ausgehend von Bsp. 127A und 4-Trifluormethoxybenzylbromid]* | $^1$H-NMR (400 MHz, DMSO-$d_6$, $\delta$/ppm): 12.82 (1H, br. s), 12.77 (1H, br. s), 7.81 (2H, d), 7.68 (2H, d), 7.49 (2H, d), 7.44 (1H, d), 7.39-7.29 (4H, m), 7.26 (2H, d), 7.18 (1H, t), 7.02 (1H, d), 6.92 (1H, t), 6.49 (1H, d), 6.19-6.07 (1H, m), 5.12 (2H, s), 2.93-2.82 (1H, m), 2.79-2.44 (4H, m), 1.88-1.73 (1H, m), 1.73-1.58 (1H, m).<br>LC-MS (Methode 2): $R_t$ = 2.86 min; m/z = 631 ($M+H^+$). |

(fortgesetzt)

| Beispiel Nr. | Beispiel-Struktur *[Edukte]* | Analytische Daten |
|---|---|---|
| **101** | 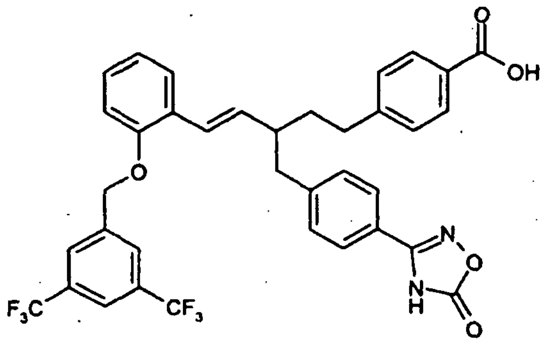<br>*(Enantiomer 1)*<br>*[ausgehend von Bsp. 127A und 3,5-Bis(trifluormethyl) benzylbromid]* | $^1$H-NMR (400 MHz, DMSO-$d_6$, $\delta$/ppm): 12.80 (1H, br. s), 12.76 (1H, br. s), 8.18-7.95 (3H, m), 7.76 (2H, d), 7.63 (2H, d), 7.44 (1H, d), 7.32 (2H, d), 7.21 (3H, d), 7.06(1H, d), 6.97(1H, t), 6.51 (1H, d), 6.21-6.08 (1H, m), 5.48-5.23 (2H, m), 2.94-2.81 (1H, m), 2.79-2.65 (2H, m), 2.65-2.41 (2H, m), 1.87-1.72 (1H, m), 1.71-1.58 (1H, m).<br><br>LC-MS (Methode 6): $R_t$ = 3.13 min; m/z = 683 (M+H$^+$). |

[Methoden zur Enantiomerentrennung:

*Beispiele 92 und 93:*

Säule: Daicel Chiralpak AD-H 250 mm x 20 mm, 5 μm; Elutionsmittel: Isopropanol (mit 1% Wasser und 0.2% Trifluoressigsäure) / Isohexan 30:70 (v/v); Fluss: 15 ml/min; UV-Detektion: 220 nm; Temperatur: 25°C.

*Beispiele 94 und 95:*

Säule: Chirale Kieselgel-Phase basierend auf dem Selektor Poly(*N*-methacryloyl-L-leucin-*tert*.-butylamid), 670 mm x 40 mm; Elutionsmittel: Essigsäureethylester; Fluss: 80 ml/min; UV-Detektion: 270 nm; Temperatur: 24°C.

*Beispiele 96-99:*

Säule: Daicel Chiralpak AD-H 250 mm x 20 mm, 5 μm; Elutionsmittel: Isopropanol (mit 1% Wasser und 0.2% Trifluoressigsäure) / Isohexan 30:70 (v/v); Fluss: 15 ml/min; UV-Detektion: 220 nm; Temperatur: 35°C].

B. Bewertung der pharmakologischen Wirksamkeit

Die pharmakologische Wirkung der erfindungsgemäßen Verbindungen kann in folgenden Assays gezeigt werden:

B-1. Gefäßrelaxierende Wirkung *in vitro*:

Kaninchen werden durch intravenöse Injektion von Thiopental-Natrium narkotisiert bzw. getötet (ca. 50 mg/kg) und entblutet. Die Arteria Saphena wird entnommen und in 3 mm breite Ringe geteilt. Die Ringe werden einzeln auf je einem triangelförmigen, am Ende offenen Häkchenpaar aus 0.3 mm starkem Spezialdraht (Remanium®) montiert. Jeder Ring wird unter Vorspannung in 5 ml-Organbäder mit 37°C warmer, carbogenbegaster Krebs-Henseleit-Lösung folgender Zusammensetzung gebracht: NaCl 119 mM; KCl 4.8 mM; $CaCl_2$ x 2 $H_2O$ 1 mM; $MgSO_4$ x 7 $H_2O$ 1.4 mM; $KH_2PO_4$ 1.2 mM; $NaHCO_3$ 25 mM; Glucose 10 mM; Rinderserumalbumin 0.001%. Die Kontraktionskraft wird mit Statham UC2-Zellen erfasst, verstärkt und über A/D-Wandler (DAS-1802 HC, Keithley Instruments, München) digitalisiert sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Test-substanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50% zu reduzieren ($IC_{50}$-Wert). Das Standard-Applikationsvolumen beträgt 5 μl. Der DMSO-Anteil in der Badlösung entspricht 0.1%.

Repräsentative Ergebnisse zu den erfindungsgemäßen Verbindungen sind in Tabelle 1 aufgeführt:

Tabelle 1: Gefäßrelaxierende Wirkung *in vitro*

| Beispiel Nr. | $IC_{50}$ [nM] |
|---|---|
| 1 | 26 |
| 11 | 724 |
| 18 | 490 |
| 19 | 850 |
| 51 | 505 |
| 55 | 146 |
| 56 | 635 |
| 57 | 714 |
| 66 | 263 |
| 67 | 153 |
| 71 | 898 |
| 74 | 290 |
| 76 | 851 |
| 78 | 13 |
| 80 | 74 |
| 81 | 422 |
| 85 | 58 |
| 92 | 390 |
| 95 | 410 |
| 96 | 1333 |

B-2. Stimulation der rekombinanten löslichen Guanylatcyclase (sGC) *in vitro:*

Die Untersuchungen zur Stimulation der rekombinanten löslichen Guanylatcyclase (sGC) durch die erfindungsgemäßen Verbindungen mit und ohne Natriumnitroprussid sowie mit und ohne den Häm-abhängigen sGC-Inhibitor 1*H*-1,2,4-Oxadiazol-(4,3a)-chinoxalin-1-on (ODQ) werden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch, "Purified soluble guanylyl cyclase expressed in a baculovirus/Sf9 system: Stimulation by YC-1, nitric oxide, and carbon oxide", J. Mol. Med. 77 (1999), 14-23. Die Häm-freie Guanylatcyclase wird durch Zugabe von Tween 20 zum Probenpuffer (0.5% in der Endkonzentration) erhalten.

Die Aktivierung der sGC durch eine Prüfsubstanz wird als n-fache Stimulation der Basalaktivität angegeben. Das Ergebnis für Beispiel 55 ist in Tabelle 2 gezeigt:

Tabelle 2: Stimulation (n-fach) der rekombinanten löslichen Guanylatcyclase (sGC) *in vitro* durch Beispiel 55

| Konzentration Beispiel 55 [μM] | Häm-haltige sGC | | | | Häm-freie sGC |
|---|---|---|---|---|---|
| | Basal | + 0.1 μM DEA/NO | + 0.01 μM DEA/NO | + 10 μM ODQ | Basal |
| 0.0 | 1.0 | 52.0 | 10.7 | 3.0 | 1.0 |
| 0.001 | 1.4 | 50.3 | 10.6 | | 3.7 |
| 0.01 | 3.1 | 53.4 | 13.0 | 12.7 | 24.0 |
| 0.1 | 15.5 | 66.5 | 25.5 | 66.3 | 98.1 |
| 1 | 25.4 | 75.1 | 35.4 | 107.4 | 132.6 |

(fortgesetzt)

| Konzentration Beispiel 55 [μM] | Häm-haltige sGC | | | | Häm-freie sGC |
|---|---|---|---|---|---|
| | Basal | + 0.1 μM DEA/NO | + 0.01 μM DEA/NO | + 10 μM ODQ | Basal |
| 10 | 33.0 | 81.5 | 42.5 | 105.5 | 137.9 |
| [DEA/NO = 2-(*N,N*-Diethylamino)diazenolat-2-oxid; ODQ = 1*H*-1,2,4-Oxadiazol-(4,3a)chinoxalin-1-on]. | | | | | |

Aus Tabelle 2 ist ersichtlich, dass eine Stimulation sowohl des Häm-haltigen als auch des Hämfreien Enzyms erreicht wird. Weiterhin zeigt die Kombination aus Beispiel 55 und 2-(*N*,*N*-Diethylamino)diazenolat-2-oxid (DEA/NO), einem NO-Donor, keinen synergistischen Effekt, d.h. die Wirkung von DEA/NO wird nicht potenziert, wie dies bei einem über einen Häm-abhängigen Mechanismus wirkenden sGC-Aktivator zu erwarten wäre. Darüber hinaus wird die Wirkung des erfindungsgemäßen sGC-Aktivators durch den Häm-abhängigen Inhibitor der löslichen Guanylatcyclase ODQ nicht blockiert, sondern sogar gesteigert. Die Ergebnisse aus Tabelle 2 belegen somit den Wirkmechanismus der erfindungsgemäßen Verbindungen als Aktivatoren der löslichen Guanylatcyclase.

B-3. Radiotelemetrische Messung von Blutdruck und Herzfrequenz an wachen SH-Ratten

Für die im Folgenden beschriebenen Messungen an wachen SH-Ratten wird ein im Handel erhältliches Telemetriesystem der Firma Data Sciences International DSI, USA, eingesetzt.

Das System besteht aus 3 Hauptkomponenten: (*1*) Implantierbare Sender, (2) Empfänger, die über einen Multiplexer mit einem (*3*) Datenakquisitionscomputer verbunden sind. Die Telemetrieanlage ermöglicht eine kontinuierliche Erfassung von Blutdruck und Herzfrequenz an wachen Tieren in ihrem gewohnten Lebensraum.

Die Untersuchungen werden an ausgewachsenen weiblichen, spontan-hypertensiven Ratten (SH-Ratten) mit einem Körpergewicht von >200 g durchgeführt. Die Versuchstiere werden nach Senderimplantation einzeln in Makrolon-Käfigen Typ 3 gehalten. Sie haben freien Zugang zu Standardfutter und Wasser. Der Tag/Nacht-Rhythmus im Versuchslabor wird per Raumbeleuchtung um 6:00 Uhr morgens und um 19:00 Uhr abends gewechselt.

Die eingesetzten Telemetriesender (TAM PA-C40, DSI) werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobarbital (Nembutal, Sanofi, 50 mg/kg i.p.) narkotisiert und an der Bauchseite weiträumig rasiert und desinfiziert. Nach Eröffnung des Bauchraumes entlang der Linea alba wird der flüssigkeitsgefüllte Messkatheter des Systems oberhalb der Bifurcation nach cranial in die Aorta descendens eingesetzt und mit Gewebekleber (VetBonD™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum verabreicht (Tardomyocel COMP, Bayer, 1 ml/kg s.c.).

Versuchsablauf:

Die zu untersuchenden Substanzen werden jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert. Eine Lösungsmittel-behandelte Gruppe von Tieren wird als Kontrolle eingesetzt.

Die Telemetrie-Messeinrichtung ist für 24 Tiere konfiguriert. Jeder Versuch wird unter einer Versuchsnummer registriert.

Den in der Anlage lebenden instrumentierten Ratten ist jeweils eine eigene Empfangsantenne zugeordnet (1010 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI) online erfasst und entsprechend aufgearbeitet werden. Die Ablage der Daten erfolgt jeweils in einem hierfür eröffneten Ordner, der die Versuchsnummer trägt.

Im Standardablauf werden über je 10 Sekunden Dauer gemessen: (1) systolischer Blutdruck (SBP), (2) diastolischer Blutdruck (DBP), (3) arterieller Mitteldruck (MAP) und (*4*) Herzfrequenz (HR).

Die Messwerterfassung wird rechnergesteuert in 5 Minuten-Abständen wiederholt. Die als Absolutwert erhobenen Quelldaten werden im Diagramm mit dem aktuell gemessenen Barometerdruck korrigiert und in Einzeldaten abgelegt. Weitere technische Details sind in der Dokumentation der Herstellerfirma (DSI) aufgeführt.

Die Verabreichung der Prüfsubstanzen erfolgt am Versuchstag um 9:00 Uhr. Im Anschluss an die Applikation werden die oben beschriebenen Parameter über 24 Stunden gemessen. Nach Versuchsende werden die erhobenen Einzeldaten mit der Analysis-Software (Dataquest™ A.R.T. Analysis) sortiert. Als Leerwert wird der Zeitpunkt 2 Stunden

vor Substanz-Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten-Mittelwert, 30 Minuten-Mittelwert) und als Textdatei auf einen Datenträger übertragen. Die so vorsortierten und komprimierten Messwerte werden in Excel-Vorlagen übertragen und tabellarisch dargestellt.

## C. **Ausführungsbeispiele für pharmazeutische Zusammensetzungen**

Die erfindungsgemäßen Verbindungen können folgendermaßen in pharmazeutische Zubereitungen überführt werden:

Tablette:

Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

**Oral applizierbare Suspension**:

Zusammensetzung:

1000 mg der erfindungsgemäßen Verbindung, 1000 mg Ethanol (96%), 400 mg Rhodigel® (Xanthan gum der Firma FMC, Pennsylvania, USA) und 99 g Wasser.

Einer Einzeldosis von 100 mg der erfindungsgemäßen Verbindung entsprechen 10 ml orale Suspension.

Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

**Oral applizierbare Lösung:**

Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

**i.v.-Lösung:**

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

**Patentansprüche**

1. Verbindung der Formel (I-A)

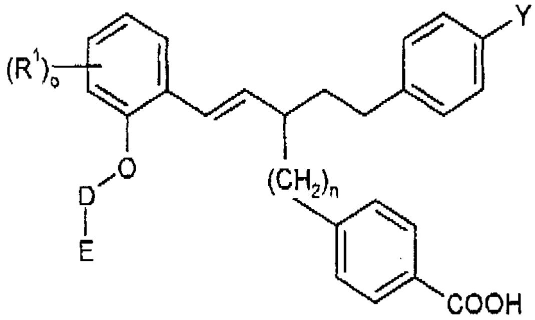

(I-A),

in welcher

D für ($C_1$-$C_7$)-Alkandiyl, welches ein- oder mehrfach mit Fluor substituiert sein kann, steht,
E für Wasserstoff, Trifluormethyl oder für eine Gruppe der Formel

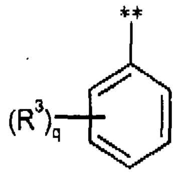

oder

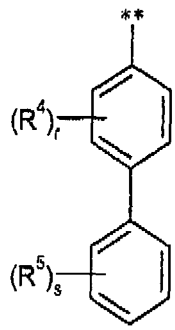

steht, worin** die Verknüpfungsstelle mit der Gruppe D bedeutet,
Y für eine Gruppe der Formel

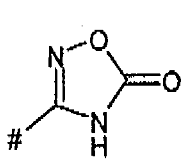

,

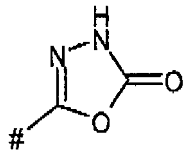

oder

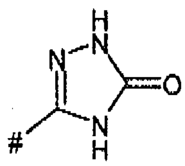

,

worin # die Verknüpfungsstelle mit dem Phenylring bedeutet, steht,
n für die Zahl 1 oder 2 steht,
$R^1$, $R^3$, $R^4$ und $R^5$ unabhängig voneinander für einen Substituenten ausgewählt aus der Reihe Fluor, Chlor, Brom, Methyl, *tert.*-Butyl, Trifluormethyl, Methoxy und Trifluormethoxy stehen,
und
o, q, r und s unabhängig voneinander jeweils für die Zahl 0, 1 oder 2 stehen, wobei für den Fall, dass $R^1$, $R^3$, $R^4$ oder $R^5$ mehrfach auftreten, ihre Bedeutungen jeweils gleich oder verschieden sein können,

sowie ihre Salze, Solvate und Solvate der Salze.

2. Verbindung der Formel (I-B)

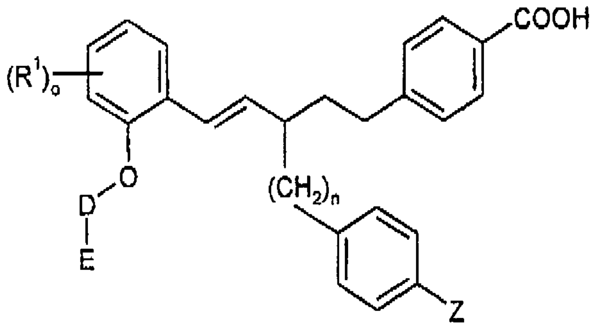

(I-B),

in welcher

D für ($C_1$-$C_7$)-Alkandiyl, welches ein- oder mehrfach mit Fluor substituiert sein kann, steht, E für Wasserstoff, Trifluormethyl oder für eine Gruppe der Formel

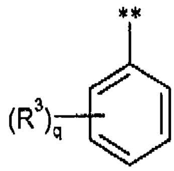

oder

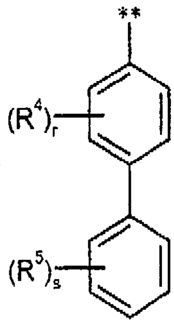

steht, worin ** die Verknüpfungsstelle mit der Gruppe D bedeutet, Z für eine Gruppe der Formel

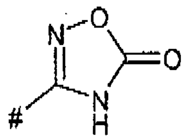

,

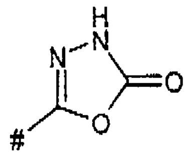

oder

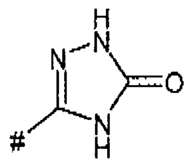

,

worin # die Verknüpfungsstelle mit dem Phenylring bedeutet, steht, n für die Zahl 1 oder 2 steht, $R^1$, $R^3$, $R^4$ und $R^5$ unabhängig voneinander für einen Substituenten ausgewählt aus der Reihe Fluor, Chlor, Brom, Methyl, *tert.*-Butyl, Trifluormethyl, Methoxy und Tri-fluormethoxy stehen, und o, q, r und s unabhängig voneinander jeweils für die Zahl 0, 1 oder 2 stehen, wobei für den Fall, dass $R^1$, $R^3$, $R^4$ oder $R^5$ mehrfach auftreten, ihre Bedeutungen jeweils gleich oder verschieden sein können,

sowie ihre Salze, Solvate und Solvate der Salze.

3. Verfahren zur Herstellung einer Verbindung der Formel (I-A) und (I-B), wie in den Ansprüchen 1 und 2 definiert, **dadurch gekennzeichnet, dass** man entweder

[A-1] Verbindungen der Formel (II-1)

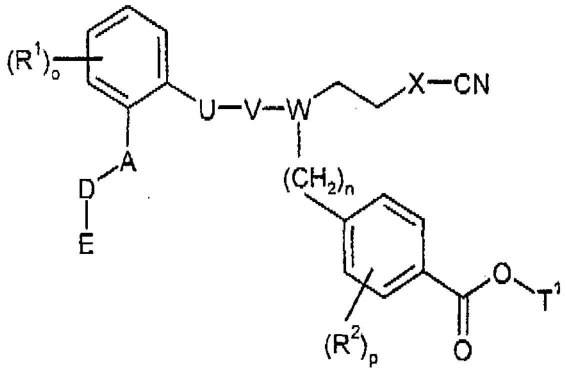

(II-1),

in welcher $R^1$, $R^2$, A, D, E, U, V, W, X, n, o und p jeweils die in den Ansprüchen 1 und 2 angegebenen Bedeutungen haben und

$T^1$ für $(C_1-C_4)$-Alkyl steht,
zunächst in einem inerten Lösungsmittel mit Hydroxylamin in Verbindungen der Formel (III-1)

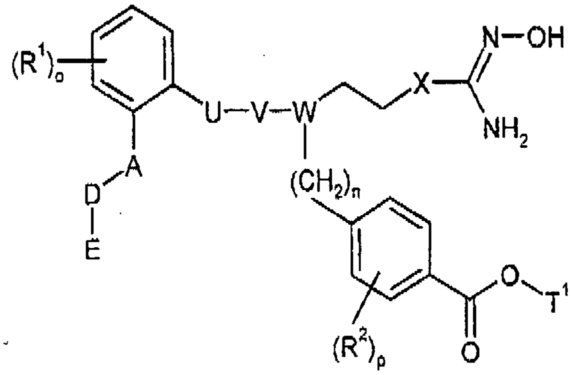

(III-1),

in welcher $R^1$, $R^2$, A, D, E, U, V, W, X, n, o, p und $T^1$ jeweils die oben angegebenen Bedeutungen haben, überführt und dann in einem inerten Lösungsmittel in Gegenwart einer Base mit einem Chlorameisensäureester der Formel (IV)

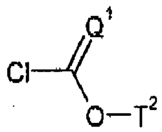

(IV),

in welcher
$Q^1$ für O oder S
und
$T^2$ für $(C_1-C_{10})$-Alkyl steht,

sowie gegebenenfalls durch nachfolgendes Erhitzen in einem inerten Lösungsmittel zu Verbindungen der Formel (V-1)

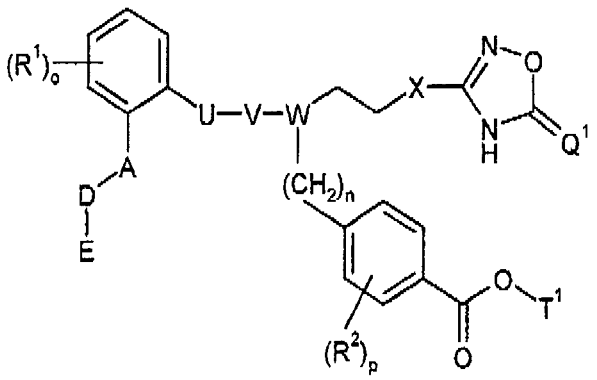

(V-1),

in welcher $R^1$, $R^2$, A, D, E, U, V, W, X, n, o, p, $Q^1$ und $T^1$ jeweils die oben angegebenen Bedeutungen haben, umsetzt

oder

[A-2] Verbindungen der Formel (II-2)

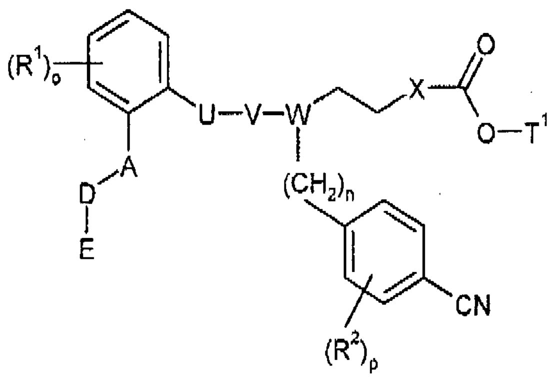

(II-2),

in welcher $R^1$, $R^2$, A, D, E, U, V, W, X, n, o, p und $T^1$ jeweils die oben angegebenen Bedeutungen haben, auf zum Verfahren [A-1] analoge Weise in Verbindungen der Formel (V-2)

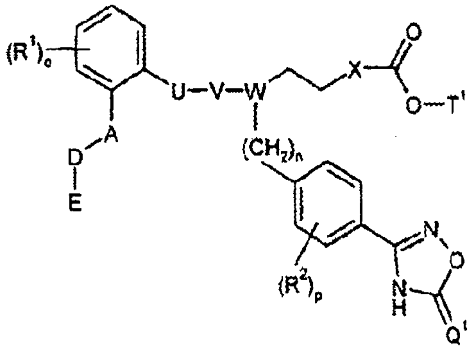

(V-2),

in welcher $R^1$, $R^2$, A, D, E, U, V, W, X, n, o, p, $Q^1$ und $T^1$ jeweils die oben angegebenen Bedeutungen haben. überführt

oder

[B-1] Verbindungen der Formel (VI-1)

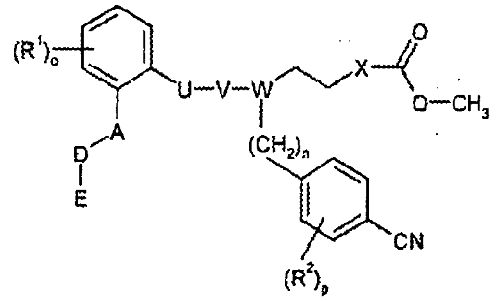

(VI-1),

in welcher $R^1$, $R^2$, A, D, E, U, V, W, X, n, o und p jeweils die in den Ansprüchen 1 und 2 angegebenen Bedeutungen haben,
zunächst in einem inenen Lösungsmittel mit Hydrazin in Verbindungen der Formel (VII-1)

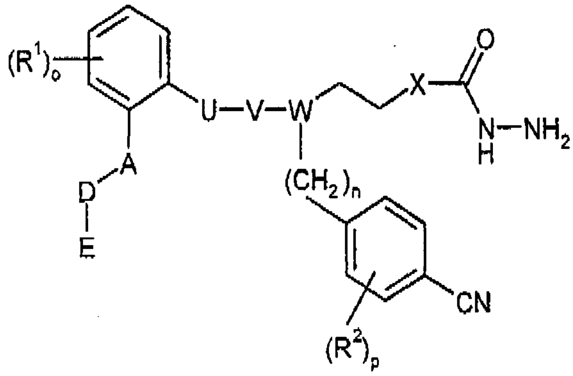

(VII-1),

in welcher $R^1$, $R^2$, A, D, E, U, V, W, X, n, o und p jeweils die oben angegebenen Bedeutungen haben,
überführt und dann in einem inerten Lösungsmittel mit Phosgen oder einem Phosgen-Derivat zu Verbindungen der Formel (VIII-1)

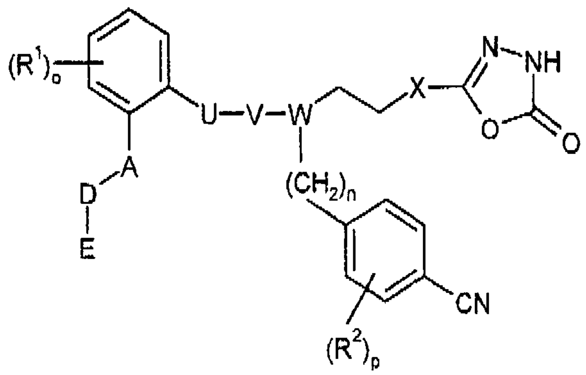

(VIII-1),

in welcher $R^1$, $R^2$, A, D, E, U, V, W, X, n, o und p jeweils die oben angegebenen Bedeutungen haben,
umsetzt
oder
[B-2] Verbindungen der Formel (VI-2)

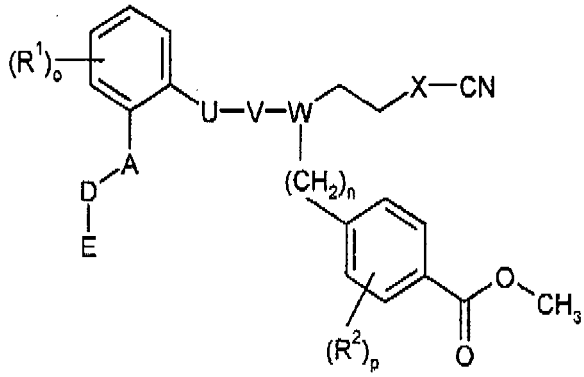

(VI-2),

in welcher $R^1$, $R^2$, A, D, E, U, V, W, X, n, o und p jeweils die oben angegebenen Bedeutungen haben, auf zum Verfahren [B-1] analoge Weise in Verbindungen der Formel (VIII-2)

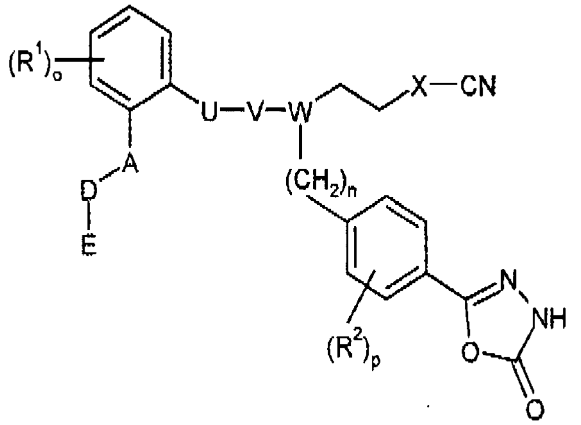

(VIII-2),

in welcher $R^1$, $R^2$, A, D, E, U, V, W, X, n, o und p jeweils die oben angegebenen Bedeutungen haben, überführt

oder

[C-1] Verbindungen der Formel (IX-1)

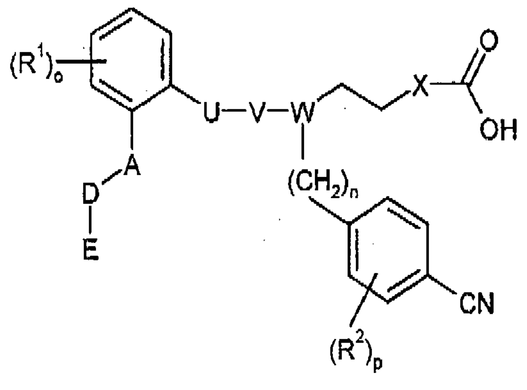

(IX-1),

in welcher $R^1$, $R^2$, A, D, E, U, V, W, X, n, o und p jeweils die in den Ansprüchen 1 und 2 angegebenen Bedeutungen haben,

zunächst in einem inerten Lösungsmittel mit Oxalylchlorid, Thionylchlorid oder Phosphorylchlorid in die entsprechenden carbonsäurechloride der Formel (X-1)

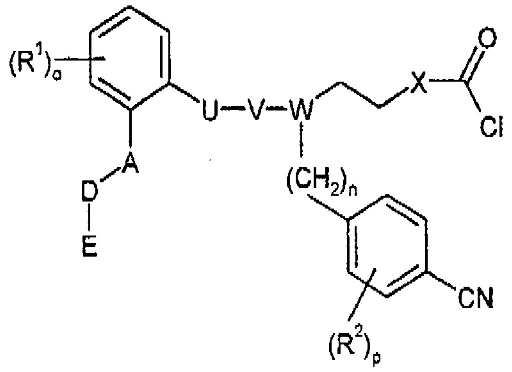

(X-1),

in welcher $R^1$, $R^2$, A, D, E, U, V, W, X, n, o und p jeweils die oben angegebenen Bedeutungen haben, überführt, diese dann in einem inerten Lösungsmittel mit einem Semicarbazid der Formel (XI)

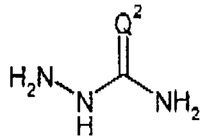

(XI),

in welcher

$Q^2$ für 0, S oder NH steht,

zu Verbindungen der Formel (XII-1)

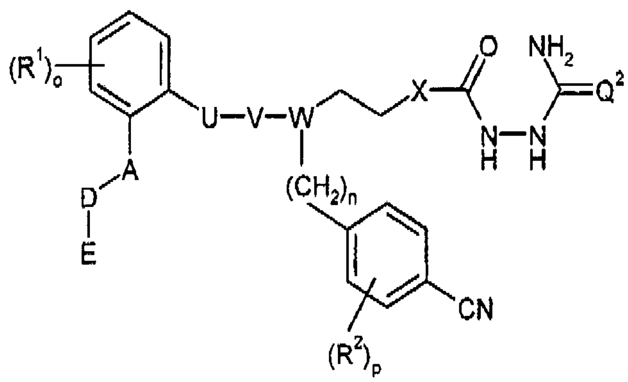

(XII-1),

in welcher $R^1$, $R^2$, A, D, E, U, V, W, X, n, o, p und $Q^2$ jeweils die oben angegebenen Bedeutungen haben, umsetzt und anschließend in Gegenwart einer Base unter gleichzeitiger Hydrolyse der Nitril-Gruppe zu Verbindungen der Formel (XIII-1)

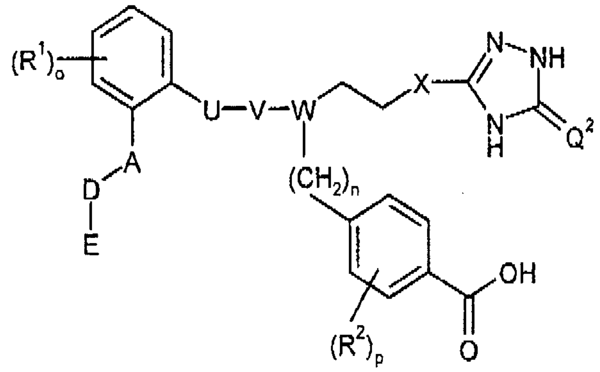

(XIII-1),

in welcher $R^1$, $R^2$, A, D, E, U, V, W, X, n, o, p und $Q^2$ jeweils die oben angegebenen Bedeutungen haben, cyclisiert
oder
[C-2] Verbindungen der Formel (IX-2)

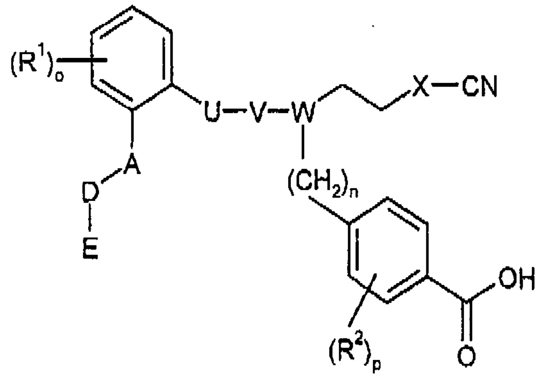

(IX-2),

in welcher $R^1$, $R^2$, A, D, E, U, V, W, X, n, o und p jeweils die oben angegebenen Bedeutungen haben, auf zum Verfahren [C-1] analoge Weise in Verbindungen der Formel (XIII-2)

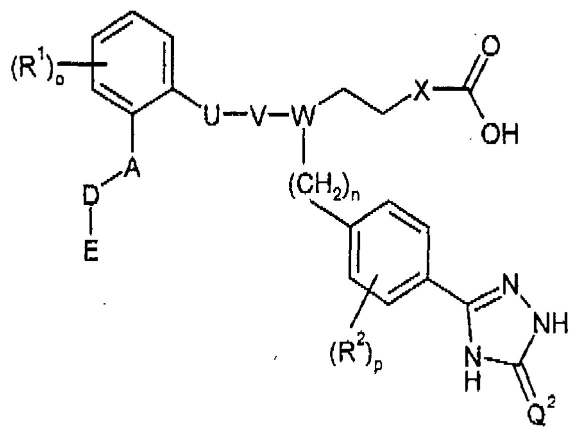

(XIII-2),

in welcher $R^1$, $R^2$, A, D, E, U, V, W, X, n, o, p und $Q^2$ jeweils die oben angegebenen Bedeutungen haben, überführt
und die jeweils resultierenden Verbindungen der Formel (V-1), (V-2), (VIII-1) bzw. (VIII-2) durch Hydrolyse der Ester-Gruppierung -C(O)O$T^1$ bzw. Hydrolyse der Nitril-Gruppe in die entsprechenden Carbonsäuren der Formel (I) überführt
und die Verbindungen der Formel (I), einschließlich der Verbindungen der Formeln (XIII-1) und (XIII-2), gegebenenfalls mit den entsprechenden (i) Lösungsmitteln und/oder (ii) Basen oder Säuren zu ihren Solvaten, Salzen

und/oder Solvaten der Salze umsetzt.

4. Verbindung, wie in Anspruch 1 oder 2 definiert, zur Behandlung und/oder Prävention von Krankheiten.

5. Verwendung einer Verbindung, wie in Anspruch 1 oder 2 definiert, zur Herstellung eines Arzneimittels zur Behandlung und/oder Prävention von Herzinsuffizienz, Angina pectoris, Hypertonie; pulmonaler Hypertonie, Ischämien, Gefäßerkrankungen, thromboembolischen Erkrankungen und Arteriosklerose.

6. Arzneimittel enthaltend eine Verbindung, wie in Anspruch 1 oder 2 definiert, in Kombination mit einem inerten, nichttoxischen, pharmazeutisch geeigneten Hilfsstoff.

7. Arzneimittel enthaltend eine Verbindung, wie in Anspruch 1 oder 2 definiert, in Kombination mit einem weiteren Wirkstoff ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck seakenden Mitteln sowie den Fettstoffwechsel verändernden Mitteln.

8. Arzneimittel nach Anspruch 6 oder 7 zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

## Claims

1. Compound of the formula (I-A)

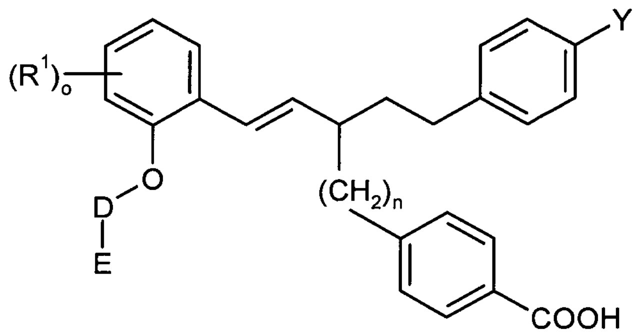

(I-A),

in which

D is ($C_1$-$C_7$)-alkanediyl which may be substituted one or more times by fluorine,
E is hydrogen, trifluoromethyl or is a group of the formula

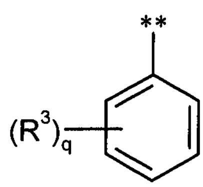

or

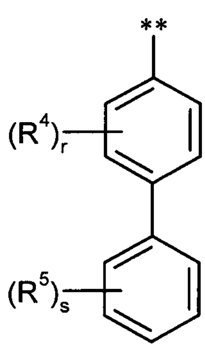

in which ** means the point of linkage to the group D,
Y is a group of the formula

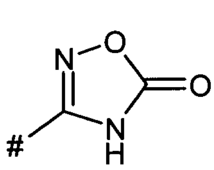, 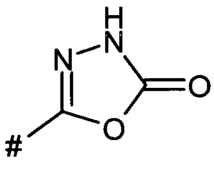 or 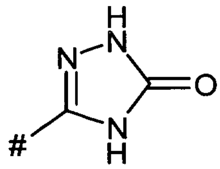,

in which # means the point of linkage to the phenyl ring,
n is the number 1 or 2,
$R^1$, $R^3$, $R^4$ and $R^5$ are independently of one another a substituent selected from the series fluorine, chlorine, bromine, methyl, tert-butyl, trifluoromethyl, methoxy and trifluoromethoxy,
and
o, q, r and s are independently of one another each the number 0, 1 or 2,
where in the case that $R^1$, $R^3$, $R^4$ or $R^5$ occur more than once, their meanings may in each case be identical or different,

and the salts, solvates and solvates of the salts thereof.

**2.** Compound of the formula (I-B)

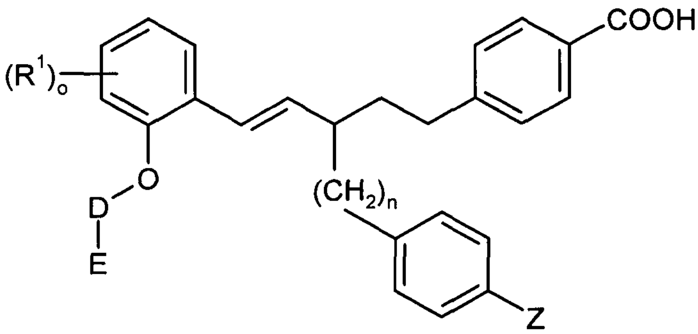

(I-B),

in which

D is ($C_1$-$C_7$)-alkanediyl which may be substituted one or more times by fluorine,
E is hydrogen, trifluoromethyl or is a group of the formula

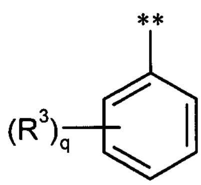 or 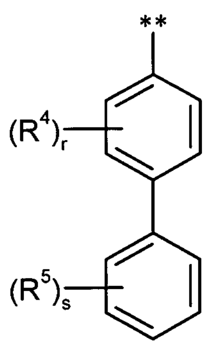

in which ** means the point of linkage to the group D,
Z is a group of the formula

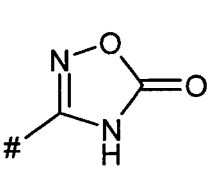 , 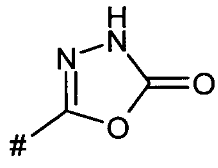 or 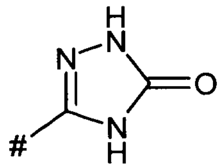 ,

in which # means the point of linkage to the phenyl ring,
n is the number 1 or 2,
$R^1$, $R^3$, $R^4$ and $R^5$ are independently of one another a substituent selected from the series fluorine, chlorine, bromine, methyl, *tert*-butyl, trifluoromethyl, methoxy and trifluoromethoxy,
and
o, q, r and s are independently of one another each the number 0, 1 or 2,

where in the case that $R^1$, $R^3$, $R^4$ or $R^5$ occur more than once, their meanings may in each case be identical or different, and the salts, solvates and solvates of the salts thereof.

**3.** Process for preparing a compound of the formula (I-A) or (I-B) as defined in Claims 1 and 2, **characterized in that** either

[A-1] compounds of the formula (II-1)

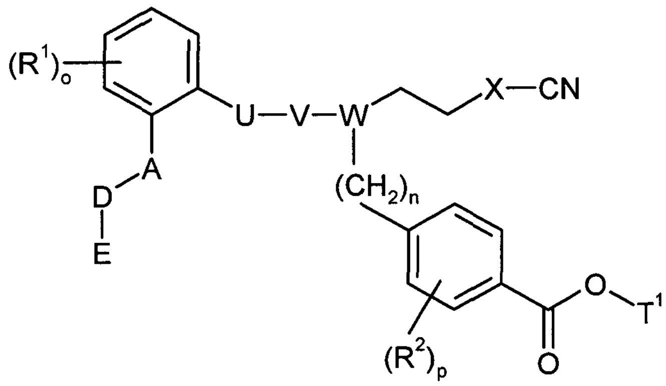 (II-1),

in which $R^1$, $R^2$, A, D, E, U, V, W, X, n, o and p each have the meanings indicated in Claims 1 and 2, and

$T^1$ is ($C_1$-$C_4$) -alkyl,

are initially converted with hydroxylamine in an inert solvent into compounds of the formula (III-1)

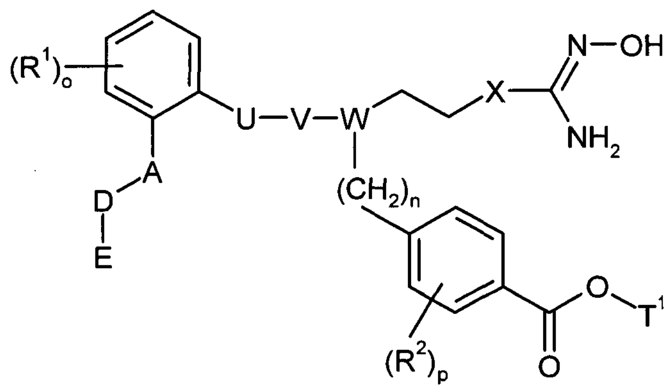 (III-1),

in which $R^1$, $R^2$, A, D, E, U, V, W, X, n, o, p and $T^1$ each have the meanings indicated above,
and then reacted in an inert solvent in the presence of a base with a chloroformic ester of the formula (IV)

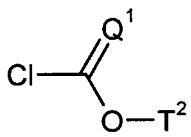

(IV),

in which

$Q^1$ is O or S
and
$T^2$ is $(C_1\text{-}C_{10})$ -alkyl,

and where appropriate after subsequent heating in an inert solvent to give compounds of the formula (V-1)

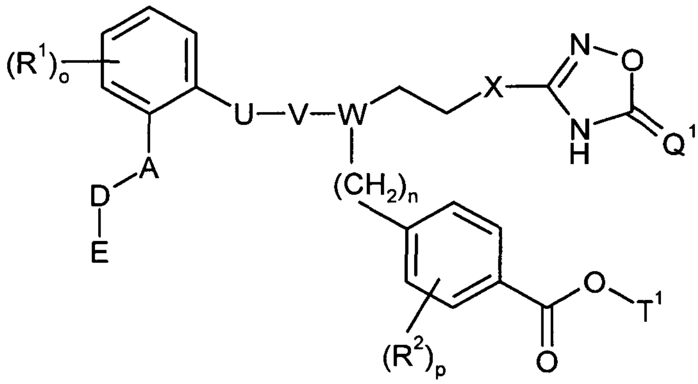

(V-1),

in which $R^1$, $R^2$, A, D, E, U, V, W, X, n, o, p, $Q^1$ and $T^1$ each have the meanings indicated above,
or
[A-2] compounds of the formula (II-2)

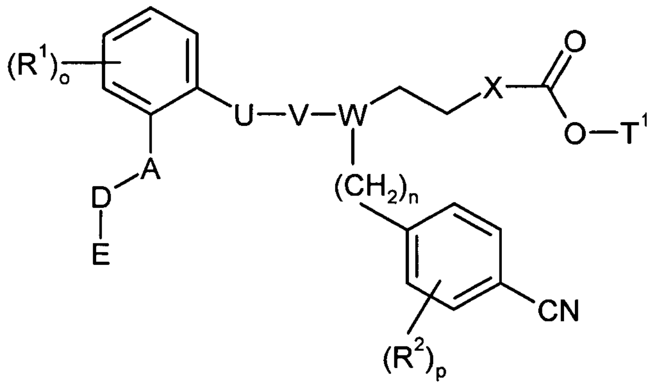

(II-2),

in which $R^1$, $R^2$, A, D, E, U, V, W, X, n, o, p and $T^1$ each have the meanings indicated above,
are converted in a manner analogous to process [A-1] into compounds of the formula (V-2)

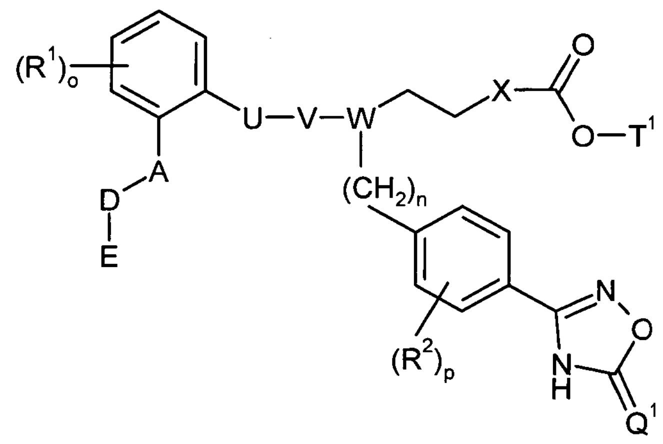

(V-2),

in which $R^1$, $R^2$, A, D, E, U, V, W, X, n, o, p, $Q^1$ and $T^1$ each have the meanings indicated above, or

[B-1] compounds of the formula (VI-1)

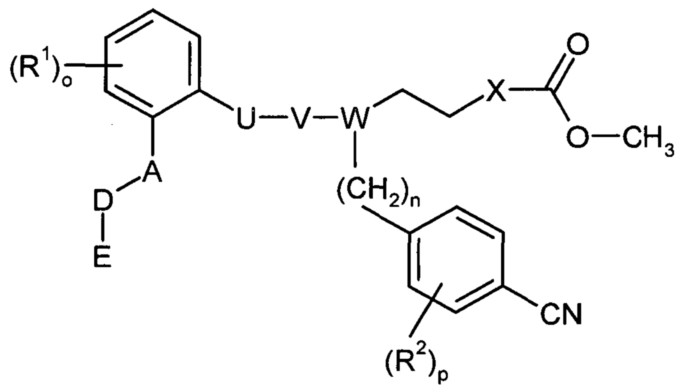

(VI-1),

in which $R^1$, $R^2$, A, D, E, U, V, W, X, n, o and p each have the meanings indicated in Claims 1 and 2, are initially converted with hydrazine in an inert solvent into compounds of the formula (VII-1)

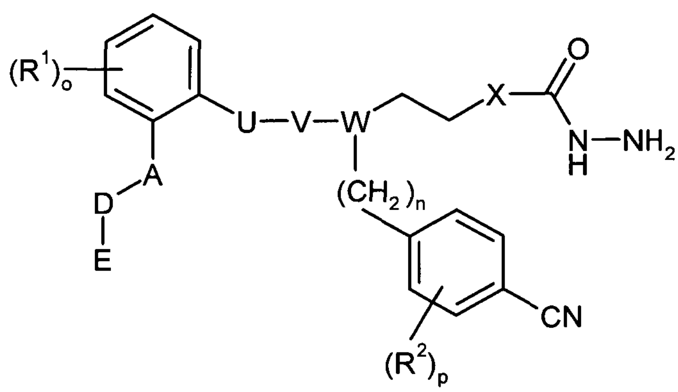

VII-1),

in which $R^1$, $R^2$, A, D, E, U, V, W, X, n, o and p each have the meanings indicated above, and then reacted in an inert solvent with phosgene or a phosgene derivative to give compounds of the formula (VIII-1)

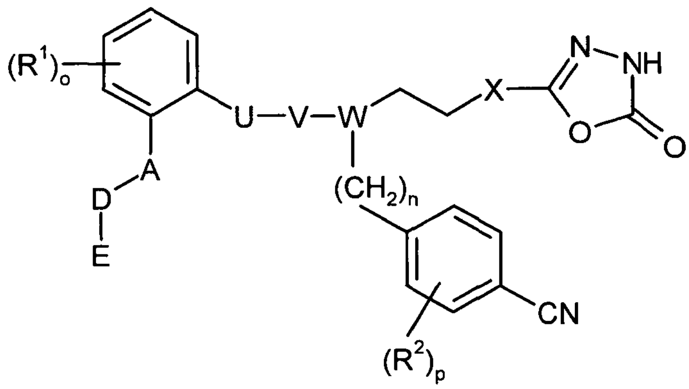

III-1),

in which $R^1$, $R^2$, A, D, E, U, V, W, X, n, o and p each have the meanings indicated above,
or
[B-2] compounds of the formula (VI-2)

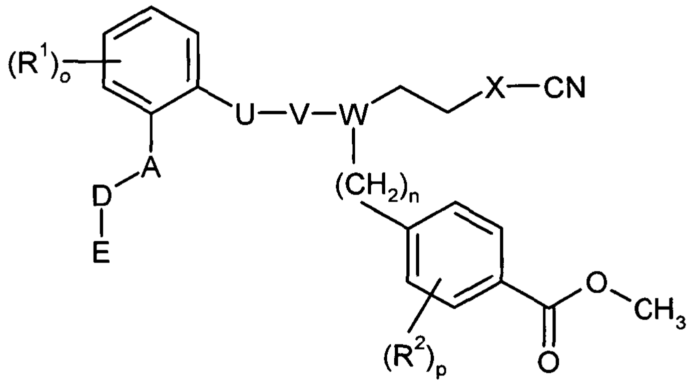

(VI-2),

in which $R^1$, $R^2$, A, D, E, U, V, W, X, n, o and p each have the meanings indicated above,
are converted in a manner analogous to process [B-1] into compounds of the formula (VIII-2)

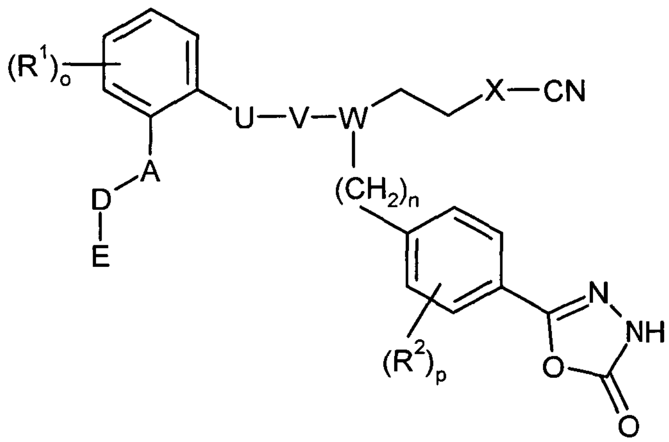

III-2),

in which $R^1$, $R^2$, A, D, E, U, V, W, X, n, o and p each have the meanings indicated above,
or
[C-1] compounds of the formula (IX-1)

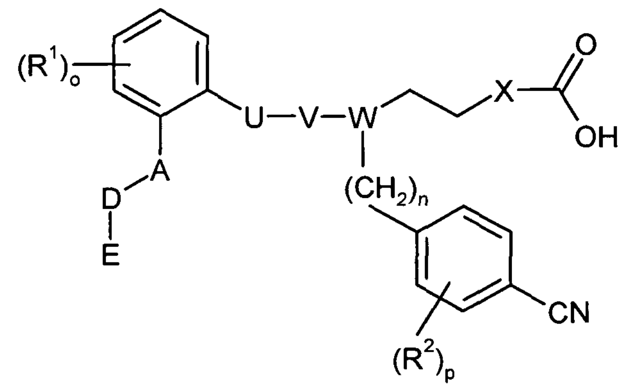

(IX-1),

in which $R^1$, $R^2$, A, D, E, U, V, W, X, n, o and p each have the meanings indicated in Claims 1 and 2, are initially converted in an inert solvent with oxalyl chloride, thionyl chloride or phosphoryl chloride into the corresponding carbonyl chlorides of the formula (X-1)

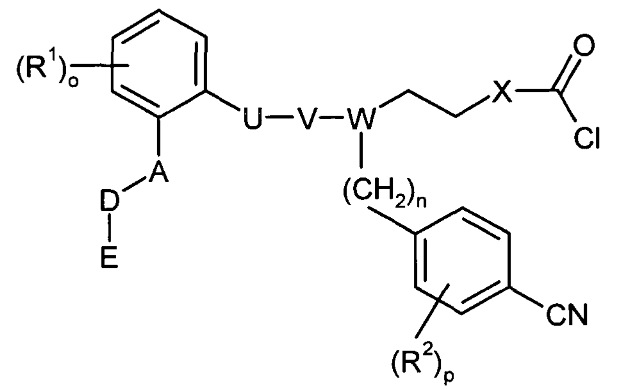

(X-1),

in which $R^1$, $R^2$, A, D, E, U, V, W, X, n, o and p each have the meanings indicated above, these are then reacted in an inert solvent with a semicarbazide of the formula (XI)

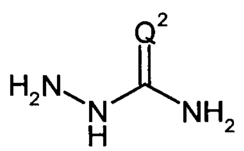

(XI),

in which

$Q^2$ is O, S or NH,

to give compounds of the formula (XII-1)

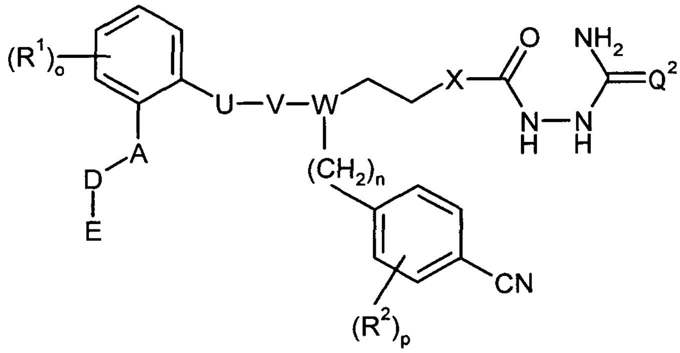

(XII-1),

in which $R^1$, $R^2$, A, D, E, U, V, W, X, n, o, p and $Q^2$ each have the meanings indicated above, and subsequently cyclized in the presence of a base with simultaneous hydrolysis of the nitrile group to give compounds of the formula (XIII-1)

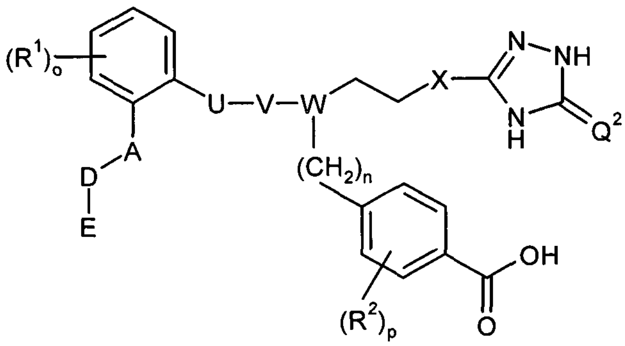

(XIII-1),

in which $R^1$, $R^2$, A, D, E, U, V, W, X, n, o, p and $Q^2$ each have the meanings indicated above,
or
[C-2] compounds of the formula (IX-2)

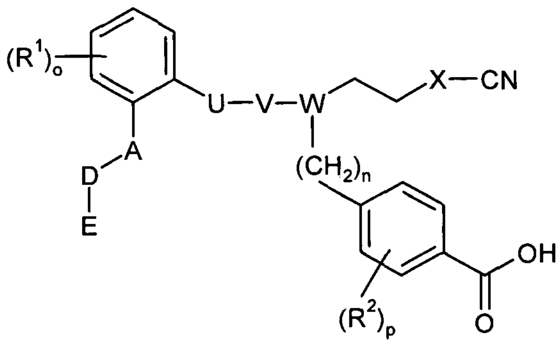

(IX-2),

in which $R^1$, $R^2$, A, D, E, U, V, W, X, n, o and p each have the meanings indicated above, are converted in a manner analogous to process [C-1] into compounds of the formula (XIII-2)

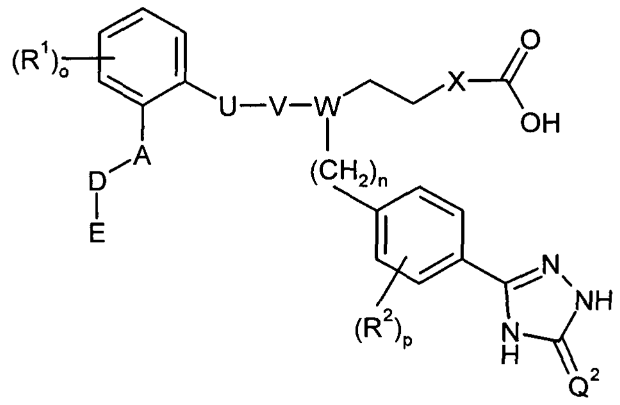

(XIII-2),

in which $R^1$, $R^2$, A, D, E, U, V, W, X, n, o, p and $Q^2$ each have the meanings indicated above,

and the compounds of the formula (V-1), (V-2), (VIII-1) or (VIII-2) resulting in each case are converted by hydrolysis of the ester group - C(O)$OT^1$ or hydrolysis of the nitrile group into the corresonding carboxylic acids of the formula (I), and the compounds of the formula (I), including the compounds of the formulae (XIII-1) and (XIII-2), are reacted where appropriate with the appropriate (i) solvents and/or (ii) bases or acids to give the solvates, salts and/or solvates of the salts thereof.

**4.** Compound as defined in Claim 1 or 2 for the treatment and/or prevention of diseases.

**5.** Use of a compound as defined in Claim 1 or 2 for producing a medicament for the treatment and/or prevention of heart failure, angina pectoris, hypertension, pulmonary hypertension, ischemias, vascular disorders, thromboembolic disorders and arteriosclerosis.

**6.** Medicament comprising a compound as defined in Claim 1 or 2 in combination with an inert, nontoxic, pharmaceutically suitable excipient.

**7.** Medicament comprising a compound as defined in Claim 1 or 2 in combination with a further active ingredient selected from the group consisting of organic nitrates, NO donors, cGMP-PDE inhibitors, stimulators of guanylate cyclase, agents having antithrombotic activity, agents lowering blood pressure, and agents altering lipid metabolism.

**8.** Medicament according to Claim 6 or 7 for the treatment and/or prevention of heart failure, angina pectoris, hypertension, pulmonary hypertension, ischemias, vascular disorders, thromboembolic disorders and arteriosclerosis.

**Revendications**

**1.** Composé de formule (I-A)

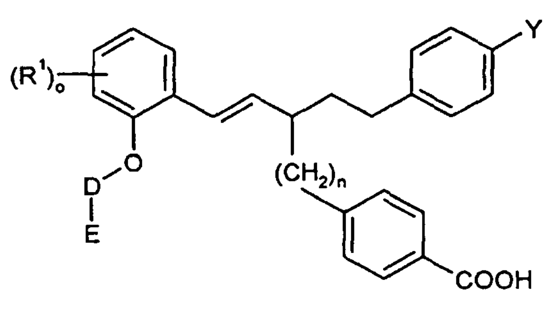

(I-A),

dans laquelle

D représente ($C_1$-$C_7$)-alcanediyle, qui peut être monosubstitué ou polysubstitué par fluor,

E représente hydrogène, trifluorométhyle ou un groupe de formule

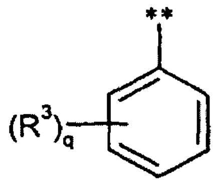

ou

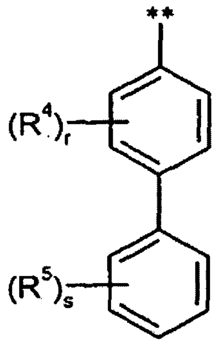

où ** signifie le site de liaison avec le groupe D,
Y représente un groupe de formule

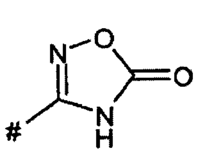

,

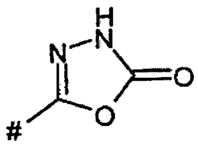

ou

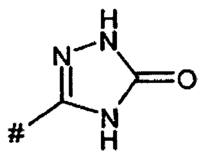

,

où # représente le site de liaison avec le cycle phényle,
n vaut le nombre 1 ou 2,
$R^1$, $R^3$, $R^4$ et $R^5$ représentent, indépendamment les uns des autres, un substituant choisi dans la série fluor, chlore, brome, méthyle, tert-butyle, trifluorométhyle, méthoxy et trifluorométhoxy, et
o, q, r et s représentent, indépendamment les uns des autres, à chaque fois le nombre 0, 1, ou 2, où, pour le cas où $R^1$, $R^3$, $R^4$ ou $R^5$ apparaissent plusieurs fois, leurs significations peuvent à chaque fois être identiques ou différentes,

ainsi que ses sels, solvates et les solvates des sels.

**2.** Composé de formule (I-B)

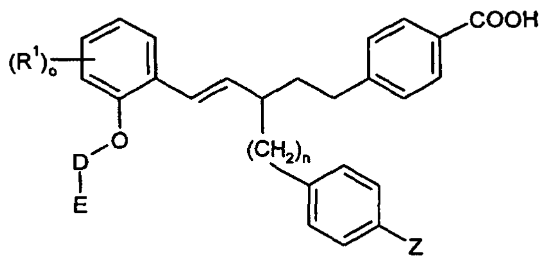

(I-B),

dans laquelle

D représente ($C_1$-$C_7$)-alcanediyle, qui peut être monosubstitué ou polysubstitué par fluor,
E représente hydrogène, trifluorométhyle ou un groupe de formule

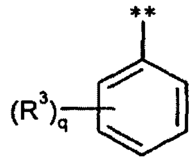

ou

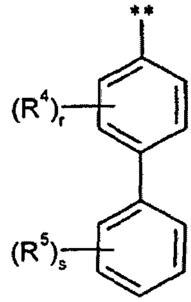

dans laquelle ** signifie le site de liaison avec le groupe D,
Z représente un groupe de formule

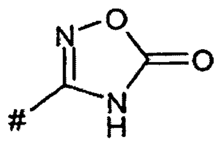 , 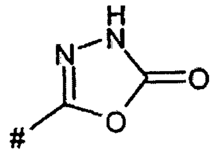 ou 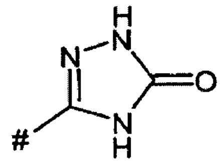 ,

où # représente le site de liaison avec le cycle phényle,
n vaut le nombre 1 ou 2,
$R^1$, $R^3$, $R^4$ et $R^5$ représentent, indépendamment les uns des autres, un substituant choisi dans la série fluor, chlore, brome, méthyle, tert-butyle, trifluorométhyle, méthoxy et trifluorométhoxy, et
o, q, r et s représentent, indépendamment les uns des autres, à chaque fois le nombre 0, 1, ou 2, où, pour le cas où $R^1$, $R^3$, $R^4$ ou $R^5$ apparaissent plusieurs fois, leurs significations peuvent à chaque fois être identiques ou différentes, ainsi que ses sels, solvates et les solvates des sels.

**3.** Procédé pour la préparation d'un composé de formule (I-A) et (I-B) tel que défini dans les revendications 1 et 2, **caractérisé en ce qu'**on transforme soit

[A-1] des composés de formule (II-1)

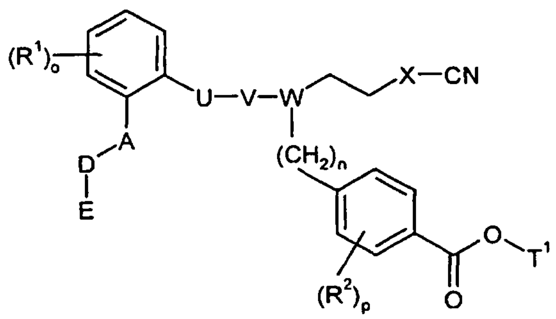

(II-1),

dans laquelle $R^1$, $R^2$, A, D, E, U, V, W, X, n, o et p présentent à chaque fois les significations indiquées dans les revendications 1 et 2 et
$T^1$ représente $(C_1\text{-}C_4)$-alkyle,
d'abord dans un solvant inerte avec une hydroxylamine en composés de formule (III-1)

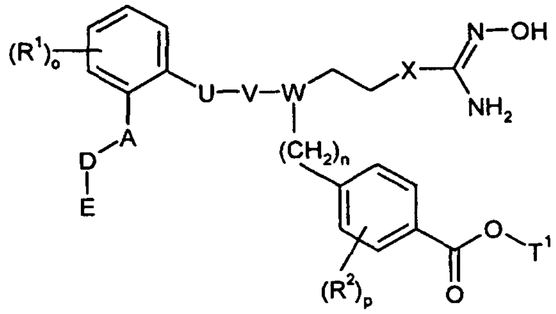

(III-1),

dans laquelle $R^1$, $R^2$, A, D, E, U, V, W, X, n, o, p et $T^1$ présentent à chaque fois les significations indiquées ci-dessus,
puis dans un solvant inerte en présence d'une base avec un ester de l'acide chloroformique de formule (IV)

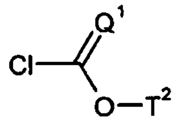

(IV),

dans laquelle
$Q^1$ représente O ou S et
$T^2$ représente $(C_1\text{-}C_{10})$ -alkyle,
ainsi que le cas échéant par chauffage consécutif dans un solvant inerte en composés de formule (V-1)

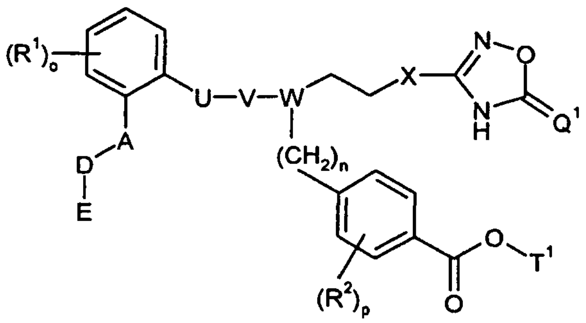

(V-1),

dans laquelle $R^1$, $R^2$, A, D, E, U, V, W, X, n, o, p, $Q^1$ et $T^1$ présentent à chaque fois les significations indiquées ci-dessus,
soit
[A-2] des composés de formule (II-2)

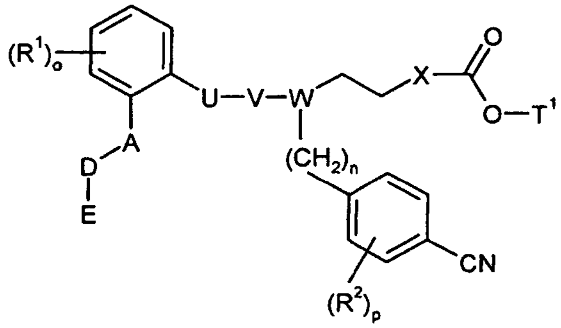

(II-2),

dans laquelle $R^1$, $R^2$, A, D, E, U, V, W, X, n, o, p et $T^1$ présentent à chaque fois les significations indiquées ci-dessus,

de manière analogue au procédé [A-1] en composés de formule (V-2)

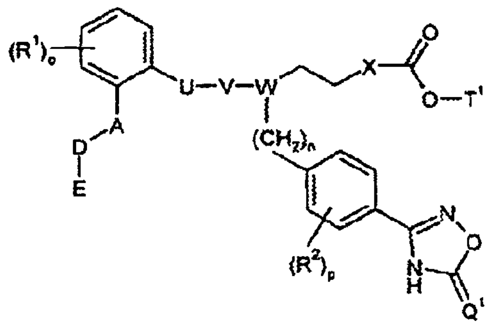

(V-2),

dans laquelle $R^1$, $R^2$, A, D, E, U, V, W, X, n, o, p, $Q^1$ et $T^1$ présentent à chaque fois les significations indiquées ci-dessus,
soit
[B-1] des composés de formule (VI-1)

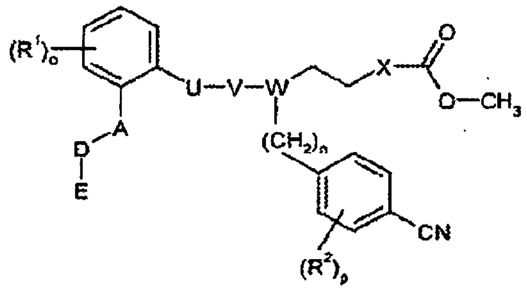

(VI-1),

dans laquelle $R^1$, $R^2$, A, D, E, U, V, W, X, n, o et p présentent à chaque fois les significations indiquées dans les revendications 1 et 2,
d'abord dans un solvant inerte avec de l'hydrazine en composés de formule (VII-1)

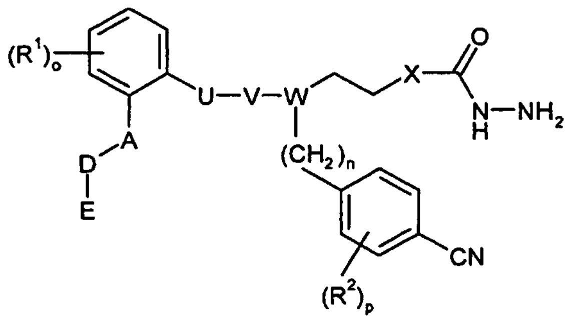

(VII-1),

dans laquelle $R^1$, $R^2$, A, D, E, U, V, W, X, n, o et p présentent à chaque fois les significations indiquées ci-dessus, puis dans un solvant inerte avec du phosgène ou un dérivé de phosgène en composés de formule (VIII-1)

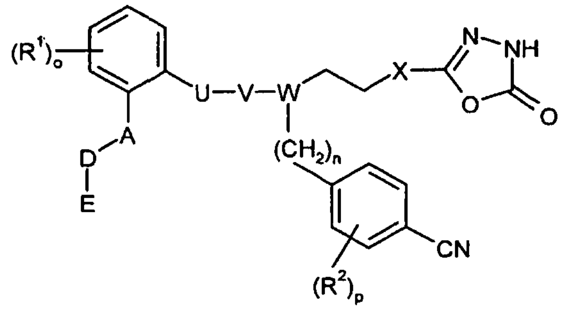

(VIII-1),

dans laquelle $R^1$, $R^2$, A, D, E, U, V, W, X, n, o et p présentent à chaque fois les significations indiquées ci-dessus, soit

[B-2] des composés de formule (VI-2)

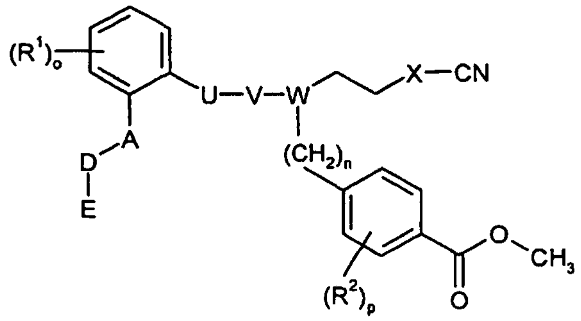

(VI-2),

dans laquelle $R^1$, $R^2$, A, D, E, U, V, W, X, n, o et p présentent à chaque fois les significations indiquées ci-dessus, de manière analogue au procédé [B-1] en composés de formule (VIII-2)

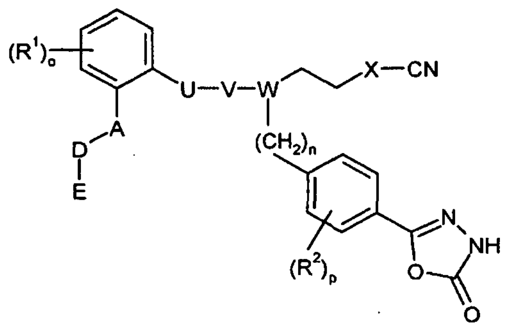

(VIII-2),

dans laquelle $R^1$, $R^2$, A, D, E, U, V, W, X, n, o et p présentent à chaque fois les significations indiquées ci-dessus, soit

[C-1] des composés de formule (IX-1)

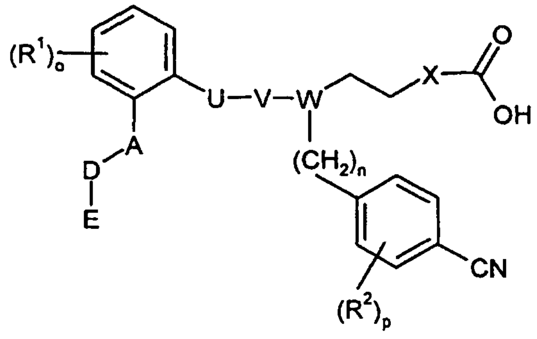

(IX-1),

dans laquelle $R^1$, $R^2$, A, D, E, U, V, W, X, n, o et p présentent à chaque fois les significations indiquées dans les revendications 1 et 2,
d'abord dans un solvant inerte avec du chlorure d'oxalyle, du chlorure de thionyle ou du chlorure de phosphoryle en chlorures d'acide carboxylique correspondants de formule (X-1)

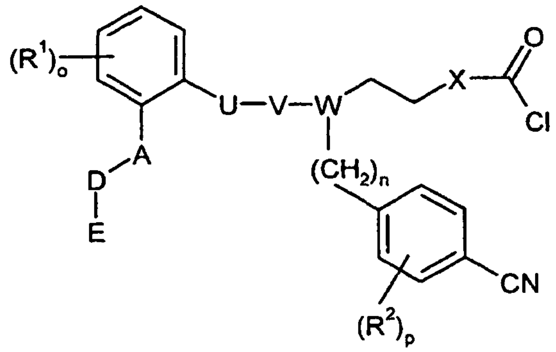

(X-1),

dans laquelle $R^1$, $R^2$, A, D, E, U, V, W, X, n, o et p présentent à chaque fois les significations indiquées ci-dessus, on transforme ceux-ci ensuite dans un solvant inerte avec un semicarbazide de formule (XI)

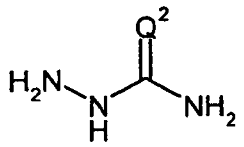

(XI),

dans laquelle
$Q^2$ représente O, S ou NH, en composés de formule (XII-1)

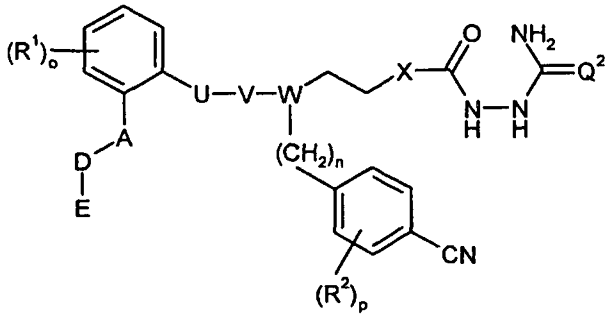

(XII-1),

dans laquelle $R^1$, $R^2$, A, D, E, U, V, W, X, n, o, p et $Q^2$ présentent à chaque fois les significations indiquées ci-dessus,

puis on cyclise en présence d'une base, avec hydrolyse simultanée du groupe nitrile, en composés de formule (XIII-1)

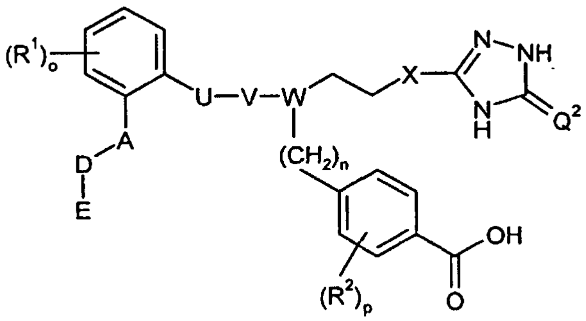

(XIII-1),

dans laquelle $R^1$, $R^2$, A, D, E, U, V, W, X, n, o, p et $Q^2$ présentent à chaque fois les significations indiquées ci-dessus,
ou
[C-2] des composés de formule (IX-2)

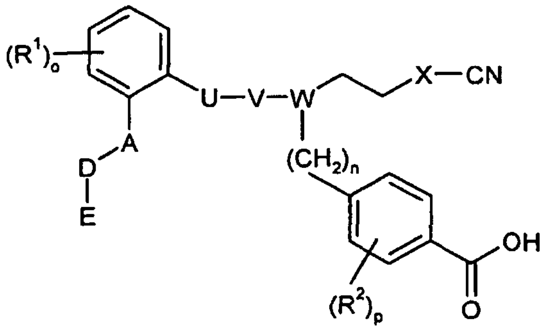

(IX-2),

dans laquelle $R^1$, $R^2$, A, D, E, U, V, W, X, n, o et p présentent à chaque fois les significations indiquées ci-dessus, de manière analogue au procédé [C-1] en composés de formule (XIII-2)

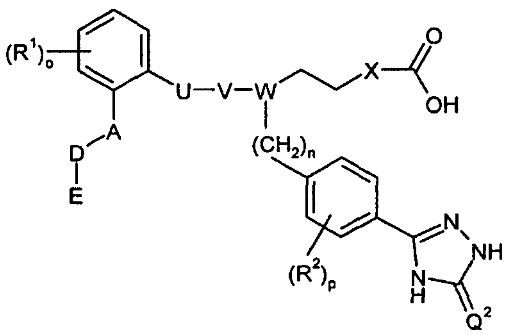

(XIII-2),

dans laquelle $R^1$, $R^2$, A, D, E, U, V, W, X, n, o, p et $Q^2$ présentent à chaque fois les significations indiquées ci-dessus,

et on transforme les composés à chaque fois résultants respectivement de formule (V-1), (V-2), (VIII-1) ou (VIII-2) par hydrolyse du groupement ester $-C(O)OT^1$ ou par hydrolyse du groupe nitrile en acides carboxyliques correspondants de formule (I)
et on transforme les composés de formule (I), y compris les composés des formules (XIII-1) et (XIII-2), le cas échéant avec (i) les solvants et/ou (ii) les bases ou acides correspondants en leurs solvates, sels et/ou les solvates des sels.

**4.** Composé, tel que défini dans la revendication 1 ou 2, destiné au traitement et/ou à la prévention de maladies.

5. Utilisation d'un composé tel que défini dans la revendication 1 ou 2 pour la préparation d'un médicament destiné au traitement et/ou à la prévention de l'insuffisance cardiaque, de l'angine de poitrine, de l'hypertonie, de l'hypertonie pulmonaire, des ischémies, des maladies vasculaires, des maladies thromboemboliques et de l'artériosclérose.

6. Médicament contenant un composé tel que défini dans la revendication 1 ou 2, en combinaison avec un adjuvant inerte, non toxique, pharmaceutiquement approprié.

7. Médicament contenant un composé tel que défini dans la revendication 1 ou 2, en combinaison avec une autre substance active choisie dans le groupe constitué par les nitrates organiques, les donneurs de NO, les inhibiteurs de cGMP-PDE, les stimulateurs de la guanylate-cyclase, les agents à effet antithrombotique, les agents hypotenseurs ainsi que les agents modifiant le métabolisme des lipides.

8. Médicament selon la revendication 6 ou 7, destiné au traitement et/ou à la prévention de l'insuffisance cardiaque, de l'angine de poitrine, de l'hypertonie, de l'hypertonie pulmonaire, des ischémies, des maladies vasculaires, des maladies thromboemboliques et de l'artériosclérose.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9816223 A
- WO 9816507 A
- WO 9823619 A
- EP 0341551 A1
- WO 0119355 A
- WO 0119776 A
- WO 0119778 A
- WO 0119780 A
- WO 02070462 A
- WO 02070510 A
- WO 0006568 A
- WO 0006569 A
- WO 0242301 A
- WO 03095451 A


### In der Beschreibung aufgeführte Nicht-Patentliteratur


- **WU et al.** *Blood,* 1994, vol. 84, 4226
- **MÜLSCH et al.** *Br. J. Pharmacol.,* 1997, vol. 120, 681
- **GOLDBERG et al.** *J. Biol. Chem.,* 1977, vol. 252, 1279
- **PETTIBONE et al.** *Eur. J. Pharmacol.,* 1985, vol. 116, 307
- **YU et al.** *Br. J. Pharmacol.,* 1995, vol. 114, 1587
- **GERZER et al.** *FEBS Lett.,* 1981, vol. 132, 71
- **HOENICKA et al.** *J. Mol. Med.,* 1999, vol. 77, 14
- **IGNARRO et al.** *Adv. Pharmacol.,* 1994, vol. 26, 35
- **MÜLSCH et al.** *Naunyn Schmiedebergs Arch. Pharmacol.,* vol. 355, R47
- **M. HOENICKA ; E.M. BECKER ; H. APELER ; T. SIRICHOKE ; H. SCHROEDER ; R. GERZER ; J.-P. STASCH.** Purified soluble guanylyl cyclase expressed in a baculovirus/Sf9 system: Stimulation by YC-1, nitric oxide, and carbon oxide. *J. Mol. Med.,* 1999, vol. 77, 14-23